# EUROPEAN PATENT APPLICATION

(11) **EP 3 009 144 A1**
(43) Date of publication of application: **20.04.2016**
(21) Application number: 14306637.1
(22) Date of filing: 15.10.2014
(51) Int. Cl.: A61K 39/00, C12N 15/86, C07K 14/005

(54) **Lentiviral vectors for inducing CD4+ and CD8+ immune responses in vaccination of humans**

(71) Applicant: Theravectys, 75015 Paris (FR)
(72) Inventor: Bauche, Cécile, 75013 Paris (FR); Agaugue, Sophie, 75019 Paris (FR)
(74) Representative: Nony

(57) **Abstract**

The present invention relates to the intramuscular injection of a lentiviral vector into a human. The lentiviral vector contains a promoter directing the transcription of a transgene, which preferably encodes a microbial or tumor antigen to be expressed in a mammalian cell host, preferably APC (DCs). The invention encompasses these vectors, methods of making the vectors, and methods of using them, including medicinal uses.

SEQUENCE LISTING

<110>  THERAVECTYS

<120>  LENTIVIRAL VECTORS FOR INDUCING CD4+ and CD8+ IMMUNE
        RESPONSES IN VACCINATION OF HUMANS

<130>  THERA14.3

<160>  40

<170>  PatentIn version 3.5

<210>  1
<211>  220
<212>  DNA
<213>  Homo sapiens

<400>  1
attggggagt cccagccttg gggattcccc aactccgcag tttcttttct ccctctccca      60

acctatgtag ggtccttctt cctggatact cacgacgcgg acccagttct cactcccatt     120

gggtgtcggg tttccagaga agccaatcag tgtcgtcgcg gtcgcggttc taaagtccgc     180

acgcacccac cgggactcag attctcccca gacgccgagg                           220


<210>  2
<211>  197
<212>  DNA
<213>  Homo sapiens

<400>  2
ggggaggcgc agcgttgggg attccccact cccctgagtt tcacttcttc tcccaacttg      60

tgtcgggtcc ttcttccagg atactcgtga cgcgtcccca cttcccactc ccattgggta     120

ttggatatct agagaagcca atcagcgtcg ccgcggtccc agttctaaag tccccacgca     180

cccacccgga ctcagag                                                    197


<210>  3
<211>  216
<212>  DNA
<213>  Homo sapiens

<400>  3
cactggggag gcgccgcgtt gaggattctc cactcccctc agtttcactt cttctcccaa      60

cctgcgtcgg gtccttcttc ctgaatactc atgacgcgtc cccaattccc actcccattg     120

ggtgtcgggt tctagagaag ccaatcagcg tctccgcagt cccggtctaa agtccccagt     180

cacccacccg gactcagatt ctccccagac gccgag                               216


<210>  4
<211>  205
<212>  DNA
<213>  Homo sapiens

<400> 4
taagaactgc tgattgctgg gaaactctgc agtttcccgt tcctctcgta acctggtcat 60

gtgtccttct tcctggatac tcatgacgca gactcagttc tcattcccaa tgggtgtcgg 120

gtttctagag aagccaatca gcgtcgccac gactcccgac tataaagtcc ccatccggac 180

tcaagaagtt ctcaggactc agagg 205


<210> 5
<211> 252
<212> DNA
<213> Homo sapiens

<400> 5
aggccccgag gcggtgtctg gggttggaag gctcagtatt gagaattccc catctcccca 60

gagtttctct ttctctccca acccgtgtca ggtccttcat cctggatact cataacgcgg 120

ccccatttct cactcccatt gggcgtcgcg tttctagaga agccaatcag tgtcgccgca 180

gttcccaggt tctaaagtcc cacgcacccc gcgggactca tatttttccc agacgcggag 240

gttggggtca tg 252


<210> 6
<211> 192
<212> DNA
<213> Homo sapiens

<400> 6
aacatcacga gactctaaga aaaggaaact gaaaacggga aagtccctct ctctaacctg 60

gcactgcgtc gctggcttgg agacaggtga cggtccctgc gggccttgtc ctgattggct 120

gggcacgcgt ttaatataag tggaggcgtc gcgctggcgg gcattcctga agctgacagc 180

attcgggccg ag 192


<210> 7
<211> 33
<212> DNA
<213> Artificial Sequence

<220>
<223> PCR Primer

<400> 7
cttactagtt ggaagggcta attcactccc aac 33


<210> 8
<211> 32
<212> DNA
<213> Artificial Sequence

<220>
<223> PCR Primer

<400> 8

cattctagaa ctgctagaga ttttccacac tg                                    32

<210>  9
<211>  42
<212>  DNA
<213>  Artificial Sequence

<220>
<223>  PCR Primer

<400>  9
taccccgggc catggcctcc aaaaaagcct cctcactact tc                          42

<210>  10
<211>  44
<212>  DNA
<213>  Artificial Sequence

<220>
<223>  PCR Primer

<400>  10
actcccgggt aatttttttt atttatgcag aggccgaggc cgcc                         44

<210>  11
<211>  35
<212>  DNA
<213>  Artificial Sequence

<220>
<223>  PCR Primer

<400>  11
tacacgcgtg gagttccgcg ttacataact tacgg                                  35

<210>  12
<211>  39
<212>  DNA
<213>  Artificial Sequence

<220>
<223>  PCR Primer

<400>  12
cgtggatccg atcgcggtgt cttctatgga ggtcaaaac                              39

<210>  13
<211>  37
<212>  DNA
<213>  Artificial Sequence

<220>
<223>  PCR Primer

<400>  13
tacacgcgtg gcctccgcgc cgggttttgg cgcctcc                                37

<210>  14
<211>  40
<212>  DNA
<213>  Artificial Sequence

<220>
<223>  PCR Primer

<400>  14
cgtggatccg atcggtctaa caaaaaagcc aaaaacggcc                              40


<210>  15
<211>  37
<212>  DNA
<213>  Artificial Sequence

<220>
<223>  PCR Primer

<400>  15
tacacgcgtt gcgtgaggct ccggtgcccg tcagtgg                                 37


<210>  16
<211>  35
<212>  DNA
<213>  Artificial Sequence

<220>
<223>  PCR Primer

<400>  16
cgtggatccg atcgtcacga cacctgaaat ggaag                                   35


<210>  17
<211>  34
<212>  DNA
<213>  Artificial Sequence

<220>
<223>  PCR Primer

<400>  17
gccggcgcgc cgagaaaccc tgcagggaat tccc                                    34


<210>  18
<211>  41
<212>  DNA
<213>  Artificial Sequence

<220>
<223>  PCR Primer

<400>  18
cgtggatccg atcgctcggc ccgaatgctg tcagcttcag g                            41


<210>  19
<211>  517
<212>  DNA

<213>  Artificial Sequence

<220>
<223>  Amplified human B2m promoter sequence

<400>  19
gagaaaccct gcagggaatt ccccagctgt agttataaac agaagttctc cttctgctag      60

gtagcattca aagatcttaa tcttctgggt ttccgttttc tcgaatgaaa aatgcaggtc     120

cgagcagtta actggcgggg gcaccattag caagtcactt agcatctctg gggccagtct     180

gcaaagcgag ggggcagcct taatgtgcct ccagcctgaa gtcctagaat gagcgcccgg     240

tgtcccaagc tggggcgcgc accccagatc ggagggcgcc gatgtacaga cagcaaactc     300

acccagtcta gtgcatgcct tcttaaacat cacgagactc taagaaaagg aaactgaaaa     360

cgggaaagtc cctctctcta acctggcact gcgtcgctgg cttggagaca ggtgacggtc     420

cctgcgggcc ttgtcctgat tggctgggca cgcgtttaat ataagtggag gcgtcgcgct     480

ggcgggcatt cctgaagctg acagcattcg ggccgag                              517


<210>  20
<211>  47
<212>  DNA
<213>  Artificial Sequence

<220>
<223>  PCR Primer

<400>  20
atgcatgcgt cgacctcgag ttaatcctca tcctgtctac ttgccac                    47


<210>  21
<211>  65
<212>  DNA
<213>  Artificial Sequence

<220>
<223>  PCR Primer

<400>  21
gcatgcatcg gccggggcgg cgactggtga gaggccacca tgggtgcgag agcgtcagta      60

ttaag                                                                  65


<210>  22
<211>  34
<212>  DNA
<213>  Artificial Sequence

<220>
<223>  PCR Primer

<400>  22
gccggcgcgc cgagaaaccc tgcagggaat tccc                                  34

<210>  23
<211>  30
<212>  DNA
<213>  Artificial Sequence

<220>
<223>  PCR Primer

<400>  23
ttctcgagca ttacacggcg atctttccgc                                        30


<210>  24
<211>  19
<212>  DNA
<213>  Artificial Sequence

<220>
<223>  PCR Primer

<400>  24
cccaagaacc caaggaaca                                                    19


<210>  25
<211>  25
<212>  DNA
<213>  Artificial Sequence

<220>
<223>  PCR Primer

<400>  25
agacaagata gaggaagagc aaaac                                             25


<210>  26
<211>  27
<212>  DNA
<213>  Artificial Sequence

<220>
<223>  LENTI TM Probe

<400>  26
aaccattagg agtagcaccc accaagg                                           27


<210>  27
<211>  24
<212>  DNA
<213>  Artificial Sequence

<220>
<223>  PCR Primer

<400>  27
cggtgaggat cttcatgagg tagt                                              24


<210>  28
<211>  20
<212>  DNA

<213>  Artificial Sequence

<220>
<223>  PCR Primer

<400>  28
aacaccccag ccatgtacgt                                                   20


<210>  29
<211>  22
<212>  DNA
<213>  Artificial Sequence

<220>
<223>  HUMURA ACT TM Probe

<400>  29
ccagccaggt ccagacgcag ga                                                22


<210>  30
<211>  586
<212>  PRT
<213>  Artificial Sequence

<220>
<223>  HIV antigen

<400>  30

Met Pro Ile Val Gln Asn Leu Gln Gly Gln Met Val His Gln Ala Ile
1               5                   10                  15


Ser Pro Arg Thr Leu Asn Ala Trp Val Lys Val Val Glu Glu Lys Ala
            20                  25                  30


Phe Ser Pro Glu Val Ile Pro Met Phe Ser Ala Leu Ser Glu Gly Ala
        35                  40                  45


Thr Pro Gln Asp Leu Asn Thr Met Leu Asn Thr Val Gly Gly His Gln
    50                  55                  60


Ala Ala Met Gln Met Leu Lys Glu Thr Ile Asn Glu Glu Ala Ala Glu
65                  70                  75                  80


Trp Asp Arg Leu His Pro Val His Ala Gly Pro Ile Ala Pro Gly Gln
                85                  90                  95


Met Arg Glu Pro Arg Gly Ser Asp Ile Ala Gly Thr Thr Ser Thr Leu
            100                 105                 110


Gln Glu Gln Ile Gly Trp Met Thr Asn Asn Pro Pro Ile Pro Val Gly
        115                 120                 125

Glu Ile Tyr Lys Arg Trp Ile Ile Leu Gly Leu Asn Lys Ile Val Arg
    130                 135                 140

Met Tyr Ser Pro Thr Ser Ile Leu Asp Ile Arg Gln Gly Pro Lys Glu
145                 150                 155                 160

Pro Phe Arg Asp Tyr Val Asp Arg Phe Tyr Lys Thr Leu Arg Ala Glu
                165                 170                 175

Gln Ala Ser Gln Glu Val Lys Asn Trp Met Thr Glu Thr Leu Leu Val
            180                 185                 190

Gln Asn Ala Asn Pro Asp Cys Lys Thr Ile Leu Lys Ala Leu Gly Pro
        195                 200                 205

Ala Ala Thr Leu Glu Glu Met Met Thr Ala Cys Gln Gly Val Gly Gly
    210                 215                 220

Pro Gly His Lys Ala Arg Val Leu Ala Glu Ala Met Ser Gln Val Thr
225                 230                 235                 240

Asn Ser Ala Thr Ile Met Met Gln Arg Gly Asn Phe Arg Asn Gln Arg
                245                 250                 255

Lys Thr Val Lys Cys Phe Asn Cys Gly Lys Glu Gly His Ile Ala Lys
            260                 265                 270

Asn Cys Arg Ala Pro Arg Lys Lys Gly Cys Trp Lys Cys Gly Lys Glu
        275                 280                 285

Gly His Gln Met Lys Asp Cys Thr Glu Arg Gln Ala Asn Phe Leu Gly
    290                 295                 300

Lys Ile Trp Pro Ser His Lys Gly Arg Pro Gly Asn Phe Leu Gln Ser
305                 310                 315                 320

Arg Pro Glu Pro Thr Ala Pro Pro Glu Glu Ser Phe Arg Phe Gly Glu
                325                 330                 335

Glu Thr Thr Thr Pro Ser Gln Lys Gln Glu Pro Ile Asp Lys Glu Leu
            340                 345                 350

Tyr Pro Leu Ala Ser Leu Arg Ser Leu Phe Gly Asn Asp Lys Lys Ser
        355                 360                 365

Val Thr Val Leu Asp Val Gly Asp Ala Tyr Phe Ser Val Pro Leu Asp
    370                 375                 380

Lys Asp Phe Arg Lys Tyr Thr Ala Phe Thr Ile Pro Ser Ile Asn Asn
385                 390                 395                 400

Glu Thr Pro Gly Ile Arg Tyr Gln Tyr Asn Val Leu Pro Gln Gly Trp
                405                 410                 415

Lys Gly Ser Pro Ala Ile Phe Gln Ser Ser Met Thr Lys Ile Leu Glu
            420                 425                 430

Pro Phe Arg Lys Gln Asn Pro Asp Ile Val Ile Tyr Gln Tyr Met Asp
        435                 440                 445

Asp Leu Tyr Val Gly Ser Asp Leu Glu Ile Gly Gln His Arg Thr Glu
    450                 455                 460

Ile Leu Lys Glu Pro Val His Gly Val Tyr Val Gly Phe Pro Val Arg
465                 470                 475                 480

Pro Gln Val Pro Leu Arg Pro Met Thr Tyr Lys Ala Ala Val Asp Leu
                485                 490                 495

Ser His Phe Leu Lys Glu Lys Gly Gly Leu Glu Gly Leu Ile Tyr Ser
            500                 505                 510

Gln Lys Arg Gln Asp Ile Leu Asp Leu Trp Val Tyr His Thr Gln Gly
        515                 520                 525

Tyr Phe Pro Asp Trp Gln Asn Tyr Thr Pro Gly Pro Gly Ile Arg Tyr
    530                 535                 540

Pro Leu Thr Phe Gly Trp Cys Phe Lys Leu Val Pro Val Asp Pro Glu
545                 550                 555                 560

Lys Glu Val Leu Val Trp Lys Phe Asp Ser Arg Leu Ala Phe His His
                565                 570                 575

Met Ala Arg Glu Leu His Pro Glu Tyr Tyr
            580                 585

<210>  31
<211>  4856
<212>  RNA
<213>  Artificial Sequence

<220>
<223>  Proviral RNA from construct

<400>  31
accuucccga uuaagugagg guuucuucug uucuauagga acuagacacc uagauggugu        60

guguuccgau gaagggacua aucgucuuga uguguggucc cgguccccag ucuauaggug 120

acuggaaacc uaccacgaug uucgaucaug gucaacucgg ucuauuccau cuucuccggu 180

uauuuccucu cuuguggucg aacaaugugg gacacucgga cguacccuac cuacugggcc 240

ucucucuuca caaucucacc uccaaacugu cggcggaucg uaaaguagug caccgggcuc 300

ucgacguagg ccucaugaag uucuugacga cuauagcucg aacgauguuc ccugaaaggc 360

gaccccugaa aggucccucc gcaccggacc cgcccugacc ccucaccgcu cgggagucua 420

ggacguauau ucgucgacga aaaacggaca ugacccagag agaccaaucu ggucuagacu 480

cggacccucg agagaccgau ugaucccuug ggugacgaau ucggaguuau uucgaacgga 540

acucacgaag uucaucacac acgggcagac aacacacuga gaccauugau cucuagggag 600

ucugggaaaa ucagucacac cuuuuagaga ucgucaccgc gggcuugucc cugaacuuuc 660

gcuuucccuu uggucuccuc gagagagcug cguccugagc cgaacgacuu cgcgcgugcc 720

guucuccgcu ccccgccgcu gaccacucau gcgguuuuua aaacugaucg ccuccgaucu 780

uccucucucu acccacgcuc ucgcagucau aauucgcccc cucuuaaucu agcgcuaccc 840

uuuuuuaagc caauuccggu cccccuuucu uuuuuauauu uaauuuugua uaucauaccc 900

guucgucccu cgaucuugcu aagcgucaau uaggaccgga caaucuuugu agucuuccga 960

caucuguuua ugacccuguc gauguuggua gggaagucug uccuagucuu cuugaaucua 1020

guaauauauu augucaucgu ugggagauaa cacacguagu uuccuaucuc uauuuucugu 1080

gguuccuucg aaaucuguuc uaucuccuuc ucguuuuguu uucauucugg uggcgugucg 1140

uucgccggcg acuagaaguc uggaccuccu ccucuauacu cccuguuaac cucuucacuu 1200

aauauauuua uauuucauca uuuuuaacuu gguaauccuc aucgugggug guuccguuuc 1260

ucuucucacc acgucucucu uuuuucucgu cacccuuauc cucgaaacaa ggaacccaag 1320

aacccucguc guccuucgug auacccgcgu cgcaguuacu gcgacugcca uguccggucu 1380

guuaauaaca gaccauauca cgucgucguc uuguuaaacg acucccgaua acuccgcguu 1440

gucguagaca acguugagug ucagaccccg uaguucgucg agguccguuc uuaggaccga 1500

caccuuucua uggauuuccu aguugucgag gaccccuaaa ccccaacgag accuuuugag 1560

uaaacguggu gacgacacgg aaccuuacga ucaaccucau uauuuagaga ccuugucuaa 1620

accuuagugu gcuggaccua ccucacccug ucucuuuaau uguuaaugug uucgaauuau 1680

gugaggaauu aacuucuuag cguuuugguc guucuuuucu uacuuguucu uaauaaccuu 1740

aaucuauuua cccguucaaa caccuuaacc aaauuguauu guuuaaccga caccauauau 1800

uuuaauaagu auuacuauca uccuccgaac cauccaaauu cuuaucaaaa acgacaugaa 1860

agauaucacu uaucucaauc cgucccuaua agugguaaua gcaaagucug gguggagggu 1920

uggggcuccc cugggcuguc cgggcuuccu uaucuucuuc uuccaccucu cucucugucu 1980

cugucuaggu aagcuaauca cuugccuaga gcugccauag cggcuuaagu guuuaccguc 2040

auaaguaggu guuaaaauuu ucuuuucccc ccuaaccccc caugucacgu ccccuuucuu 2100

aucaucugua uuaucguugu cuguauguuu gauuucuuaa uguuuuuguu uaauguuuuu 2160

aaguuuaaaa agcccaaaua augucccugu cgucucuagg ugaaaccgac uaugcgcggc 2220

ucuuugggac gucccuuaag ggucgacauu caauauuugu cuucaagagg aagacgaucc 2280

aucguaaguu ucuagaauua gaagacccaa aggcaaaaga gcuuacuuuu uacguccagg 2340

cucgucaauu gaccgccccc gugguaaucg uucagugaau cguagagacc ccggucagac 2400

guuucgcucc cccgucggaa uuacacggag gucggacuuc aggaucuuac ucgcgggcca 2460

caggguucga ccccgcgcgu ggggucuagc cucccgcggc uacaugucug ucguuugagu 2520

gggucagauc acguacggaa gaauuuguag ugcucugaga uucuuuuccu uugacuuuug 2580

cccuuucagg gagagagauu ggaccgugac gcagcgaccg aaccucuguc cacugccagg 2640

gacgcccgga acaggacuaa ccgacccgug cgcaaauuau auucaccucc gcagcgcgac 2700

cgcccguaag gacuucgacu gucguaagcc cggcucgcga gccuaggcgg ugguacgggu 2760

agcacgucuu ggacgucccg gucuaccacg ugguccggua gucgggggcc ugggacuugc 2820

ggacccacuu ccaccaccuc cuuuuccgga agucggggcu ccacuagggg uacaagucgc 2880

gggacucgcu cccgcggugu gggguccugg acuuguggua cgacuugugg cacccgccgg 2940

ugguccggcg guacgucuac gacuuucucu guuaguugcu ccuucggcgg cucacccugg 3000

ccgacguggg acacgugcgg ccuggauagc ggggaccggu cuacgcccuc gggucccegu 3060

cgcuauagcg gccguggugg aggugugacg uccuugucua gccgaccuac ugguuguugg 3120

ggggguaggg gcacccgcuc uagauguucg ccaccuagua ggacccggac uuguucuagc 3180

acgccuacau gucggggugg agguaggacc uguaggccgu cccgggguuu cucgggaagg 3240

cccugaugca ccuggccaag auguucuggg acgcccggcu cguccggucg guccuucacu 3300

ucuugaccua cuggcucugu gacgaccacg ucuugcgguu ggggcugacg uucugguagg 3360

acuuccggga cccgggacgg cggugggacc uucucuacua cuggcggacg gucccgcacc 3420

cgccuggacc gguguuccgg gcccacgacc ggcuccggua cucgguccac ugguugucgc 3480

ggugguagua cuacgucgcc ccguugaagg ccuuggucuc uuucuggcac uucacgaagu 3540

ugacgccguu ucucccggug uaacgguucu ugacgucucg gggguccuuc uucccgacga 3600

ccuucacacc guuucuuccc guggucuacu uccugacgug gcucgccguc cgguugaagg 3660

acccuuuuua aaccggaagg guguucccgu ccgggccguu gaaggacguc ucguccgggc 3720

ucggguggcg gggagggcuc cuuucgaagg ccaagccgcu ccuuuggugg ugggggucgg 3780

ucuucguccu uggguagcug uuucucgaca ugggggaccg gagggacucu ucggacaagc 3840

cguugcuguu cuuuucgcac uggcacgacc ugcacccgcu gcggaugaag ucgcacgggg 3900

accuauuccu gaaggccuuc auguggcgga agugguaggg gucguaguug uugcucugug 3960

ggccguaguc uauggucaug uugcacgacg gggucccgac cuucccgucg gggcgguaga 4020

aggucucguc guacugguuc uaggaccuug ggaaggccuu cgucuugggg cuguagcacu 4080

agauggucau guaccugcug gacaugcacc cgucgcugga ccuuuagccg gucguggccu 4140

gucucuagga cuuucuuggg cacgugccgc acaugcaccc gaaggggcac ucuggugucc 4200

acggugacuc uggguacugg auguuccggc ggcaccugga cucggugaag gacuuccucu 4260

ucccgccgga ccuuccggac uagaugucgg ucuucuccgu ccuguaggac cuggacaccc 4320

acauggugug ggucccgaug aaggggcuga ccgucuugau guggggaccg ggaccguagg 4380

cgauggggga cuggaagccg accacgaagu ucgaccacgg gcaccugggg cucuuccuuc 4440

acgaccacac cuucaagcug ucggccgacc ggaagguggu guaccggucu cucgacgugg 4500

ggcucaugau gacuacugag cucuccaugg aaauucuggu uacugaaugu uccgucgaca 4560

ucuagaaucg gugaaaaauu uucuuuuccc cccugaccuu cccgauuaag ugaggguugc 4620

uucuguuuua gcagcucucu acgacguaua uucgucgacg aaaaacgaac augacccaga 4680

gagaccaauc uggucuagac ucggacccuc gagagaccga uugaucccuu gggugacgaa 4740

uucggaguua uuucgaacgg aacucacgaa guucaucaca cacgggcaga caacacacug 4800

agaccauuga ucucuaggga gucugggaaa aucagucaca ccuuuuagag aucguc 4856

<210> 32
<211> 5256
<212> RNA
<213> Artificial Sequence

<220>
<223> Proviral RNA from construct

<400> 32
accuucccga uuaagugagg guuucuucug uucuauagga acuagacacc uagauggugu 60

guguuccgau gaagggacua aucgucuuga uguguggucc cgguccccag ucuauaggug 120

acuggaaacc uaccacgaug uucgaucaug gucaacucgg ucuauuccau cuucuccggu 180

uauuuccucu cuuguggucg aacaaugugg gacacucgga cguacccuac cuacugggcc 240

ucucucuuca caaucucacc uccaaacugu cggcggaucg uaaaguagug caccgggcuc 300

ucgacguagg ccucaugaag uucuugacga cuauagcucg aacgauguuc ccugaaaggc 360

gaccccugaa aggucccucc gcaccggacc cgcccugacc ccucaccgcu cgggagucua 420

ggacguauau ucgucgacga aaaacggaca ugacccagag agaccaaucu ggucuagacu 480

cggacccucg agagaccgau ugaucccuug ggugacgaau ucggaguuau uucgaacgga 540

acucacgaag uucaucacac acgggcagac aacacacuga gaccauugau cucuagggag 600

ucugggaaaa ucagucacac cuuuuagaga ucgucaccgc gggcuugucc cugaacuuuc 660

gcuuucccuu uggucuccuc gagagagcug cguccugagc cgaacgacuu cgcgcgugcc 720

guucuccgcu ccccgccgcu gaccacucau gcgguuuuua aaacugaucg ccuccgaucu 780

uccucucucu acccacgcuc ucgcagucau aauucgcccc cucuuaaucu agcgcuaccc 840

uuuuuuaagc caauuccggu cccccuuucu uuuuuauauu uaauuuugua uaucauaccc 900

guucgucccu cgaucuugcu aagcgucaau uaggaccgga caaucuuugu agucuuccga 960

caucuguuua ugacccuguc gauguuggua gggaagucug uccuagucuu cuugaaucua 1020

guaauauauu augucaucgu ugggagauaa cacacguagu uuccuaucuc uauuuucugu 1080

gguuccuucg aaaucuguuc uaucuccuuc ucguuuuguu uucauucugg uggcgugucg 1140

uucgccggcg acuagaaguc uggaccuccu ccucuauacu cccuguuaac cucuucacuu 1200

aauauauuua uauuucauca uuuuuaacuu gguaauccuc aucgugggug guuccguuuc 1260

ucuucucacc acgucucucu uuuuucucgu cacccuuauc cucgaaacaa ggaacccaag 1320

aacccucguc guccuucgug auacccgcgu cgcaguuacu gcgacugcca uguccggucu 1380

guuaauaaca gaccauauca cgucgucguc uuguuaaacg acucccgaua acuccgcguu 1440

gucguagaca acguugagug ucagaccccg uaguucgucg agguccguuc uuaggaccga 1500

caccuuucua uggauuuccu aguugucgag gaccccuaaa ccccaacgag accuuuugag 1560

uaaacguggu gacgacacgg aaccuuacga ucaaccucau uauuuagaga ccuugucuaa 1620

accuuagugu gcuggaccua ccucacccug ucucuuuaau uguuaaugug uucgaauuau 1680

gugaggaauu aacuucuuag cguuuugguc guucuuuucu uacuuguucu uaauaaccuu 1740

aaucuauuua cccguucaaa caccuuaacc aaauuguauu guuuaaccga caccauauau 1800

uuuaauaagu auuacuauca uccuccgaac cauccaaauu cuuaucaaaa acgacaugaa 1860

agauaucacu uaucucaauc cgucccuaua agugguaaua gcaaagucug gguggagggu 1920

uggggcuccc cugggcuguc cgggcuuccu uaucuucuuc uuccaccucu cucucugucu 1980

cugucuaggu aagcuaauca cuugccuaga gcugccauag cggcuuaagu guuuaccguc 2040

auaaguaggu guuaaaauuu ucuuuucccc ccuaaccccc caugucacgu ccccuuucuu 2100

aucaucugua uuaucguugu cuguauguuu gauuucuuaa uguuuuuguu uaauguuuuu 2160

aaguuuuaaa agcccaaaua augucccugu cgucucuagg ugaaaccgac uaugcgcggc 2220

ucuuugggac gucccuuaag ggucgacauu caauauuugu cuucaagagg aagacgaucc 2280

aucguaaguu ucuagaauua gaagacccaa aggcaaaaga gcuuacuuuu uacguccagg 2340

cucgucaauu gaccgccccc gugguaaucg uucagugaau cguagagacc ccggucagac 2400

guuucgcucc cccgucggaa uuacacggag gucggacuuc aggaucuuac ucgcgggcca 2460

caggguucga ccccgcgcgu ggggucuagc cucccgcggc uacaugucug ucguuugagu 2520

gggucagauc acguacggaa gaauuuguag ugcucugaga uucuuuuccu uugacuuuug 2580

cccuuucagg gagagagauu ggaccgugac gcagcgaccg aaccucuguc cacugccagg 2640

gacgcccgga acaggacuaa ccgacccgug cgcaaauuau auucaccucc gcagcgcgac 2700

cgcccguaag gacuucgacu gucguaagcc cggcucgcga gccuaggcgg ugguacgggu 2760

agcacgucuu ggacgucccg gucuaccacg ugguccggua gucgggggcc ugggacuugc 2820

ggacccacuu ccaccaccuc cuuuuccgga agucggggcu ccacuagggg uacaagucgc 2880

gggacucgcu cccgcggugu gggguccugg acuuguggua cgacuugugg cacccgccgg 2940

ugguccggcg guacgucuac gacuuucucu guuaguugcu ccuucggcgg cucacccugg 3000

ccgacguggg acacgugcgg ccuggauagc ggggaccggu cuacgcccuc ggguccccgu 3060

cgcuauagcg gccguggugg aggugugacg uccuugucua gccgaccuac ugguuguugg 3120

ggggguaggg gcacccgcuc uagauguucg ccaccuagua ggacccggac uuguucuagc 3180

acgccuacau gucggggugg agguaggacc uguaggccgu cccgggguuu cucgggaagg 3240

cccugaugca ccuggccaag auguucuggg acgcccggcu cguccggucg guccuucacu 3300

ucuugaccua cuggcucugu gacgaccacg ucuugcgguu ggggcugacg uucugguagg 3360

acuuccggga cccgggacgg cggugggacc uucucuacua cuggcggacg gucccgcacc 3420

cgccuggacc gguguuccgg gcccacgacc ggcuccggua cucgguccac ugguugucgc 3480

ggugguagua cuacgucgcc ccguugaagg ccuuggucuc uuucuggcac uucacgaagu 3540

ugacgccguu ucucccggug uaacgguucu ugacgucucg gggguccuuc uucccgacga 3600

ccuucacacc guuucuuccc guggucuacu uccugacgug gcucgccguc cgguugaagg 3660

acccuuuuua aaccggaagg guguucccgu ccgggccguu gaaggacguc ucguccgggc 3720

ucggguggcg gggagggcuc cuuucgaagg ccaagccgcu ccuuuggugg ugggggucgg 3780

ucuucguccu uggguagcug uuucucgaca ugggggaccg gagggacucu ucggacaagc 3840

cguugcuguu cuuuucgcac uggcacgacc ugcacccgcu gcggaugaag ucgcacgggg 3900

accuauuccu gaaggccuuc auguggcgga agugguaggg gucguaguug uugcucugug 3960

ggccguaguc uauggucaug uugcacgacg gggucccgac cuucccgucg gggcgguaga 4020

aggucucguc guacugguuc uaggaccuug ggaaggccuu cgucuugggg cuguagcacu 4080

agauggucau guaccugcug gacaugcacc cgucgcugga ccuuuagccg gucguggccu 4140

gucucuagga cuuucuuggg cacgugccgc acaugcaccc gaaggggcac ucuggugucc 4200

acggugacuc uggguacugg auguuccggc ggcaccugga cucggugaag gacuuccucu 4260

ucccgccgga ccuuccggac uagaugucgg ucuucuccgu ccuguaggac cuggacaccc 4320

acauggugug ggucccgaug aaggggcuga ccgucuugau guggggaccg ggaccguagg 4380

cgauggggga cuggaagccg accacgaagu ucgaccacgg gcaccugggg cucuuccuuc 4440

acgaccacac cuucaagcug ucggccgacc ggaagguggu guaccggucu cucgacgugg 4500

ggcucaugau gacuacugag cucuuuaagg gcuauuaguu ggagaccuaa uguuuuaaac 4560

acuuucuaac ugaccauaag aauugauaca acgaggaaaa ugcgauacac cuaugcgacg 4620

aaauuacgga aacauaguac gauaacgaag ggcauaccga aaguaaaaga ggaggaacau 4680

auuuaggacc aacgacagag aaauacuccu caacaccggg caacaguccg uugcaccgca 4740

ccacacguga cacaaacgac ugcguugggg gugaccaacc ccguaacggu gguggacagu 4800

cgaggaaagg cccugaaagc gaaaggggga gggauaacgg ugccgccuug aguagcggcg 4860

gacggaacgg gcgacgaccu guccccgagc cgacaacccg ugacuguuaa ggcaccacca 4920

acagccaugg aaauucuggu uacugaaugu uccgucgaca ucuagaaucg gugaaaaauu 4980

uucuuuuccc cccugaccuu cccgauuaag ugaggguugc uucuguuuua gcagcucucu 5040

acgacguaua uucgucgacg aaaaacgaac augacccaga gagaccaauc uggucuagac 5100

ucggacccuc gagagaccga uugaucccuu gggugacgaa uucggaguua uuucgaacgg 5160

aacucacgaa guucaucaca cacgggcaga caacacacug agaccauuga ucucuaggga 5220

gucugggaaa aucagucaca ccuuuuagag aucguc 5256

<210> 33
<211> 2171
<212> RNA
<213> Artificial Sequence

<220>
<223> Antigen RNA sequence from integrated provirus

<400> 33
uacggguagc acgucuugga cgucccgguc uaccacgugg uccgguaguc gggggccugg 60

gacuugcgga cccacuucca ccaccuccuu uuccggaagu cggggcucca cuagggguac 120

aagucgcggg acucgcuccc gcgguguggg guccuggacu ugugguacga cuuguggcac 180

ccgccggugg uccggcggua cgucuacgac uuucucuguu aguugcuccu ucggcggcuc 240

acccuggccg acgugggaca cgugcggccu ggauagcggg gaccggucua cgcccucggg 300

uccccgucgc uauagcggcc gugguggagg ugugacgucc uugucuagcc gaccuacugg 360

uuguuggggg gguaggggca cccgcucuag auguucgcca ccuaguagga cccggacuug 420

uucuagcacg ccuacauguc gggguggagg uaggaccugu aggccguccc gggguuucuc 480

gggaaggccc ugaugcaccu ggccaagaug uucugggacg cccggcucgu ccggucgguc 540

cuucacuucu ugaccuacug gcucugugac gaccacgucu ugcgguuggg gcugacguuc 600

ugguaggacu uccgggaccc gggacggcgg ugggaccuuc ucuacuacug gcggacgguc 660

ccgcacccgc cuggaccggu guuccgggcc cacgaccggc uccgguacuc gguccacugg 720

uugucgcggu gguaguacua cgucgccccg uugaaggccu uggucucuuu cuggcacuuc 780

acgaaguuga cgccguuucu cccgguguaa cgguucuuga cgucucgggg guccuucuuc 840

ccgacgaccu ucacaccguu ucuucccgug gucuacuucc ugacguggcu cgccguccgg 900

uugaaggacc cuuuuuaaac cggaagggug uucccguccg ggccguugaa ggacgucucg 960

uccgggcucg gguggcgggg agggcuccuu ucgaaggcca agccgcuccu uuggguggug 1020

gggucggucu ucguccuugg guagcuguuu cucgacaugg gggaccggag ggacucuucg 1080

gacaagccgu ugcuguucuu uucgcacugg cacgaccugc acccgcugcg gaugaagucg 1140

cacggggacc uauuccugaa ggccuucaug uggcggaagu gguagggguc guaguuguug 1200

cucugugggc cguagucuau ggucauguug cacgacgggg ucccgaccuu cccgucgggg 1260

cgguagaagg ucucgucgua cugguucuag gaccuuggga aggccuucgu cuuggggcug 1320

uagcacuaga uggucaugua ccugcuggac augcacccgu cgcuggaccu uuagccgguc 1380

guggccuguc ucuaggacuu ucuugggcac gugccgcaca ugcacccgaa ggggcacucu 1440

gguguccacg gugacucugg guacuggaug uuccggcggc accuggacuc ggugaaggac 1500

uuccucuucc cgccggaccu uccggacuag augucggucu ucuccguccu guaggaccug 1560

gacacccaca uggugugggu cccgaugaag gggcugaccg ucuugaugug gggaccggga 1620

ccguaggcga ugggggacug gaagccgacc acgaaguucg accacgggca ccuggggcuc 1680

uuccuucacg accacaccuu caagcugucg gccgaccgga agguggugua ccggucucuc 1740

gacguggggc ucaugaugac uacugagcuc uuuaagggcu auuaguugga gaccuaaugu 1800

uuuaaacacu uucuaacuga ccauaagaau ugauacaacg aggaaaaugc gauacaccua 1860

ugcgacgaaa uuacggaaac auaguacgau aacgaagggc auaccgaaag uaaaagagga 1920

ggaacauauu uaggaccaac gacagagaaa uacuccucaa caccgggcaa caguccguug 1980

caccgcacca cacgugacac aaacgacugc guugggggug accaaccccg uaacgguggu 2040

ggacagucga ggaaaggccc ugaaagcgaa agggggaggg auaacggugc cgccuugagu 2100

agcggcggac ggaacgggcg acgaccuguc cccgagccga caacccguga cuguuaaggc 2160

accaccaaca g 2171

<210> 34
<211> 2104
<212> RNA
<213> Artificial Sequence

<220>
<223> Antigen RNA sequence from integrated provirus

<400> 34

uacggguagc acgucuugga cgucccgguc uaccacgugg uccgguaguc gggggccugg 60

gacuugcgga cccacuucca ccaccuccuu uuccggaagu cggggcucca cuagggguac 120

aagucgcggg acucgcuccc gcgguguggg guccuggacu ugugguacga cuuguggcac 180

ccgccggugg uccggcggua cgucuacgac uuucucuguu aguugcuccu ucggcggcuc 240

acccuggccg acguggacaa cgugcggccu ggauagcggg gaccggucua cgcccucggg 300

uccccgucgc uauagcggcc gugguggagg ugugacgucc uugucuagcc gaccuacugg 360

uuguuggggg gguaggggca cccgcucuag auguucgcca ccuaguagga cccggacuug 420

uucuagcacg ccuacauguc gggguggagg uaggaccugu aggccguccc gggguuucuc 480

gggaaggccc ugaugcaccu ggccaagaug uucugggacg cccggcucgu ccggucgguc 540

cuucacuucu ugaccuacug gcucugugac gaccacgucu ugcgguuggg gcugacguuc 600

ugguaggacu uccgggaccc gggacggcgg ugggaccuuc ucuacuacug gcggacgguc 660

ccgcacccgc cuggaccggu guuccgggcc cacgaccggc uccgguacuc gguccacugg 720

uugucgcggu gguaguacua cgucgccccg uugaaggccu uggucucuuu cuggcacuuc 780

acgaaguuga cgccguuucu cccgguguaa cgguucuuga cgucucgggg guccuucuuc 840

ccgacgaccu ucacaccguu ucuucccgug gucuacuucc ugacguggcu cgccguccgg 900

uugaaggacc cuuuuuaaac cggaagggug uucccguccg ggccguugaa ggacgucucg 960

uccgggcucg gguggcgggg agggcuccuu ucgaaggcca agccgcuccu uugguggugg 1020

gggucggucu ucguccuugg guagcuguuu cucgacaugg gggaccggag ggacucuucg 1080

gacaagccgu ugcuguucuu uucgcacugg cacgaccugc acccgcugcg gaugaagucg 1140

cacggggacc uauuccugaa ggccuucaug uggcggaagu gguagggguc guaguuguug 1200

cucugugggc cguagucuau ggucauguug cacgacgggg ucccgaccuu cccgucgggg 1260

cgguagaagg ucucgucgua cugguucuag gaccuuggga aggccuucgu cuuggggcug 1320

uagcacuaga uggucaugua ccugcuggac augcacccgu cgcuggaccu uuagccgguc 1380

guggccuguc ucuaggacuu ucuugggcac gugccgcaca ugcacccgaa ggggcacucu 1440

gguguccacg gugacucugg guacuggaug uuccggcggc accuggacuc ggugaaggac 1500

uuccucuucc cgccggaccu uccggacuag augucggucu ucuccguccu guaggaccug 1560

gacacccaca uggugugggu cccgaugaag gggcugaccg ucuugaugug gggaccggga 1620

ccguaggcga ugggggacug gaagccgacc acgaaguucg accacgggca ccuggggcuc 1680

uuccuucacg accacaccuu caagcugucg gccgaccgga agguggugua ccggucucuc 1740

gacguggggc ucaugaugac uacugagcuc uccauggaaa uucugguuac ugaauguucc 1800

gucgacaucu agaaucggug aaaaauuuuc uuuucccccc ugaccuuccc gauuaaguga 1860

ggguugcuuc uguuuuagca gcucucuacg acguauauuc gucgacgaaa aacgaacaug 1920

acccagagag accaaucugg ucuagacucg gacccucgag agaccgauug aucccuuggg 1980

ugacgaauuc ggaguuauuu cgaacggaac ucacgaaguu caucacacac gggcagacaa 2040

cacacugaga ccauugaucu cuagggaguc ugggaaaauc agucacaccu uuuagagauc 2100

guca 2104

<210> 35
<211> 2504
<212> RNA
<213> Artificial Sequence

<220>
<223> Antigen RNA sequence from integrated provirus

<400> 35
uacggguagc acgucuugga cgucccgguc uaccacgugg uccgguaguc gggggccugg 60

gacuugcgga cccacuucca ccaccuccuu uuccggaagu cggggcucca cuagggguac 120

aagucgcggg acucgcuccc gcgguguggg guccuggacu ugugguacga cuuguggcac 180

ccgccggugg uccggcggua cgucuacgac uuucucuguu aguugcuccu ucggcggcuc 240

acccuggccg acgugggaca cgugcggccu ggauagcggg gaccggucua cgcccucggg 300

uccccgucgc uauagcggcc gugguggagg ugugacgucc uugucuagcc gaccuacugg 360

uuguuggggg gguaggggca cccgcucuag auguucgcca ccuaguagga cccggacuug 420

uucuagcacg ccuacauguc gggguggagg uaggaccugu aggccguccc gggguuucuc 480

gggaaggccc ugaugcaccu ggccaagaug uucugggacg cccggcucgu ccggucgguc 540

cuucacuucu ugaccuacug gcucugugac gaccacgucu ugcgguuggg gcugacguuc 600

ugguaggacu uccgggaccc gggacggcgg ugggaccuuc ucuacuacug gcggacgguc 660

ccgcacccgc cuggaccggu guuccgggcc cacgaccggc uccgguacuc gguccacugg 720

uugucgcggu gguaguacua cgucgccccg uugaaggccu uggucucuuu cuggcacuuc 780

acgaaguuga cgccguuucu cccgguguaa cgguucuuga cgucucgggg guccuucuuc 840

ccgacgaccu ucacaccguu ucuucccgug gucuacuucc ugacguggcu cgccguccgg 900

uugaaggacc cuuuuuaaac cggaagggug uucccguccg ggccguugaa ggacgucucg 960

uccgggcucg gguggcgggg agggcuccuu ucgaaggcca agccgcuccu uugguggugg 1020

gggucggucu ucguccuugg guagcuguuu cucgacaugg gggaccggag ggacucuucg 1080

gacaagccgu ugcuguucuu uucgcacugg cacgaccugc acccgcugcg gaugaagucg 1140

cacggggacc uauuccugaa ggccuucaug uggcggaagu gguagggguc guaguuguug 1200

cucugugggc cguagucuau ggucauguug cacgacgggg ucccgaccuu cccgucgggg 1260

cgguagaagg ucucgucgua cugguucuag gaccuuggga aggccuucgu cuuggggcug 1320

uagcacuaga uggucaugua ccugcuggac augcacccgu cgcuggaccu uuagccgguc 1380

guggccuguc ucuaggacuu ucuugggcac gugccgcaca ugcacccgaa ggggcacucu 1440

gguguccacg gugacucugg guacuggaug uuccggcggc accuggacuc ggugaaggac 1500

uuccucuucc cgccggaccu uccggacuag augucggucu ucuccguccu guaggaccug 1560

gacacccaca uggugugggu cccgaugaag gggcugaccg ucuugaugug gggaccggga 1620

ccguaggcga ugggggacug gaagccgacc acgaaguucg accacgggca ccuggggcuc 1680

uuccuucacg accacaccuu caagcugucg gccgaccgga agguggugua ccggucucuc 1740

gacguggggc ucaugaugac uacugagcuc uuuaagggcu auuaguugga gaccuaaugu 1800

uuuaaacacu uucuaacuga ccauaagaau ugauacaacg aggaaaaugc gauacaccua 1860

ugcgacgaaa uuacggaaac auaguacgau aacgaagggc auaccgaaag uaaaagagga 1920

ggaacauauu uaggaccaac gacagagaaa uacuccucaa caccgggcaa caguccguug 1980

caccgcacca cacgugacac aaacgacugc guugggggug accaaccccg uaacgguggu 2040

ggacagucga ggaaaggccc ugaaagcgaa agggggaggg auaacggugc cgccuugagu 2100

agcggcggac ggaacgggcg acgaccuguc cccgagccga caacccguga cuguuaaggc 2160

accaccaaca gccauggaaa uucugguuac ugaauguucc gucgacaucu agaaucggug 2220

aaaaauuuuc uuuucccccc ugaccuuccc gauuaaguga ggguugcuuc uguuuuagca 2280

gcucucuacg acguauauuc gucgacgaaa aacgaacaug acccagagag accaaucugg 2340

ucuagacucg gacccucgag agaccgauug aucccuuggg ugacgaauuc ggaguuauuu 2400

cgaacggaac ucacgaaguu caucacacac gggcagacaa cacacugaga ccauugaucu 2460

cuagggaguc ugggaaaauc agucacaccu uuuagagauc guca 2504

<210> 36
<211> 1764
<212> RNA
<213> Artificial Sequence

<220>
<223> Antigen RNA sequence from integrated provirus

<400> 36
uacggguagc acgucuugga cgucccgguc uaccacgugg uccgguaguc gggggccugg 60

gacuugcgga cccacuucca ccaccuccuu uuccggaagu cggggcucca cuagggguac 120

aagucgcggg acucgcuccc gcgguguggg guccuggacu ugugguacga cuuguggcac 180

ccgccggugg uccggcggua cgucuacgac uuucucuguu aguugcuccu ucggcggcuc 240

acccuggccg acgugggaca cgugcggccu ggauagcggg gaccggucua cgcccucggg 300

uccccgucgc uauagcggcc gugguggagg ugugacgucc uugucuagcc gaccuacugg 360

uuguuggggg gguaggggca cccgcucuag auguucgcca ccuaguagga cccggacuug 420

uucuagcacg ccuacauguc ggggugaggg uaggaccugu aggccguccc gggguuucuc 480

gggaaggccc ugaugcaccu ggccaagaug uucugggacg cccggcucgu ccggucgguc 540

cuucacuucu ugaccuacug gcucugugac gaccacgucu ugcgguuggg gcugacguuc 600

ugguaggacu uccgggaccc gggacggcgg ugggaccuuc ucuacuacug gcggacgguc 660

ccgcacccgc cuggaccggu guuccgggcc cacgaccggc uccgguacuc gguccacugg 720

uugucgcggu gguaguacua cgucgccccg uugaaggccu uggucucuuu cuggcacuuc 780

acgaaguuga cgccguuucu cccgguguaa cgguucuuga cgucucgggg guccuucuuc 840

ccgacgaccu ucacaccguu ucuucccgug gucuacuucc ugacguggcu cgccguccgg 900

uugaaggacc cuuuuuaaac cggaagggug uucccguccg ggccguugaa ggacgucucg 960

uccgggcucg gguggcgggg agggcuccuu ucgaaggcca agccgcuccu uugguggugg 1020

gggucggucu ucguccuugg guagcuguuu cucgacaugg gggaccggag ggacucuucg 1080

gacaagccgu ugcuguucuu uucgcacugg cacgaccugc acccgcugcg gaugaagucg 1140

cacggggacc uauuccugaa ggccuucaug uggcggaagu gguagggguc guaguuguug 1200

cucugugggc cguagucuau ggucauguug cacgacgggg ucccgaccuu cccgucgggg 1260

cgguagaagg ucucgucgua cugguucuag gaccuuggga aggccuucgu cuuggggcug 1320

uagcacuaga uggucaugua ccugcuggac augcacccgu cgcuggaccu uuagccgguc 1380

guggccuguc ucuaggacuu ucuugggcac gugccgcaca ugcacccgaa ggggcacucu 1440

gguguccacg gugacucugg guacuggaug uuccggcggc accuggacuc ggugaaggac 1500

uuccucuucc cgccggaccu uccggacuag augucggucu ucuccguccu guaggaccug 1560

gacacccaca uggugugggu cccgaugaag gggcugaccg ucuugaugug gggaccggga 1620

ccguaggcga uggggacug gaagccgacc acgaaguucg accacggca ccugggcuc 1680

uuccuucacg accacaccuu caagcugucg gccgaccgga agguggugua ccggucucuc 1740

gacguggggc ucaugaugac uacu 1764

<210> 37
<211> 5256
<212> DNA
<213> Artificial Sequence

<220>
<223> Plasmid proviral sequence

<400> 37
tggaagggct aattcactcc caaagaagac aagatatcct tgatctgtgg atctaccaca 60

cacaaggcta cttccctgat tagcagaact acacaccagg gccaggggtc agatatccac 120

tgacctttgg atggtgctac aagctagtac cagttgagcc agataaggta gaagaggcca 180

ataaaggaga gaacaccagc ttgttacacc ctgtgagcct gcatgggatg gatgacccgg 240

agagagaagt gttagagtgg aggtttgaca gccgcctagc atttcatcac gtggcccgag 300

agctgcatcc ggagtacttc aagaactgct gatatcgagc ttgctacaag ggactttccg 360

ctggggactt tccagggagg cgtggcctgg gcgggactgg ggagtggcga gccctcagat 420

cctgcatata agcagctgct ttttgcctgt actgggtctc tctggttaga ccagatctga 480

gcctgggagc tctctggcta actagggaac ccactgctta agcctcaata aagcttgcct 540

tgagtgcttc aagtagtgtg tgcccgtctg ttgtgtgact ctggtaacta gagatccctc 600

agaccctttt agtcagtgtg gaaaatctct agcagtggcg cccgaacagg gacttgaaag 660

cgaaagggaa accagaggag ctctctcgac gcaggactcg gcttgctgaa gcgcgcacgg 720

caagaggcga ggggcggcga ctggtgagta cgccaaaaat tttgactagc ggaggctaga 780

aggagagaga tgggtgcgag agcgtcagta ttaagcgggg gagaattaga tcgcgatggg 840

aaaaaattcg gttaaggcca gggggaaaga aaaaatataa attaaaacat atagtatggg 900

caagcaggga gctagaacga ttcgcagtta atcctggcct gttagaaaca tcagaaggct 960

gtagacaaat actgggacag ctacaaccat cccttcagac aggatcagaa gaacttagat 1020

cattatataa tacagtagca accctctatt gtgtgcatca aaggatagag ataaaagaca 1080

ccaaggaagc tttagacaag atagaggaag agcaaaacaa aagtaagacc accgcacagc 1140

aagcggccgc tgatcttcag acctggagga ggagatatga gggacaattg gagaagtgaa 1200

ttatataaat ataaagtagt aaaaattgaa ccattaggag tagcacccac caaggcaaag 1260

agaagagtgg tgcagagaga aaaaagagca gtgggaatag gagctttgtt ccttgggttc 1320

ttgggagcag caggaagcac tatgggcgca gcgtcaatga cgctgacggt acaggccaga 1380

caattattgt ctggtatagt gcagcagcag aacaatttgc tgagggctat tgaggcgcaa 1440

cagcatctgt tgcaactcac agtctggggc atcaagcagc tccaggcaag aatcctggct 1500

gtggaaagat acctaaagga tcaacagctc ctggggattt ggggttgctc tggaaaactc 1560

atttgcacca ctgctgtgcc ttggaatgct agttggagta ataaatctct ggaacagatt 1620

tggaatcaca cgacctggat ggagtgggac agagaaatta acaattacac aagcttaata 1680

cactccttaa ttgaagaatc gcaaaaccag caagaaaaga atgaacaaga attattggaa 1740

ttagataaat gggcaagttt gtggaattgg tttaacataa caaattggct gtggtatata 1800

aaattattca taatgatagt aggaggcttg gtaggtttaa gaatagtttt tgctgtactt 1860

tctatagtga atagagttag gcagggatat tcaccattat cgtttcagac ccacctccca 1920

accccgaggg gacccgacag gcccgaagga atagaagaag aaggtggaga gagagacaga 1980

gacagatcca ttcgattagt gaacggatct cgacggtatc gccgaattca caaatggcag 2040

tattcatcca caattttaaa agaaaagggg ggattggggg gtacagtgca ggggaaagaa 2100

tagtagacat aatagcaaca gacatacaaa ctaaagaatt acaaaaacaa attacaaaaa 2160

ttcaaaattt tcgggtttat tacagggaca gcagagatcc actttggctg atacgcgccg 2220

agaaaccctg cagggaattc cccagctgta gttataaaca gaagttctcc ttctgctagg 2280

tagcattcaa agatcttaat cttctgggtt tccgttttct cgaatgaaaa atgcaggtcc 2340

gagcagttaa ctggcggggg caccattagc aagtcactta gcatctctgg ggccagtctg 2400

caaagcgagg gggcagcctt aatgtgcctc cagcctgaag tcctagaatg agcgcccggt 2460

gtcccaagct ggggcgcgca ccccagatcg gagggcgccg atgtacagac agcaaactca 2520

cccagtctag tgcatgcctt cttaaacatc acgagactct aagaaaagga aactgaaaac 2580

gggaaagtcc ctctctctaa cctggcactg cgtcgctggc ttggagacag gtgacggtcc 2640

ctgcgggcct tgtcctgatt ggctgggcac gcgtttaata taagtggagg cgtcgcgctg 2700

gcgggcattc ctgaagctga cagcattcgg gccgagcgct cggatccgcc accatgccca 2760

tcgtgcagaa cctgcagggc cagatggtgc accaggccat cagcccccgg accctgaacg 2820

cctgggtgaa ggtggtggag gaaaaggcct tcagccccga ggtgatcccc atgttcagcg 2880

ccctgagcga gggcgccaca ccccaggacc tgaacaccat gctgaacacc gtgggcggcc 2940

accaggccgc catgcagatg ctgaaagaga caatcaacga ggaagccgcc gagtgggacc 3000

ggctgcaccc tgtgcacgcc ggacctatcg cccctggcca gatgcgggag cccaggggca 3060

gcgatatcgc cggcaccacc tccacactgc aggaacagat cggctggatg accaacaacc 3120

cccccatccc cgtgggcgag atctacaagc ggtggatcat cctgggcctg aacaagatcg 3180

tgcggatgta cagccccacc tccatcctgg acatccggca gggccccaaa gagcccttcc 3240

gggactacgt ggaccggttc tacaagaccc tgcgggccga gcaggccagc caggaagtga 3300

agaactggat gaccgagaca ctgctggtgc agaacgccaa ccccgactgc aagaccatcc 3360

tgaaggccct gggccctgcc gccaccctgg aagagatgat gaccgcctgc cagggcgtgg 3420

gcggacctgg ccacaaggcc cgggtgctgg ccgaggccat gagccaggtg accaacagcg 3480

ccaccatcat gatgcagcgg ggcaacttcc ggaaccagag aaagaccgtg aagtgcttca 3540

actgcggcaa agagggccac attgccaaga actgcagagc ccccaggaag aagggctgct 3600

ggaagtgtgg caaagaaggg caccagatga aggactgcac cgagcggcag gccaacttcc 3660

tgggaaaaat ttggccttcc cacaagggca ggcccggcaa cttcctgcag agcaggcccg 3720

agcccaccgc ccctcccgag gaaagcttcc ggttcggcga ggaaaccacc acccccagcc 3780

agaagcagga acccatcgac aaagagctgt accccctggc ctccctgaga agcctgttcg 3840

gcaacgacaa gaaaagcgtg accgtgctgg acgtgggcga cgcctacttc agcgtgcccc 3900

tggataagga cttccggaag tacaccgcct tcaccatccc cagcatcaac aacgagacac 3960

ccggcatcag ataccagtac aacgtgctgc cccagggctg gaagggcagc cccgccatct 4020

tccagagcag catgaccaag atcctggaac ccttccggaa gcagaacccc gacatcgtga 4080

tctaccagta catggacgac ctgtacgtgg gcagcgacct ggaaatcggc cagcaccgga 4140

cagagatcct gaaagaaccc gtgcacggcg tgtacgtggg cttccccgtg agaccacagg 4200

tgccactgag acccatgacc tacaaggccg ccgtggacct gagccacttc ctgaaggaga 4260

agggcggcct ggaaggcctg atctacagcc agaagaggca ggacatcctg gacctgtggg 4320

tgtaccacac ccagggctac ttccccgact ggcagaacta cacccctggc cctggcatcc 4380

gctaccccct gaccttcggc tggtgcttca agctggtgcc cgtggacccc gagaaggaag 4440

tgctggtgtg gaagttcgac agccggctgg ccttccacca catggccaga gagctgcacc 4500

ccgagtacta ctgatgactc gagaaattcc cgataatcaa cctctggatt acaaaatttg 4560

tgaaagattg actggtattc ttaactatgt tgctcctttt acgctatgtg gatacgctgc 4620

tttaatgcct ttgtatcatg ctattgcttc ccgtatggct ttcattttct cctccttgta 4680

taaatcctgg ttgctgtctc tttatgagga gttgtggccc gttgtcaggc aacgtggcgt 4740

ggtgtgcact gtgtttgctg acgcaacccc cactggttgg ggcattgcca ccacctgtca 4800

gctcctttcc gggactttcg ctttccccct ccctattgcc acggcggaac tcatcgccgc 4860

ctgccttgcc cgctgctgga caggggctcg gctgttgggc actgacaatt ccgtggtggt 4920

tgtcggtacc tttaagacca atgacttaca aggcagctgt agatcttagc cactttttaa 4980

aagaaaaggg gggactggaa gggctaattc actcccaacg aagacaaaat cgtcgagaga 5040

tgctgcatat aagcagctgc tttttgcttg tactgggtct ctctggttag accagatctg 5100

agcctgggag ctctctggct aactagggaa cccactgctt aagcctcaat aaagcttgcc 5160

ttgagtgctt caagtagtgt gtgcccgtct gttgtgtgac tctggtaact agagatccct 5220

cagacccttt tagtcagtgt ggaaaatctc tagcag 5256

<210> 38
<211> 4856
<212> DNA
<213> Artificial Sequence

<220>
<223> Plasmid proviral sequence

<400> 38
tggaagggct aattcactcc caaagaagac aagatatcct tgatctgtgg atctaccaca 60

cacaaggcta cttccctgat tagcagaact acacaccagg gccaggggtc agatatccac 120

tgacctttgg atggtgctac aagctagtac cagttgagcc agataaggta gaagaggcca 180

ataaaggaga gaacaccagc ttgttacacc ctgtgagcct gcatgggatg gatgacccgg 240

agagagaagt gttagagtgg aggtttgaca gccgcctagc atttcatcac gtggcccgag 300

agctgcatcc ggagtacttc aagaactgct gatatcgagc ttgctacaag ggactttccg 360

ctggggactt tccagggagg cgtggcctgg gcgggactgg ggagtggcga gccctcagat 420

cctgcatata agcagctgct ttttgcctgt actgggtctc tctggttaga ccagatctga 480

gcctgggagc tctctggcta actagggaac ccactgctta agcctcaata aagcttgcct 540

tgagtgcttc aagtagtgtg tgcccgtctg ttgtgtgact ctggtaacta gagatccctc 600

agaccctttt agtcagtgtg gaaaatctct agcagtggcg cccgaacagg gacttgaaag 660

cgaaagggaa accagaggag ctctctcgac gcaggactcg gcttgctgaa gcgcgcacgg 720

caagaggcga ggggcggcga ctggtgagta cgccaaaaat tttgactagc ggaggctaga 780

aggagagaga tgggtgcgag agcgtcagta ttaagcgggg gagaattaga tcgcgatggg 840

aaaaaattcg gttaaggcca gggggaaaga aaaaatataa attaaaacat atagtatggg 900

caagcaggga gctagaacga ttcgcagtta atcctggcct gttagaaaca tcagaaggct 960

gtagacaaat actgggacag ctacaaccat cccttcagac aggatcagaa gaacttagat 1020

cattatataa tacagtagca accctctatt gtgtgcatca aaggatagag ataaaagaca 1080

ccaaggaagc tttagacaag atagaggaag agcaaaacaa aagtaagacc accgcacagc 1140

aagcggccgc tgatcttcag acctggagga ggagatatga gggacaattg gagaagtgaa 1200

ttatataaat ataaagtagt aaaaattgaa ccattaggag tagcacccac caaggcaaag 1260

agaagagtgg tgcagagaga aaaaagagca gtgggaatag gagctttgtt ccttgggttc 1320

ttgggagcag caggaagcac tatgggcgca gcgtcaatga cgctgacggt acaggccaga 1380

caattattgt ctggtatagt gcagcagcag aacaatttgc tgagggctat tgaggcgcaa 1440

cagcatctgt tgcaactcac agtctggggc atcaagcagc tccaggcaag aatcctggct 1500

gtggaaagat acctaaagga tcaacagctc ctggggattt ggggttgctc tggaaaactc 1560

atttgcacca ctgctgtgcc ttggaatgct agttggagta ataaatctct ggaacagatt 1620

tggaatcaca cgacctggat ggagtgggac agagaaatta acaattacac aagcttaata 1680

cactccttaa ttgaagaatc gcaaaaccag caagaaaaga atgaacaaga attattggaa 1740

ttagataaat gggcaagttt gtggaattgg tttaacataa caaattggct gtggtatata 1800

aaattattca taatgatagt aggaggcttg gtaggtttaa gaatagtttt tgctgtactt 1860

tctatagtga atagagttag gcagggatat tcaccattat cgtttcagac ccacctccca 1920

accccgaggg gacccgacag gcccgaagga atagaagaag aaggtggaga gagagacaga 1980

gacagatcca ttcgattagt gaacggatct cgacggtatc gccgaattca caaatggcag 2040

tattcatcca caattttaaa agaaaagggg ggattggggg gtacagtgca ggggaaagaa 2100

tagtagacat aatagcaaca gacatacaaa ctaaagaatt acaaaaacaa attacaaaaa 2160

ttcaaaattt tcgggtttat tacagggaca gcagagatcc actttggctg atacgcgccg 2220

agaaaccctg cagggaattc cccagctgta gttataaaca gaagttctcc ttctgctagg 2280

tagcattcaa agatcttaat cttctgggtt tccgttttct cgaatgaaaa atgcaggtcc 2340

gagcagttaa ctggcggggg caccattagc aagtcactta gcatctctgg ggccagtctg 2400

caaagcgagg gggcagcctt aatgtgcctc cagcctgaag tcctagaatg agcgcccggt 2460

gtcccaagct ggggcgcgca ccccagatcg gagggcgccg atgtacagac agcaaactca 2520

cccagtctag tgcatgcctt cttaaacatc acgagactct aagaaaagga aactgaaaac 2580

gggaaagtcc ctctctctaa cctggcactg cgtcgctggc ttggagacag gtgacggtcc 2640

ctgcgggcct tgtcctgatt ggctgggcac gcgtttaata taagtggagg cgtcgcgctg 2700

gcgggcattc ctgaagctga cagcattcgg gccgagcgct cggatccgcc accatgccca 2760

tcgtgcagaa cctgcagggc cagatggtgc accaggccat cagcccccgg accctgaacg 2820

cctgggtgaa ggtggtggag gaaaaggcct tcagccccga ggtgatcccc atgttcagcg 2880

ccctgagcga gggcgccaca ccccaggacc tgaacaccat gctgaacacc gtgggcggcc 2940

accaggccgc catgcagatg ctgaaagaga caatcaacga ggaagccgcc gagtgggacc 3000

ggctgcaccc tgtgcacgcc ggacctatcg cccctggcca gatgcgggag cccaggggca 3060

gcgatatcgc cggcaccacc tccacactgc aggaacagat cggctggatg accaacaacc 3120

cccccatccc cgtgggcgag atctacaagc ggtggatcat cctgggcctg aacaagatcg 3180

tgcggatgta cagccccacc tccatcctgg acatccggca gggccccaaa gagcccttcc 3240

gggactacgt ggaccggttc tacaagaccc tgcgggccga gcaggccagc caggaagtga 3300

agaactggat gaccgagaca ctgctggtgc agaacgccaa ccccgactgc aagaccatcc 3360

tgaaggccct gggccctgcc gccaccctgg aagagatgat gaccgcctgc cagggcgtgg 3420

gcggacctgg ccacaaggcc cgggtgctgg ccgaggccat gagccaggtg accaacagcg 3480

ccaccatcat gatgcagcgg ggcaacttcc ggaaccagag aaagaccgtg aagtgcttca 3540

actgcggcaa agagggccac attgccaaga actgcagagc ccccaggaag aagggctgct 3600

ggaagtgtgg caaagaaggg caccagatga aggactgcac cgagcggcag gccaacttcc 3660

tgggaaaaat ttggccttcc cacaagggca ggcccggcaa cttcctgcag agcaggcccg 3720

agcccaccgc ccctcccgag gaaagcttcc ggttcggcga ggaaaccacc acccccagcc 3780

agaagcagga acccatcgac aaagagctgt accccctggc ctccctgaga agcctgttcg 3840

gcaacgacaa gaaaagcgtg accgtgctgg acgtgggcga cgcctacttc agcgtgcccc 3900

tggataagga cttccggaag tacaccgcct tcaccatccc cagcatcaac aacgagacac 3960

ccggcatcag ataccagtac aacgtgctgc cccagggctg gaagggcagc cccgccatct 4020

tccagagcag catgaccaag atcctggaac ccttccggaa gcagaacccc gacatcgtga 4080

tctaccagta catggacgac ctgtacgtgg gcagcgacct ggaaatcggc cagcaccgga 4140

cagagatcct gaaagaaccc gtgcacggcg tgtacgtggg cttccccgtg agaccacagg 4200

tgccactgag acccatgacc tacaaggccg ccgtggacct gagccacttc ctgaaggaga 4260

agggcggcct ggaaggcctg atctacagcc agaagaggca ggacatcctg gacctgtggg 4320

tgtaccacac ccagggctac ttccccgact ggcagaacta cacccctggc cctggcatcc 4380

gctaccccct gaccttcggc tggtgcttca agctggtgcc cgtggacccc gagaaggaag 4440

tgctggtgtg gaagttcgac agccggctgg ccttccacca catggccaga gagctgcacc 4500

ccgagtacta ctgatgactc gagaggtacc tttaagacca atgacttaca aggcagctgt 4560

agatcttagc cactttttaa aagaaaaggg gggactggaa gggctaattc actcccaacg 4620

aagacaaaat cgtcgagaga tgctgcatat aagcagctgc tttttgcttg tactgggtct 4680

ctctggttag accagatctg agcctgggag ctctctggct aactagggaa cccactgctt 4740

aagcctcaat aaagcttgcc ttgagtgctt caagtagtgt gtgcccgtct gttgtgtgac 4800

tctggtaact agagatccct cagacccttt tagtcagtgt ggaaaatctc tagcag 4856

<210> 39
<211> 4810
<212> DNA
<213> Artificial Sequence

<220>
<223> Integrated proviral sequence

<400> 39
   tgtactgggt ctctctggtt agaccagatc tgagcctggg agctctctgg ctaactaggg 60
   aacccactgc ttaagcctca ataaagcttg ccttgagtgc ttcaagtagt gtgtgcccgt 120
   ctgttgtgtg actctggtaa ctagagatcc ctcagaccct tttagtcagt gtggaaaatc 180
   tctagcagtg gcgcccgaac agggacttga aagcgaaagg gaaaccagag gagctctctc 240
   gacgcaggac tcggcttgct gaagcgcgca cggcaagagg cgaggggcgg cgactggtga 300
   gtacgccaaa aattttgact agcggaggct agaaggagag agatgggtgc gagagcgtca 360
   gtattaagcg ggggagaatt agatcgcgat gggaaaaaat tcggttaagg ccagggggaa 420
   agaaaaaata taaattaaaa catatagtat gggcaagcag ggagctagaa cgattcgcag 480
   ttaatcctgg cctgttagaa acatcagaag gctgtagaca aatactggga cagctacaac 540
   catcccttca gacaggatca gaagaactta gatcattata taatacagta gcaaccctct 600
   attgtgtgca tcaaaggata gagataaaag acaccaagga agctttagac aagatagagg 660
   aagagcaaaa caaaagtaag accaccgcac agcaagcggc cgctgatctt cagacctgga 720
   ggaggagata tgagggacaa ttggagaagt gaattatata aatataaagt agtaaaaatt 780
   gaaccattag gagtagcacc caccaaggca aagagaagag tggtgcagag agaaaaaaga 840
   gcagtgggaa taggagcttt gttccttggg ttcttgggag cagcaggaag cactatgggc 900
   gcagcgtcaa tgacgctgac ggtacaggcc agacaattat tgtctggtat agtgcagcag 960
   cagaacaatt tgctgagggc tattgaggcg caacagcatc tgttgcaact cacagtctgg 1020
   ggcatcaagc agctccaggc aagaatcctg gctgtggaaa gatacctaaa ggatcaacag 1080
   ctcctgggga tttggggttg ctctggaaaa ctcatttgca ccactgctgt gccttggaat 1140
   gctagttgga gtaataaatc tctggaacag atttggaatc acacgacctg gatggagtgg 1200
   gacagagaaa ttaacaatta cacaagctta atacactcct taattgaaga atcgcaaaac 1260
   cagcaagaaa agaatgaaca agaattattg gaattagata aatgggcaag tttgtggaat 1320
   tggtttaaca taacaaattg gctgtggtat ataaaattat tcataatgat agtaggaggc 1380
   ttggtaggtt taagaatagt ttttgctgta ctttctatag tgaatagagt taggcaggga 1440
   tattcaccat tatcgtttca gacccacctc ccaaccccga ggggacccga caggcccgaa 1500
   ggaatagaag aagaaggtgg agagagagac agagacagat ccattcgatt agtgaacgga 1560
   tctcgacggt atcgccgaat tcacaaatgg cagtattcat ccacaatttt aaaagaaaag 1620
   gggggattgg ggggtacagt gcaggggaaa gaatagtaga cataatagca acagacatac 1680
   aaactaaaga attacaaaaa caaattacaa aaattcaaaa ttttcgggtt tattacaggg 1740
   acagcagaga tccactttgg ctgatacgcg ccgagaaacc ctgcagggaa ttccccagct 1800
   gtagttataa acagaagttc tccttctgct aggtagcatt caaagatctt aatcttctgg 1860
   gtttccgttt tctcgaatga aaatgcaggt tccgagcagt taactggcgg gggcaccatt 1920
   agcaagtcac ttagcatctc tggggccagt ctgcaaagcg agggggcagc cttaatgtgc 1980
   ctccagcctg aagtcctaga atgagcgccc ggtgtcccaa gctggggcgc gcaccccaga 2040
   tcggagggcg ccgatgtaca gacagcaaac tcacccagtc tagtgcatgc cttcttaaac 2100
   atcacgagac tctaagaaaa ggaaactgaa aacgggaaag tccctctctc taacctggca 2160
   ctgcgtcgct ggcttggaga caggtgacgg tccctgcggg ccttgtcctg attggctggg 2220
   cacgcgttta atataagtgg aggcgtcgcg ctggcgggca ttcctgaagc tgacagcatt 2280
   cgggccgagc gatcggatcc gccaccatgc ccatcgtgca gaacctgcag ggccagatgg 2340
   tgcaccaggc catcagcccc cggaccctga acgcctgggt gaaggtggtg gaggaaaagg 2400
   ccttcagccc cgaggtgatc cccatgttca gcgccctgag cgagggcgcc acaccccagg 2460
   acctgaacac catgctgaac accgtgggcg gccaccaggc cgccatgcag atgctgaaag 2520
   agacaatcaa cgaggaagcc gccgagtggg accggctgca ccctgtgcac gccggaccta 2580
   tcgcccctgg ccagatgcgg gagcccaggg gcagcgatat cgccggcacc acctccacac 2640
   tgcaggaaca gatcggctgg atgaccaaca acccccccat ccccgtgggc gagatctaca 2700
   agcggtggat catcctgggc ctgaacaaga tcgtgcggat gtacagcccc acctccatcc 2760
   tggacatccg gcagggcccc aaagagccct tccgggacta cgtggaccgg ttctacaaga 2820
   ccctgcgggc cgagcaggcc agccaggaag tgaagaactg gatgaccgag acactgctgg 2880
   tgcagaacgc caaccccgac tgcaagacca tcctgaaggc cctgggccct gccgccaccc 2940
   tggaagagat gatgaccgcc tgccagggcg tgggcggacc tggccacaag gcccgggtgc 3000
   tggccgaggc catgagccag gtgaccaaca gcgccaccat catgatgcag cggggcaact 3060
   tccggaacca gagaaagacc gtgaagtgct tcaactgcgg caaagagggc cacattgcca 3120
   agaactgcag agcccccagg aagaagggct gctggaagtg tggcaaagaa gggcaccaga 3180
   tgaaggactg caccgagcgg caggccaact tcctgggaaa aatttggcct tcccacaagg 3240
   gcaggcccgg caacttcctg cagagcaggc ccgagcccac cgcccctccc gaggaaagct 3300
   tccggttcgg cgaggaaacc accaccccca gccagaagca ggaacccatc gacaaagagc 3360
   tgtaccccct ggcctccctg agaagcctgt tcggcaacga caagaaaagc gtgaccgtgc 3420
   tggacgtggg cgacgcctac ttcagcgtgc ccctggataa ggacttccgg aagtacaccg 3480
   ccttcaccat ccccagcatc aacaacgaga cacccggcat cagataccag tacaacgtgc 3540
   tgccccaggg ctggaagggc agccccgcca tcttccagag cagcatgacc aagatcctgg 3600
   aacccttccg gaagcagaac cccgacatcg tgatctacca gtacatggac gacctgtacg 3660
   tgggcagcga cctggaaatc ggccagcacc ggacagagat cctgaaagaa cccgtgcacg 3720
   gcgtgtacgt gggcttcccc gtgagaccac aggtgccact gagacccatg acctacaagg 3780
   ccgccgtgga cctgagccac ttcctgaagg agaagggcgg cctggaaggc ctgatctaca 3840
   gccagaagag gcaggacatc ctggacctgt gggtgtacca cacccagggc tacttccccg 3900
   actggcagaa ctacacccct ggccctggca tccgctaccc cctgaccttc ggctggtgct 3960
   tcaagctggt gcccgtggac cccgagaagg aagtgctggt gtggaagttc gacagccggc 4020
   tggccttcca ccacatggcc agagagctgc accccgagta ctactgatga ctcgagaaat 4080
   tcccgataat caacctctgg attacaaaat ttgtgaaaga ttgactggta ttcttaacta 4140
   tgttgctcct tttacgctat gtggatacgc tgctttaatg cctttgtatc atgctattgc 4200
   ttcccgtatg gctttcattt tctcctcctt gtataaatcc tggttgctgt ctctttatga 4260
   ggagttgtgg cccgttgtca ggcaacgtgg cgtggtgtgc actgtgtttg ctgacgcaac 4320
   ccccactggt tggggcattg ccaccacctg tcagctcctt tccgggactt tcgctttccc 4380
   cctccctatt gccacggcgg aactcatcgc cgcctgcctt gcccgctgct ggacaggggc 4440
   tcggctgttg ggcactgaca attccgtggt ggttgtcggt acctttaaga ccaatgactt 4500
   acaaggcagc tgtagatctt agccactttt taaaagaaaa ggggggactg gaagggctaa 4560
   ttcactccca acgaagacaa gatcgtcgag agatgctgca tataagcagc tgctttttgc 4620
   ttgtactggg tctctctggt tagaccagat ctgagcctgg gagctctctg gctaactagg 4680
   gaacccactg cttaagcctc aataaagctt gccttgagtg cttcaagtag tgtgtgcccg 4740
   tctgttgtgt gactctggta actagagatc cctcagaccc ttttagtcag tgtggaaaat 4800
   ctctagcagt 4810

<210> 40
<211> 4410
<212> DNA
<213> Artificial Sequence

<220>
<223> Integrated proviral sequence

<400> 40
   tgtactgggt ctctctggtt agaccagatc tgagcctggg agctctctgg ctaactaggg 60
   aacccactgc ttaagcctca ataaagcttg ccttgagtgc ttcaagtagt gtgtgcccgt 120
   ctgttgtgtg actctggtaa ctagagatcc ctcagaccct tttagtcagt gtggaaaatc 180
   tctagcagtg gcgcccgaac agggacttga aagcgaaagg gaaaccagag gagctctctc 240
   gacgcaggac tcggcttgct gaagcgcgca cggcaagagg cgaggggcgg cgactggtga 300
   gtacgccaaa aattttgact agcggaggct agaaggagag agatgggtgc gagagcgtca 360
   gtattaagcg ggggagaatt agatcgcgat gggaaaaaat tcggttaagg ccagggggaa 420
   agaaaaaata taaattaaaa catatagtat gggcaagcag ggagctagaa cgattcgcag 480
   ttaatcctgg cctgttagaa acatcagaag gctgtagaca aatactggga cagctacaac 540
   catcccttca gacaggatca gaagaactta gatcattata taatacagta gcaaccctct 600
   attgtgtgca tcaaaggata gagataaaag acaccaagga agctttagac aagatagagg 660
   aagagcaaaa caaaagtaag accaccgcac agcaagcggc cgctgatctt cagacctgga 720
   ggaggagata tgagggacaa ttggagaagt gaattatata aatataaagt agtaaaaatt 780
   gaaccattag gagtagcacc caccaaggca aagagaagag tggtgcagag agaaaaaaga 840
   gcagtgggaa taggagcttt gttccttggg ttcttgggag cagcaggaag cactatgggc 900
   gcagcgtcaa tgacgctgac ggtacaggcc agacaattat tgtctggtat agtgcagcag 960
   cagaacaatt tgctgagggc tattgaggcg caacagcatc tgttgcaact cacagtctgg 1020
   ggcatcaagc agctccaggc aagaatcctg gctgtggaaa gatacctaaa ggatcaacag 1080
   ctcctgggga tttggggttg ctctggaaaa ctcatttgca ccactgctgt gccttggaat 1140
   gctagttgga gtaataaatc tctggaacag atttggaatc acacgacctg gatggagtgg 1200
   gacagagaaa ttaacaatta cacaagctta atacactcct taattgaaga atcgcaaaac 1260
   cagcaagaaa agaatgaaca agaattattg gaattagata aatgggcaag tttgtggaat 1320
   tggtttaaca taacaaattg gctgtggtat ataaaattat tcataatgat agtaggaggc 1380
   ttggtaggtt taagaatagt ttttgctgta ctttctatag tgaatagagt taggcaggga 1440
   tattcaccat tatcgtttca gacccacctc ccaaccccga ggggacccga caggcccgaa 1500
   ggaatagaag aagaaggtgg agagagagac agagacagat ccattcgatt agtgaacgga 1560
   tctcgacggt atcgccgaat tcacaaatgg cagtattcat ccacaatttt aaaagaaaag 1620
   gggggattgg ggggtacagt gcaggggaaa gaatagtaga cataatagca acagacatac 1680
   aaactaaaga attacaaaaa caaattacaa aaattcaaaa ttttcgggtt tattacaggg 1740
   acagcagaga tccactttgg ctgatacgcg ccgagaaacc ctgcagggaa ttccccagct 1800
   gtagttataa acagaagttc tccttctgct aggtagcatt caaagatctt aatcttctgg 1860
   gtttccgttt tctcgaatga aaaatgcagg tccgagcagt taactggcgg gggcaccatt 1920
   agcaagtcac ttagcatctc tggggccagt ctgcaaagcg agggggcagc cttaatgtgc 1980
   ctccagcctg aagtcctaga atgagcgccc ggtgtcccaa gctggggcgc gcaccccaga 2040
   tcggagggcg ccgatgtaca gacagcaaac tcacccagtc tagtgcatgc cttcttaaac 2100
   atcacgagac tctaagaaaa ggaaactgaa aacgggaaag tccctctctc taacctggca 2160
   ctgcgtcgct ggcttggaga caggtgacgg tccctgcggg ccttgtcctg attggctggg 2220
   cacgcgttta atataagtgg aggcgtcgcg ctggcgggca ttcctgaagc tgacagcatt 2280
   cgggccgagc gatcggatcc gccaccatgc ccatcgtgca gaacctgcag ggccagatgg 2340
   tgcaccaggc catcagcccc cggaccctga acgcctgggt gaaggtggtg gaggaaaagg 2400
   ccttcagccc cgaggtgatc cccatgttca gcgccctgag cgagggcgcc acaccccagg 2460
   acctgaacac catgctgaac accgtgggcg gccaccaggc cgccatgcag atgctgaaag 2520
   agacaatcaa cgaggaagcc gccgagtggg accggctgca ccctgtgcac gccggaccta 2580
   tcgcccctgg ccagatgcgg gagcccaggg gcagcgatat cgccggcacc acctccacac 2640
   tgcaggaaca gatcggctgg atgaccaaca acccccccat ccccgtgggc gagatctaca 2700
   agcggtggat catcctgggc ctgaacaaga tcgtgcggat gtacagcccc acctccatcc 2760
   tggacatccg gcagggcccc aaagagccct tccgggacta cgtggaccgg ttctacaaga 2820
   ccctgcgggc cgagcaggcc agccaggaag tgaagaactg gatgaccgag acactgctgg 2880
   tgcagaacgc caaccccgac tgcaagacca tcctgaaggc cctgggccct gccgccaccc 2940
   tggaagagat gatgaccgcc tgccagggcg tgggcggacc tggccacaag gcccgggtgc 3000
   tggccgaggc catgagccag gtgaccaaca gcgccaccat catgatgcag cggggcaact 3060
   tccggaacca gagaaagacc gtgaagtgct tcaactgcgg caaagagggc cacattgcca 3120
   agaactgcag agcccccagg aagaagggct gctggaagtg tggcaaagaa gggcaccaga 3180
   tgaaggactg caccgagcgg caggccaact tcctgggaaa aatttggcct tcccacaagg 3240
   gcaggcccgg caacttcctg cagagcaggc ccgagcccac cgcccctccc gaggaaagct 3300
   tccggttcgg cgaggaaacc accaccccca gccagaagca ggaacccatc gacaaagagc 3360
   tgtaccccct ggcctccctg agaagcctgt tcggcaacga caagaaaagc gtgaccgtgc 3420
   tggacgtggg cgacgcctac ttcagcgtgc ccctggataa ggacttccgg aagtacaccg 3480
   ccttcaccat ccccagcatc aacaacgaga cacccggcat cagataccag tacaacgtgc 3540
   tgccccaggg ctggaagggc agccccgcca tcttccagag cagcatgacc aagatcctgg 3600
   aacccttccg gaagcagaac cccgacatcg tgatctacca gtacatggac gacctgtacg 3660
   tgggcagcga cctggaaatc ggccagcacc ggacagagat cctgaaagaa cccgtgcacg 3720
   gcgtgtacgt gggcttcccc gtgagaccac aggtgccact gagacccatg acctacaagg 3780
   ccgccgtgga cctgagccac ttcctgaagg agaagggcgg cctggaaggc ctgatctaca 3840
   gccagaagag gcaggacatc ctggacctgt gggtgtacca cacccagggc tacttccccg 3900
   actggcagaa ctacacccct ggccctggca tccgctaccc cctgaccttc ggctggtgct 3960
   tcaagctggt gcccgtggac cccgagaagg aagtgctggt gtggaagttc gacagccggc 4020
   tggccttcca ccacatggcc agagagctgc accccgagta ctactgatga ctcgagaggt 4080
   acctttaaga ccaatgactt acaaggcagc tgtagatctt agccactttt taaaagaaaa 4140
   ggggggactg gaagggctaa ttcactccca acgaagacaa gatcgtcgag agatgctgca 4200
   tataagcagc tgctttttgc ttgtactggg tctctctggt tagaccagat ctgagcctgg 4260
   gagctctctg gctaactagg gaacccactg cttaagcctc aataaagctt gccttgagtg 4320
   cttcaagtag tgtgtgcccg tctgttgtgt gactctggta actagagatc cctcagaccc 4380
   ttttagtcag tgtggaaaat ctctagcagt 4410

## Description

### TECHNICAL FIELD

The present invention is in the field of recombinant vaccine technology and relates to improvements of lentiviral vectors, which can be used as therapeutic and prophylactic vaccines. The vectors provide improved immune responses over other vectors.

### BACKGROUND

Recombinant vaccines have been developed with the progress of recombinant DNA technology, allowing the modification of viral genomes to produce modified viruses. In this manner, it has been possible to introduce genetic sequences into non-pathogenic viruses, so that they encode immunogenic proteins to be expressed in target cells upon infection or transduction, in order to develop a specific immune response in their host.

Such vaccines constitute a major advance in vaccine technology (Kutzler et al., Nat Rev Genet, 9(10): 776-788, 2008). In particular, they have the advantage over traditional vaccines of avoiding live (attenuated) virus and eliminating risks associated with the manufacture of inactivated vaccines.

Gene delivery using modified retroviruses (retroviral vectors) was introduced in the early 1980s by Mann et al. (Cell, 33(1):153-9, 1983). The most commonly used oncogenic retroviral vectors are based on the Moloney murine leukemia virus (MLV). They have a simple genome from which the polyproteins Gag, Pol and Env are produced and are required in *trans for* viral replication (Breckpot et al., 2007, Gene Ther, 14(11):847-62; He et al. 2007, Expert Rev vaccines, 6(6):913-24). Sequences generally required in *cis* are the long terminal repeats (LTRs) and its vicinity: the inverted repeats (IR or att sites) required for integration, the packaging sequence ψ, the transport RNA-binding site (primer binding site, PBS), and some additional sequences involved in reverse transcription (the repeat R within the LTRs, and the polypurine tracts, PPT, necessary for plus strand initiation). To generate replication-defective retroviral vectors, the *gag, pol,* and *env* genes are generally entirely deleted and replaced with an expression cassette.

Retroviral vectors deriving from lentivirus genomes (i.e. lentiviral vectors) have emerged as promising tools for both gene therapy and immunotherapy purposes, because they exhibit several advantages over other viral systems. In particular, lentiviral vectors themselves are not toxic and, unlike other retroviruses, lentiviruses are capable of transducing non-dividing cells, in particular dendritic cells (He et al. 2007, Expert Rev vaccines, 6(6):913-24), allowing antigen presentation through the endogenous pathway.

Lentiviruses are linked by similarities in genetic composition, molecular mechanisms of replication and biological interactions with their hosts. They are best known as agents of slow disease syndromes that begin insidiously after prolonged periods of subclinical infection and progress slowly; thus, they are referred to as the "slow" viruses (Narayan et al., 1989, J Gen Virol, 70(7):1617-39). They have the same basic organization as all retroviruses but are more complex due to the presence of accessory genes (e.g., *vif, vpr, vpu, nef, tat,* and *rev),* which play key roles in lentiviral replication *in vivo.*

Lentiviruses represent a genus of slow viruses of the Retroviridae family, which includes the human immunodeficiency viruses (HIV), the simian immunodeficiency virus (SIV), the equine infectious encephalitis virus (EIAV), the caprine arthritis encephalitis virus (CAEV), the bovine immunodeficiency virus (BIV) and the feline immunodeficiency virus (FIV). Lentiviruses can persist indefinitely in their hosts and replicate continuously at variable rates during the course of the lifelong infection. Persistent replication of the viruses in their hosts depends on their ability to circumvent host defenses.

The design of recombinant integrating lentiviral vectors is based on the separation of the *cis-* and trans-acting sequences of the lentivirus. Efficient transduction in non-dividing cells requires the presence of two *cis*-acting sequences in the lentiviral genome, the central polypurine tract (cPPT) and the central termination sequence (CTS). These lead to the formation of a triple-stranded DNA structure called the central DNA "flap", which maximizes the efficiency of gene import into the nuclei of non-dividing cells, including dendritic cells (DCs) (Zennou et al., 2000, Cell, 101 (2) 173-85; Arhel et al., 2007, EMBO J, 26(12):3025-37).

Dendritic cells are of primary importance for antigen presentation because they constitute the main class of antigen presenting cells (APCs) whose primary function is to present antigens and initiate an immune response.

To generate an immune response, antigenic proteins must be processed by cells into peptides that are displayed on the cell surface by major histocompatibility complex proteins (MHCs). Circulating APCs present the peptide-MHC complexes to T cells in the draining lymph nodes, where they interact with T cell receptors, and, in conjunction with co-stimulatory signals, activate the T cells.

A variety of studies have shown that inoculation with lentiviral vectors leads to antigen presentation by DCs and strong activation of antigen specific cytotoxic T lymphocytes (CTLs; CD8⁺ T cells). Therefore, lentiviral vectors have been engineered for the last 10 years for gene transfer and immunotherapy applications.

The vectors routinely contain strong constitutive promoters containing enhancers, such as the CMV promoter. Michelini et al., Vaccine 27(34):4622-29 (2009); Karwacz et al., J. Virol. 83(7):30943103 (2009); Negri et al., Molecular Therapy 15(9):1716-23 (2007); and Buffa et al., J. General Virology 87:1625-1634 (2006).

Lentiviral vectors have been improved in their safety by removal of the LTR U3 sequence, resulting in *"self-inactivating"* vectors that are entirely devoid of viral promoter and enhancer sequences originally present within the LTRs.

The lentiviral particles, which contain lentiviral vectors, can be produced by recombinant technology upon transient transfection of cells, for example HEK 293T human cultured cells, by different DNA plasmids:
(i) a packaging plasmid, which expresses at least the Gag, Pol Rev, Tat and, in some cases, structural and enzymatic proteins necessary for the packaging of the transfer construct;
(ii) a proviral transfer plasmid, containing an expression cassette and HIV cis-acting factors necessary for packaging, reverse transcription, and integration; and
(iii) an envelope-encoding plasmid, in most cases the glycoprotein of vesicular stomatitis virus (VSV.G), a protein that allows the formation of mixed particles (pseudotypes) that can target a wide variety of cells, especially major histocompatibility (MHC) antigen-presenting cells (APCs), including DCs.

This procedure allows obtaining transient production of lentiviral particle vectors by the transfected cells. However, the lentiviral particle vectors may also be continuously produced by cells by stably inserting the packaging genes, the proviral coding DNA, and the envelope gene into the cellular genome. This allows the continuous production of lentiviral particle vectors by the cells without the need for transient transfection. Of course, a combination of these procedures can be used, with some of the DNAs/plasmids integrated into the cellular genome and others provided by transient transfection.

Non-integrating lentiviral vectors have been designed to mitigate the risks of potential oncogenesis linked to insertional mutagenesis events, particularly for vaccination purposes. Examples of non-integrating lentiviral vectors are provided in Coutant et al., PLOS ONE 7(11):e48644 (2102), Karwacz et al., J. Virol. 83(7):3094-3103 (2009), Negri et al., Molecular Therapy 15(9):1716-1723 (2007); Hu et al., Vaccine 28:6675-6683 (2010). Consequently, it has been reported that a non-integrating lentiviral vector system greatly mitigates the potential risk of insertional mutagenesis as compared to an integrating system. Hu et al., Vaccine 28:6675-6683 (2010). It has been further reported that functional analysis indicates that both the magnitude and quality of the immune responses elicited by DC-directed integration-defective lentiviral vectors (IDLVs) are comparable to that of its integrating counterpart. *Id.* Thus, integration-defective lentiviral vectors (IDLVs) have been considered safer vectors than integrating vectors for human administration, with comparable effectiveness.

In addition, deletion in the U3 region of the 3' LTR of the viral promoter and enhancer sequences in self-inactivating lentiviral vectors limits the likelihood of endogenous promoter activation. These concerns with safety directly address the experiences gained from the SCID-X1 gene therapy trial carried out in 1998-1999, performed with Moloney virus-based retroviral vectors on children suffering from a rare form of X-linked (SCID-X1 gene) severe immunodeficiency disease (Cavazzana-Calvo et al., 2000, Science., 288(5466):669-72). During this trial, four of nine children developed leukemia as a result of the integration of the Moloney-derived retroviral vector at close proximity to the human LM02 proto-oncogene (Hacein-Bey-Abina et al., 2008, J.Clin.Invest., 118(9):3132-3142). It was demonstrated that malignancy was the consequence of the proximity of the viral U3 promoter/enhancer to the LM02 proto-oncogene. As a result, safety is a major concern for the administration of lentivectors to humans.

Enhancers are *cis*-acting sequences, which can act as transcriptional activators at a distance. They have been widely employed in viral derived vectors because they appear to be the most efficient for obtaining transgene strong expression in a variety of cell types, in particular DCs (Chinnasamy et al., 2000, Hum Gene Ther 11(13):1901-9; Rouas et al., 2008, Cancer Gene Ther 9(9):715-24; Kimura et al., 2007, Mol Ther 15(7):1390-9; Gruh et al., 2008, J Gene Med 10(1) 21-32). However, given the safety issue of insertional mutagenesis, such transcriptional enhancer sequences should be deleted from the lentiviral vector constructs to abolish the risk of insertional mutagenesis by enhancer proximity effect. This enhancer proximity effect is by far the most frequent mechanism of insertional mutagenesis and is the only effect described in human or animal cases of tumorigenic events after gene transfer.

Thus, there is a need to develop retroviral, particularly lentiviral vectors, which do not include viral enhancers, but still allow sufficient expression of transgenes encoding immunogenic peptides, if possible, as much expression as that observed when using the CMV promoter.

Recent studies has reported on the replacement of viral promoters by DC-specific promoters deriving from major histocompatibility complex class II genes (MHC class II) (Kimura et al., 2007, Mol Ther 15(7):1390-9) and dectin-2 genes (Lopes et al., 2008, J Virol 82(1):86-95). The dectin-2 gene promoter used in Lopes et al. contains a putative enhancer and an adenoviral conserved sequence (inverted terminal repeats in adenovirus promoter) (Bonkabara et al., 2001, J. Immunology, 167:6893-6900). The MHC class II gene promoter used by Kimura *et al.* does not contain any known enhancer.

Yet, without an enhancer, the MHC class II promoter was found not to provide sufficient transgene expression in DCs, when administered intravenously. In particular, lentiviral vectors including MHC class II promoters did not provoke an immune reaction in immunocompetent C57BL/6 mice, in contrast to the immune responses observed with CMV promoters/enhancers. Although integration and persistent transgene expression were observed after injection in mice, the lentiviral vectors transcribed through MHC class II promoters failed to stimulate an antigen-specific CD8+ cytotoxic T-lymphocyte response, even after vaccination boost. The authors of these studies therefore concluded that the use of MHC class II promoters was of interest only for applications where persistence of expression is sought as in gene replacement therapy, but not in the context of immunotherapy. Of note, MHC class II promoters are expressed poorly in most cell types.

Thus, the MHC class II promoter is not an adequate promoter for lentiviral vectors for induction of an immune response against an antigen via IV injection. Moreover, the dectin-2 promoter is expressed poorly in most cell types and appears to contain an enhancer. Thus, the dectin-2 promoter is not a good promoter for lentiviral vectors for safety reasons.

Preferably, in immunotherapy, lentiviral vectors provide effective expression of the transgene that elicits a desired specific immune response. This requires that the expression is at a high level in APCs, such as dendritic cells.

It is also preferable that the cells transduced by the lentiviral vectors are eliminated by the immune response to provide a higher degree of safety. That is, the transgene can elicit an immune response in the host sufficient to eliminate the cells that are transduced by the lentiviral vectors. The elimination of transduced cells eliminates the persistence of the lentiviral vector in the host, and possible secondary effects of the vector. In order for the transduced cells to be eliminated, expression is required in non-dendritic cells at a level that allows elimination by the immune response. Thus, appropriate expression of an antigen is desirable.

At the same time, the promoter should maximize immune stimulation through the key cells (i.e., dendritic cells) involved in the activation of naïve and memory T cells, and should minimize the risk of insertional mutagenesis and genotoxicity in stem cells, leading to malignancies. Thus, the promoter should have sufficiently high activity in dendritic and other cells, but not contain an enhancer. Based on these criteria, viral promoters, such as the CMV promoter, are not ideal because of the presence of strong enhancers. These criteria are summarized as follows:
1. high expression in antigen presenting cells, including dendritic cells, to induce maximal immune responses;
2. expression in other transduced cell types sufficient for elimination by the induced immune response; and
3. lack of an enhancer element to avoid insertional effects.

Thus, a need exists in the art for improved vectors and methods for immunizing humans. The present invention fulfills these needs in the art.

### SUMMARY OF THE INVENTION

The invention encompasses compositions comprising lentiviral vectors and methods of using the vectors. The invention encompasses methods for inducing an immune response in a human. In one embodiment, the method comprises intramuscularly administering integrating lentiviral vector particles comprising a lentiviral vector to a human. In one embodiment, the intramuscular administration is in a priming dose and a boosting dose.

The invention encompasses compositions comprising a dose of 5 x 10⁶ to 5 x 10⁸ TU of lentiviral vector particles for use in the treatment of a microbial infection or a cancer by induction of a CD4+ and/or CD8+ T cell response against an antigen encoded by the lentiviral vector. Preferably, the treatment is by repetitive intramuscular administration to a human,

The lentiviral vector particles can comprise a functional integrase protein and a lentiviral vector. The DNA of the lentiviral vector can comprise a β2m or MHC class I promoter directing expression of a microbial or tumor antigen, wherein the promoter does not contain an enhancer. Preferably, the repetitive intramuscular administration induces CD4+ and/or CD8+ T-specific responses against the antigen.

Preferably, the lentiviral vector encodes a viral antigen, preferably a human immunodeficiency virus type I antigen, a bacterial antigen, or a tumor antigen, preferably a human telomerase antigen.

In various embodiments, the method comprises injecting at least 10⁷ or 5 x 10⁷ lentiviral vector particles.

The invention further encompasses compositions comprising a lentiviral vector or lentiviral vector particle according to the invention for use as a medicament or vaccine.

The invention encompasses a composition or use, wherein the lentiviral vector comprises a viral antigen, particularly a human immunodeficiency virus type I antigen, a bacterial antigen, or a tumor antigen, particularly a human telomerase antigen.

The invention encompasses a composition or use comprising at least 10⁷ or 5 x 10⁷ lentiviral vector particles.

The invention encompasses nucleic acid molecules and vectors encoding a polypeptide comprising the amino acid sequence of SEQ ID NO:30 and methods of using the nucleic acid molecules and vectors.

Preferably, the vector is a lentiviral vector. In one embodiment, the vector comprises a β2-microglobulin promoter. In one embodiment, the vector comprises a Woodchuck PostTranscriptional Regulatory Element (WPRE).

Preferably, the vector encodes an HIV-1 (e.g., Gag, Pol, or Nef) polypeptide.

In one embodiment, the vector is a DNA. Preferably, the vector comprises or encodes the nucleic acid sequence of SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, or SEQ ID NO:40.

The invention encompasses a lentiviral vector particle encoding a polypeptide comprising the amino acid sequence of SEQ ID NO:30. Preferably, the lentiviral vector particle comprises the nucleic acid sequence of SEQ ID NO:31 or SEQ ID NO:32.

In one embodiment, the lentiviral vector particle comprises a functional lentiviral integrase protein. In one embodiment, the lentiviral vector particle comprises a vesicular stomatitis virus glycoprotein. In one embodiment, the lentiviral vector particle comprises HIV-1 subtype D Gag and Pol proteins.

The invention encompasses an isolated cell comprising a vector of the invention.

The invention encompasses the use of a vector of the invention for inducing an immune response in a human by intramuscular administration.

The invention encompasses a method for inducing an immune response in a human comprising intramuscularly administering a lentiviral vector particle of the invention to a human.

### BRIEF DESCRIPTION OF THE DRAWINGS

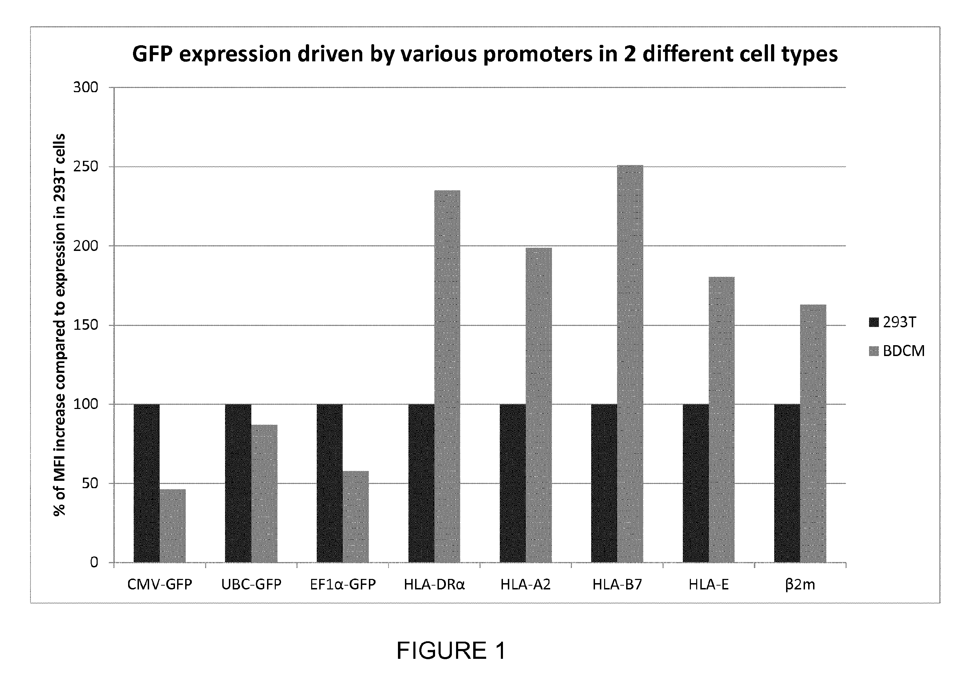

**Figure 1****.** Evaluation of the GFP expression driven by various internal promoters. Lentivector constructions encompassing various promoters were used to transduce HEK293T (a fibroblast cell line) and BDCM (a dendritic-like cell line) cells, and the promoters' ability to drive the GFP expression was evaluated by flow cytometry. MFI (Mean Fluorescence intensity) in BDCM cells are represented in % of the MFI obtained in 293T cells. CMV: cytomegalovirus, β2m: β2-microglobulin, UBC: Ubiquitin C and EF1α: Elongating factor 1α. See http://biogps.org/#goto=welcome for their corresponding expression profiles in human tissues.
**Figure 2****.** T-cell specific immune response (ELISpot IFN-γ) elicited in C57BI/6j mice by lentiviral vectors wherein the antigen (HIV-1) expression is driven by various promoters (1 10⁶TU/mouse, cumulative response, median).
**Figure 3****.** In vivo evaluation of the GFP expression in DCs isolated from muscle or draining lymph node three days after mice intramuscular immunization with 10⁸ TU of lentiviral vector encompassing the CMV or the β2m promoter.
**Figure 4****.** Specific CD4+ and CD8+ response elicited against the P24 and Nef part of the HIV-1 antigen, after intramuscular injection of 2 10⁷ TU of the lentivector (β2m-HIV) in Sprague Dawley rats (ELISpot IFN-γ).
**Figure 5****.** Long lasting specific T-cell response elicited after intramuscular injection of 2.68 10⁷ TU of the vaccine candidate (β2m-HIV) in C57BI/6j mice (ELISpot IFN-γ, cumulative response).
**Figure 6****.** Diversified specific T-cell response elicited after intramuscular injection of 2.68 10⁷ TU of the vaccine candidate (β2m-HIV) in C57BI/6j mice (ELISpot IFN-γ, cumulative response).
**Figure 7****.** Evaluation of the cross neutralization of lentivectors pseudotyped with New Jersey or Indiana VSV-G glycoprotein using serum from mice injected with vectors pseudotyped with Indiana VSV-G glycoprotein.
**Figure 8****.** Evaluation of the cross neutralization of lentivectors pseudotyped with New Jersey or Indiana VSV glycoprotein using serum from mice injected with vectors pseudotyped with New Jersey VSV glycoprotein.
**Figure 9****.** Specific T-cell response (ELISpot IFN-γ) after intramuscular injection of (2 10⁶ TU) in Sprague-Dawley rats (n=6) in a prime-boost regimen (THV01-1 and THV01-2 injected 6 weeks apart).
**Figure 10****.** Specific T-cell response (ELISpot IFN-γ, cumulative response) elicited in Sprague-Dawley rats after injection of 3 different lentiviral doses (2 10⁵, 2 10⁶ and 2 10⁷ TU) displayed in 2 different volumes (n=20 and 400µL).
**Figure 11****.** Specific T-cell response (ELISpot IFN-γ, cumulative response) elicited in SD rats (n=6) after intramuscular injection of 2 10⁶ TU of lentiviral vector displayed in 1, 2, 3 or 4 injection sites.

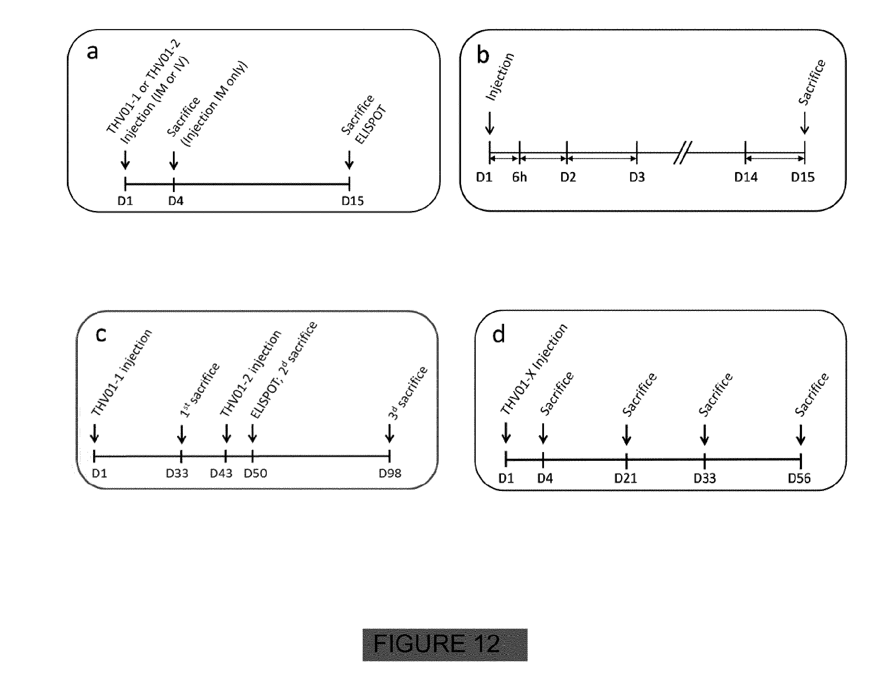

**Figure 12****.** Schematic depiction of GLP preclinical studies performed in Sprague-Dawley rats. **a**. Design of the acute single dose study: MAD injection of the preclinical batches of THV01-1 and THV01-2, intramuscularly as it is clinical route and intravenously to evaluate worst case scenario. Sacrifices were performed at day 4 for IM group only to evaluate microscopic and macroscopic toxicity and the local tolerance and at day 15 for IM and IV groups to evaluate the immunogenicity and the recovery after injection. **b**. Design of the vector shedding in urines: urines were collected in GMP conditions in animal participating in the acute single dose study. **c**. Design of the treatment toxicity study: clinical treatment was mimicked in rats, with MAD and ½ MAD THV01-1 prime injection followed by THV01-2 boost injection 6 weeks later. Sacrifices for toxicology studies were performed before and after the boost injection, and 17 weeks post prime. During this study, blood was collected before and after injections and samples were used to evaluate the cytokines levels. d. GLP biodistribution study design: Rats were injected with THV01-1 or THV01-2 (MAD) and sacrifices were performed at day 4, 21, 33 and 56. 13 organs (brain, ovaries or testes, heart, lung, liver, spleen, adrenal, kidneys, bone marrow, skeletal muscle -dorsal-, lymph node -iliac-, subcutis -injection site-, skeletal muscle -injection site-) and 3 body fluids (blood, urines and feces) were collected, and the presence of proviral sequence was evaluated with specific qPCR.

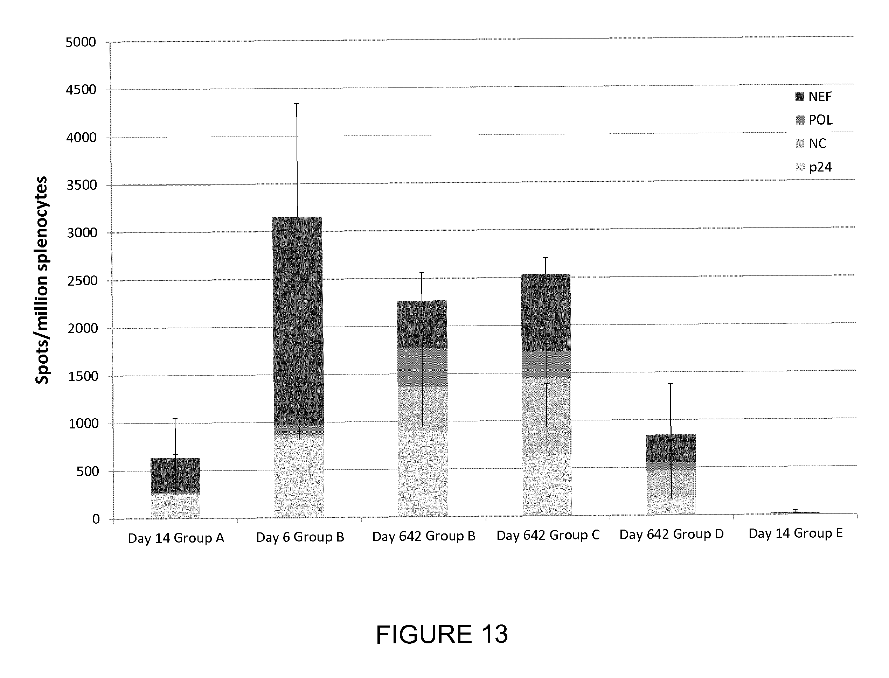

**Figure 13****.** Specific T-cell response elicited after MAD (400µL) intramuscular injections of THV01-1 (corresponding to 1.06 10⁸ TU) and THV01-2 (corresponding to 1.88.10⁸ TU) lentiviral vectors in Sprague Dawley rats (ELISPot IFN-γ). Group A: THV01-1 alone, Group B THV01-1 and THV01-2 injected 6 weeks apart (sacrifice day 49, 6 days post THV01-2 boost injection), Group C: Group B that received a second boost injection at day 637 (day 599 post THV01-2 boost) with THV01-3, the HIV-1 vaccine candidate pseudotyped with Cocal, a third VSV-G serotype, MAD corresponding to 5,3.10⁷ TU), Group D: THV01-3 alone injected at day 628 (MAD, corresponding to 5,3.10⁷) and Group E : control (PBS-Lactose).
**Figure 14****.** GLP biodistribution (qPCR) of the lentiviral proviral sequences after MAD intramuscular injections of THV01-1 (A) and THV01-2 (B). Variations between animals (males and females) can be observed, as the lentiviral vector diffusion is reduced after intramuscular administration and the injection sites were sometimes hard to localize and sample precisely at necropsy.
**Figure 15****.** Bioluminescence monitoring of LV after IM injections of 1 10⁶ TU of the lentiviral vector in C57BI/6j mice.
**Figure 16****.** Comparison of the specific T-cell specific immune responses (ELISpot IFN-γ, cumulative response) elicited by the injection of a lentiviral vector (β2m-HIV) following 3 administration routes (intramuscular, subcutaneous and intranasal) in Sprague Dawley rat (2 10⁶ TU/rat, n=4/6).
**Figure 17****.** Evaluation of the specific T-cell specific immune responses (ELISpot IFN-γ, cumulative response) elicited by the injection of a lentiviral vector (β2m-HIV) following 5 administration routes (intramuscular, subcutaneous, intranasal, intravaginal and intrarectal) in Sprague Dawley rat (2 10⁷ TU/rat, n=2/8).
**Figure 18****.** Evaluation of the specific T-cell specific immune responses (ELISpot IFN-γ, median of responses against p24, NC, POL and NEF) elicited by the injection of a lentiviral vector (β2m-HIV) injected following 5 administration routes (intramuscular, subcutaneous, intranasal, intravaginal and intrarectal) in Sprague Dawley rat (2 10⁷ TU/rat, n=2/8).
**Figure 19****.** Dose-response in respect of the immunogenicity of a non-integrating vector (β2m-HIV integrase mutant), compared to the effective dose of the corresponding integrating vector (β2m-HIV). Mice (5 per group) were inoculated by intramuscular injection with the indicated doses of viral particles. Splenocytes were isolated from the mice after 12 days and stimulated for 18 h with pools of peptides. All reactions were performed in triplicate.
**Figure 20****.** Dose-response in respect of the immunogenicity of an Adenovector (Ad5-β2m-HIV adenoviral vector), compared to an effective dose of the corresponding integrating lentiviral vector (β2m-HIV). Mice (5 per group) were inoculated by intramuscular injection with the indicated doses of viral particles. Splenocytes were isolated from the mice after 12 days and stimulated for 18 h with pools of peptides. All reactions were performed in triplicate.

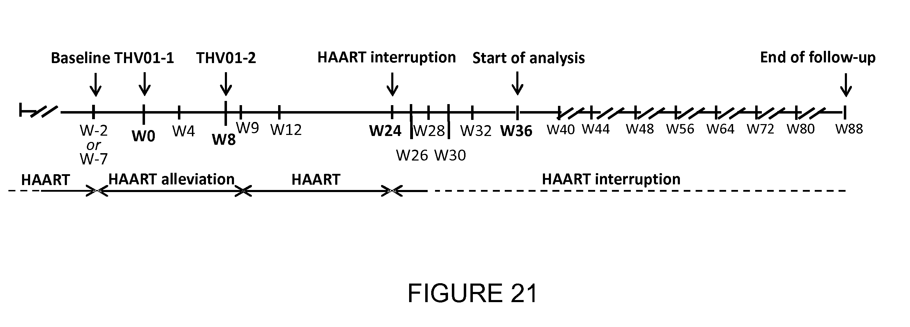

**Figure 21****.** Clinical trial regimen. Schematically, four steps can be defined: i. the screening phase; ii. HAART alleviation: patient is enrolled at his baseline visit and stops taking all antiretroviral treatments except a boosted protease inhibitor. Baseline visit takes place 2 weeks before the injection of THV01-1 except for patients taking Tenofovir for whom it must take place 7 weeks before the injection because of a longer intracellular half-life. Patient receives THV01-1 then 8 weeks later THV01-2 at the same dose; iii. HAART resumption: 1 week after the vaccination with THV01-2, patient resumes its full antiretroviral therapy and iv. Patient stops all antiretroviral treatments 16 weeks after the second vaccination. Stringent monitoring is implemented to guaranty patient's safety; patient will resume HAART as soon as HAART resumption criteria are met.
**Figure 22****.** A: Kinetics of in vivo expression of (CMV-Luciferase) after direct IM injection into C57BI/6j mice (left flank exposure) - mean. B: Kinetics of in vivo expression of (β2-m HIV antigen IRES luciferase) after direct IM injection into C56bl/- mice (left flank exposure -mean).
**Figure 23****.** Long-term biodistribution of integrated lentiviral vector sequence following direct IM injection of MAD in rats (muscle, lymphnode and subcutis) were evaluated.
**Figure 24****.** Cell-specific expression of various promoters was analyzed using the data sets at biogps.org. The expression levels were normalized to expression in skeletal muscle (=1.0).
**Figure 25****.** Kinetics of prime-boost immunization (influenza vaccine) was evaluated. Evaluation of specific T-cell specific immune response (ELISpot IFN-γ, cumulative response) of a lentiviral vector with an influenza antigen in a kinetic evaluation, in C57BI/6j mouse (5 10⁶ TU/mouse in prime and 310⁶ TU/mouse in boost, n=5/8).
**Figure 26 A-D.** Depiction of constructs expressing HIV antigens. A) Proviral RNA sequence from production plasmid (5'LTR to 3'-LTR) with WPRE (SEQ ID NO:37). B) Proviral RNA sequence from production plasmid (5'LTR to 3'-LTR) without WPRE (SEQ ID NO:38). C) Antigen RNA sequence from integrated provirus with WPRE (SEQ ID NO:39). D) Antigen RNA sequence from integrated provirus without WPRE (SEQ ID NO:40).
**Figure 27 A-U.** Depiction of stimulations with peptides from the antigens included in the vaccine (GAG, POL and Nef pools). Patients 103-001, 103-003, 202-004, 105-003, 106-001, and 201-006 were given placebo.
**Figure 28****.** Depiction of clinical study design.
**Figure 29 A-D.** Patient RNA and CD4+ T cell levels at various time points during the study for four different patients. The week at which the patient left the study is indicated within the box at the upper right. The P within the darkened circle indicates the patient given the placebo. These patients were given 2 doses of 5 x 10⁶ of the lentivector or the placebo.
**Figure 30 A-D.** Patient RNA and CD4+ T cell levels at various time points during the study for four different patients. The week at which the patient left the study is indicated within the box at the upper right. The P within the darkened circle indicates the patient given the placebo. These patients were given 2 doses of 5 x 10⁶ of the lentivector or the placebo.
**Figure 31 A-D.** Patient RNA and CD4+ T cell levels at various time points during the study for four different patients. The week at which the patient left the study is indicated within the box at the upper right. The P within the darkened circle indicates the patient given the placebo. These patients were given 2 doses of 5 x 10⁶ of the lentivector or the placebo.
**Figure 32 A-D.** Patient RNA and CD4+ T cell levels at various time points during the study for four different patients. The week at which the patient left the study is indicated within the box at the upper right. The P within the darkened circle indicates the patient given the placebo. These patients were given 2 doses of 5 x 10⁷ of the lentivector or the placebo.
**Figure 33 A-D.** Patient RNA and CD4+ T cell levels at various time points during the study for four different patients. The week at which the patient left the study is indicated within the box at the upper right. The P within the darkened circle indicates the patient given the placebo. These patients were given 2 doses of 5 x 10⁷ of the lentivector or the placebo.
**Figure 34 A-D.** Patient RNA and CD4+ T cell levels at various time points during the study for four different patients. The week at which the patient left the study is indicated within the box at the upper right. The P within the darkened circle indicates the patient given the placebo. These patients were given 2 doses of 5 x 10⁷ of the lentivector or the placebo.
**Figure 35 A-D.** Patient RNA and CD4+ T cell levels at various time points during the study for four different patients. The week at which the patient left the study is indicated within the box at the upper right. These patients were given 2 doses of 5 x 10⁸ of the lentivector or the placebo.
**Figure 36 A-D.** Patient RNA and CD4+ T cell levels at various time points during the study for four different patients. These patients were given 2 doses of 5 x 10⁸ of the lentivector or the placebo.
**Figure 37 A-D.** Patient RNA and CD4+ T cell levels at various time points during the study for four different patients. These patients were given 2 doses of 5 x 10⁸ of the lentivector or the placebo.
**Figure 38 A-B.** Patient RNA and CD4+ T cell levels at various time points during the study for two different patients. These patients were given 2 doses of 5 x 10⁸ of the lentivector or the placebo.
**Figure 39 A-B.** Summary of pre-week 24 CD4+ and CD8+ immune responses for dose 1 (A) and dose 2 (B).
**Figure 40 A-B.** Summary of pre- and post-week 24 CD4+ and CD8+ immune responses for dose 1 (A) and dose 2 (B).

### DETAILED DESCRIPTION OF THE INVENTION

The inventors have discovered that the intramuscular administration to an animal, including a human, of an integrating lentivector with specialized promoters driving expression of an immunogenic protein results in an unexpectedly high and prolonged immune response against the protein that leads to elimination of the integrated vector from the animal, including a human. A clinical study in humans has shown that the intramuscular administration of a lentivector to a human is safe. Thus, the invention provides for new lentivectors having high and prolonged immune responses and increased safety for human administration.

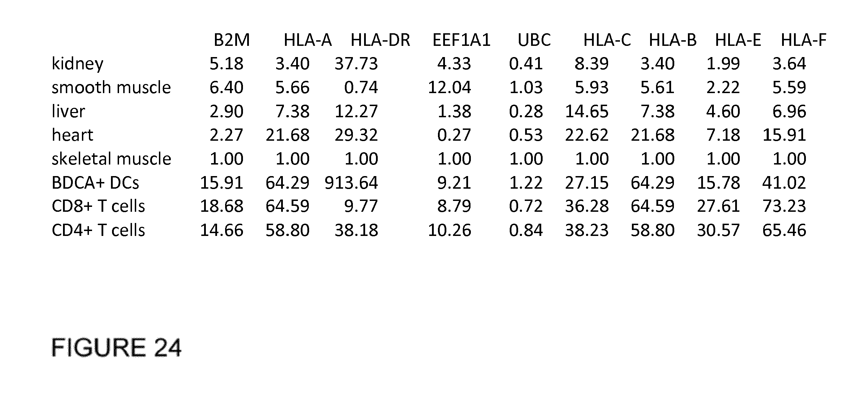

In order to determine if promoters existed that might meet the criteria of high expression in dendritic cells to induce maximal immune responses; expression in other transduced cell types sufficient for elimination by the induced immune response; and lack of an enhancer element to avoid insertional effects, human gene promoters were investigated for their utility in lentiviral vectors (Fig. 24). Human promoters were analyzed for their expression levels in dendritic cells and in other tissues. *Id.* Promoters with expected high levels of dendritic cell expression and relatively high mean expression in all tissues were selected. The promoters were further selected for lack of enhancer sequences.

The selection of promoters was initially for those with high expression in dendritic cells. Desirable promoters were selected to have greater expression in BDCA+ dendritic cells than in other cell types, such as kidney, smooth muscle, liver, heart and skeletal muscle. Desirable promoters were further selected to have some residual expression in other cell types. One group of promoters that meets these criteria is MHC Class I and β2-microglobulin (β2m) promoters (Fig. 24).

The MHC Class I promoters show conservation of NF-Kb binding sites, an interferon stimulated response element (ISRE), and an SXY regulatory module (SXY). The human β2-microglobulin (β2m) promoter shows some similarity to the MHC Class I promoters, as it contains an ISRE, albeit upstream of a single NF-Kb binding site.

MHC Class II promoters are considered to be antigen presenting cell (including dendritic cell)-specific promoters. Although MHC class II promoters contain an SXY module, they do not contain NF-Kb binding sites or an ISRE (Van den Helsen et al, 1998, Immunogenetics, 48:208-221). Thus, MHC Class II promoters are quite different from MHC Class I promoters. As a result, they also have very different cell expression patterns (Fig. 24).

Another antigen presenting cell-specific promoter, dectin-2, contains an interferon stimulated response element (ISRE); but does not contain an SXY module (Bonkabara et al., 2001, J. Immunology, 167:6893-6900).

The sequences of various mammalian (human) MHC class I promoters are shown below:
**HLA-A2 (MHC I):** 
attgggagtcccagccttggggattccccaactccgcagtttcttttctccctctcccaacct
atgtagggtccttcttcctggatactcacgacgcggacccagttctcactcccattgggtgtcg
ggtttccagagaagccaatcagtgtcgtcgcggtcgcggttctaaagtccgcacgcacccaccg
ggactcagattctccccagacgccgagg
(SEQ ID NO: 1)
**HLA-B7 (MHC I):** 
ggggaggcgcagcgttggggattccccactcccctgagtttcacttcttctcccaacttgtgtc
gggtccttcttccaggatactcgtgacgcgtccccacttcccactcccattgggtattggatat
ctagagaagccaatcagcgtcgccgcggtcccagttctaaagtccccacgcacccacccggact
cagag (SEQ ID NO: 2)
**HLA-Cw5 (MHC I):** 
cactgggaggcgccgcgttgaggattctccactcccctcagtttcacttcttctcccaacctg
cgtcgggtccttcttcctgaatactcatgacgcgtccccaattcccactcccattgggtgtcgg
gttctagagaagccaatcagcgtctccgcagtcccggtctaaagtccccagtcacccacccgga
ctcagattctccccagacgccgag
(SEQ ID NO: 3)
**HLA-E (MHC I):** 
taagaactgctgattgctgggaaactctgcagtttcccgttcctctcgtaacctggtcatgtgt
ccttcttcctggatactcatgacgcagactcagttctcattcccaatgggtgtcggtttctag
agaagccaatcagcgtcgccacgactcccgactataaagtccccatccggactcaagaagttct
caggactcagagg  (SEQ ID NO: 4)
**HLA-F (MHC I):**

aggccccgaggcggtgtctggggttggaaggctcagtattgagaattccccatctccccagagt
ttctctttctctcccaacccgtgtcaggtccttcatcctggatactcataacgcggccccattt
ctcactcccattgggcgtcgcgtttctagagaagccaatcagtgtcgccgcagttcccaggttc
taaagtcccacgcaccccgcgggactcatatttttcccagacgcggaggttggggtcatg
(SEQ ID NO: 5)

A sequence of the human β2-microglobulin promoter is shown below:

aacatcacgagactctaagaaaaggaaactgaaaacggaaagtccctctctaacctggcac
tgcgtcgctggcttggagacaggtgacggtccctgcgggccttgtcctgattggctgggcacgc
gtttaatataagtggaggcgtcgcgctggcgggcattcctgaagctgacagcattcgggccgag
(SEQ ID NO:6).

The MHCI and β2m promoters do not contain an enhancer. Moreover, these promoters are dendritic-specific in that expression of the promoter in BDCA+ dendritic cells is higher than the expression in kidney, smooth muscle, liver, and heart cells (http://biogps.org; Fig. 24). They also have relatively high expression in other transduced cell types, for example, expression of the promoter in BDCA+ dendritic cells is only 12-100 times the expression of that promoter in skeletal muscle cells, in contrast to 900 times with the MHCII HLA-DRα promoter. *Id.* Consequently, the MHCI and β2m promoters were assessed to determine their utility in a lentiviral vector.

MHCI promoters (HLA-A2, HLA-B7 or HLA-E) or a β2m promoter were inserted into a lentiviral vector. For comparison, the promoters of the ubiquitously expressed EF1α and Ubiquitin (UBC) genes were also inserted into the lentiviral vector. EF1 a and Ubiquitin promoters do not contain any identified enhancers, and do not contain an SXY module, NF-Kb binding sites or an ISRE. Rather, the EF1α and Ubiquitin (UBC) promoters instead contain Sp1 and Ap1 binding sites. The EF1α promoter does not have high expression in dendritic cells (http://biogps.org; Fig. 24). In fact, expression in smooth muscle is greater than expression in BDCA+ DCs. *Id.* The UBC promoter does not have expression in BDCA+ dendritic cells that is 12-100 times the expression of that promoter in skeletal muscle cells (http://biogps.org; Fig. 24). Rather, the expression in skeletal muscle is similar to that in BDCA+ DCs. *Id.* A vector containing the CMV promoter/enhancer was also generated. A vector containing an MHCII promoter (HLA-DRα) was also generated. This promoter has extremely high expression in dendritic cells, but not in other cells, such as kidney, smooth muscle, liver, heart and skeletal muscle (http://biogps.org). In these vectors, the promoters drive expression of green fluorescent protein (GFP).

To look for dendritic cell-specific expression, the lentivectors were packaged by cotransfection in HEK 293 T cells with an encapsidation plasmid and a plasmid providing VSV.G envelope, essentially as described in Naldini et al, 1996, Science 272:263-7. Both HEK 293 T and BDCM cells (a dendritic-like cell line) were then transduced with particles of the different vectors. Expression was detected in HEK 293 T cells with all vectors.

Vectors with a CMV promoter, an UBC promoter, or an EF1α promoter demonstrated higher expression in HEK 293 T cells than in BDCM cells (Fig. 1). However, vectors with an MHCII promoter (HLA-DRα), MHCI promoter (HLA-A2, HLA-B7 or HLA-E), or β2 microglobulin promoter (β2m) showed higher expression in BDCM cells than in HEK 293 T cells. Thus, these promoters all exhibited a dendritic cell-specific overexpression.

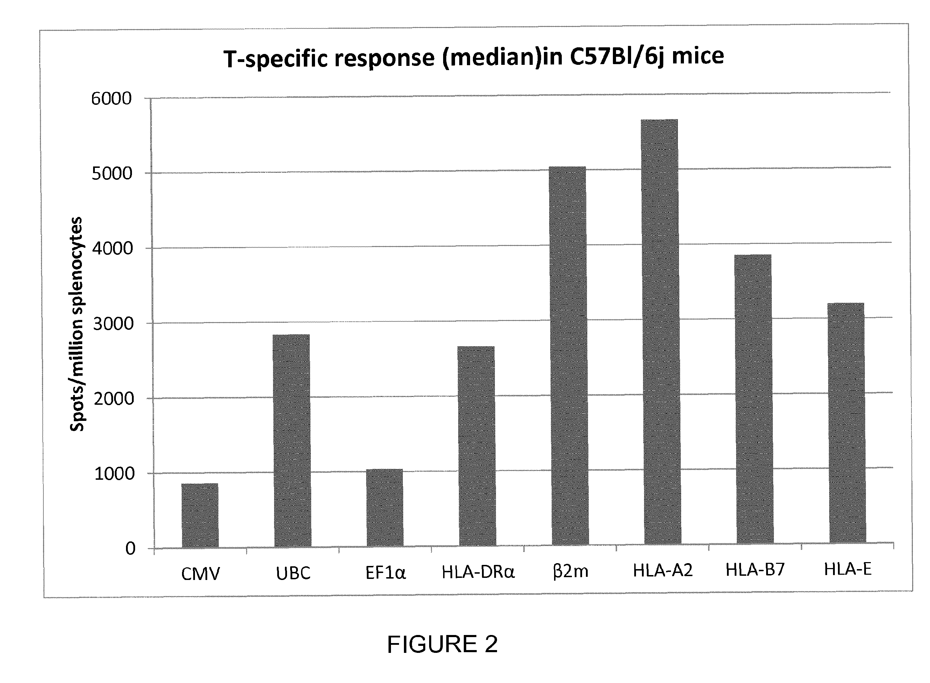

The ability of these vectors to produce specific immune responses was then determined. Mice were immunized with lentivectors in which HIV antigen expression is driven by various promoters. 12 days after immunization, the specific T-cell responses were monitored in mice splenocytes by IFN-γ ELISPOT. As shown in Figure 2, immunization with a lentivector with an MHCI promoter gave the highest specific T-cell response.

As seen in Figure 2, the T-specific response with the MHCI promoters was higher than with CMV, EF1 a, Ubiquitin, or MHCII promoters, and more than 3 fold higher than that with EF1 a or CMV promoters. The T-specific response with the β2m promoter was similar to the MHCI promoters. These results indicate that the MHCI and β2m promoters are unexpectedly superior to the EF1 a, Ubiquitin, CMV, or MHCII promoters for use in lentivectors for maximizing the T-specific response in vivo.

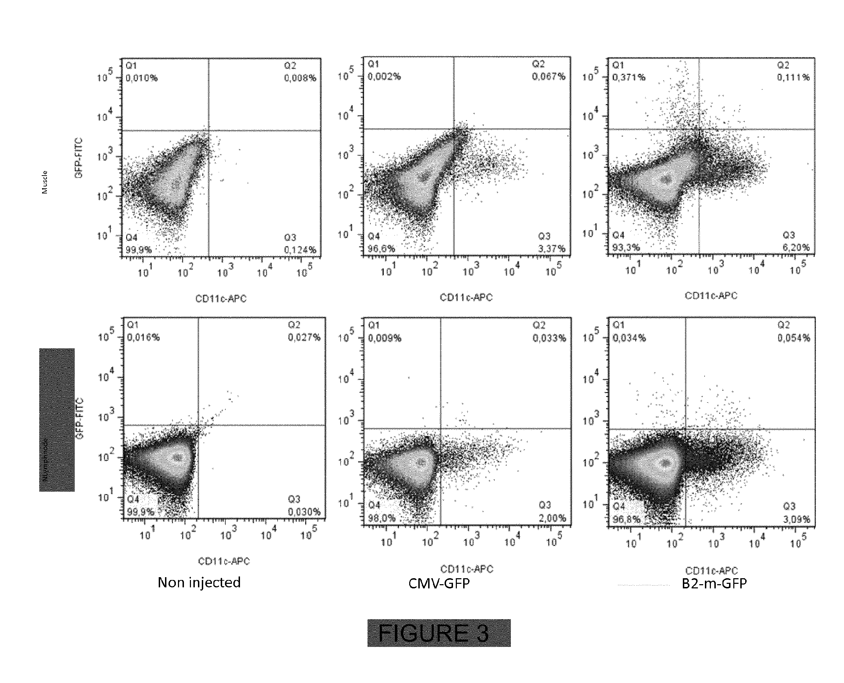

The in vivo GFP expression in DCs isolated from muscle or draining lymph node, three days after mice were intramuscularly administered 10⁸ TU of lentiviral vector with the CMV or the β2m promoter driving GFP expression was analyzed. Figure 3 shows FACS analysis of DCs isolated from muscle or draining lymph node, three days after administration. Compared to the CMV promoter, the β2m promoter showed substantially higher expression of GFP in DCs, in both muscle and draining lymph node.

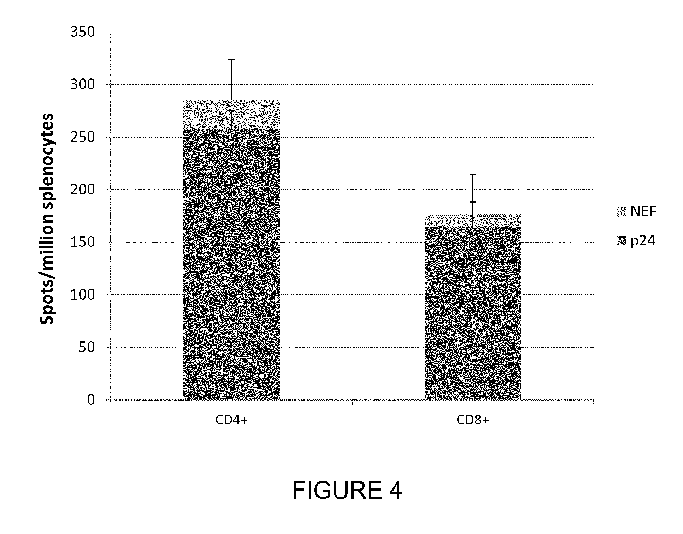

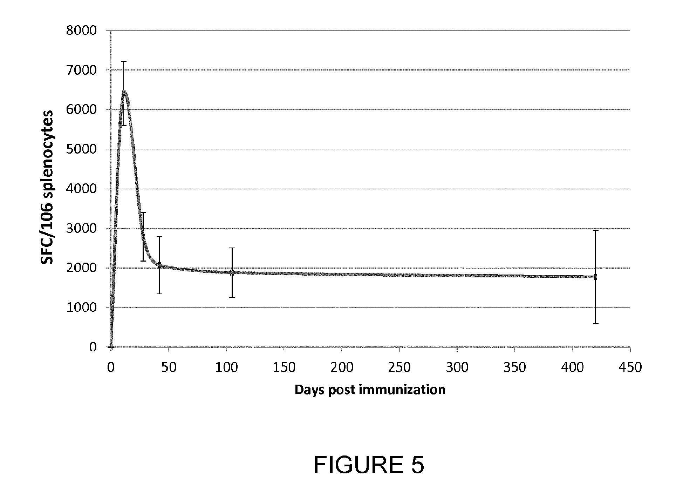

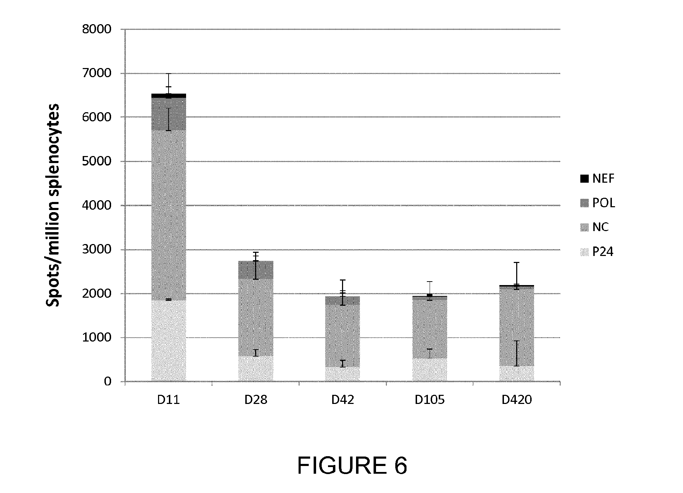

Specific CD4+ and CD8+ responses elicited against the P24 and Nef part of the anti-HIV-1 antigen, were analyzed after injection of 2 x 10⁷ TU of the lentivector β2m-HIV in Sprague Dawley rats. As can be seen in Figure 4, both CD4 and CD8 responses were induced using the β2m promoter. Long lasting and diversified specific T-cell response was elicited after intramuscular injection of 2.68 x 10⁷ TU of the lentivector β2m-HIV in C57BI/6j mice (Figures 5 and 6). The T-specific responses at 420 days after administration achieved levels of more than 25% of the responses at 11 days after administration and more than 75% of the responses at 42 days after administration.

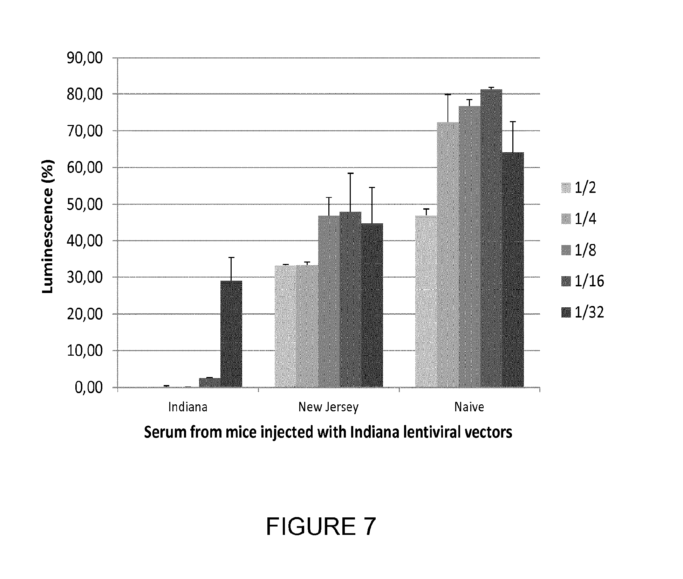

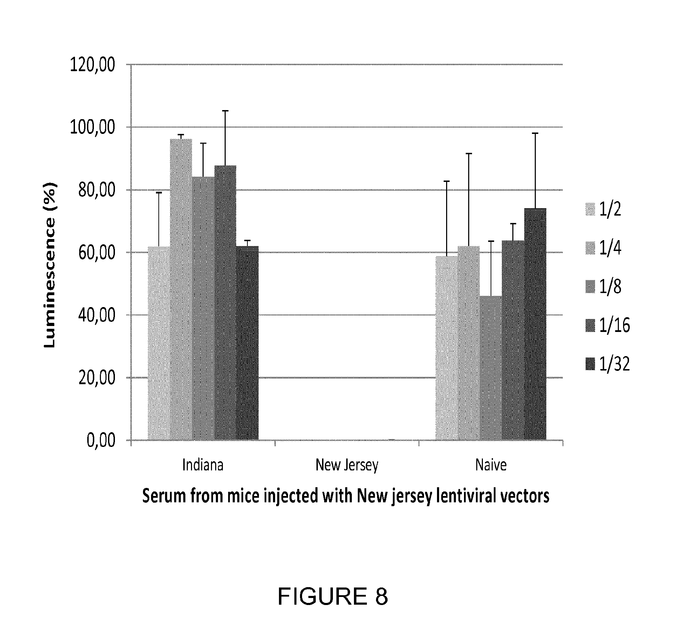

The ability of antibodies generated against lentivectors pseudotyped with Indiana and New Jersey VSV-G glycoproteins to interfere with a second administration of a lentivector pseudotyped with the same, but not a different, VSV glycoprotein was shown (Figures 7 and 8).

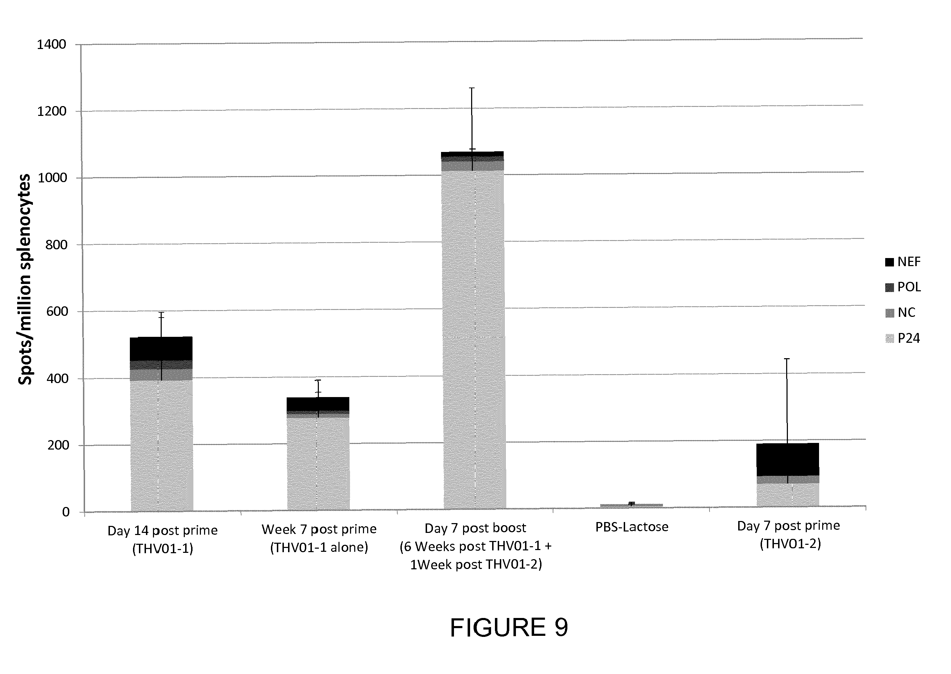

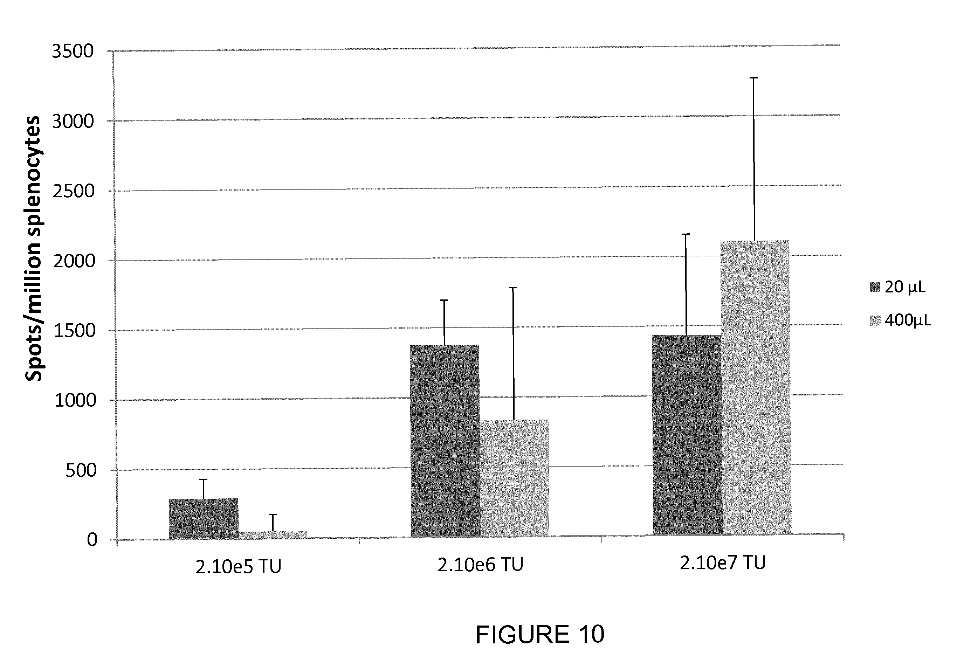

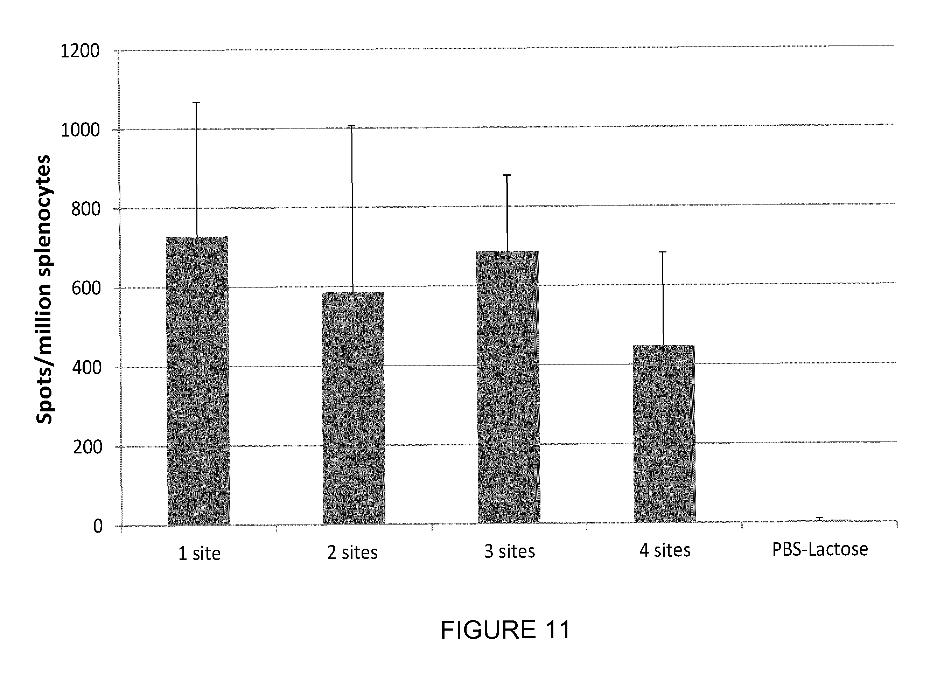

Specific T-cell responses were shown after intramuscular injection of (2 x 10⁶ TU) in Sprague-Dawley rats (n=6) in a prime-boost regimen (THV01-1: β2m-HIV pseudotyped with Indiana VSV-G and THV01-2: β2m-HIV pseudotyped with New Jersey VSV-G) injected 6 weeks apart)(Figure 9). Specific T-cell responses were elicited in Sprague-Dawley rats after injection of 3 different lentiviral doses (2 x 10⁵, 2 x 10⁶ and 2 x 10⁷ TU) displayed in 2 different volumes (n=20 and 400µL)(Figure 10). Specific T-cell responses were elicited in SD rats (n=6) after intramuscular injection of 2 10⁶ TU of lentiviral vector displayed in 1, 2, 3 or 4 injection sites (Figure 11).

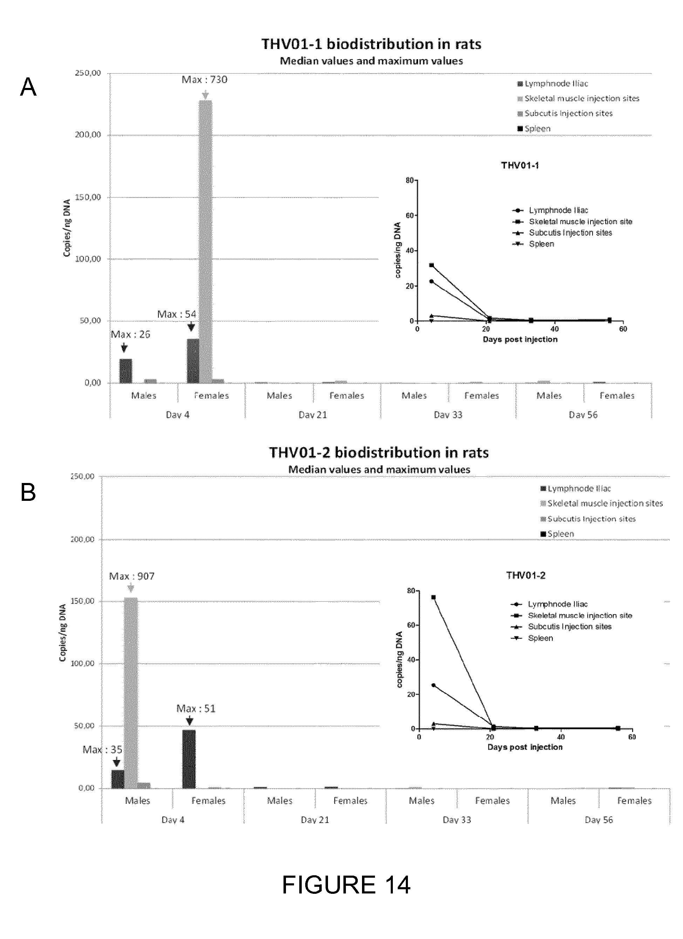

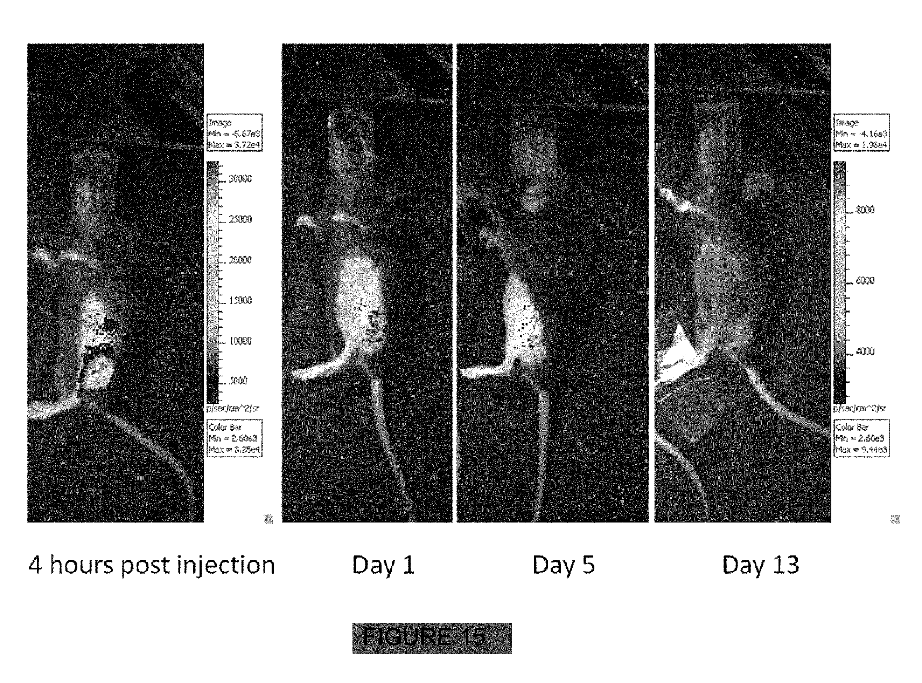

Specific T-cell responses were elicited after MAD (400µL) intramuscular injections of THV01-1 (corresponding to 1.06 x 10⁸ TU) and THV01-2 (corresponding to 1.88 x 10⁸ TU) lentiviral vectors in Sprague Dawley rats (Figure 12). Group A: THV01-1 alone, Group B THV01-1 and THV01-2 injected 6 weeks apart (sacrifice day 49, 6 days post THV01-2 boost injection), Group C: Group B that received a second boost injection at day 637 (day 599 post THV01-2 boost) with THV01-3, the HIV-1 vaccine candidate (β2m-HIV) pseudotyped with Cocal, a third VSV-G serotype, MAD corresponding to 5,3.10⁷ TU), Group D: THV01-3 alone injected at day 628 (MAD, corresponding to 5,3.10⁷) and Group E : control (PBS-Lactose)(Figure 13). GLP biodistribution (qPCR) of the lentiviral proviral sequences after MAD intramuscular injections of THV01-1 and THV01-2 was determined (Figure 14). Variations between animals (males and females) can be observed, as the lentiviral vector diffusion is reduced after intramuscular administration and the injection sites were sometimes hard to localize and sample precisely at necropsy. Bioluminescence monitoring of LV after IM injections of 10⁶ TU of the lentiviral vector in C57BI/6j mice was performed (Figure 15). As can be seen in Figures 14 and 15, the expression of the lentivectors in vivo was seen soon after transduction, and decreased quickly to nearly undetectable levels. Thus, even though the vectors are integrating, they are being eliminated rapidly in vivo.

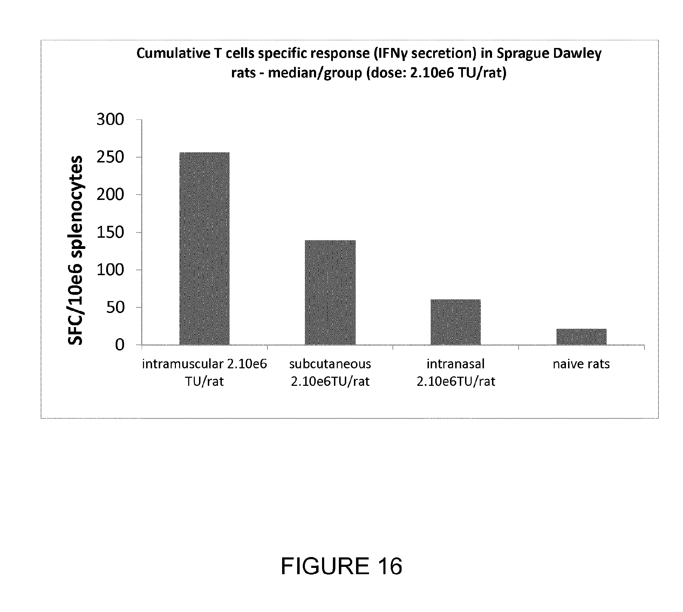

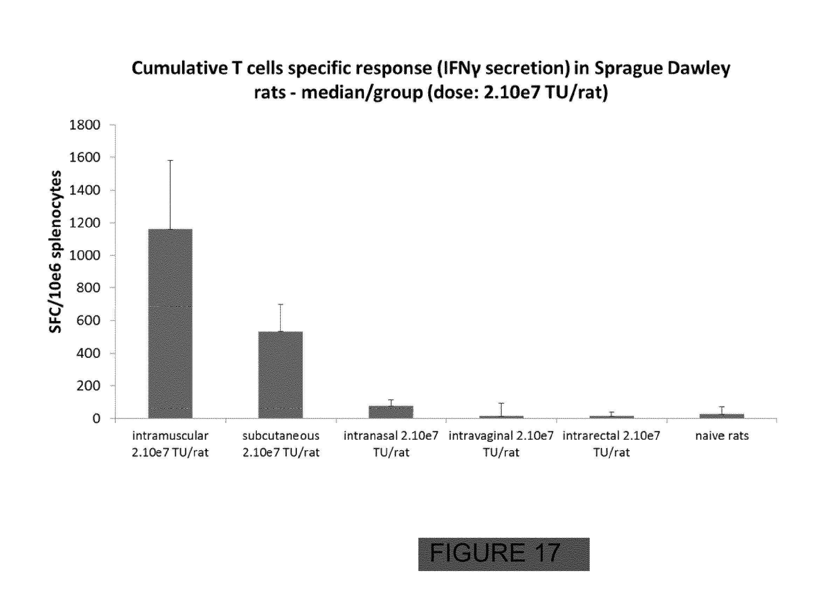

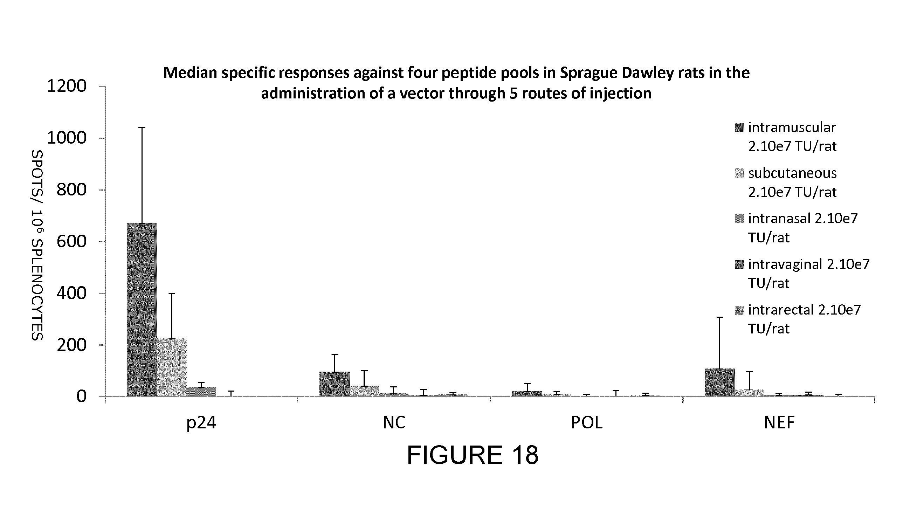

Specific T-cell responses induced by various routes of administration were compared (Figures 16-18). Unexpectedly, intramuscular administration was shown to be substantially better than other routes of administration.

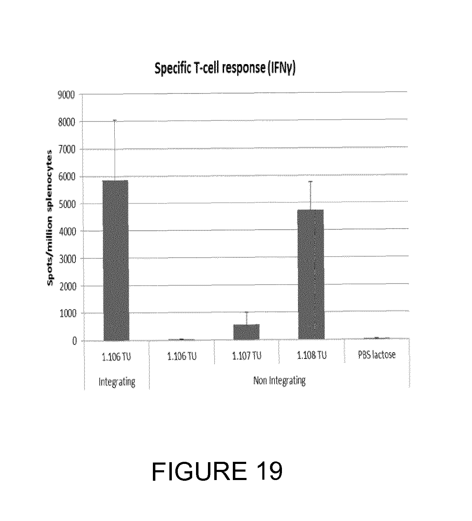

It has been reported that non-integrating vectors have comparable efficiency as integrating vectors, and have improved safety due to their lack of integration. Specific T-cell responses induced by integrating and non-integrating lentivectors were compared (Figure 19). 100 times as much of the non-integrating vector was required to induce a similar level of specific T-cell response.

In contrast to the present results, other groups have demonstrated similar levels of immunogenicity elicited after direct injections of integrating and non-integrating lentiviral vectors in animals. In the prior studies, the lentivector doses used to perform these experiments were quantified with the ELISA P24 detection kit; and the injected doses are expressed in ng of P24 protein per ml. This method of titration is poorly representative of viral titer, as it doesn't discriminate the functional lentiviral particles from the non-functional ones and from the free P24 protein present in the solution. Furthermore, the ELISA P24 detection kit is calibrated to measure the presence of P24 in patient's blood. As the P24 protein is far more concentrated in lentiviral batches, strong dilutions need to be applied before performing the titration (up to 10⁻⁷), increasing the inaccuracy of the method. Hence lentiviral batches can show great variability when P24 measurements are performed at different times. To overcome this problem, a new method of titration based on qPCR detection of non-integrating lentiviral vectors was implemented. This method was used in the present study to determine precisely and reliably the titer of non-integrating lentiviral vectors over time, and to evaluate more precisely the immunogenicity after direct injection into animals.

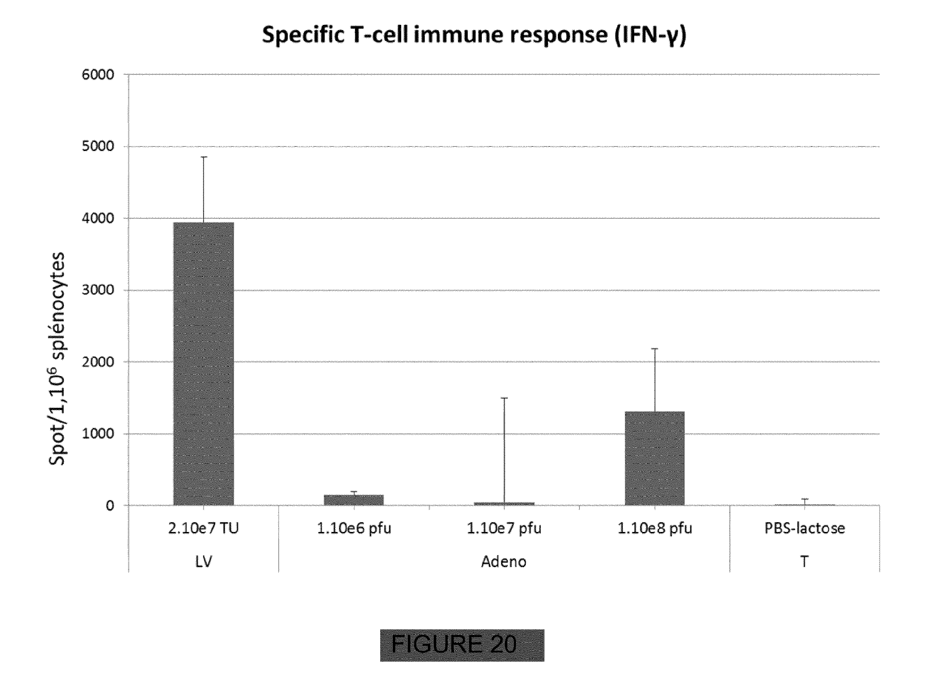

Specific T-cell responses induced by an integrating lentivector were compared to a comparable adenovirus (non-integrating) vector (Figure 20). 100 times as much of the non-integrating adenovirus vector did not even induce a similar level of specific T-cell response. Thus, an integrating lentivector is unexpectedly superior to a non-integrating vector.

A clinical trial plan was developed and implemented in humans (Figure 21).

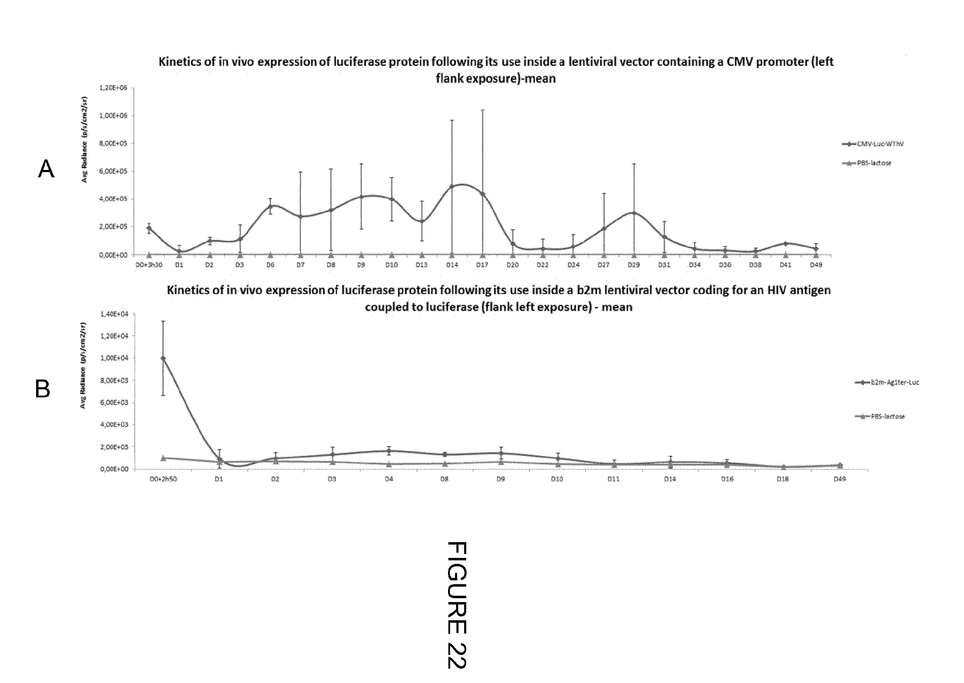

To determine the effect of expression of a poorly immunogenic protein on elimination of expression of integrated lentivector, mice were intramuscularly administered a CMV-luciferase lentivector (Figure 22). The expression of the CMV-luciferase lentivector was expressed at higher levels for longer than the β2m-HIV vector.

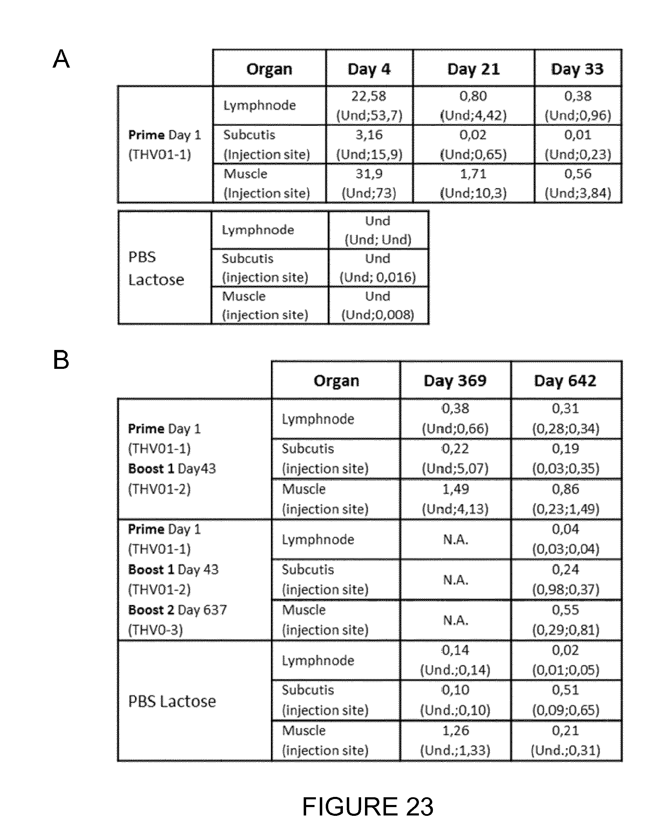

Integrated DNA persistence in injected mice was also measured (Figure 23). Although detectable at day 4 post-administration, (for THV01-1: 2,13 10⁻⁵% of the injected particles can be found in the draining lymph nodes, 2,98 10⁻⁶% in the subcutis and 3,02 10⁻⁵% in the muscle; and for THV01-2, 1,35 10⁻⁵% of the injected particles can be found in the draining lymph nodes, 1,60 10⁻⁶% in the subcutis and 4,04 10⁻⁵% in the muscle), the bulk of the integrated DNA was eliminated by 21 days post-administration (for THV01-1: 7,55 10⁻⁷% of the injected particles can be found in the draining lymph nodes, 1,89 10⁻⁸% in the subcutis and 1,61 10⁻⁶% in the muscle; and for THV01-2, 7,55 10⁻⁷ % of the injected particles can be found in the draining lymph nodes, 3,72 10⁻⁸% in the subcutis and 0% in the muscle).

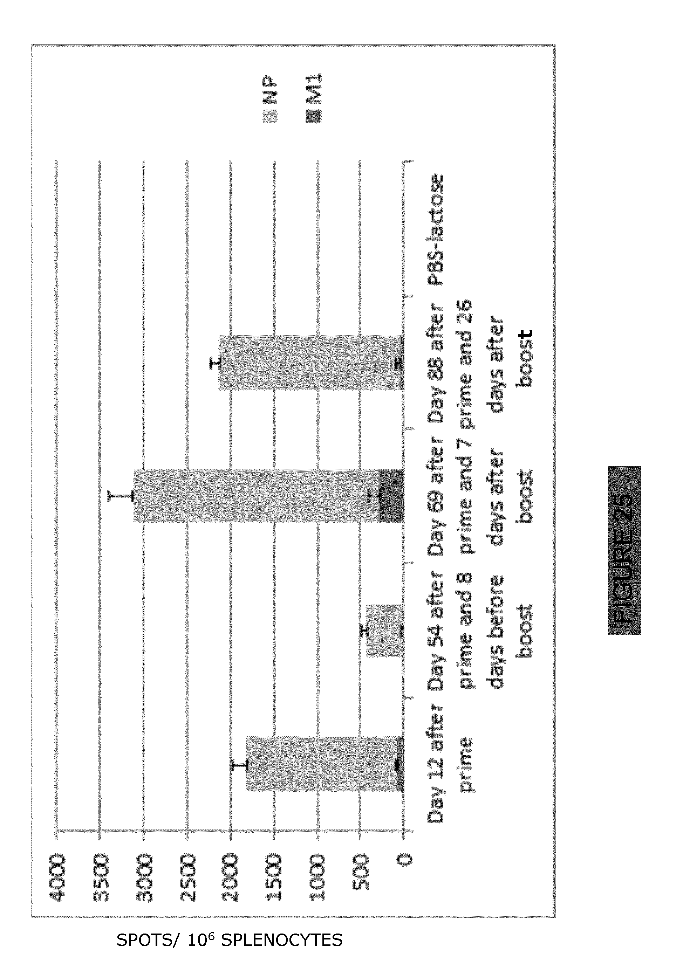

To evaluate long term expression of another antigen, a lentiviral vector expressing influenza M1 and NP antigens was administered intramuscularly to mice in priming and boosting doses. Long term specific T cell responses against influenza antigens were observed (Figure 25).

After mice immunization with the lentivector coding for the HIV antigen, the number of mice T-cells responding to a GAG peptide stimulation appeared to be larger than expected from previously published studies using either lentiviral vectors (Yang et al, PNAS 2006) or other clinically used viral vectors (Chen X. et al, J. Virol 2005; Gherardi M M et al. J. Virol. 2003; Maamary J et al. J. Virol. 2011; Xin KQ et al. Gene Therapy 2005; Rodriguez A.M. et al, PLOS one 2012). Furthermore, this immune response appears to be sustainable in time in immunized animals, compared to the one observed in animals immunized with other clinically used viral vectors (Xin KQ et al. Gene Therapy 2005, Lin SW et al, J. Clin. Invest 2007).

Similar results have been seen in mice immunized with the lentivector coding for the influenza M1 and NP antigen, as it elicited a stronger (Chen et al, J. Virol 2005; Vitelli A. et al, PLOS one 2013) and a more sustainable T cell response compared to other clinically used viral vectors (Zhou D et al, Mol. Ther 2010).

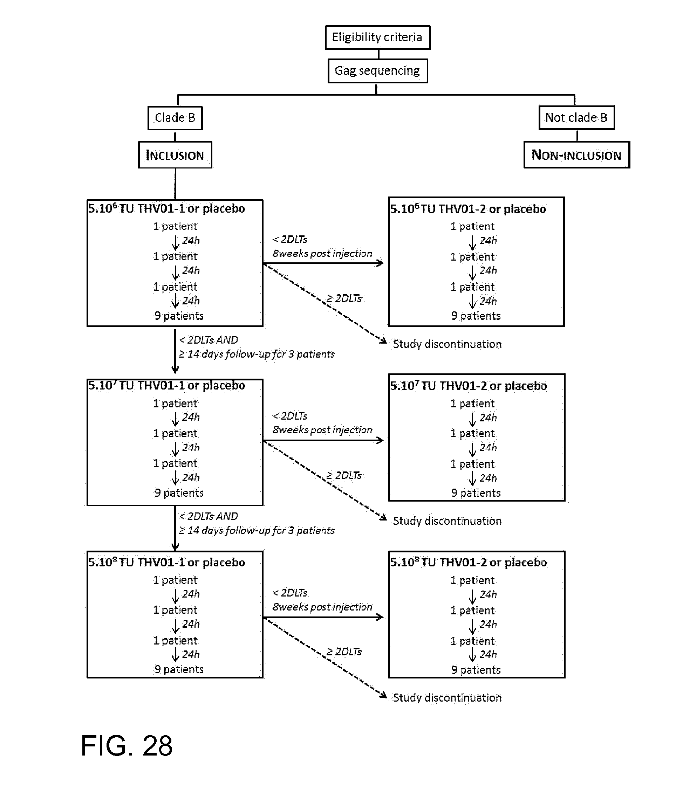

The patients in the clinical trial plan (Figures 21 & 28) were analysed for adverse effects to determine the safety of the lentiviral administration. The safety profile of patients treated with the lentiviral vector was comparable to patients that received the Placebo. In addition, neither Serious Adverse Event nor additional severe Adverse Events were seen among the patients treated with the third dose. Thus, the lentiviral vector is safe for use in humans.

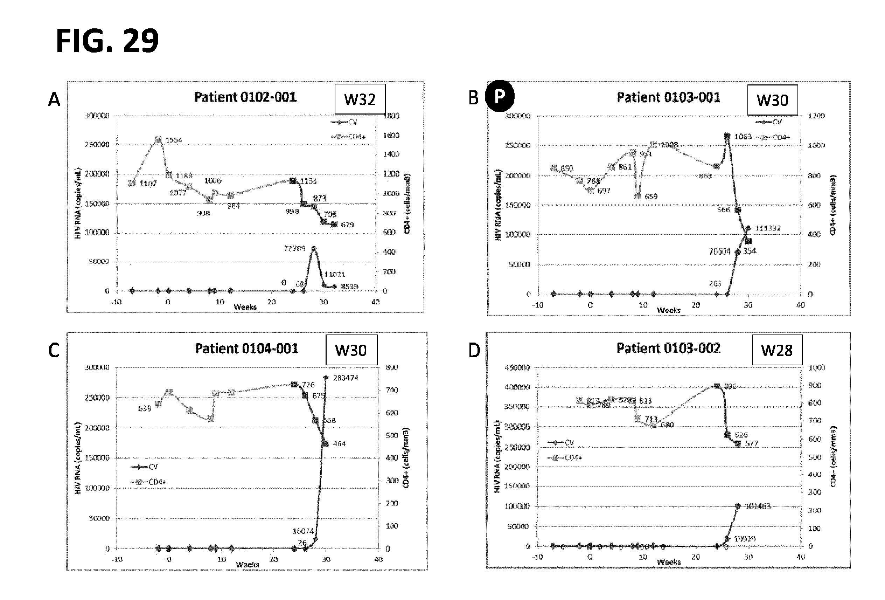

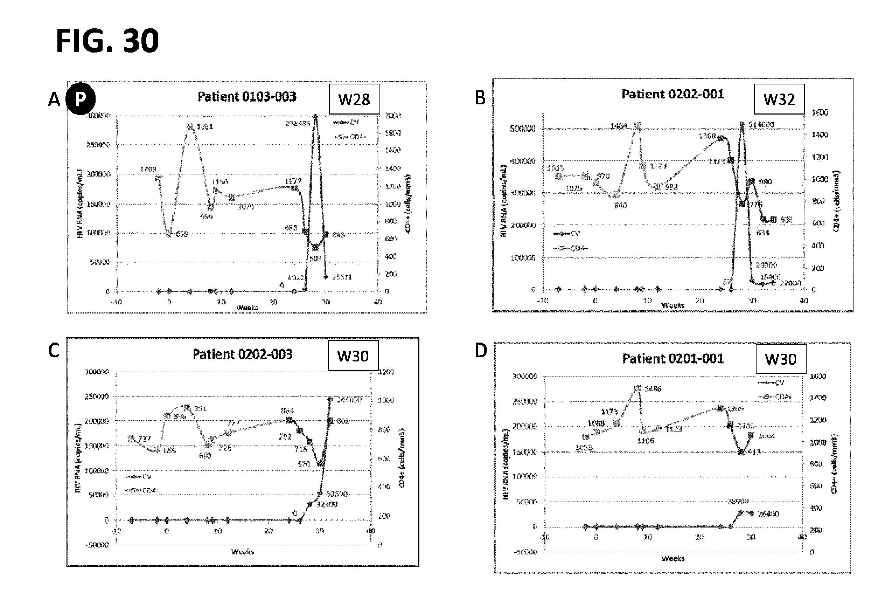

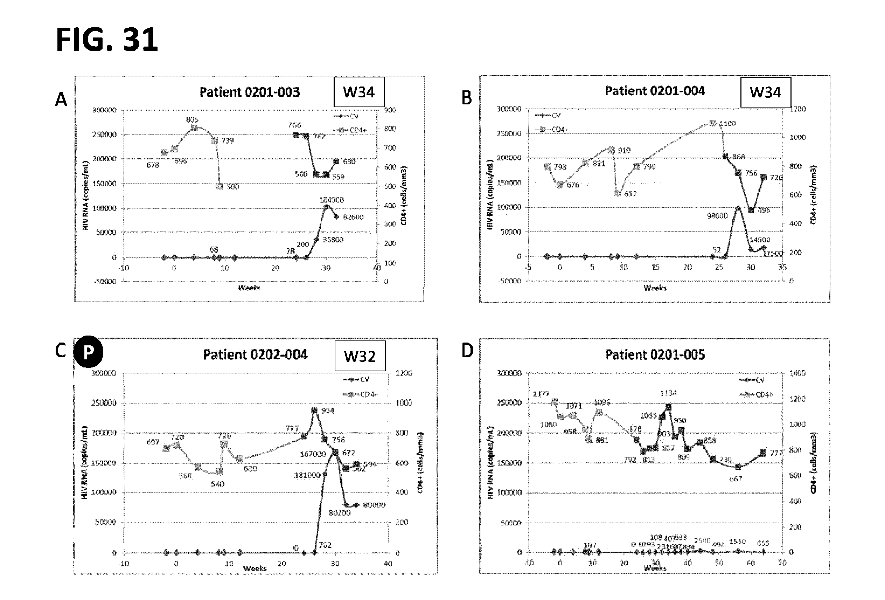

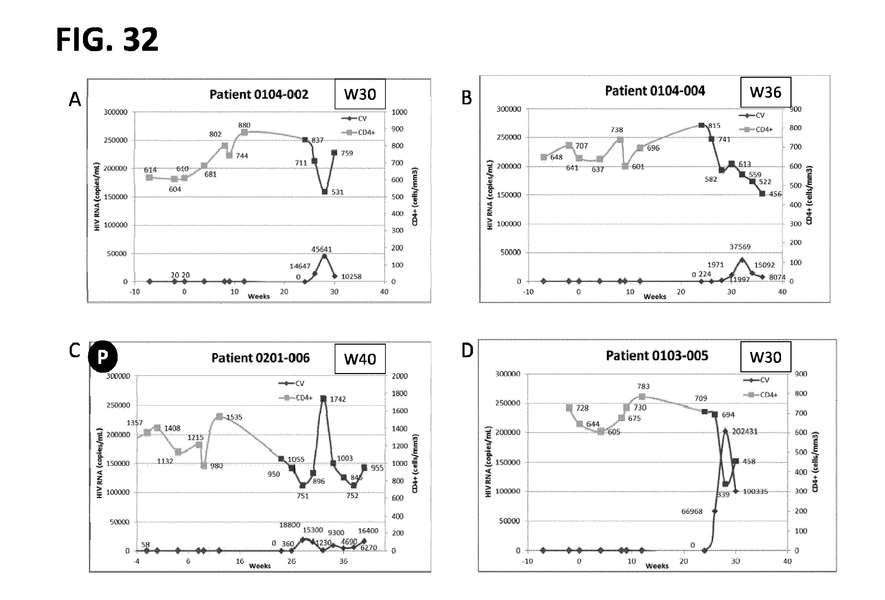

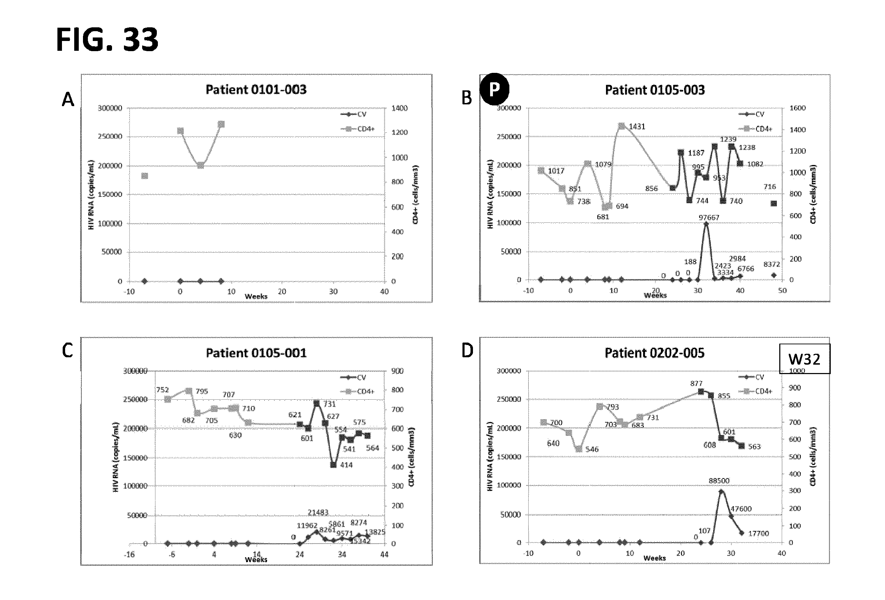

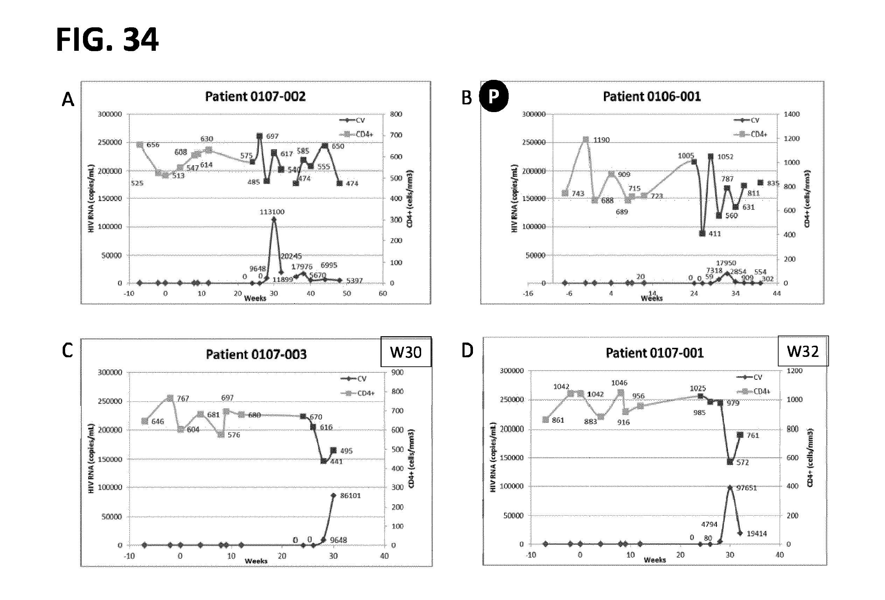

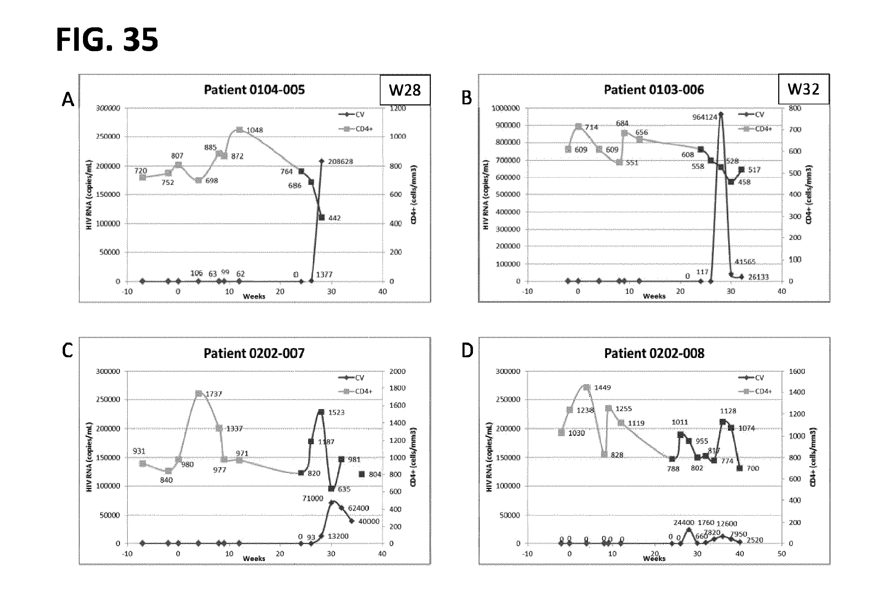

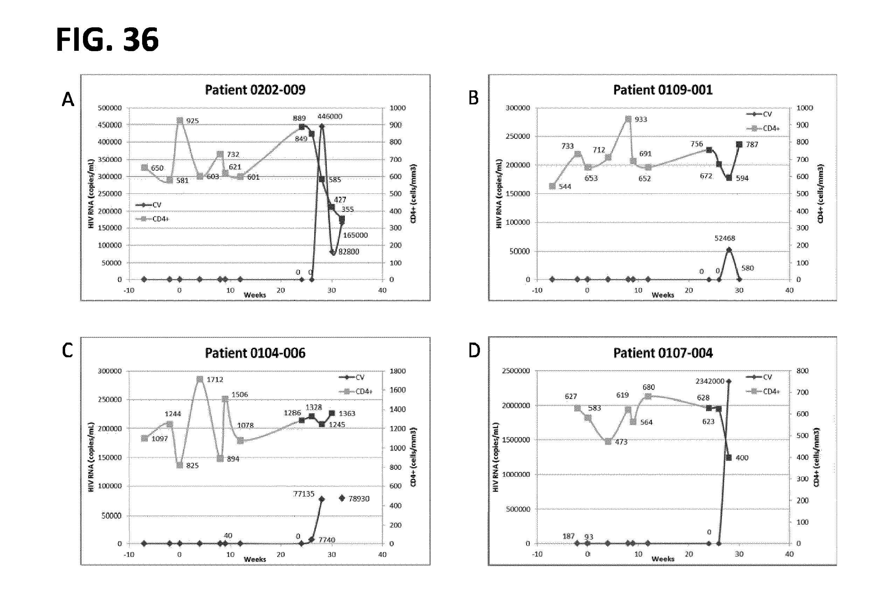

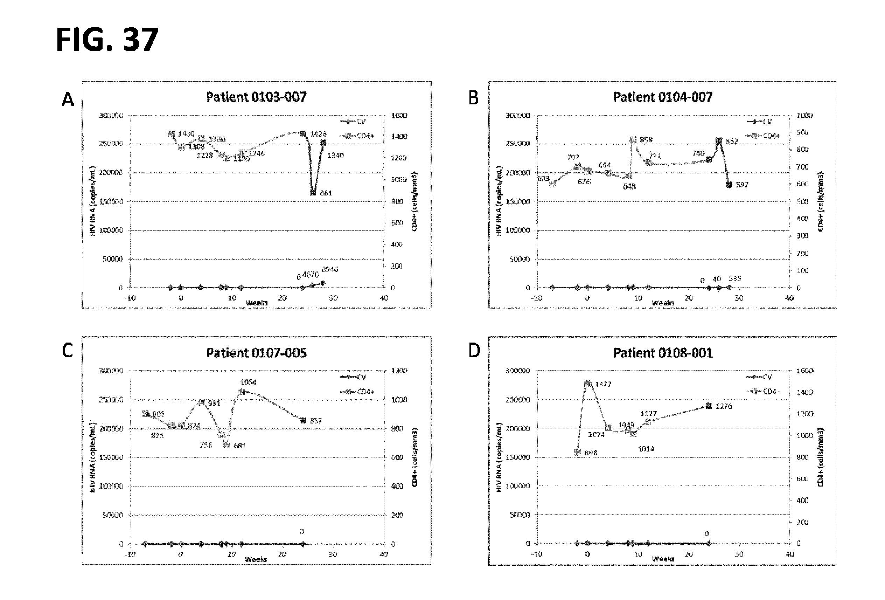

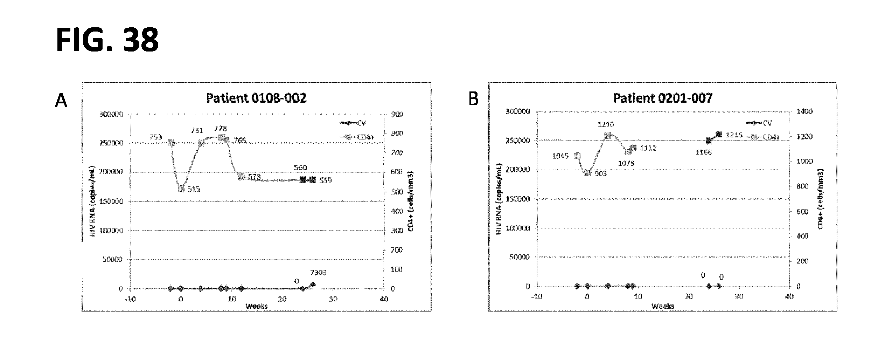

The patients in the clinical trial plan (Figures 21 & 28) were analysed for viral load (PCR) and CD4+ T cell counts. The results are depicted in Figures 29-38. Control of viral replication and stabilization of CD4+ T cell counts was observed after HAART removal in many patients. The viral rebound was delayed with dose 3 relative to dose 2 and with dose 2 relative to dose 1. The viral rebound with dose 1 was the same as with the placebo.

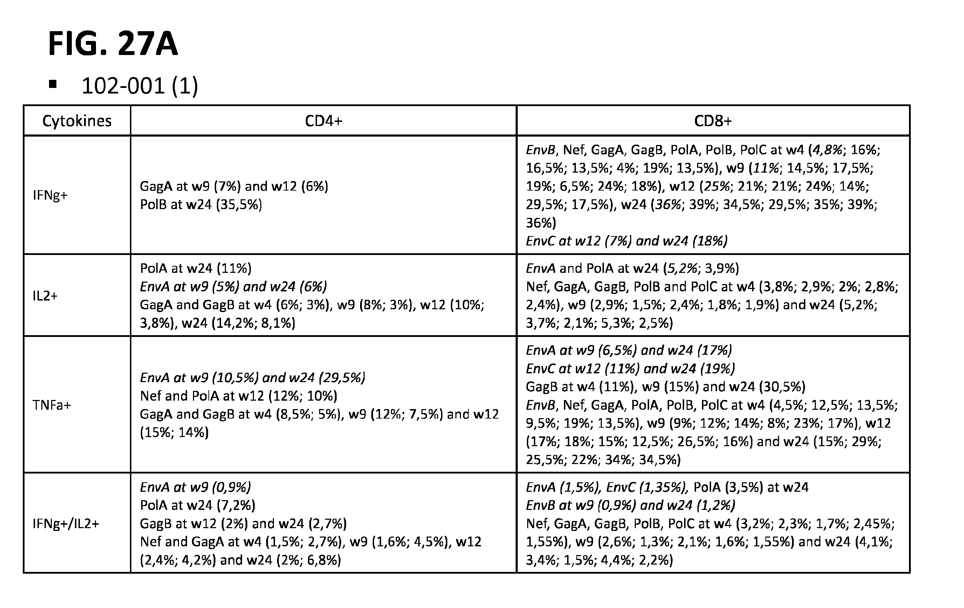

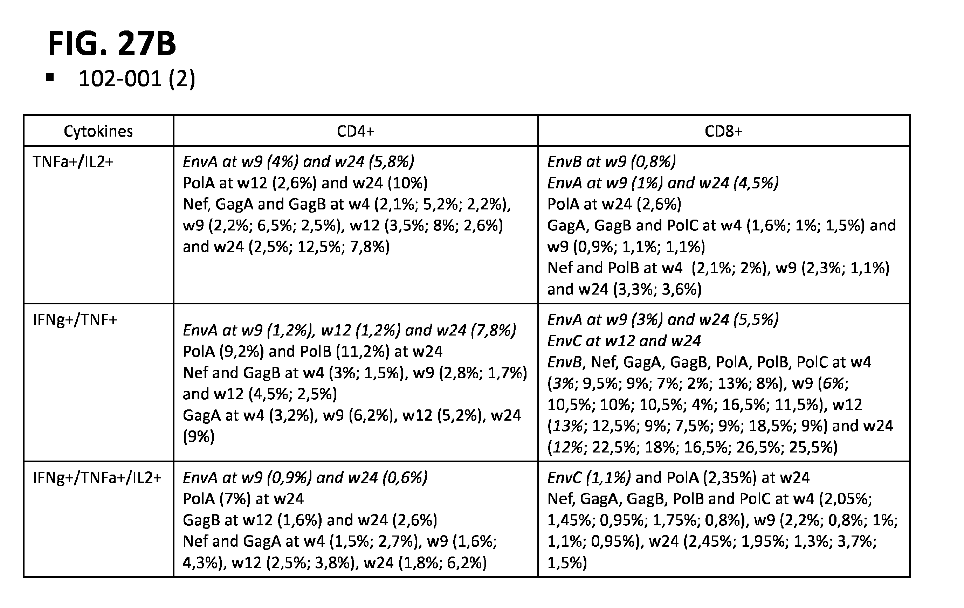

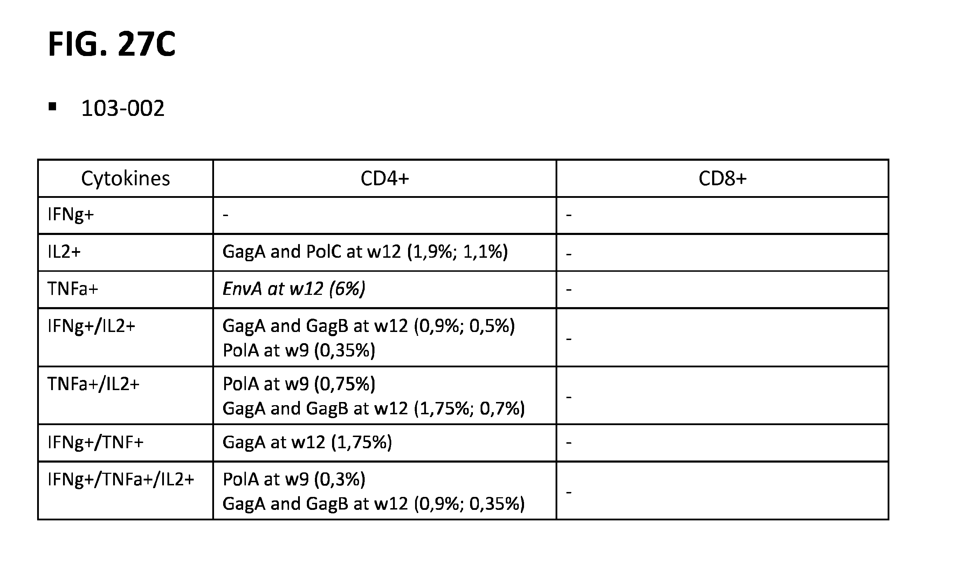

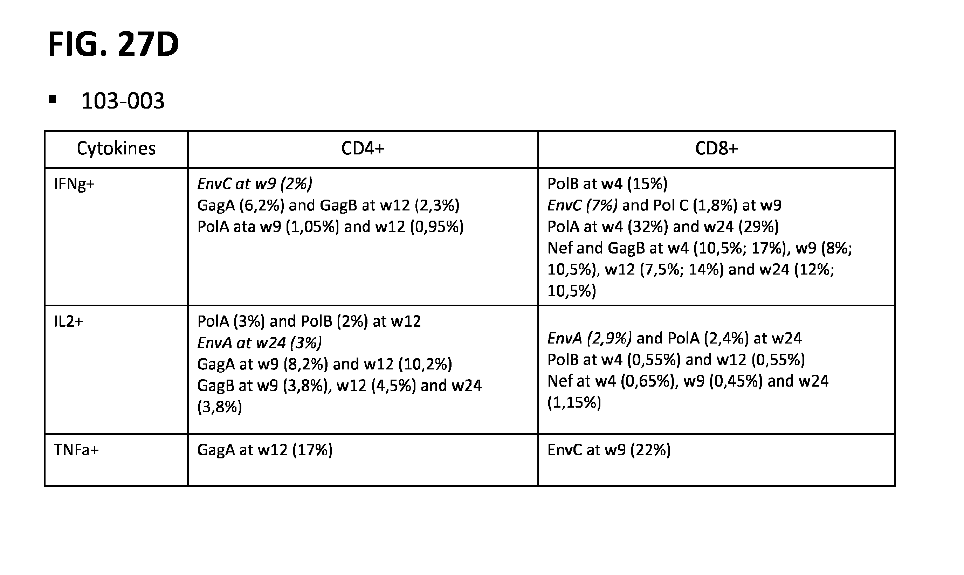

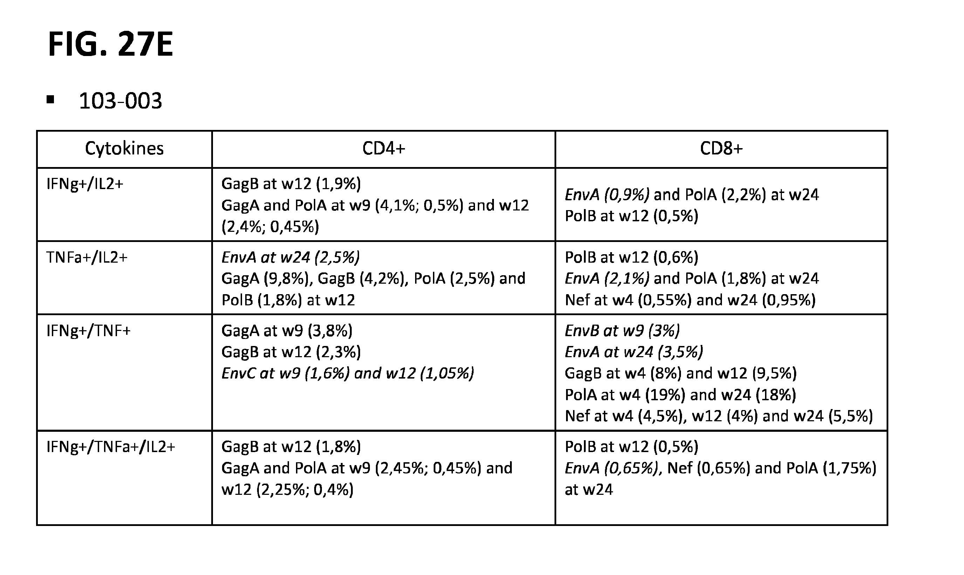

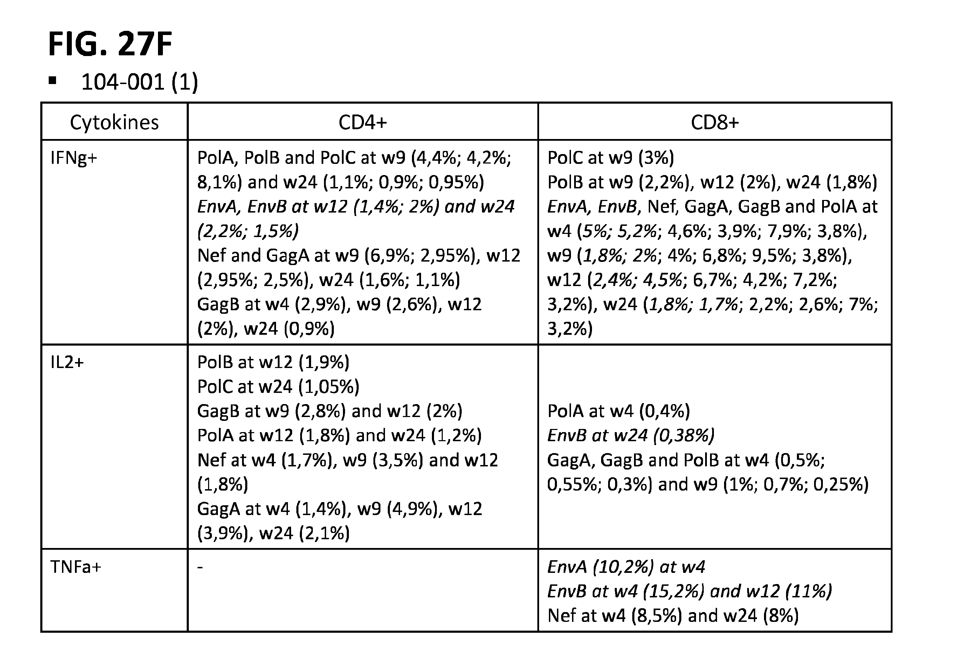

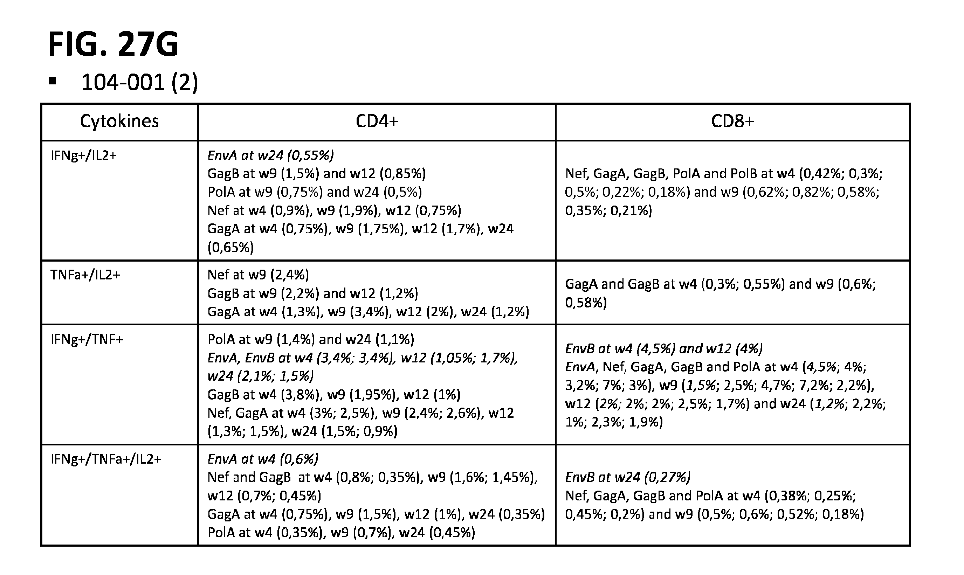

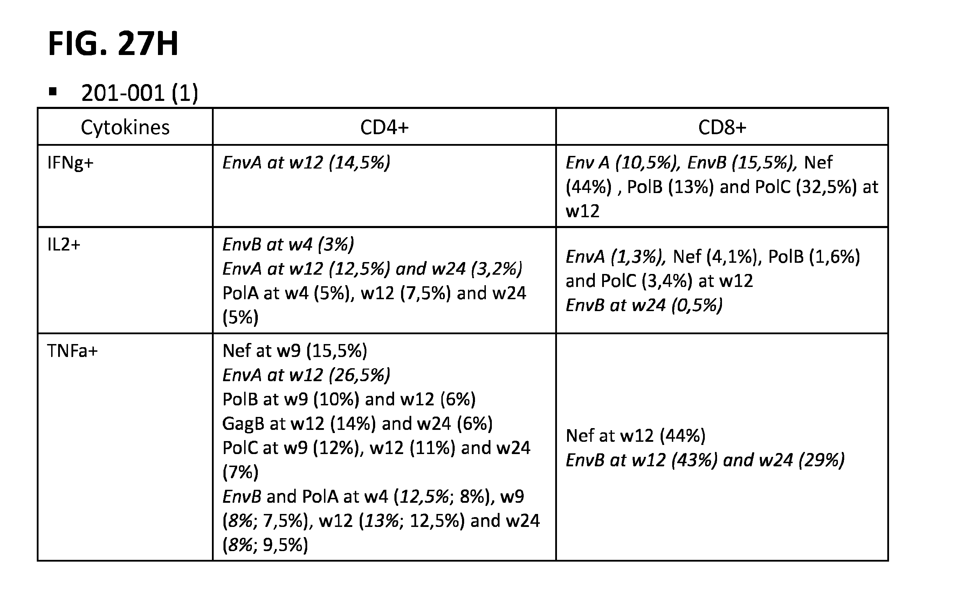

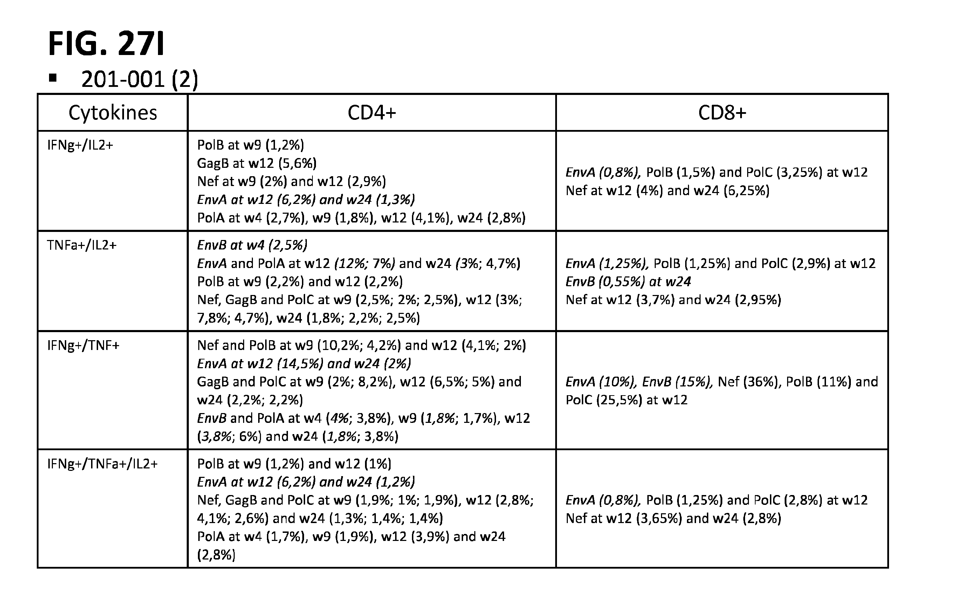

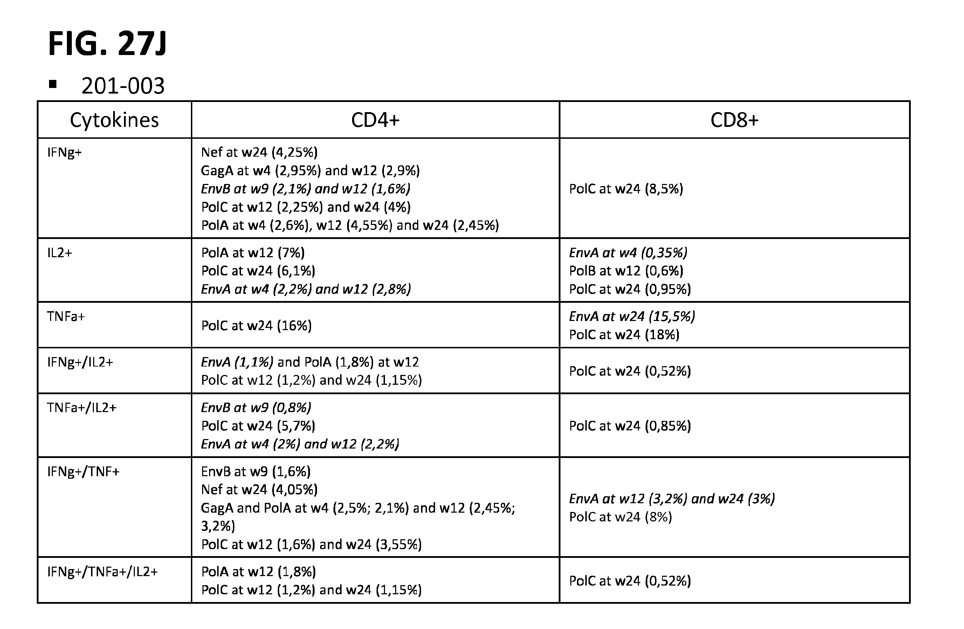

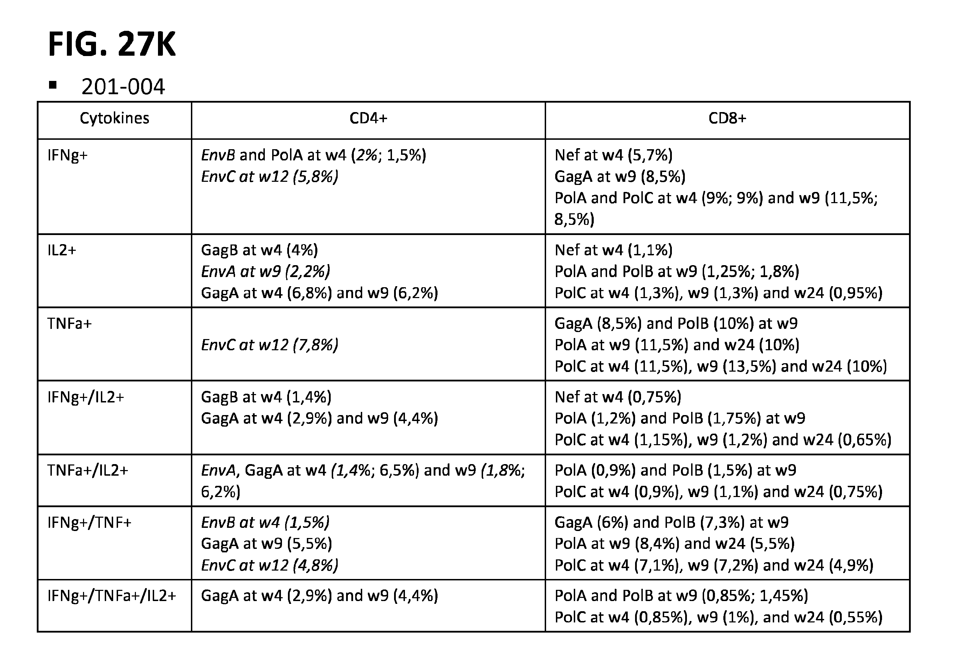

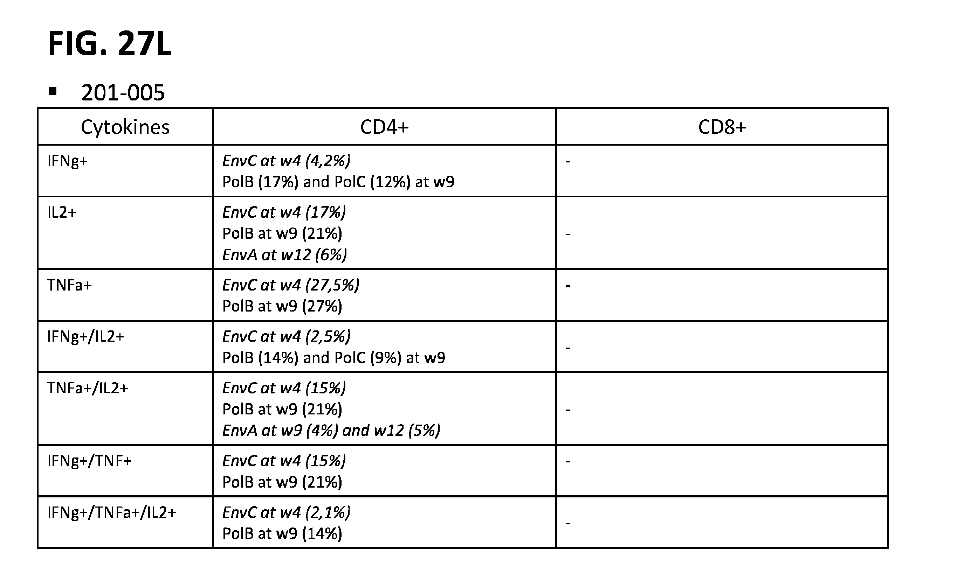

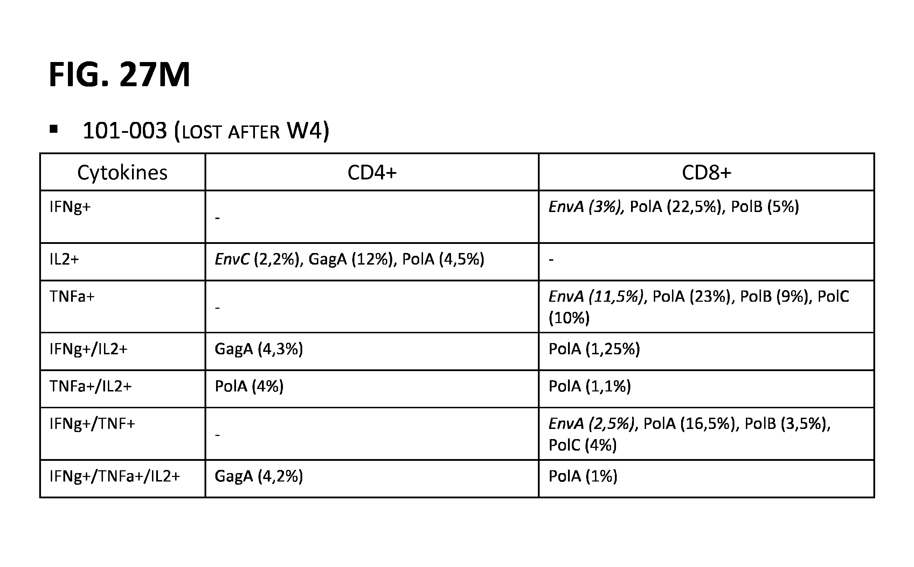

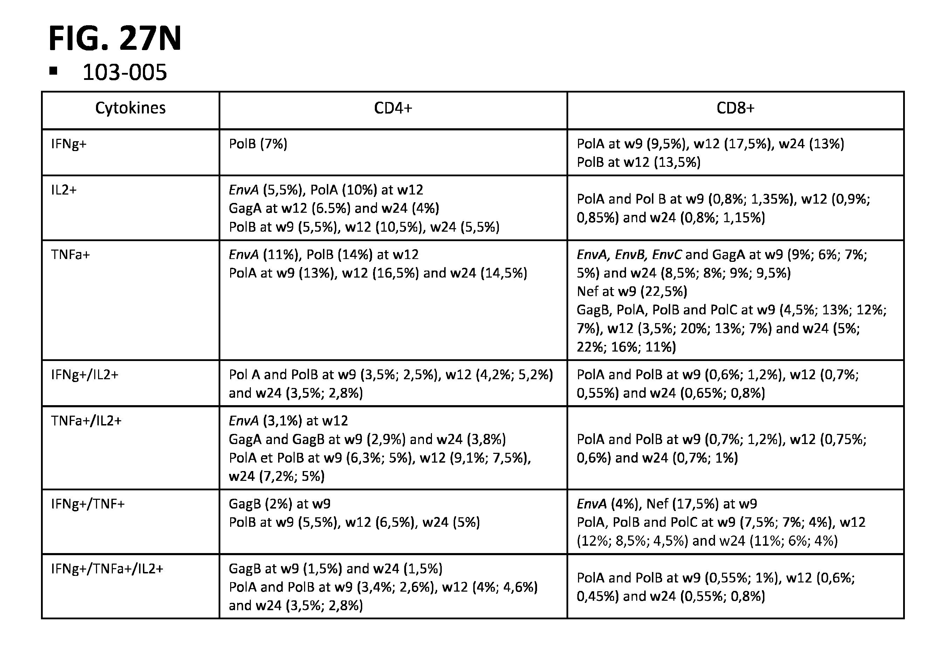

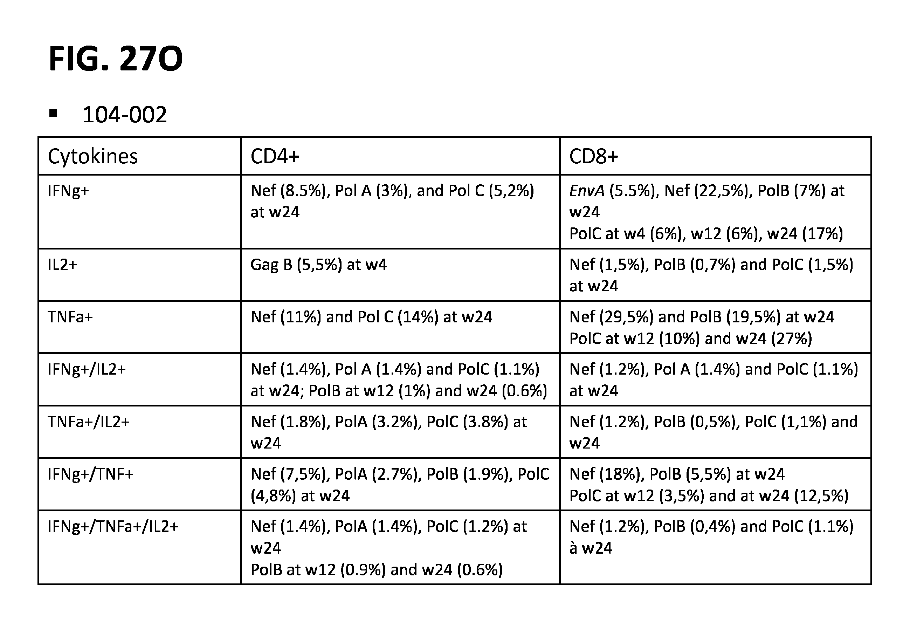

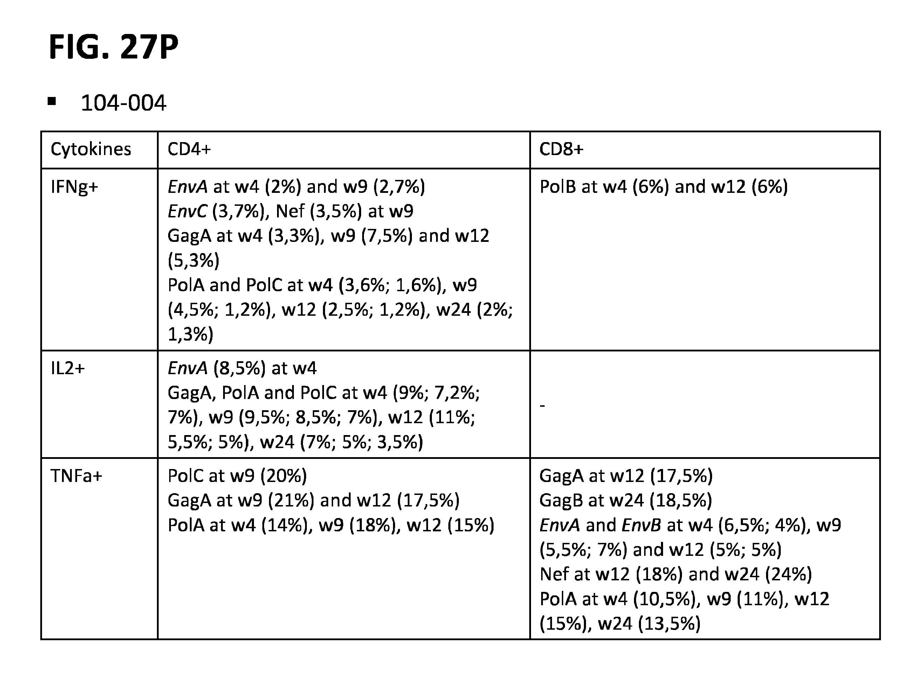

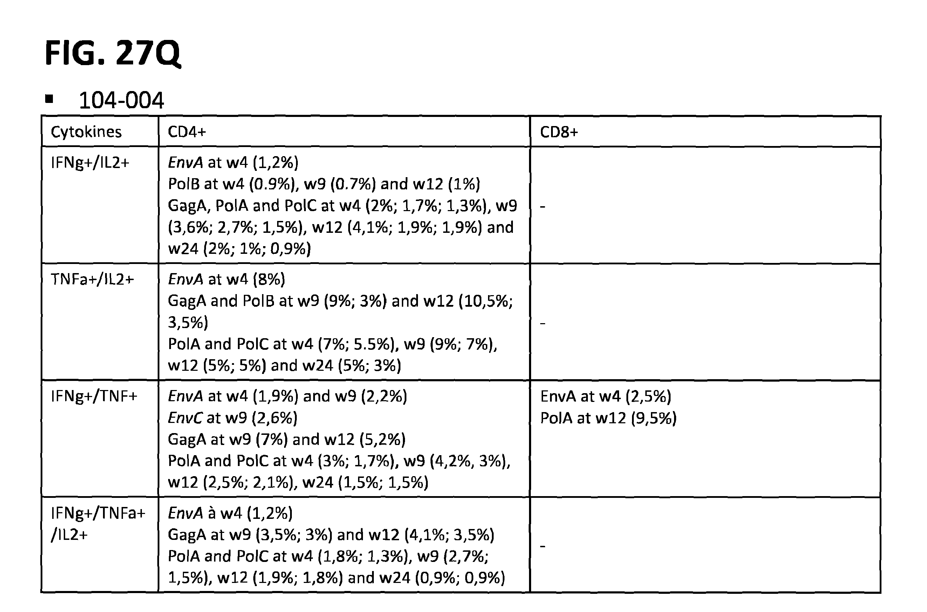

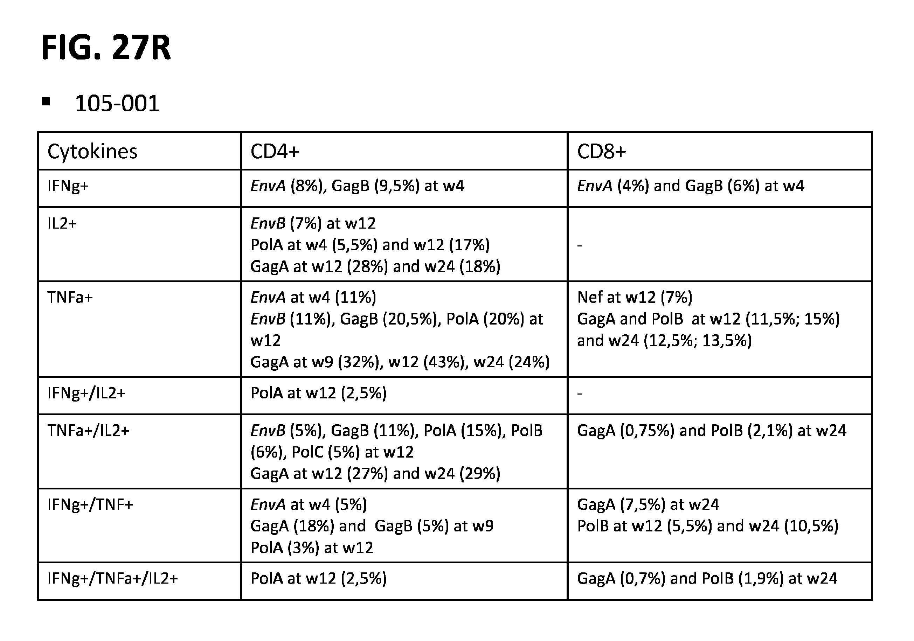

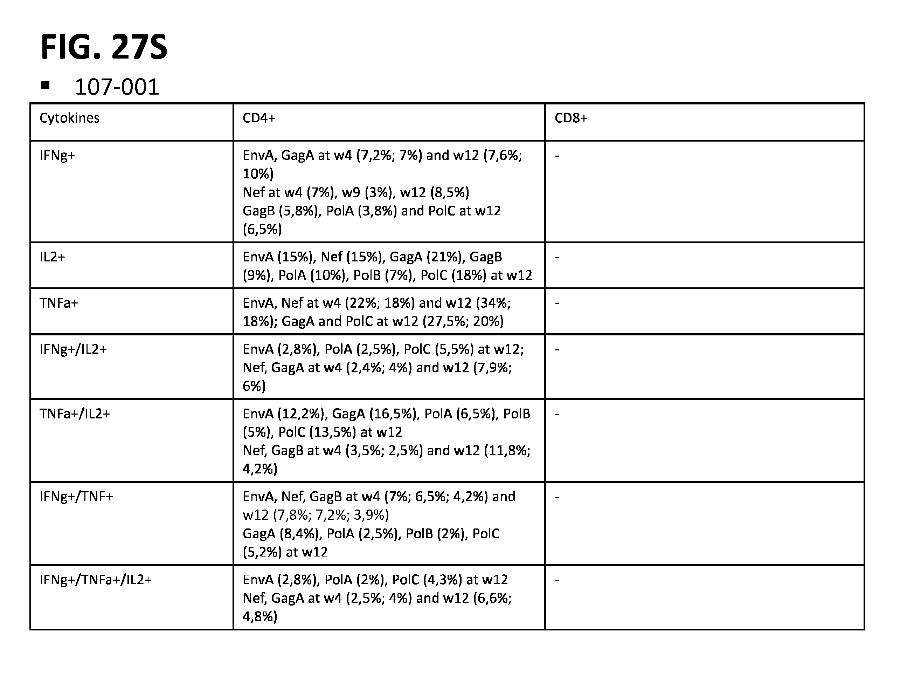

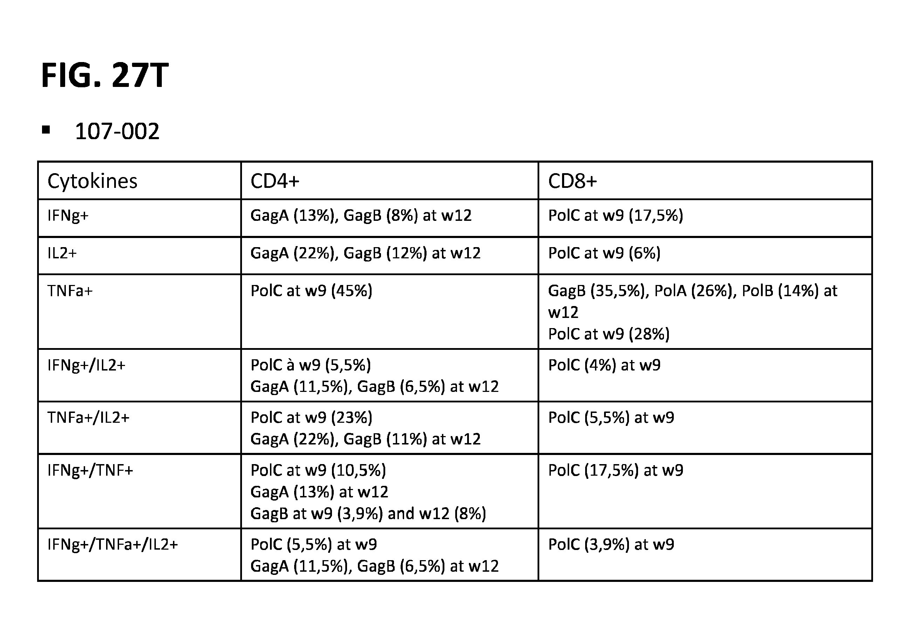

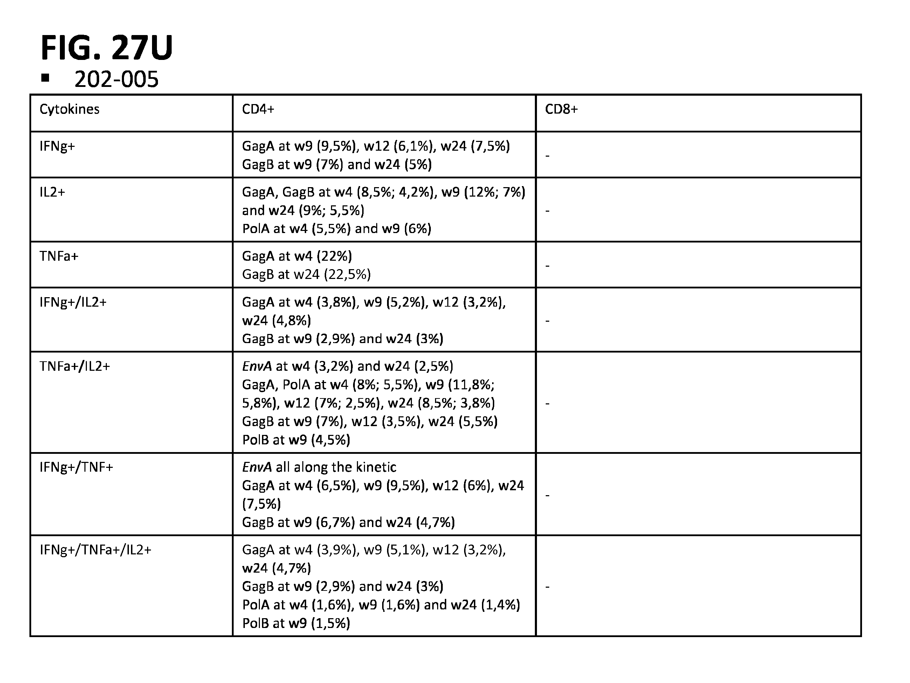

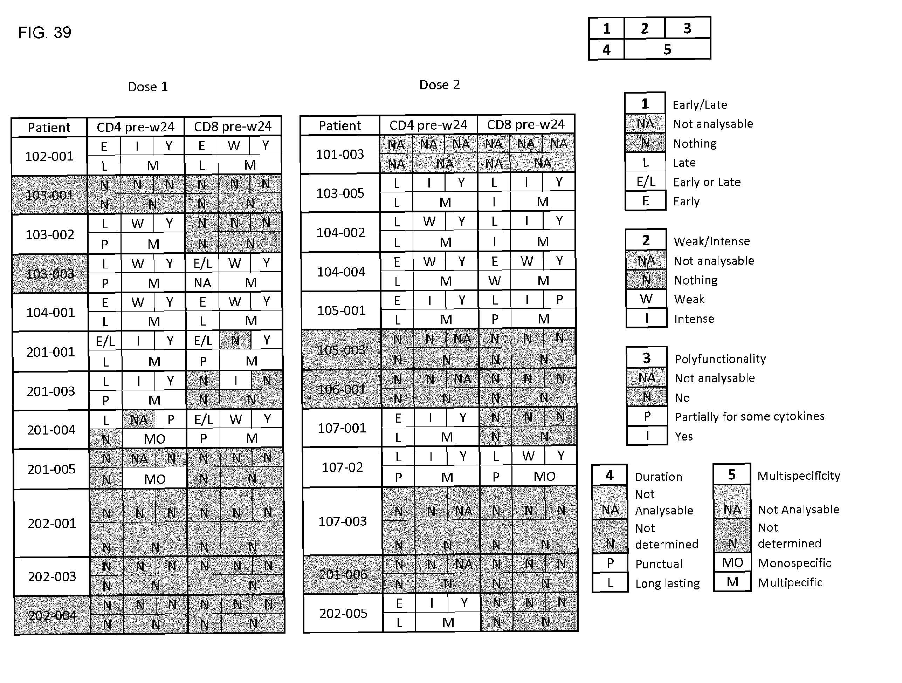

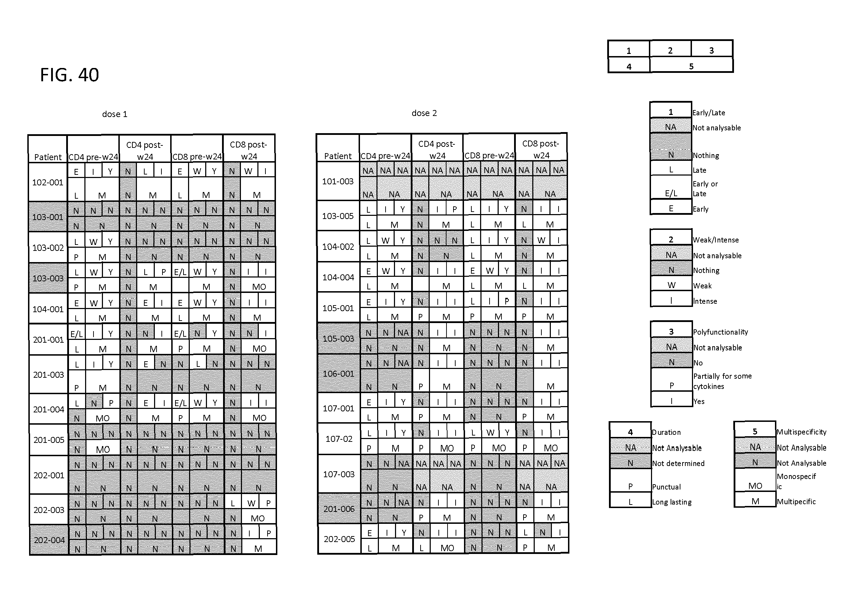

Samples taken from patients in the clinical trial plan (Figures 21 & 28) were analysed for specific immune responses. The results are depicted in Figure 27 and summarized in Figures 39 and 40.

The analysis of the Immunogenicity profile distinguishes between placebo vs vaccinated patients. Only 1 of 6 placebo patients demonstrated an immune response pre-week 24; whereas 6 of 9 of the dose 1 patients (Dose 1: 5 x 10⁶) and 7 of 8 (one patient couldn't be analyzed) of the dose 2 patients (Dose 2: 5 x 10⁷) demonstrated an immune response pre-week 24.

The immunogenicity profile of the vaccine is good. Multispecific polyfunctional CD4 and CD8 T cell responses are induced in many patients. Dose 2 was found to be more efficient than dose 1, especially in CD8 T cell responses where it induces higher and longer responses. Analyses are ongoing to correlate this immunogenicity profile to viral load post week 24.

Consequently, the intramuscular administration to a human of a lentiviral vector comprising a β2m promoter can induce dose-dependent CD4+ and CD8+ T cell responses against each of the encoded antigens. Although 5 x 10⁶ TU of the lentiviral vector is sufficient to observe responses, 5 x 10⁷ TU demonstrated better responses. 5 x 10⁸ TU can demonstrate even better responses.

The present invention encompasses lentiviral vectors comprising MHCI and β2m promoters, and their use for the induction of immune responses in a host by intramuscular administration to a human.

The present invention encompasses a lentiviral vector comprising a promoter sequence from a class I MHC or β2m gene promoter that directs the transcription of a transgene, which preferably encodes an immunogenic polypeptide, in a cell of a host, preferably in dendritic cells. Preferably, the lentiviral vector encodes at least one HIV-1 antigen, preferably an HIV-1 Nef, Pol, NC, or CA antigen or immunogenic polypeptide, most preferably comprising the amino acid sequence of SEQ ID NO:30.

### PROTEINS

The invention encompasses proteins comprising the amino acid sequence of SEQ ID NO:30. Preferably, the protein consists of the amino acid sequence of SEQ ID NO:30. 
MPIVQNLQGQMVHQAISPRTLNAWVKVVEEKAFSPEVIPMFSALSEGATPQDLNT
MLNTVGGHQAAMQMLKETINEEAAEWDRLHPVHAGPIAPGQMREPRGSDIAGTTSTLQEQIGWM
TNNPPIPVGEIYKRWIILGLNKIVRMYSPTSILDIRQGPKEPFRDYVDRFYKTLRAEQASQEVK
NWMTETLLVQNANPDCKTILKALGPAATLEEMMTACQGVGGPGHKARVLAEAMSQVTNSATIMM
QRGNFRNQRKTVKCFNCGKEGHIAKNCRAPRKKGCWKCGKEGHQMKDCTERQANFLGKIWPSHK
GRPGNFLQSRPEPTAPPEESFRFGEETTTPSQKQEPIDKELYPLASLRSLFGNDKKSVTVLDVG
DAYFSVPLDKDFRKYTAFTIPSINNETPGIRYQYNVLPQGWKGSPAIFQSSMTKILEPFRKQNP
 
DIVIYQYMDDLYVGSDLEIGQHRTEILKEPVHGVYVGFPVRPQVPLRPMTYKAAVDLSHFLKEK
GGLEGLIYSQKRQDILDLWVYHTQGYFPDWQNYTPGPGIRYPLTFGWCFKLVPVDPEKEVLVWK
FDSRLAFHHMARELHPEYY (SEQ ID NO:30).
 This sequence comprises Gag, Pol, and Nef sequences, in that order. The Pol sequences are in bold.

The protein can be purified. Preferably, the purified protein is more than 50%, 75%, 85%, 90%, 95%, 97%, 98%, or 99% pure. Within the context of this invention, a purified protein that is more than 50% (etc.) pure means a purified protein sample containing less than 50% (etc.) other proteins not comprising SEQ ID NO:30. For example, a sample of a protein comprising SEQ ID NO:30 can be 99% pure if it contains less than 1% contaminating host cell proteins.

### NUCLEIC ACIDS

The invention encompasses nucleic acids encoding the amino acid sequence of SEQ ID NO:30. The nucleic acid can be single-stranded or double-stranded. The nucleic acid can be an RNA or DNA molecule. Preferred nucleic acids comprise, or encode the nucleic acid sequence of any of the sequences disclosed herein.

Preferred nucleic acids comprise or encode any of the following nucleic acid sequences.

Proviral RNA sequence from production plasmid (5'LTR to 3'-LTR) without WPRE:

ACCUUCCCGAUUAAGUGAGGGUUUCUUCUGUUCUAUAGGAACUAGACACCUAGAUGGUG
UGUGUUCCGAUGAAGGGACUAAUCGUCUUGAUGUGUGGUCCCGGUCCCCAGUCUAUAGGUGACU
GGAAACCUACCACGAUGUUCGAUCAUGGUCAACUCGGUCUAUUCCAUCUUCUCCGGUUAUUUCC
UCUCUUGUGGUCGAACAAUGUGGACACUCGGACGUACCCUACCUACUGGGCCUCUCUCUUCAC
AAUCUCACCUCCAAACUGUCGGCGGAUCGUAAAGUAGUGCACCGGGCUCUCGACGUAGGCCUCA
UGAAGUUCUUGACGACUAUAGCUCGAACGAUGUUCCCUGAAAGGCGACCCCUGAAAGGUCCCUC
CGCACCGGACCCGCCCUGACCCCUCACCGCUCGGGAGUCUAGGACGUAUAUUCGUCGACGAAAA
ACGGACAUGACCCAGAGAGACCAAUCUGGUCUAGACUCGGACCCUCGAGAGACCGAUUGAUCCC
UUGGGUGACGAAUUCGGAGUUAUUUCGAACGGAACUCACGAAGUUCAUCACACACGGGCAGACA
ACACACUGAGACCAUUGAUCUCUAGGGAGUCUGGGAAAAUCAGUCACACCUUUUAGAGAUCGUC

ACCGCGGGCUUGUCCCUGAACUUUCGCUUUCCCUUUGGUCUCCUCGAGAGAGCUGCGUCCUGAG
CCGAACGACUUCGCGCGUGCCGUUCUCCGCUCCCCGCCGCUGACCACUCAUGCGGUUUUUAAAA
CUGAUCGCCUCCGAUCUUCCUCUCUCUACCCACGCUCUCGCAGUCAUAAUUCGCCCCCUCUUAA
UCUAGCGCUACCCUUUUUUAAGCCAAUUCCGGUCCCCCUUUCUUUUUUAUAUUUAAUUUUGUAU
AUCAUACCCGUUCGUCCCUCGAUCUUGCUAAGCGUCAAUUAGGACCGGACAAUCUUUGUAGUCU
UCCGACAUCUGUUUAUGACCCUGUCGAUGUUGGUAGGAAGUCUGUCCUAGUCUUCUUGAAUCU
AGUAAUAUAUUAUGUCAUCGUUGGGAGAUAACACACGUAGUUUCCUAUCUCUAUUUUCUGUGGU
UCCUUCGAAAUCUGUUCUAUCUCCUUCUCGUUUUGUUUUCAUUCUGGUGGCGUGUCGUUCGCCG
GCGACUAGAAGUCUGGACCUCCUCCUCUAUACUCCCUGUUAACCUCUUCACUUAAUAUAUUUAU
AUUUCAUCAUUUUUAACUUGGUAAUCCUCAUCGUGGGUGGUUCCGUUUCUCUUCUCACCACGUC
UCUCUUUUUUCUCGUCACCCUUAUCCUCGAAACAAGGAACCCAAGAACCCUCGUCGUCCUUCGU
GAUACCCGCGUCGCAGUUACUGCGACUGCCAUGUCCGGUCUGUUAAUAACAGACCAUAUCACGU
CGUCGUCUUGUUAAACGACUCCCGAUAACUCCGCGUUGUCGUAGACAACGUUGAGUGUCAGACC
CCGUAGUUCGUCGAGGUCCGUUCUUAGGACCGACACCUUUCUAUGGAUUUCCUAGUUGUCGAGG
ACCCCUAAACCCCAACGAGACCUUUGAGUAAACGUGGUGACGACACGGAACCUUACGAUCAAC
CUCAUUAUUUAGAGACCUUGUCUAAACCUUAGUGUGCUGGACCUACCUCACCCUGUCUCUUUAA
UUGUUAAUGUGUUCGAAUUAUGUGAGGAAUUAACUUCUUAGCGUUUUGGUCGUUCUUUUCUUAC
UUGUUCUUAAUAACCUUAAUCUAUUUACCCGUUCAAACACCUUAACCAAAUUGUAUUGUUUAAC
CGACACCAUAUAUUUUAAUAAGUAUUACUAUCAUCCUCCGAACCAUCCAAAUUCUUAUCAAAAA
CGACAUGAAAGAUAUCACUUAUCUCAAUCCGUCCCUAUAAGUGGUAAUAGCAAAGUCUGGGUGG
AGGGUUGGGGCUCCCCUGGGCUGUCCGGGCUUCCUUAUCUUCUUCUUCCACCUCUCUCUCUGUC
UCUGUCUAGGUAAGCUAAUCACUUGCCUAGAGCUGCCAUAGCGGCUUAAGUGUUUACCGUCAUA
AGUAGGUGUUAAAAUUUUCUUUUCCCCCCUAACCCCCCAUGUCACGUCCCCUUUCUUAUCAUCU
GUAUUAUCGUUGUCUGUAUGUUUGAUUUCUUAAUGUUUUUGUUUAAUGUUUUUAAGUUUUAAAA
GCCCAAAUAAUGUCCCUGUCGUCUCUAGGUGAAACCGACUAUGCGCGGCUCUUUGGACGUCCC
UUAAGGGGUCGACAUCAAUAUUUGUCUUCAAGAGGAAGACGAUCCAUCGUAAGUUUCUAGAAUU
AGAAGACCCAAAGGCAAAAGAGCUUACUUUUUACGUCCAGGCUCGUCAAUUGACCGCCCCCGUG
GUAAUCGUUCAGUGAAUCGUAGAGACCCCGGUCAGACGUUUCGCUCCCCCGUCGGAAUUACACG
GAGGUCGGACUUCAGGAUCUUACUCGCGGCCACAGGGUUCGACCCCGCGCGUGGGGUCUAGCC
UCCCGCGGCUACAUGUCUGUCGUUUGAGUGGGUCAGAUCACGUACGGAAGAAUUUGUAGUGCUC
UGAGAUUCUUUUCCUUUGACUUUUGCCCUUUCAGGGAGAGAGAUUGGACCGUGACGCAGCGACC
GAACCUCUGUCCACUGCCAGGGACGCCCGGAACAGGACUAACCGACCCGUGCGCAAAUUAUAUU

CACCUCCGCAGCGCGACCGCCCGUAAGGACUUCGACUGUCGUAAGCCCGGCUCGCGAGCCUAGG
CGGUGGUACGGGUAGCACGUCUUGGACGUCCCGGUCUACCACGUGGUCCGGUAGUCGGGGGCCU
GGGACUUGCGGACCCACUUCCACCACCUCCUUUUCCGGAAGUCGGGGCUCCACUAGGGUACAA
GUCGCGGGACUCGCUCCCGCGGUGUGGGGUCCUGGACUUGUGGUACGACUUGUGGCACCCGCCG
GUGGUCCGGCGGUACGUCUACGACUUUCUCUGUUAGUUGCUCCUUCGGCGGCUCACCCUGGCCG
ACGUGGGACACGUGCGGCCUGGAUAGCGGGGACCGGUCUACGCCCUCGGGUCCCCGUCGCUAUA
GCGGCCGUGGUGGAGGUGUGACGUCCUUGUCUAGCCGACCUACUGGUUGUUGGGGGGGUAGGGG
CACCCGCUCUAGAUGUUCGCCACCUAGUAGGACCCGGACUUGUUCUAGCACGCCUACAUGUCGG
GGUGGAGGUAGGACCUGUAGGCCGUCCCGGGGUUUCUCGGGAAGGCCCUGAUGCACCUGGCCAA
GAUGUUCUGGGACGCCCGGCUCGUCCGGUCGGUCCUUCACUUCUUGACCUACUGGCUCUGUGAC
GACCACGUCUUGCGGUUGGGGCUGACGUUCUGGUAGGACUUCCGGGACCCGGGACGGCGGUGGG
ACCUUCUCUACUACUGGCGGACGGUCCCGCACCCGCCUGGACCGGUGUUCCGGGCCCACGACCG
GCUCCGGUACUCGGUCCACUGGUUGUCGCGGUGGUAGUACUACGUCGCCCCGUUGAAGGCCUUG
GUCUCUUUCUGGCACUUCACGAAGUUGACGCCGUUUCUCCCGGUGUAACGGUUCUUGACGUCUC
GGGGGUCCUUCUUCCCGACGACCUUCACACCGUUUCUUCCCGUGGUCUACUUCCUGACGUGGCU
CGCCGUCCGGUUGAAGGACCCUUUUAAACCGGAAGGGUGUUCCCGUCCGGGCCGUUGAAGGAC
GUCUCGUCCGGGCUCGGGUGGCGGGGAGGGCUCCUUUCGAAGGCCAAGCCGCUCCUUUGGUGGU
GGGGGUCGGUCUUCGUCCUUGGGUAGCUGUUUCUCGACAUGGGGGACCGGAGGGACUCUUCGGA
CAAGCCGUUGCUGUUCUUUUCGCACUGGCACGACCUGCACCCGCUGCGGAUGAAGUCGCACGGG
GACCUAUUCCUGAAGGCCUUCAUGUGGCGGAAGUGGUAGGGGUCGUAGUUGUUGCUCUGUGGGC
CGUAGUCUAUGGUCAUGUUGCACGACGGGGUCCCGACCUUCCCGUCGGGGCGGUAGAAGGUCUC
GUCGUACUGGUUCUAGGACCUUGGGAAGGCCUUCGUCUUGGGGCUGUAGCACUAGAUGGUCAUG
UACCUGCUGGACAUGCACCCGUCGCUGGACCUUUAGCCGGUCGUGGCCUGUCUCUAGGACUUUC
UUGGGCACGUGCCGCACAUGCACCCGAAGGGGCACUCUGGUGUCCACGGUGACUCUGGGUACUG
GAUGUUCCGGCGGCACCUGGACUCGGUGAAGGACUUCCUCUUCCCGCCGGACCUUCCGGACUAG
AUGUCGGUCUUCUCCGUCCUGUAGGACCUGGACACCCACAUGGUGUGGGUCCCGAUGAAGGGGC
UGACCGUCUUGAUGUGGGGACCGGGACCGUAGGCGAUGGGGGACUGGAAGCCGACCACGAAGUU
CGACCACGGGCACCUGGGGCUCUUCCUUCACGACCACACCUUCAAGCUGUCGGCCGACCGGAAG
GUGGUGUACCGGUCUCUCGACGUGGGGCUCAUGAUGACUACUGAGCUCUCCAUGGAAAUUCUGG
UUACUGAAUGUUCCGUCGACAUCUAGAAUCGGUGAAAAUUUUCUUUUCCCCCCUGACCUUCCC
GAUUAAGUGAGGGUUGCUUCUGUUUUAGCAGCUCUCUACGACGUAUAUUCGUCGACGAAAAACG
AACAUGACCCAGAGAGACCAAUCUGGUCUAGACUCGGACCCUCGAGAGACCGAUUGAUCCCUUG

GGUGACGAAUUCGGAGUUAUUUCGAACGGAACUCACGAAGUUCAUCACACACGGGCAGACAACA
CACUGAGACCAUUGAUCUCUAGGGAGUCUGGGAAAAUCAGUCACACCUUUUAGAGAUCGUC
(SEQ ID NO:31).

Proviral RNA sequence from production plasmid (5'LTR to 3'-LTR) with WPRE:

ACCUUCCCGAUUAAGUGAGGGUUUCUUCUGUUCUAUAGGAACUAGACACCUAGAUGGUG
UGUGUUCCGAUGAAGGGACUAAUCGUCUUGAUGUGUGGUCCCGGUCCCAGUCUAUAGGUGACU
GGAAACCUACCACGAUGUUCGAUCAUGGUCAACUCGGUCUAUUCCAUCUUCUCCGGUUAUUUCC
UCUCUUGUGGUCGAACAAUGUGGGACACUCGGACGUACCCUACCUACUGGGCCUCUCUCUUCAC
AAUCUCACCUCCAAACUGUCGGCGGAUCGUAAAGUAGUGCACCGGGCUCUCGACGUAGGCCUCA
UGAAGUUCUUGACGACUAUAGCUCGAACGAUGUUCCCUGAAAGGCGACCCCUGAAAGGUCCCUC
CGCACCGGACCCGCCCUGACCCCUCACCGCUCGGGAGUCUAGGACGUAUAUUCGUCGACGAAAA
ACGGACAUGACCCAGAGAGACCAAUCUGGUCUAGACUCGGACCCUCGAGAGACCGAUUGAUCCC
UUGGGUGACGAAUUCGGAGUUAUUUCGAACGGAACUCACGAAGUUCAUCACACACGGGCAGACA
ACACACUGAGACCAUUGAUCUCUAGGGAGUCUGGGAAAAUCAGUCACACCUUUUAGAGAUCGUC
ACCGCGGGCUUGUCCCUGAACUUUCGCUUUCCCUUUGGUCUCCUCGAGAGAGCUGCGUCCUGAG
CCGAACGACUUCGCGCGUGCCGUUCUCCGCUCCCCGCCGCUGACCACUCAUGCGGUUUUUAAAA
CUGAUCGCCUCCGAUCUUCCUCUCUCUACCCACGCUCUCGCAGUCAUAAUUCGCCCCCUCUUAA
UCUAGCGCUACCCUUUUUUAAGCCAAUUCCGGUCCCCCUUUCUUUUUUAUAUUUAAUUUUGUAU
AUCAUACCCGUUCGUCCCUCGAUCUUGCUAAGCGUCAAUUAGGACCGGACAAUCUUUGUAGUCU
UCCGACAUCUGUUUAUGACCCUGUCGAUGUUGGUAGGGAAGUCUGUCCUAGUCUUCUUGAAUCU
AGUAAUAUAUUAUGUCAUCGUUGGGAGAUAACACACGUAGUUUCCUAUCUCUAUUUUCUGUGGU
UCCUUCGAAAUCUGUUCUAUCUCCUUCUCGUUUUGUUUUCAUUCUGGUGGCGUGUCGUUCGCCG
GCGACUAGAAGUCUGGACCUCCUCCUCUAUACUCCCUGUUAACCUCUUCACUUAAUAUAUUUAU
AUUUCAUCAUUUUUAACUUGGUAAUCCUCAUCGUGGGUGGUUCCGUUUCUCUUCUCACCACGUC
UCUCUUUUUUCUCGUCACCCUUAUCCUCGAAACAAGGAACCCAAGAACCCUCGUCGUCCUUCGU
GAUACCCGCGUCGCAGUUACUGCGACUGCCAUGUCCGGUCUGUUAAUAACAGACCAUAUCACGU
CGUCGUCUUGUUAAACGACUCCCGAUAACUCCGCGUUGUCGUAGACAACGUUGAGUGUCAGACC
CCGUAGUUCGUCGAGGUCCGUUCUUAGGACCGACACCUUUCUAUGGAUUUCCUAGUUGUCGAGG
ACCCCUAAACCCCAACGAGACCUUUUGAGUAAACGUGGUGACGACACGGAACCUUACGAUCAAC
CUCAUUAUUUAGAGACCUUGUCUAAACCUUAGUGUGCUGGACCUACCUCACCCUGUCUCUUUAA

UUGUUAAUGUGUUCGAAUUAUGUGAGGAAUUAACUUCUUAGCGUUUUGGUCGUUCUUUUCUUAC
UUGUUCUUAAUAACCUUAAUCUAUUUACCCGUUCAAACACCUUAACCAAAUUGUAUUGUUUAAC
CGACACCAUAUAUUUUAAUAAGUAUUACUAUCAUCCUCCGAACCAUCCAAAUUCUUAUCAAAAA
CGACAUGAAAGAUAUCACUUAUCUCAAUCCGUCCCUAUAAGUGGUAAUAGCAAAGUCUGGGUGG
AGGGUUGGGGCUCCCCUGGGCUGUCCGGGCUUCCUUAUCUUCUUCUUCCACCUCUCUCUCUGUC
UCUGUCUAGGUAAGCUAAUCACUUGCCUAGAGCUGCCAUAGCGGCUUAAGUGUUUACCGUCAUA
AGUAGGUGUUAAAAUUUUCUUUUCCCCCCUAACCCCCCAUGUCACGUCCCCUUUCUUAUCAUCU
GUAUUAUCGUUGUCUGUAUGUUUGAUUUCUUAAUGUUUUUGUUUAAUGUUUUUAAGUUUUAAAA
GCCCAAAUAAUGUCCCUGUCGUCUCUAGGUGAAACCGACUAUGCGCGGCUCUUUGGACGUCCC
UUAAGGGGUCGACAUCAAUAUUUGUCUUCAAGAGGAAGACGAUCCAUCGUAAGUUUCUAGAAUU
AGAAGACCCAAAGGCAAAAGAGCUUACUUUUUACGUCCAGGCUCGUCAAUUGACCGCCCCCGUG
GUAAUCGUUCAGUGAAUCGUAGAGACCCCGGUCAGACGUUUCGCUCCCCCGUCGGAAUUACACG
GAGGUCGGACUUCAGGAUCUUACUCGCGGGCCACAGGGUUCGACCCCGCGCGUGGGGUCUAGCC
UCCCGCGGCUACAUGUCUGUCGUUUGAGUGGGUCAGAUCACGUACGGAAGAAUUUGUAGUGCUC
UGAGAUUCUUUUCCUUUGACUUUUGCCCUUUCAGGGAGAGAGAUUGGACCGUGACGCAGCGACC
GAACCUCUGUCCACUGCCAGGGACGCCCGGAACAGGACUAACCGACCCGUGCGCAAAUUAUAUU
CACCUCCGCAGCGCGACCGCCCGUAAGGACUUCGACUGUCGUAAGCCCGGCUCGCGAGCCUAGG
CGGUGGUACGGGUAGCACGUCUUGGACGUCCCGGUCUACCACGUGGUCCGGUAGUCGGGGGCCU
GGGACUUGCGGACCCACUUCCACCACCUCCUUUUCCGGAAGUCGGGGCUCCACUAGGGGUACAA
GUCGCGGGACUCGCUCCCGCGGUGUGGGGUCCUGGACUUGUGGUACGACUUGUGGCACCCGCCG
GUGGUCCGGCGGUACGUCUACGACUUUCUCUGUUAGUUGCUCCUUCGGCGGCUCACCCUGGCCG
ACGUGGGACACGUGCGGCCUGGAUAGCGGGGACCGGUCUACGCCCUCGGGUCCCCGUCGCUAUA
GCGGCCGUGGUGGAGGUGUGACGUCCUUGUCUAGCCGACCUACUGGUUGUUGGGGGGUAGGGG
CACCCGCUCUAGAUGUUCGCCACCUAGUAGGACCCGGACUUGUUCUAGCACGCCUACAUGUCGG
GGUGGAGGUAGGACCUGUAGGCCGUCCCGGGGUUUCUCGGGAAGGCCCUGAUGCACCUGGCCAA
GAUGUUCUGGGACGCCCGGCUCGUCCGGUCGGUCCUUCACUUCUUGACCUACUGGCUCUGUGAC
GACCACGUCUUGCGGUUGGGGCUGACGUUCUGGUAGGACUUCCGGGACCCGGGACGGCGGUGGG
ACCUUCUCUACUACUGGCGGACGGUCCCGCACCCGCCUGGACCGGUGUUCCGGGCCCACGACCG
GCUCCGGUACUCGGUCCACUGGUUGUCGCGGUGGUAGUACUACGUCGCCCCGUUGAAGGCCUUG
GUCUCUUUCUGGCACUUCACGAAGUUGACGCCGUUUCUCCCGGUGUAACGGUUCUUGACGUCUC
GGGGGUCCUUCUUCCCGACGACCUUCACACCGUUUCUUCCCGUGGUCUACUUCCUGACGUGGCU
CGCCGUCCGGUUGAAGGACCCUUUUUAAACCGGAAGGGUGUUCCCGUCCGGGCCGUUGAAGGAC

GUCUCGUCCGGGCUCGGGUGGCGGGGAGGGCUCCUUUCGAAGGCCAAGCCGCUCCUUUGGUGGU
GGGGGUCGGUCUUCGUCCUUGGGUAGCUGUUUCUCGACAUGGGGGACCGGAGGGACUCUUCGGA
CAAGCCGUUGCUGUUCUUUUCGCACUGGCACGACCUGCACCCGCUGCGGAUGAAGUCGCACGGG
GACCUAUUCCUGAAGGCCUUCAUGUGGCGGAAGUGGUAGGGGUCGUAGUUGUUGCUCUGUGGGC
CGUAGUCUAUGGUCAUGUUGCACGACGGGGUCCCGACCUUCCCGUCGGGGCGGUAGAAGGUCUC
GUCGUACUGGUUCUAGGACCUUGGGAAGGCCUUCGUCUUGGGGCUGUAGCACUAGAUGGUCAUG
UACCUGCUGGACAUGCACCCGUCGCUGGACCUUUAGCCGGUCGUGGCCUGUCUCUAGGACUUUC
UUGGGCACGUGCCGCACAUGCACCCGAAGGGGCACUCUGGUGUCCACGGUGACUCUGGGUACUG
GAUGUUCCGGCGGCACCUGGACUCGGUGAAGGACUUCCUCUUCCCGCCGGACCUUCCGGACUAG
AUGUCGGUCUUCUCCGUCCUGUAGGACCUGGACACCCACAUGGUGUGGGUCCCGAUGAAGGGGC
UGACCGUCUUGAUGUGGGACCGGACCGUAGGCGAUGGGGGACUGGAAGCCGACCACGAAGUU
CGACCACGGGCACCUGGGGCUCUUCCUUCACGACCACACCUUCAAGCUGUCGGCCGACCGGAAG
GUGGUGUACCGGUCUCUCGACGUGGGGCUCAUGAUGACUACUGAGCUCUUUAAGGGCUAUUAGU
UGGAGACCUAAUGUUUAAACACUUUCUAACUGACCAUAAGAAUUGAUACAACGAGGAAAAUGC
GAUACACCUAUGCGACGAAAUUACGGAAACAUAGUACGAUAACGAAGGGCAUACCGAAAGUAAA
AGAGGAGGAACAUAUUUAGGACCAACGACAGAGAAAUACUCCUCAACACCGGGCAACAGUCCGU
UGCACCGCACCACACGUGACACAAACGACUGCGUUGGGGUGACCAACCCCGUAACGGUGGUGG
ACAGUCGAGGAAAGGCCCUGAAAGCGAAAGGGGGAGGAUAACGGUGCCGCCUUGAGUAGCGGC
GGACGGAACGGGCGACGACCUGUCCCCGAGCCGACAACCCGUGACUGUUAAGGCACCACCAACA
GCCAUGGAAAUUCUGGUUACUGAAUGUUCCGUCGACAUCUAGAAUCGGUGAAAAUUUUCUUUU
CCCCCCUGACCUUCCCGAUUAAGUGAGGGUUGCUUCUGUUUUAGCAGCUCUCUACGACGUAUAU
UCGUCGACGAAAAACGAACAUGACCCAGAGAGACCAAUCUGGUCUAGACUCGGACCCUCGAGAG
ACCGAUUGAUCCCUUGGGUGACGAAUUCGGAGUUAUUUCGAACGGAACUCACGAAGUUCAUCAC
ACACGGGCAGACAACACACUGAGACCAUUGAUCUCUAGGGAGUCUGGGAAAAUCAGUCACACCU
UUUAGAGAUCGUC (SEQ ID NO:32).

The WPRE in this sequence is in bold. This sequence is shorter than the WPRE sequences previously used in lentiviral constructs.

Antigen RNA sequence from integrated provirus with WPRE:

UACGGGUAGCACGUCUUGGACGUCCCGGUCUACCACGUGGUCCGGUAGUCGGGGCCUG
GGACUUGCGGACCCACUUCCACCACCUCCUUUCCGGAAGUCGGGCUCCACUAGGGUACAAG

UCGCGGGACUCGCUCCCGCGGUGUGGGUCCUGGACUUGUGGUACGACUUGUGGCACCCGCCGG
UGGUCCGGCGGUACGUCUACGACUUUCUCUGUUAGUUGCUCCUUCGGCGGCUCACCCUGGCCGA
CGUGGGACACGUGCGGCCUGGAUAGCGGGACCGGUCUACGCCCUCGGUCCCCGUCGCUAUAG
CGGCCGUGGUGGAGGUGUGACGUCCUUGUCUAGCCGACCUACUGGUUGUUGGGGGGUAGGGGC
ACCCGCUCUAGAUGUUCGCCACCUAGUAGGACCCGGACUUGUUCUAGCACGCCUACAUGUCGGG
GUGGAGGUAGGACCUGUAGGCCGUCCCGGGGUUUCUCGGGAAGGCCCUGAUGCACCUGGCCAAG
AUGUUCUGGGACGCCCGGCUCGUCCGGUCGGUCCUUCACUUCUUGACCUACUGGCUCUGUGACG
ACCACGUCUUGCGGUUGGGGCUGACGUUCUGGUAGGACUUCCGGGACCCGGGACGGCGGUGGGA
CCUUCUCUACUACUGGCGGACGGUCCCGCACCCGCCUGGACCGGUGUUCCGGGCCCACGACCGG
CUCCGGUACUCGGUCCACUGGUUGUCGCGGUGGUAGUACUACGUCGCCCCGUUGAAGGCCUUGG
UCUCUUUCUGGCACUUCACGAAGUUGACGCCGUUUCUCCCGGUGUAACGGUUCUUGACGUCUCG
GGGGUCCUUCUUCCCGACGACCUUCACACCGUUUCUUCCCGUGGUCUACUUCCUGACGUGGCUC
GCCGUCCGGUUGAAGGACCCUUUUAAACCGGAAGGGUGUUCCCGUCCGGGCCGUUGAAGGACG
UCUCGUCCGGGCUCGGGUGGCGGGGAGGGCUCCUUUCGAAGGCCAAGCCGCUCCUUUGGUGGUG
GGGGUCGGUCUUCGUCCUUGGGUAGCUGUUUCUCGACAUGGGGGACCGGAGGGACUCUUCGGAC
AAGCCGUUGCUGUUCUUUUCGCACUGGCACGACCUGCACCCGCUGCGGAUGAAGUCGCACGGGG
ACCUAUUCCUGAAGGCCUUCAUGUGGCGGAAGUGGUAGGGGUCGUAGUUGUUGCUCUGUGGGCC
GUAGUCUAUGGUCAUGUUGCACGACGGGGUCCCGACCUUCCCGUCGGGGCGGUAGAAGGUCUCG
UCGUACUGGUUCUAGGACCUUGGGAAGGCCUUCGUCUUGGGGCUGUAGCACUAGAUGGUCAUGU
ACCUGCUGGACAUGCACCCGUCGCUGGACCUUUAGCCGGUCGUGGCCUGUCUCUAGGACUUUCU
UGGGCACGUGCCGCACAUGCACCCGAAGGGGCACUCUGGUGUCCACGGUGACUCUGGGUACUGG
AUGUUCCGGCGGCACCUGGACUCGGUGAAGGACUUCCUCUUCCCGCCGGACCUUCCGGACUAGA
UGUCGGUCUUCUCCGUCCUGUAGGACCUGGACACCCACAUGGUGUGGGUCCCGAUGAAGGGGCU
GACCGUCUUGAUGUGGGGACCGGGACCGUAGGCGAUGGGGGACUGGAAGCCGACCACGAAGUUC
GACCACGGGCACCUGGGGCUCUUCCUUCACGACCACACCUUCAAGCUGUCGGCCGACCGGAAGG
UGGUGUACCGGUCUCUCGACGUGGGGCUCAUGAUGACUACUGAGCUCUUUAAGGGCUAUUAGUU
GGAGACCUAAUGUUUUAAACACUUUCUAACUGACCAUAAGAAUUGAUACAACGAGGAAAAUGCG
AUACACCUAUGCGACGAAAUUACGGAAACAUAGUACGAUAACGAAGGGCAUACCGAAAGUAAAA
GAGGAGGAACAUAUUUAGGACCAACGACAGAGAAAUACUCCUCAACACCGGGCAACAGUCCGUU
GCACCGCACCACACGUGACACAAACGACUGCGUUGGGGGUGACCAACCCCGUAACGGUGGUGGA
CAGUCGAGGAAAGGCCCUGAAAGCGAAAGGGGGAGGAUAACGGUGCCGCCUUGAGUAGCGGCG
GACGGAACGGGCGACGACCUGUCCCCGAGCCGACAACCCGUGACUGUUAAGGCACCACCAACAG

(SEQ ID NO:33).

Antigen RNA sequence from integrated provirus up to the U5 region, without WPRE:

UACGGGUAGCACGUCUUGGACGUCCCGGUCUACCACGUGGUCCGGUAGUCGGGGGCCUG
GGACUUGCGGACCCACUUCCACCACCUCCUUUCCGGAAGUCGGGGCUCCACUAGGGUACAAG
UCGCGGGACUCGCUCCCGCGGUGUGGGUCCUGGACUUGUGGUACGACUUGUGGCACCCGCCGG
UGGUCCGGCGGUACGUCUACGACUUUCUCUGUUAGUUGCUCCUUCGGCGGCUCACCCUGGCCGA
CGUGGGACACGUGCGGCCUGGAUAGCGGGGACCGGUCUACGCCCUCGGGUCCCCGUCGCUAUAG
CGGCCGUGGUGGAGGUGUGACGUCCUUGUCUAGCCGACCUACUGGUUGUUGGGGGGUAGGGGC
ACCCGCUCUAGAUGUUCGCCACCUAGUAGGACCCGGACUUGUUCUAGCACGCCUACAUGUCGGG
GUGGAGGUAGGACCUGUAGGCCGUCCCGGGGUUUCUCGGGAAGGCCCUGAUGCACCUGGCCAAG
AUGUUCUGGGACGCCCGGCUCGUCCGGUCGGUCCUUCACUUCUUGACCUACUGGCUCUGUGACG
ACCACGUCUUGCGGUUGGGGCUGACGUUCUGGUAGGACUUCCGGGACCCGGACGGCGGUGGGA
CCUUCUCUACUACUGGCGGACGGUCCCGCACCCGCCUGGACCGGUGUUCCGGGCCCACGACCGG
CUCCGGUACUCGGUCCACUGGUUGUCGCGGUGGUAGUACUACGUCGCCCCGUUGAAGGCCUUGG
UCUCUUUCUGGCACUUCACGAAGUUGACGCCGUUUCUCCCGGUGUAACGGUUCUUGACGUCUCG
GGGGUCCUUCUUCCCGACGACCUUCACACCGUUUCUUCCCGUGGUCUACUUCCUGACGUGGCUC
GCCGUCCGGUUGAAGGACCCUUUUUAAACCGGAAGGGUGUUCCCGUCCGGGCCGUUGAAGGACG
UCUCGUCCGGGCUCGGGUGGCGGGAGGGCUCCUUUCGAAGGCCAAGCCGCUCCUUUGGUGGUG
GGGGUCGGUCUUCGUCCUUGGGUAGCUGUUUCUCGACAUGGGGGACCGGAGGGACUCUUCGGAC
AAGCCGUUGCUGUUCUUUUCGCACUGGCACGACCUGCACCCGCUGCGGAUGAAGUCGCACGGGG
ACCUAUUCCUGAAGGCCUUCAUGUGGCGGAAGUGGUAGGGGUCGUAGUUGUUGCUCUGUGGGCC
GUAGUCUAUGGUCAUGUUGCACGACGGGGUCCCGACCUUCCCGUCGGGGCGGUAGAAGGUCUCG
UCGUACUGGUUCUAGGACCUUGGGAAGGCCUUCGUCUUGGGGCUGUAGCACUAGAUGGUCAUGU
ACCUGCUGGACAUGCACCCGUCGCUGGACCUUUAGCCGGUCGUGGCCUGUCUCUAGGACUUUCU
UGGGCACGUGCCGCACAUGCACCCGAAGGGGCACUCUGGUGUCCACGGUGACUCUGGGUACUGG
AUGUUCCGGCGGCACCUGGACUCGGUGAAGGACUUCCUCUUCCCGCCGGACCUUCCGGACUAGA
UGUCGGUCUUCUCCGUCCUGUAGGACCUGGACACCCACAUGGUGUGGGUCCCGAUGAAGGGGCU
GACCGUCUUGAUGUGGGACCGGGACCGUAGGCGAUGGGGACUGGAAGCCGACCACGAAGUUC
GACCACGGGCACCUGGGGCUCUUCCUUCACGACCACACCUUCAAGCUGUCGGCCGACCGGAAGG

UGGUGUACCGGUCUCUCGACGUGGGGCUCAUGAUGACUACUGAGCUCUCCAUGGAAAUUCUGGU
UACUGAAUGUUCCGUCGACAUCUAGAAUCGGUGAAAAAUUUCUUUUCCCCCCUGACCUUCCCG
AUUAAGUGAGGGUUGCUUCUGUUUUAGCAGCUCUCUACGACGUAUAUUCGUCGACGAAAAACGA
ACAUGACCCAGAGAGACCAAUCUGGUCUAGACUCGGACCCUCGAGAGACCGAUUGAUCCCUUGG
GUGACGAAUUCGGAGUUAUUUCGAACGGAACUCACGAAGUUCAUCACACACGGGCAGACAACAC
ACUGAGACCAUUGAUCUCUAGGGAGUCUGGGAAAAUCAGUCACACCUUUUAGAGAUCGUCA
(SEQ ID NO:34).

Antigen RNA sequence from integrated provirus up to the U5 region, with WPRE:

UACGGGUAGCACGUCUUGGACGUCCCGGUCUACCACGUGGUCCGGUAGUCGGGGGCCUG
GGACUUGCGGACCCACUUCCACCACCUCCUUUCCGGAAGUCGGGCUCCACUAGGGUACAAG
UCGCGGGACUCGCUCCCGCGGUGUGGGGUCCUGGACUUGUGGUACGACUUGUGGCACCCGCCGG
UGGUCCGGCGGUACGUCUACGACUUUCUCUGUUAGUUGCUCCUUCGGCGGCUCACCCUGGCCGA
CGUGGGACACGUGCGGCCUGGAUAGCGGGGACCGGUCUACGCCCUCGGGUCCCCGUCGCUAUAG
CGGCCGUGGUGGAGGUGUGACGUCCUUGUCUAGCCGACCUACUGGUUGUUGGGGGGUAGGGGC
ACCCGCUCUAGAUGUUCGCCACCUAGUAGGACCCGGACUUGUUCUAGCACGCCUACAUGUCGGG
GUGGAGGUAGGACCUGUAGGCCGUCCCGGGGUUUCUCGGGAAGGCCCUGAUGCACCUGGCCAAG
AUGUUCUGGGACGCCCGGCUCGUCCGGUCGGUCCUUCACUUCUUGACCUACUGGCUCUGUGACG
ACCACGUCUUGCGGUUGGGGCUGACGUUCUGGUAGGACUUCCGGGACCCGGGACGGCGGUGGGA
CCUUCUCUACUACUGGCGGACGGUCCCGCACCCGCCUGGACCGGUGUUCCGGGCCCACGACCGG
CUCCGGUACUCGGUCCACUGGUUGUCGCGGUGGUAGUACUACGUCGCCCCGUUGAAGGCCUUGG
UCUCUUUCUGGCACUUCACGAAGUUGACGCCGUUUCUCCCGGUGUAACGGUUCUUGACGUCUCG
GGGGUCCUUCUUCCCGACGACCUUCACACCGUUUCUUCCCGUGGUCUACUUCCUGACGUGGCUC
GCCGUCCGGUUGAAGGACCCUUUUAAACCGGAAGGGUGUUCCCGUCCGGGCCGUUGAAGGACG
UCUCGUCCGGGCUCGGGUGGCGGGGAGGGCUCCUUUCGAAGGCCAAGCCGCUCCUUUGGUGGUG
GGGGUCGGUCUUCGUCCUUGGGUAGCUGUUUCUCGACAUGGGGGACCGGAGGGACUCUUCGGAC
AAGCCGUUGCUGUUCUUUUCGCACUGGCACGACCUGCACCCGCUGCGGAUGAAGUCGCACGGGG
ACCUAUUCCUGAAGGCCUUCAUGUGGCGGAAGUGGUAGGGGUCGUAGUUGUUGCUCUGUGGGCC
GUAGUCUAUGGUCAUGUUGCACGACGGGGUCCCGACCUUCCCGUCGGGGCGGUAGAAGGUCUCG
UCGUACUGGUUCUAGGACCUUGGGAAGGCCUUCGUCUUGGGGCUGUAGCACUAGAUGGUCAUGU

ACCUGCUGGACAUGCACCCGUCGCUGGACCUUUAGCCGGUCGUGGCCUGUCUCUAGGACUUUCU
UGGGCACGUGCCGCACAUGCACCCGAAGGGGCACUCUGGUGUCCACGGUGACUCUGGUACUGG
AUGUUCCGGCGGCACCUGGACUCGGUGAAGGACUUCCUCUUCCCGCCGGACCUUCCGGACUAGA
UGUCGGUCUUCUCCGUCCUGUAGGACCUGGACACCCACAUGGUGUGGGUCCCGAUGAAGGGGCU
GACCGUCUUGAUGUGGGACCGGACCGUAGGCGAUGGGGACUGGAAGCCGACCACGAAGUUC
GACCACGGGCACCUGGGGCUCUUCCUUCACGACCACACCUUCAAGCUGUCGGCCGACCGGAAGG
UGGUGUACCGGUCUCUCGACGUGGGGCUCAUGAUGACUACUGAGCUCUUUAAGGGCUAUUAGUU
GGAGACCUAAUGUUUAAACACUUUCUAACUGACCAUAAGAAUUGAUACAACGAGGAAAAUGCG
AUACACCUAUGCGACGAAAUUACGGAAACAUAGUACGAUAACGAAGGGCAUACCGAAAGUAAAA
GAGGAGGAACAUAUUUAGGACCAACGACAGAGAAAUACUCCUCAACACCGGGCAACAGUCCGUU
GCACCGCACCACACGUGACACAAACGACUGCGUUGGGGGUGACCAACCCCGUAACGGUGGUGGA
CAGUCGAGGAAAGGCCCUGAAAGCGAAAGGGGGAGGGAUAACGGUGCCGCCUUGAGUAGCGGCG
GACGGAACGGGCGACGACCUGUCCCCGAGCCGACAACCCGUGACUGUUAAGGCACCACCAACAG
CCAUGGAAAUUCUGGUUACUGAAUGUUCCGUCGACAUCUAGAAUCGGUGAAAAUUUUCUUUUC
CCCCCUGACCUUCCCGAUUAAGUGAGGUUGCUUCUGUUUUAGCAGCUCUCUACGACGUAUAUU
CGUCGACGAAAAACGAACAUGACCCAGAGAGACCAAUCUGGUCUAGACUCGGACCCUCGAGAGA
CCGAUUGAUCCCUUGGGUGACGAAUUCGGAGUUAUUUCGAACGGAACUCACGAAGUUCAUCACA
CACGGGCAGACAACACACUGAGACCAUUGAUCUCUAGGGAGUCUGGGAAAAUCAGUCACACCUU
UUAGAGAUCGUCA (SEQ ID NO:35).

**Antigen RNA sequence from integrated provirus**

UACGGGUAGCACGUCUUGGACGUCCCGGUCUACCACGUGGUCCGGUAGUCGGGGCCUG
GGACUUGCGGACCCACUUCCACCACCUCCUUUCCGGAAGUCGGGCUCCACUAGGGUACAAG
UCGCGGGACUCGCUCCCGCGGUGUGGGGUCCUGGACUUGUGGUACGACUUGUGGCACCCGCCGG
UGGUCCGGCGGUACGUCUACGACUUUCUCUGUUAGUUGCUCCUUCGGCGGCUCACCCUGGCCGA
CGUGGGACACGUGCGGCCUGGAUAGCGGGGACCGGUCUACGCCCUCGGGUCCCCGUCGCUAUAG
CGGCCGUGGUGGAGGUGUGACGUCCUUGUCUAGCCGACCUACUGGUUGUUGGGGGGUAGGGGC
ACCCGCUCUAGAUGUUCGCCACCUAGUAGGACCCGGACUUGUUCUAGCACGCCUACAUGUCGGG
GUGGAGGUAGGACCUGUAGGCCGUCCCGGGGUUUCUCGGGAAGGCCCUGAUGCACCUGGCCAAG
AUGUUCUGGGACGCCCGGCUCGUCCGGUCGGUCCUUCACUUCUUGACCUACUGGCUCUGUGACG
ACCACGUCUUGCGGUUGGGGCUGACGUUCUGGUAGGACUUCCGGGACCCGGGACGGCGGUGGGA

CCUUCUCUACUACUGGCGGACGGUCCCGCACCCGCCUGGACCGGUGUUCCGGGCCCACGACCGG
CUCCGGUACUCGGUCCACUGGUUGUCGCGGUGGUAGUACUACGUCGCCCCGUUGAAGGCCUUGG
UCUCUUUCUGGCACUUCACGAAGUUGACGCCGUUUCUCCCGGUGUAACGGUUCUUGACGUCUCG
GGGGUCCUUCUUCCCGACGACCUUCACACCGUUUCUUCCCGUGGUCUACUUCCUGACGUGGCUC
GCCGUCCGGUUGAAGGACCCUUUUAAACCGGAAGGGUGUUCCCGUCCGGGCCGUUGAAGGACG
UCUCGUCCGGGCUCGGGUGGCGGGGAGGGCUCCUUUCGAAGGCCAAGCCGCUCCUUUGGUGGUG
GGGGUCGGUCUUCGUCCUUGGGUAGCUGUUUCUCGACAUGGGGGACCGGAGGGACUCUUCGGAC
AAGCCGUUGCUGUUCUUUUCGCACUGGCACGACCUGCACCCGCUGCGGAUGAAGUCGCACGGGG
ACCUAUUCCUGAAGGCCUUCAUGUGGCGGAAGUGGUAGGGGUCGUAGUUGUUGCUCUGUGGGCC
GUAGUCUAUGGUCAUGUUGCACGACGGGGUCCCGACCUUCCCGUCGGGGCGGUAGAAGGUCUCG
UCGUACUGGUUCUAGGACCUUGGGAAGGCCUUCGUCUUGGGGCUGUAGCACUAGAUGGUCAUGU
ACCUGCUGGACAUGCACCCGUCGCUGGACCUUUAGCCGGUCGUGGCCUGUCUCUAGGACUUUCU
UGGGCACGUGCCGCACAUGCACCCGAAGGGGCACUCUGGUGUCCACGGUGACUCUGGGUACUGG
AUGUUCCGGCGGCACCUGGACUCGGUGAAGGACUUCCUCUUCCCGCCGGACCUUCCGGACUAGA
UGUCGGUCUUCUCCGUCCUGUAGGACCUGGACACCCACAUGGUGUGGGUCCCGAUGAAGGGGCU
GACCGUCUUGAUGUGGGGACCGGGACCGUAGGCGAUGGGGGACUGGAAGCCGACCACGAAGUUC
GACCACGGGCACCUGGGGCUCUUCCUUCACGACCACACCUUCAAGCUGUCGGCCGACCGGAAGG
UGGUGUACCGGUCUCUCGACGUGGGGCUCAUGAUGACUACU (SEQ ID NO:36).

Production plasmid proviral sequence (5'LTR to 3'-LTR) with WPRE:

TGGAAGGGCTAATTCACTCCCAAAGAAGACAAGATATCCTTGATCTGTGGATCTACCAC
ACACAAGGCTACTTCCCTGATTAGCAGAACTACACACCAGGGCCAGGGGTCAGATATCCACTGA
CCTTTGGATGGTGCTACAAGCTAGTACCAGTTGAGCCAGATAAGGTAGAAGAGGCCAATAAAGG
AGAGAACACCAGCTTGTTACACCCTGTGAGCCTGCATGGGATGGATGACCCGGAGAGAGAAGTG
TTAGAGTGGAGGTTTGACAGCCGCCTAGCATTTCATCACGTGGCCCGAGAGCTGCATCCGGAGT
ACTTCAAGAACTGCTGATATCGAGCTTGCTACAAGGGACTTTCCGCTGGGGACTTTCCAGGGAG
GCGTGGCCTGGGCGGGACTGGGGAGTGGCGAGCCCTCAGATCCTGCATATAAGCAGCTGCTTTT
TGCCTGTACTGGGTCTCTCTGGTTAGACCAGATCTGAGCCTGGGAGCTCTCTGGCTAACTAGGG
AACCCACTGCTTAAGCCTCAATAAAGCTTGCCTTGAGTGCTTCAAGTAGTGTGTGCCCGTCTGT
TGTGTGACTCTGGTAACTAGAGATCCCTCAGACCCTTTTAGTCAGTGTGGAAAATCTCTAGCAG
TGGCGCCCGAACAGGGACTTGAAAGCGAAAGGAAACCAGAGGAGCTCTCTCGACGCAGGACTC

GGCTTGCTGAAGCGCGCACGGCAAGAGGCGAGGGGCGGCGACTGGTGAGTACGCCAAAAATTTT
GACTAGCGGAGGCTAGAAGGAGAGAGATGGGTGCGAGAGCGTCAGTATTAAGCGGGGGAGAATT
AGATCGCGATGGGAAAAAATTCGGTTAAGGCCAGGGGGAAAGAAAAAATATAAATTAAAACATA
TAGTATGGGCAAGCAGGGAGCTAGAACGATTCGCAGTTAATCCTGGCCTGTTAGAAACATCAGA
AGGCTGTAGACAAATACTGGGACAGCTACAACCATCCCTTCAGACAGGATCAGAAGAACTTAGA
TCATTATATAATACAGTAGCAACCCTCTATTGTGTGCATCAAAGGATAGAGATAAAAGACACCA
AGGAAGCTTTAGACAAGATAGAGGAAGAGCAAAACAAAAGTAAGACCACCGCACAGCAAGCGGC
CGCTGATCTTCAGACCTGGAGGAGGAGATATGAGGGACAATTGGAGAAGTGAATTATATAAATA
TAAAGTAGTAAAAATTGAACCATTAGGAGTAGCACCCACCAAGGCAAAGAGAAGAGTGGTGCAG
AGAGAAAAAAGAGCAGTGGGAATAGGAGCTTTGTTCCTTGGGTTCTTGGGAGCAGCAGGAAGCA
CTATGGGCGCAGCGTCAATGACGCTGACGGTACAGGCCAGACAATTATTGTCTGGTATAGTGCA
GCAGCAGAACAATTTGCTGAGGGCTATTGAGGCGCAACAGCATCTGTTGCAACTCACAGTCTGG
GGCATCAAGCAGCTCCAGGCAAGAATCCTGGCTGTGGAAAGATACCTAAAGGATCAACAGCTCC
TGGGGATTTGGGGTTGCTCTGGAAAACTCATTTGCACCACTGCTGTGCCTTGGAATGCTAGTTG
GAGTAATAAATCTCTGGAACAGATTTGGAATCACACGACCTGGATGGAGTGGGACAGAGAAATT
AACAATTACACAAGCTTAATACACTCCTTAATTGAAGAATCGCAAAACCAGCAAGAAAAGAATG
AACAAGAATTATTGGAATTAGATAAATGGGCAAGTTTGTGGAATTGGTTTAACATAACAAATTG
GCTGTGGTATATAAAATTATTCATAATGATAGTAGGAGGCTTGGTAGGTTTAAGAATAGTTTTT
GCTGTACTTTCTATAGTGAATAGAGTTAGGCAGGGATATTCACCATTATCGTTTCAGACCCACC
TCCCAACCCCGAGGGGACCCGACAGGCCCGAAGGAATAGAAGAAGAAGGTGGAGAGAGAGACAG
AGACAGATCCATTCGATTAGTGAACGGATCTCGACGGTATCGCCGAATTCACAAATGGCAGTAT
TCATCCACAATTTTAAAAGAAAAGGGGGGATTGGGGGGTACAGTGCAGGGGAAAGAATAGTAGA
CATAATAGCAACAGACATACAAACTAAAGAATTACAAAAACAAATTACAAAAATTCAAAATTTT
CGGGTTTATTACAGGGACAGCAGAGATCCACTTTGGCTGATACGCGCCGAGAAACCCTGCAGGG
AATTCCCCAGCTGTAGTTATAAACAGAAGTTCTCCTTCTGCTAGGTAGCATTCAAAGATCTTAA
TCTTCTGGGTTTCCGTTTTCTCGAATGAAAAATGCAGGTCCGAGCAGTTAACTGGCGGGGGCAC
CATTAGCAAGTCACTTAGCATCTCTGGGGCCAGTCTGCAAAGCGAGGGGGCAGCCTTAATGTGC
CTCCAGCCTGAAGTCCTAGAATGAGCGCCCGGTGTCCCAAGCTGGGGCGCGCACCCCAGATCGG
AGGGCGCCGATGTACAGACAGCAAACTCACCCAGTCTAGTGCATGCCTTCTTAAACATCACGAG
ACTCTAAGAAAAGGAAACTGAAAACGGGAAAGTCCCTCTCTCTAACCTGGCACTGCGTCGCTGG
CTTGGAGACAGGTGACGGTCCCTGCGGGCCTTGTCCTGATTGGCTGGGCACGCGTTTAATATAA
GTGGAGGCGTCGCGCTGGCGGGCATTCCTGAAGCTGACAGCATTCGGGCCGAGCGCTCGGATCC

GCCACCATGCCCATCGTGCAGAACCTGCAGGGCCAGATGGTGCACCAGGCCATCAGCCCCCGGA
CCCTGAACGCCTGGGTGAAGGTGGTGGAGGAAAAGGCCTTCAGCCCCGAGGTGATCCCCATGTT
CAGCGCCCTGAGCGAGGGCGCCACACCCCAGGACCTGAACACCATGCTGAACACCGTGGGCGGC
CACCAGGCCGCCATGCAGATGCTGAAAGAGACAATCAACGAGGAAGCCGCCGAGTGGGACCGGC
TGCACCCTGTGCACGCCGGACCTATCGCCCCTGGCCAGATGCGGGAGCCCAGGGGCAGCGATAT
CGCCGGCACCACCTCCACACTGCAGGAACAGATCGGCTGGATGACCAACAACCCCCCCATCCCC
GTGGGCGAGATCTACAAGCGGTGGATCATCCTGGGCCTGAACAAGATCGTGCGGATGTACAGCC
CCACCTCCATCCTGGACATCCGGCAGGGCCCCAAAGAGCCCTTCCGGGACTACGTGGACCGGTT
CTACAAGACCCTGCGGGCCGAGCAGGCCAGCCAGGAAGTGAAGAACTGGATGACCGAGACACTG
CTGGTGCAGAACGCCAACCCCGACTGCAAGACCATCCTGAAGGCCCTGGGCCCTGCCGCCACCC
TGGAAGAGATGATGACCGCCTGCCAGGGCGTGGGCGGACCTGGCCACAAGGCCCGGGTGCTGGC
CGAGGCCATGAGCCAGGTGACCAACAGCGCCACCATCATGATGCAGCGGGGCAACTTCCGGAAC
CAGAGAAAGACCGTGAAGTGCTTCAACTGCGGCAAAGAGGGCCACATTGCCAAGAACTGCAGAG
CCCCCAGGAAGAAGGGCTGCTGGAAGTGTGGCAAAGAAGGCACCAGATGAAGGACTGCACCGA
GCGGCAGGCCAACTTCCTGGGAAAAATTTGGCCTTCCCACAAGGGCAGGCCCGGCAACTTCCTG
CAGAGCAGGCCCGAGCCCACCGCCCCTCCCGAGGAAAGCTTCCGGTTCGGCGAGGAAACCACCA
CCCCCAGCCAGAAGCAGGAACCCATCGACAAAGAGCTGTACCCCCTGGCCTCCCTGAGAAGCCT
GTTCGGCAACGACAAGAAAAGCGTGACCGTGCTGGACGTGGGCGACGCCTACTTCAGCGTGCCC
CTGGATAAGGACTTCCGGAAGTACACCGCCTTCACCATCCCCAGCATCAACAACGAGACACCCG
GCATCAGATACCAGTACAACGTGCTGCCCCAGGGCTGGAAGGGCAGCCCCGCCATCTTCCAGAG
CAGCATGACCAAGATCCTGGAACCCTTCCGGAAGCAGAACCCCGACATCGTGATCTACCAGTAC
ATGGACGACCTGTACGTGGGCAGCGACCTGGAAATCGGCCAGCACCGGACAGAGATCCTGAAAG
AACCCGTGCACGGCGTGTACGTGGGCTTCCCCGTGAGACCACAGGTGCCACTGAGACCCATGAC
CTACAAGGCCGCCGTGGACCTGAGCCACTTCCTGAAGGAGAAGGGCGGCCTGGAAGGCCTGATC
TACAGCCAGAAGAGGCAGGACATCCTGGACCTGTGGGTGTACCACACCCAGGGCTACTTCCCCG
ACTGGCAGAACTACACCCCTGGCCCTGGCATCCGCTACCCCCTGACCTTCGGCTGGTGCTTCAA
GCTGGTGCCCGTGGACCCCGAGAAGGAAGTGCTGGTGTGGAAGTTCGACAGCCGGCTGGCCTTC
CACCACATGGCCAGAGAGCTGCACCCCGAGTACTACTGATGACTCGAGAAATTCCCGATAATCA
ACCTCTGGATTACAAAATTTGTGAAAGATTGACTGGTATTCTTAACTATGTTGCTCCTTTTACG
CTATGTGGATACGCTGCTTTAATGCCTTTGTATCATGCTATTGCTTCCCGTATGGCTTTCATTT
TCTCCTCCTTGTATAAATCCTGGTTGCTGTCTCTTTATGAGGAGTTGTGGCCCGTTGTCAGGCA
ACGTGGCGTGGTGTGCACTGTGTTTGCTGACGCAACCCCCACTGGTTGGGGCATTGCCACCACC

TGTCAGCTCCTTTCCGGGACTTTCGCTTTCCCCCTCCCTATTGCCACGGCGGAACTCATCGCCG
CCTGCCTTGCCCGCTGCTGGACAGGGGCTCGGCTGTTGGGCACTGACAATTCCGTGGTGGTTGT
CGGTACCTTTAAGACCAATGACTTACAAGGCAGCTGTAGATCTTAGCCACTTTTTAAAAGAAAA
GGGGGGACTGGAAGGGCTAATTCACTCCCAACGAAGACAAAATCGTCGAGAGATGCTGCATATA
AGCAGCTGCTTTTTGCTTGTACTGGGTCTCTCTGGTTAGACCAGATCTGAGCCTGGGAGCTCTC
TGGCTAACTAGGGAACCCACTGCTTAAGCCTCAATAAAGCTTGCCTTGAGTGCTTCAAGTAGTG
TGTGCCCGTCTGTTGTGTGACTCTGGTAACTAGAGATCCCTCAGACCCTTTTAGTCAGTGTGGA
AAATCTCTAGCAG (SEQ ID NO:37).

Production plasmid proviral sequence (5'LTR to 3'-LTR) without WPRE:

TGGAAGGGCTAATTCACTCCCAAAGAAGACAAGATATCCTTGATCTGTGGATCTACCAC
ACACAAGGCTACTTCCCTGATTAGCAGAACTACACACCAGGGCCAGGGGTCAGATATCCACTGA
CCTTTGGATGGTGCTACAAGCTAGTACCAGTTGAGCCAGATAAGGTAGAAGAGGCCAATAAAGG
AGAGAACACCAGCTTGTTACACCCTGTGAGCCTGCATGGGATGGATGACCCGGAGAGAGAAGTG
TTAGAGTGGAGGTTTGACAGCCGCCTAGCATTTCATCACGTGGCCCGAGAGCTGCATCCGGAGT
ACTTCAAGAACTGCTGATATCGAGCTTGCTACAAGGGACTTTCCGCTGGGGACTTTCCAGGGAG
GCGTGGCCTGGGCGGGACTGGGGAGTGGCGAGCCCTCAGATCCTGCATATAAGCAGCTGCTTTT
TGCCTGTACTGGGTCTCTCTGGTTAGACCAGATCTGAGCCTGGGAGCTCTCTGGCTAACTAGGG
AACCCACTGCTTAAGCCTCAATAAAGCTTGCCTTGAGTGCTTCAAGTAGTGTGTGCCCGTCTGT
TGTGTGACTCTGGTAACTAGAGATCCCTCAGACCCTTTTAGTCAGTGTGGAAAATCTCTAGCAG
TGGCGCCCGAACAGGGACTTGAAAGCGAAAGGAAACCAGAGGAGCTCTCTCGACGCAGGACTC
GGCTTGCTGAAGCGCGCACGGCAAGAGGCGAGGGGCGGCGACTGGTGAGTACGCCAAAAATTTT
GACTAGCGGAGGCTAGAAGGAGAGAGATGGGTGCGAGAGCGTCAGTATTAAGCGGGGGAGAATT
AGATCGCGATGGGAAAAAATTCGGTTAAGGCCAGGGGGAAAGAAAAAATATAAATTAAAACATA
TAGTATGGGCAAGCAGGGAGCTAGAACGATTCGCAGTTAATCCTGGCCTGTTAGAAACATCAGA
AGGCTGTAGACAAATACTGGGACAGCTACAACCATCCCTTCAGACAGGATCAGAAGAACTTAGA
TCATTATATAATACAGTAGCAACCCTCTATTGTGTGCATCAAAGGATAGAGATAAAAGACACCA
AGGAAGCTTTAGACAAGATAGAGGAAGAGCAAAACAAAAGTAAGACCACCGCACAGCAAGCGGC
CGCTGATCTTCAGACCTGGAGGAGGAGATATGAGGGACAATTGGAGAAGTGAATTATATAAATA
TAAAGTAGTAAAAATTGAACCATTAGGAGTAGCACCCACCAAGGCAAAGAGAAGAGTGGTGCAG
AGAGAAAAAAGAGCAGTGGGAATAGGAGCTTTGTTCCTTGGGTTCTTGGGAGCAGCAGGAAGCA

CTATGGGCGCAGCGTCAATGACGCTGACGGTACAGGCCAGACAATTATTGTCTGGTATAGTGCA
GCAGCAGAACAATTTGCTGAGGGCTATTGAGGCGCAACAGCATCTGTTGCAACTCACAGTCTGG
GGCATCAAGCAGCTCCAGGCAAGAATCCTGGCTGTGGAAAGATACCTAAAGGATCAACAGCTCC
TGGGGATTTGGGGTTGCTCTGGAAAACTCATTTGCACCACTGCTGTGCCTTGGAATGCTAGTTG
GAGTAATAAATCTCTGGAACAGATTTGGAATCACACGACCTGGATGGAGTGGGACAGAGAAATT
AACAATTACACAAGCTTAATACACTCCTTAATTGAAGAATCGCAAAACCAGCAAGAAAAGAATG
AACAAGAATTATTGGAATTAGATAAATGGGCAAGTTTGTGGAATTGGTTTAACATAACAAATTG
GCTGTGGTATATAAAATTATTCATAATGATAGTAGGAGGCTTGGTAGGTTTAAGAATAGTTTTT
GCTGTACTTTCTATAGTGAATAGAGTTAGGCAGGGATATTCACCATTATCGTTTCAGACCCACC
TCCCAACCCCGAGGGGACCCGACAGGCCCGAAGGAATAGAAGAAGAAGGTGGAGAGAGAGACAG
AGACAGATCCATTCGATTAGTGAACGGATCTCGACGGTATCGCCGAATTCACAAATGGCAGTAT
TCATCCACAATTTTAAAAGAAAAGGGGGGATTGGGGGGTACAGTGCAGGGGAAAGAATAGTAGA
CATAATAGCAACAGACATACAAACTAAAGAATTACAAAAACAAATTACAAAAATTCAAAATTTT
CGGGTTTATTACAGGGACAGCAGAGATCCACTTTGGCTGATACGCGCCGAGAAACCCTGCAGGG
AATTCCCCAGCTGTAGTTATAAACAGAAGTTCTCCTTCTGCTAGGTAGCATTCAAAGATCTTAA
TCTTCTGGGTTTCCGTTTTCTCGAATGAAAAATGCAGGTCCGAGCAGTTAACTGGCGGGGGCAC
CATTAGCAAGTCACTTAGCATCTCTGGGGCCAGTCTGCAAAGCGAGGGGGCAGCCTTAATGTGC
CTCCAGCCTGAAGTCCTAGAATGAGCGCCCGGTGTCCCAAGCTGGGGCGCGCACCCCAGATCGG
AGGGCGCCGATGTACAGACAGCAAACTCACCCAGTCTAGTGCATGCCTTCTTAAACATCACGAG
ACTCTAAGAAAAGGAAACTGAAAACGGGAAAGTCCCTCTCTCTAACCTGGCACTGCGTCGCTGG
CTTGGAGACAGGTGACGGTCCCTGCGGGCCTTGTCCTGATTGGCTGGGCACGCGTTTAATATAA
GTGGAGGCGTCGCGCTGGCGGGCATTCCTGAAGCTGACAGCATTCGGGCCGAGCGCTCGGATCC
GCCACCATGCCCATCGTGCAGAACCTGCAGGGCCAGATGGTGCACCAGGCCATCAGCCCCCGGA
CCCTGAACGCCTGGGTGAAGGTGGTGGAGGAAAAGGCCTTCAGCCCCGAGGTGATCCCCATGTT
CAGCGCCCTGAGCGAGGGCGCCACACCCCAGGACCTGAACACCATGCTGAACACCGTGGGCGGC
CACCAGGCCGCCATGCAGATGCTGAAAGAGACAATCAACGAGGAAGCCGCCGAGTGGGACCGGC
TGCACCCTGTGCACGCCGGACCTATCGCCCCTGGCCAGATGCGGGAGCCCAGGGGCAGCGATAT
CGCCGGCACCACCTCCACACTGCAGGAACAGATCGGCTGGATGACCAACAACCCCCCCATCCCC
GTGGGCGAGATCTACAAGCGGTGGATCATCCTGGGCCTGAACAAGATCGTGCGGATGTACAGCC
CCACCTCCATCCTGGACATCCGGCAGGGCCCCAAAGAGCCCTTCCGGGACTACGTGGACCGGTT
CTACAAGACCCTGCGGGCCGAGCAGGCCAGCCAGGAAGTGAAGAACTGGATGACCGAGACACTG
CTGGTGCAGAACGCCAACCCCGACTGCAAGACCATCCTGAAGGCCCTGGGCCCTGCCGCCACCC

TGGAAGAGATGATGACCGCCTGCCAGGGCGTGGGCGGACCTGGCCACAAGGCCCGGGTGCTGGC
CGAGGCCATGAGCCAGGTGACCAACAGCGCCACCATCATGATGCAGCGGGGCAACTTCCGGAAC
CAGAGAAAGACCGTGAAGTGCTTCAACTGCGGCAAAGAGGGCCACATTGCCAAGAACTGCAGAG
CCCCCAGGAAGAAGGGCTGCTGGAAGTGTGGCAAAGAAGGGCACCAGATGAAGGACTGCACCGA
GCGGCAGGCCAACTTCCTGGGAAAAATTTGGCCTTCCCACAAGGGCAGGCCCGGCAACTTCCTG
CAGAGCAGGCCCGAGCCCACCGCCCCTCCCGAGGAAAGCTTCCGGTTCGGCGAGGAAACCACCA
CCCCCAGCCAGAAGCAGGAACCCATCGACAAAGAGCTGTACCCCCTGGCCTCCCTGAGAAGCCT
GTTCGGCAACGACAAGAAAAGCGTGACCGTGCTGGACGTGGGCGACGCCTACTTCAGCGTGCCC
CTGGATAAGGACTTCCGGAAGTACACCGCCTTCACCATCCCCAGCATCAACAACGAGACACCCG
GCATCAGATACCAGTACAACGTGCTGCCCCAGGGCTGGAAGGGCAGCCCCGCCATCTTCCAGAG
CAGCATGACCAAGATCCTGGAACCCTTCCGGAAGCAGAACCCCGACATCGTGATCTACCAGTAC
ATGGACGACCTGTACGTGGGCAGCGACCTGGAAATCGGCCAGCACCGGACAGAGATCCTGAAAG
AACCCGTGCACGGCGTGTACGTGGGCTTCCCCGTGAGACCACAGGTGCCACTGAGACCCATGAC
CTACAAGGCCGCCGTGGACCTGAGCCACTTCCTGAAGGAGAAGGGCGGCCTGGAAGGCCTGATC
TACAGCCAGAAGAGGCAGGACATCCTGGACCTGTGGGTGTACCACACCCAGGGCTACTTCCCCG
ACTGGCAGAACTACACCCCTGGCCCTGGCATCCGCTACCCCCTGACCTTCGGCTGGTGCTTCAA
GCTGGTGCCCGTGGACCCCGAGAAGGAAGTGCTGGTGTGGAAGTTCGACAGCCGGCTGGCCTTC
CACCACATGGCCAGAGAGCTGCACCCCGAGTACTACTGATGACTCGAGAGGTACCTTTAAGACC
AATGACTTACAAGGCAGCTGTAGATCTTAGCCACTTTTTAAAAGAAAAGGGGGGACTGGAAGGG
CTAATTCACTCCCAACGAAGACAAAATCGTCGAGAGATGCTGCATATAAGCAGCTGCTTTTTGC
TTGTACTGGGTCTCTCTGGTTAGACCAGATCTGAGCCTGGGAGCTCTCTGGCTAACTAGGGAAC
CCACTGCTTAAGCCTCAATAAAGCTTGCCTTGAGTGCTTCAAGTAGTGTGTGCCCGTCTGTTGT
GTGACTCTGGTAACTAGAGATCCCTCAGACCCTTTTAGTCAGTGTGGAAAATCTCTAGCAG
(SEQ ID NO:38).

Integrated proviral sequence (5'LTR to 3'-LTR) with WPRE:

TGTACTGGGTCTCTCTGGTTAGACCAGATCTGAGCCTGGGAGCTCTCTGGCTAACTAGG
GAACCCACTGCTTAAGCCTCAATAAAGCTTGCCTTGAGTGCTTCAAGTAGTGTGTGCCCGTCTG
TTGTGTGACTCTGGTAACTAGAGATCCCTCAGACCCTTTTAGTCAGTGTGGAAAATCTCTAGCA
GTGGCGCCCGAACAGGGACTTGAAAGCGAAAGGGAAACCAGAGGAGCTCTCTCGACGCAGGACT
CGGCTTGCTGAAGCGCGCACGGCAAGAGGCGAGGGGCGGCGACTGGTGAGTACGCCAAAAATTT

TGACTAGCGGAGGCTAGAAGGAGAGAGATGGGTGCGAGAGCGTCAGTATTAAGCGGGGGAGAAT
TAGATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATCAG
AAGGCTGTAGACAAATACTGGGACAGCTACAACCATCCCTTCAGACAGGATCAGAAGAACTTAG
ATCATTATATAATACAGTAGCAACCCTCTATTGTGTGCATCAAAGGATAGAGATAAAAGACACC
AAGGAAGCTTTAGACAAGATAGAGGAAGAGCAAAACAAAAGTAAGACCACCGCACAGCAAGCGG
CCGCTGATCTTCAGACCTGGAGGAGGAGATATGAGGGACAATTGGAGAAGTGAATTATATAAAT
ATAAAGTAGTAAAAATTGAACCATTAGGAGTAGCACCCACCAAGGCAAAGAGAAGAGTGGTGCA
GAGAGAAAAAAGAGCAGTGGGAATAGGAGCTTTGTTCCTTGGGTTCTTGGGAGCAGCAGGAAGC
ACTATGGGCGCAGCGTCAATGACGCTGACGGTACAGGCCAGACAATTATTGTCTGGTATAGTGC
AGCAGCAGAACAATTTGCTGAGGGCTATTGAGGCGCAACAGCATCTGTTGCAACTCACAGTCTG
GGGCATCAAGCAGCTCCAGGCAAGAATCCTGGCTGTGGAAAGATACCTAAAGGATCAACAGCTC
CTGGGGATTTGGGGTTGCTCTGGAAAACTCATTTGCACCACTGCTGTGCCTTGGAATGCTAGTT
GGAGTAATAAATCTCTGGAACAGATTTGGAATCACACGACCTGGATGGAGTGGGACAGAGAAAT
TAACAATTACACAAGCTTAATACACTCCTTAATTGAAGAATCGCAAAACCAGCAAGAAAAGAAT
GAACAAGAATTATTGGAATTAGATAAATGGGCAAGTTTGTGGAATTGGTTTAACATAACAAATT
GGCTGTGGTATATAAAATTATTCATAATGATAGTAGGAGGCTTGGTAGGTTTAAGAATAGTTTT
TGCTGTACTTTCTATAGTGAATAGAGTTAGGCAGGGATATTCACCATTATCGTTTCAGACCCAC
CTCCCAACCCCGAGGGGACCCGACAGGCCCGAAGGAATAGAAGAAGAAGGTGGAGAGAGAGACA
GAGACAGATCCATTCGATTAGTGAACGGATCTCGACGGTATCGCCGAATTCACAAATGGCAGTA
TTCATCCACAATTTTAAAAGAAAAGGGGGGATTGGGGGGTACAGTGCAGGGGAAAGAATAGTAG
ACATAATAGCAACAGACATACAAACTAAAGAATTACAAAAACAAATTACAAAAATTCAAAATTT
TCGGGTTTATTACAGGGACAGCAGAGATCCACTTTGGCTGATACGCGCCGAGAAACCCTGCAGG
GAATTCCCCAGCTGTAGTTATAAACAGAAGTTCTCCTTCTGCTAGGTAGCATTCAAAGATCTTA
ATCTTCTGGGTTTCCGTTTTCTCGAATGAAAAATGCAGGTCCGAGCAGTTAACTGGCGGGGGCA
CCATTAGCAAGTCACTTAGCATCTCTGGGGCCAGTCTGCAAAGCGAGGGGGCAGCCTTAATGTG
CCTCCAGCCTGAAGTCCTAGAATGAGCGCCCGGTGTCCCAAGCTGGGGCGCGCACCCCAGATCG
GAGGGCGCCGATGTACAGACAGCAAACTCACCCAGTCTAGTGCATGCCTTCTTAAACATCACGA
GACTCTAAGAAAAGGAAACTGAAAACGGGAAAGTCCCTCTCTCTAACCTGGCACTGCGTCGCTG
GCTTGGAGACAGGTGACGGTCCCTGCGGGCCTTGTCCTGATTGGCTGGGCACGCGTTTAATATA
AGTGGAGGCGTCGCGCTGGCGGGCATTCCTGAAGCTGACAGCATTCGGGCCGAGCGATCGGATC
CGCCACCATGCCCATCGTGCAGAACCTGCAGGGCCAGATGGTGCACCAGGCCATCAGCCCCCGG

ACCCTGAACGCCTGGGTGAAGGTGGTGGAGGAAAAGGCCTTCAGCCCCGAGGTGATCCCCATGT
TCAGCGCCCTGAGCGAGGGCGCCACACCCCAGGACCTGAACACCATGCTGAACACCGTGGGCGG
CCACCAGGCCGCCATGCAGATGCTGAAAGAGACAATCAACGAGGAAGCCGCCGAGTGGGACCGG
CTGCACCCTGTGCACGCCGGACCTATCGCCCCTGGCCAGATGCGGGAGCCCAGGGGCAGCGATA
TCGCCGGCACCACCTCCACACTGCAGGAACAGATCGGCTGGATGACCAACAACCCCCCCATCCC
CGTGGGCGAGATCTACAAGCGGTGGATCATCCTGGGCCTGAACAAGATCGTGCGGATGTACAGC
CCCACCTCCATCCTGGACATCCGGCAGGGCCCCAAAGAGCCCTTCCGGGACTACGTGGACCGGT
TCTACAAGACCCTGCGGGCCGAGCAGGCCAGCCAGGAAGTGAAGAACTGGATGACCGAGACACT
GCTGGTGCAGAACGCCAACCCCGACTGCAAGACCATCCTGAAGGCCCTGGGCCCTGCCGCCACC
CTGGAAGAGATGATGACCGCCTGCCAGGGCGTGGGCGGACCTGGCCACAAGGCCCGGGTGCTGG
CCGAGGCCATGAGCCAGGTGACCAACAGCGCCACCATCATGATGCAGCGGGGCAACTTCCGGAA
CCAGAGAAAGACCGTGAAGTGCTTCAACTGCGGCAAAGAGGGCCACATTGCCAAGAACTGCAGA
GCCCCCAGGAAGAAGGGCTGCTGGAAGTGTGGCAAAGAAGGGCACCAGATGAAGGACTGCACCG
AGCGGCAGGCCAACTTCCTGGGAAAAATTTGGCCTTCCCACAAGGGCAGGCCCGGCAACTTCCT
GCAGAGCAGGCCCGAGCCCACCGCCCCTCCCGAGGAAAGCTTCCGGTTCGGCGAGGAAACCACC
ACCCCCAGCCAGAAGCAGGAACCCATCGACAAAGAGCTGTACCCCCTGGCCTCCCTGAGAAGCC
TGTTCGGCAACGACAAGAAAAGCGTGACCGTGCTGGACGTGGGCGACGCCTACTTCAGCGTGCC
CCTGGATAAGGACTTCCGGAAGTACACCGCCTTCACCATCCCCAGCATCAACAACGAGACACCC
GGCATCAGATACCAGTACAACGTGCTGCCCCAGGGCTGGAAGGGCAGCCCCGCCATCTTCCAGA
GCAGCATGACCAAGATCCTGGAACCCTTCCGGAAGCAGAACCCCGACATCGTGATCTACCAGTA
CATGGACGACCTGTACGTGGGCAGCGACCTGGAAATCGGCCAGCACCGGACAGAGATCCTGAAA
GAACCCGTGCACGGCGTGTACGTGGGCTTCCCCGTGAGACCACAGGTGCCACTGAGACCCATGA
CCTACAAGGCCGCCGTGGACCTGAGCCACTTCCTGAAGGAGAAGGGCGGCCTGGAAGGCCTGAT
CTACAGCCAGAAGAGGCAGGACATCCTGGACCTGTGGGTGTACCACACCCAGGGCTACTTCCCC
GACTGGCAGAACTACACCCCTGGCCCTGGCATCCGCTACCCCCTGACCTTCGGCTGGTGCTTCA
AGCTGGTGCCCGTGGACCCCGAGAAGGAAGTGCTGGTGTGGAAGTTCGACAGCCGGCTGGCCTT
CCACCACATGGCCAGAGAGCTGCACCCCGAGTACTACTGATGACTCGAGAAATTCCCGATAATC
AACCTCTGGATTACAAAATTTGTGAAAGATTGACTGGTATTCTTAACTATGTTGCTCCTTTTAC
GCTATGTGGATACGCTGCTTTAATGCCTTTGTATCATGCTATTGCTTCCCGTATGGCTTTCATT
TTCTCCTCCTTGTATAAATCCTGGTTGCTGTCTCTTTATGAGGAGTTGTGGCCCGTTGTCAGGC
AACGTGGCGTGGTGTGCACTGTGTTTGCTGACGCAACCCCCACTGGTTGGGGCATTGCCACCAC
CTGTCAGCTCCTTTCCGGGACTTTCGCTTTCCCCCTCCCTATTGCCACGGCGGAACTCATCGCC

GCCTGCCTTGCCCGCTGCTGGACAGGGGCTCGGCTGTTGGGCACTGACAATTCCGTGGTGGTTG
TCGGTACCTTTAAGACCAATGACTTACAAGGCAGCTGTAGATCTTAGCCACTTTTTAAAAGAAA
AGGGGGGACTGGAAGGGCTAATTCACTCCCAACGAAGACAAGATCGTCGAGAGATGCTGCATAT
AAGCAGCTGCTTTTTGCTTGTACTGGGTCTCTCTGGTTAGACCAGATCTGAGCCTGGGAGCTCT
CTGGCTAACTAGGGAACCCACTGCTTAAGCCTCAATAAAGCTTGCCTTGAGTGCTTCAAGTAGT
GTGTGCCCGTCTGTTGTGTGACTCTGGTAACTAGAGATCCCTCAGACCCTTTTAGTCAGTGTGG
AAAATCTCTAGCAGT (SEQ ID NO:39).

Integrated proviral sequence (5'LTR to 3'-LTR) without WPRE:

TGTACTGGGTCTCTCTGGTTAGACCAGATCTGAGCCTGGGAGCTCTCTGGCTAACTAGG
GAACCCACTGCTTAAGCCTCAATAAAGCTTGCCTTGAGTGCTTCAAGTAGTGTGTGCCCGTCTG
TTGTGTGACTCTGGTAACTAGAGATCCCTCAGACCCTTTTAGTCAGTGTGGAAAATCTCTAGCA
GTGGCGCCCGAACAGGGACTTGAAAGCGAAAGGAAACCAGAGGAGCTCTCTCGACGCAGGACT
CGGCTTGCTGAAGCGCGCACGGCAAGAGGCGAGGGGCGGCGACTGGTGAGTACGCCAAAAATTT
TGACTAGCGGAGGCTAGAAGGAGAGAGATGGGTGCGAGAGCGTCAGTATTAAGCGGGGGAGAAT
TAGATCGCGATGGGAAAAAATTCGGTTAAGGCCAGGGGGAAAGAAAAAATATAAATTAAAACAT
ATAGTATGGGCAAGCAGGGAGCTAGAACGATTCGCAGTTAATCCTGGCCTGTTAGAAACATCAG
AAGGCTGTAGACAAATACTGGGACAGCTACAACCATCCCTTCAGACAGGATCAGAAGAACTTAG
ATCATTATATAATACAGTAGCAACCCTCTATTGTGTGCATCAAAGGATAGAGATAAAAGACACC
AAGGAAGCTTTAGACAAGATAGAGGAAGAGCAAAACAAAAGTAAGACCACCGCACAGCAAGCGG
CCGCTGATCTTCAGACCTGGAGGAGGAGATATGAGGGACAATTGGAGAAGTGAATTATATAAAT
ATAAAGTAGTAAAAATTGAACCATTAGGAGTAGCACCCACCAAGGCAAAGAGAAGAGTGGTGCA
GAGAGAAAAAAGAGCAGTGGGAATAGGAGCTTTGTTCCTTGGGTTCTTGGGAGCAGCAGGAAGC
ACTATGGGCGCAGCGTCAATGACGCTGACGGTACAGGCCAGACAATTATTGTCTGGTATAGTGC
AGCAGCAGAACAATTTGCTGAGGGCTATTGAGGCGCAACAGCATCTGTTGCAACTCACAGTCTG
GGGCATCAAGCAGCTCCAGGCAAGAATCCTGGCTGTGGAAAGATACCTAAAGGATCAACAGCTC
CTGGGGATTTGGGGTTGCTCTGGAAAACTCATTTGCACCACTGCTGTGCCTTGGAATGCTAGTT
GGAGTAATAAATCTCTGGAACAGATTTGGAATCACACGACCTGGATGGAGTGGGACAGAGAAAT
TAACAATTACACAAGCTTAATACACTCCTTAATTGAAGAATCGCAAAACCAGCAAGAAAAGAAT
GAACAAGAATTATTGGAATTAGATAAATGGGCAAGTTTGTGGAATTGGTTTAACATAACAAATT
GGCTGTGGTATATAAAATTATTCATAATGATAGTAGGAGGCTTGGTAGGTTTAAGAATAGTTTT

TGCTGTACTTTCTATAGTGAATAGAGTTAGGCAGGGATATTCACCATTATCGTTTCAGACCCAC
CTCCCAACCCCGAGGGGACCCGACAGGCCCGAAGGAATAGAAGAAGAAGGTGGAGAGAGAGACA
GAGACAGATCCATTCGATTAGTGAACGGATCTCGACGGTATCGCCGAATTCACAAATGGCAGTA
TTCATCCACAATTTTAAAAGAAAAGGGGGGATTGGGGGGTACAGTGCAGGGGAAAGAATAGTAG
ACATAATAGCAACAGACATACAAACTAAAGAATTACAAAAACAAATTACAAAAATTCAAAATTT
TCGGGTTTATTACAGGGACAGCAGAGATCCACTTTGGCTGATACGCGCCGAGAAACCCTGCAGG
GAATTCCCCAGCTGTAGTTATAAACAGAAGTTCTCCTTCTGCTAGGTAGCATTCAAAGATCTTA
ATCTTCTGGGTTTCCGTTTTCTCGAATGAAAAATGCAGGTCCGAGCAGTTAACTGGCGGGGGCA
CCATTAGCAAGTCACTTAGCATCTCTGGGGCCAGTCTGCAAAGCGAGGGGGCAGCCTTAATGTG
CCTCCAGCCTGAAGTCCTAGAATGAGCGCCCGGTGTCCCAAGCTGGGGCGCGCACCCCAGATCG
GAGGGCGCCGATGTACAGACAGCAAACTCACCCAGTCTAGTGCATGCCTTCTTAAACATCACGA
GACTCTAAGAAAAGGAAACTGAAAACGGGAAAGTCCCTCTCTCTAACCTGGCACTGCGTCGCTG
GCTTGGAGACAGGTGACGGTCCCTGCGGGCCTTGTCCTGATTGGCTGGGCACGCGTTTAATATA
AGTGGAGGCGTCGCGCTGGCGGGCATTCCTGAAGCTGACAGCATTCGGGCCGAGCGATCGGATC
CGCCACCATGCCCATCGTGCAGAACCTGCAGGGCCAGATGGTGCACCAGGCCATCAGCCCCCGG
ACCCTGAACGCCTGGGTGAAGGTGGTGGAGGAAAAGGCCTTCAGCCCCGAGGTGATCCCCATGT
TCAGCGCCCTGAGCGAGGGCGCCACACCCCAGGACCTGAACACCATGCTGAACACCGTGGGCGG
CCACCAGGCCGCCATGCAGATGCTGAAAGAGACAATCAACGAGGAAGCCGCCGAGTGGGACCGG
CTGCACCCTGTGCACGCCGGACCTATCGCCCCTGGCCAGATGCGGGAGCCCAGGGGCAGCGATA
TCGCCGGCACCACCTCCACACTGCAGGAACAGATCGGCTGGATGACCAACAACCCCCCCATCCC
CGTGGGCGAGATCTACAAGCGGTGGATCATCCTGGGCCTGAACAAGATCGTGCGGATGTACAGC
CCCACCTCCATCCTGGACATCCGGCAGGGCCCCAAAGAGCCCTTCCGGGACTACGTGGACCGGT
TCTACAAGACCCTGCGGGCCGAGCAGGCCAGCCAGGAAGTGAAGAACTGGATGACCGAGACACT
GCTGGTGCAGAACGCCAACCCCGACTGCAAGACCATCCTGAAGGCCCTGGGCCCTGCCGCCACC
CTGGAAGAGATGATGACCGCCTGCCAGGGCGTGGGCGGACCTGGCCACAAGGCCCGGGTGCTGG
CCGAGGCCATGAGCCAGGTGACCAACAGCGCCACCATCATGATGCAGCGGGGCAACTTCCGGAA
CCAGAGAAAGACCGTGAAGTGCTTCAACTGCGGCAAAGAGGGCCACATTGCCAAGAACTGCAGA
GCCCCCAGGAAGAAGGGCTGCTGGAAGTGTGGCAAAGAAGGGCACCAGATGAAGGACTGCACCG
AGCGGCAGGCCAACTTCCTGGGAAAAATTTGGCCTTCCCACAAGGGCAGGCCCGGCAACTTCCT
GCAGAGCAGGCCCGAGCCCACCGCCCCTCCCGAGGAAAGCTTCCGGTTCGGCGAGGAAACCACC
ACCCCCAGCCAGAAGCAGGAACCCATCGACAAAGAGCTGTACCCCCTGGCCTCCCTGAGAAGCC
TGTTCGGCAACGACAAGAAAAGCGTGACCGTGCTGGACGTGGGCGACGCCTACTTCAGCGTGCC

CCTGGATAAGGACTTCCGGAAGTACACCGCCTTCACCATCCCCAGCATCAACAACGAGACACCC
GGCATCAGATACCAGTACAACGTGCTGCCCCAGGGCTGGAAGGGCAGCCCCGCCATCTTCCAGA
GCAGCATGACCAAGATCCTGGAACCCTTCCGGAAGCAGAACCCCGACATCGTGATCTACCAGTA
CATGGACGACCTGTACGTGGGCAGCGACCTGGAAATCGGCCAGCACCGGACAGAGATCCTGAAA
GAACCCGTGCACGGCGTGTACGTGGGCTTCCCCGTGAGACCACAGGTGCCACTGAGACCCATGA
CCTACAAGGCCGCCGTGGACCTGAGCCACTTCCTGAAGGAGAAGGGCGGCCTGGAAGGCCTGAT
CTACAGCCAGAAGAGGCAGGACATCCTGGACCTGTGGGTGTACCACACCCAGGGCTACTTCCCC
GACTGGCAGAACTACACCCCTGGCCCTGGCATCCGCTACCCCCTGACCTTCGGCTGGTGCTTCA
AGCTGGTGCCCGTGGACCCCGAGAAGGAAGTGCTGGTGTGGAAGTTCGACAGCCGGCTGGCCTT
CCACCACATGGCCAGAGAGCTGCACCCCGAGTACTACTGATGACTCGAGAGGTACCTTTAAGAC
CAATGACTTACAAGGCAGCTGTAGATCTTAGCCACTTTTTAAAAGAAAAGGGGGGACTGGAAGG
GCTAATTCACTCCCAACGAAGACAAGATCGTCGAGAGATGCTGCATATAAGCAGCTGCTTTTTG
CTTGTACTGGGTCTCTCTGGTTAGACCAGATCTGAGCCTGGGAGCTCTCTGGCTAACTAGGGAA
CCCACTGCTTAAGCCTCAATAAAGCTTGCCTTGAGTGCTTCAAGTAGTGTGTGCCCGTCTGTTG
TGTGACTCTGGTAACTAGAGATCCCTCAGACCCTTTTAGTCAGTGTGGAAAATCTCTAGCAGT
(SEQ ID NO:40).

In some embodiments, the nucleic acid comprises a DNA or RNA sequence recited herein, preferably selected from SEQ ID NOs 31-40.

In some embodiments, the nucleic acid is a DNA sequence of any of SEQ ID NOs 37-40 that encodes an RNA sequence, preferably of any of SEQ ID NOs 31-36.

The invention encompasses an isolated nucleic acid of the invention inserted into a vector.

The nucleic acid can be purified. Preferably, the purified nucleic acid is more than 50%, 75%, 85%, 90%, 95%, 97%, 98%, or 99% pure. Within the context of this invention, a purified nucleic acid that is at least 50% pure means a purified nucleic acid sample containing less than 50% other nucleic acids not encoding SEQ ID NO:30. For example, a sample of a plasmid encoding SEQ ID NO:30 can be at least 99% pure if it contains less than 1% contaminating bacterial DNA.

### VECTORS

The invention encompasses vectors encoding at least one HIV-1 antigen, preferably an HIV-1 Nef, Pol, NC, or CA antigen or immunogenic polypeptide, most preferably comprising the amino acid sequence of SEQ ID NO:30. Preferred vectors comprise or encode the nucleic acid sequence of any of SEQ ID NOs 31-40.

The vector can be an expression vector. The vector can be a plasmid vector. Preferably, the vector is a lentiviral vector.

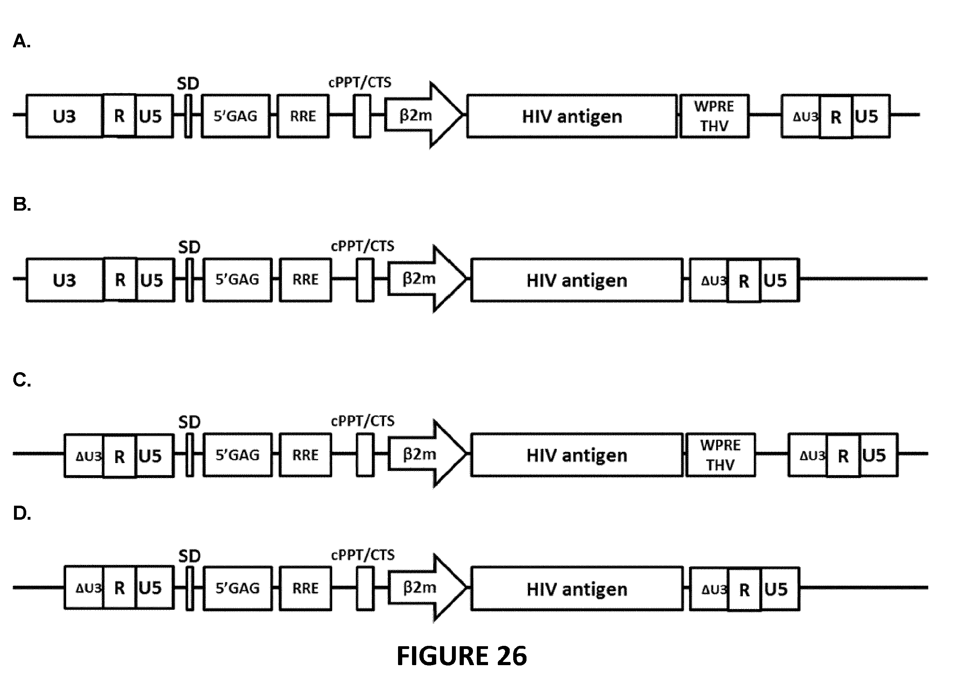

Preferably, the vector has the order, 5' to 3', of LTR, Splice donor, 5' Gag (including ψ), RRE, cPPT/CTS, promoter (preferably β2m), HIV antigen (comprising SEQ ID NO:30), LTR (ΔU3). More preferably, the vector has a structure shown in Figure 26. Most preferably, the vector comprises the nucleic acid sequence of SEQ ID NO:37 or SEQ ID NO:38.

The invention encompasses methods for generating a vector comprising inserting an nucleic acid encoding SEQ ID NO:30 into a vector, preferably a lentiviral vector.

### ISOLATED CELLS

The invention encompasses cells comprising vectors and lentiviral vector particles encoding at least one HIV-1 antigen, preferably an HIV-1 Nef, Pol, NC, or CA antigen or immunogenic polypeptide, most preferably comprising the amino acid sequence of SEQ ID NO:30. Preferably, the cells comprise or encode the nucleic acid sequence of any of SEQ ID NOs 31-40.

In one embodiment, the cell contains the vector integrated into the cellular genome. In one embodiment, the cell contains the vector transiently expressing the HIV-1 antigen, preferably an HIV-1 Nef, Pol, NC, or CA antigen or immunogenic polypeptide, most preferably comprising the amino acid sequence of SEQ ID NO:30. In one embodiment, the cell produces lentiviral vector particles encoding the HIV-1 antigen, preferably an HIV-1 Nef, Pol, NC, or CA antigen or immunogenic polypeptide, most preferably comprising the amino acid sequence of SEQ ID NO:30.

In various embodiments, the invention encompasses a cell line, a population of cells, or a cell culture comprising vectors and lentiviral vector particles encoding the HIV-1 antigen, preferably an HIV-1 Nef, Pol, NC, or CA antigen or immunogenic polypeptide, most preferably comprising the amino acid sequence of SEQ ID NO:30.

### LENTIVIRAL VECTOR

Within the context of this invention, a "lentiviral vector" means a non-replicating vector for the transduction of a host cell with a transgene comprising *cis-*acting lentiviral RNA or DNA sequences, and requiring lentiviral proteins (e.g., Gag, Pol, and/or Env) that are provided *in trans.* The lentiviral vector lacks expression of functional Gag, Pol, and Env proteins. The lentiviral vector may be present in the form of an RNA or DNA molecule, depending on the stage of production or development of said retroviral vectors.

The lentiviral vector can be in the form of a recombinant DNA molecule, such as a plasmid. The lentiviral vector can be in the form of a lentiviral particle vector, such as an RNA molecule(s) within a complex of lentiviral and other proteins. Typically, lentiviral particle vectors, which correspond to modified or recombinant lentivirus particles, comprise a genome which is composed of two copies of single-stranded RNA. These RNA sequences can be obtained by transcription from a double-stranded DNA sequence inserted into a host cell genome (proviral vector DNA) or can be obtained from the transient expression of plasmid DNA (plasmid vector DNA) in a transformed host cell.

Preferably the lentiviral vector particles have the capacity for integration. As such, they contain a functional integrase protein. Non-integrating vector particles have one or more mutations that eliminate most or all of the integrating capacity of the lentiviral vector particles. For, example, a non-integrating vector particle can contain mutations in the integrase encoded by the lentiviral pol gene that cause a reduction in integrating capacity. In contrast, an integrating vector particle comprises a functional integrase protein that does not contain any mutations that eliminate most or all of the integrating capacity of the lentiviral vector particles.

Lentiviral vectors derive from lentiviruses, in particular human immunodeficiency virus (HIV-1 or HIV-2), simian immunodeficiency virus (SIV), equine infectious encephalitis virus (EIAV), caprine arthritis encephalitis virus (CAEV), bovine immunodeficiency virus (BIV) and feline immunodeficiency virus (FIV), which are modified to remove genetic determinants involved in pathogenicity and introduce new determinants useful for obtaining therapeutic effects.

Such vectors are based on the separation of the *cis-* and *trans-acting* sequences. In order to generate replication-defective vectors, the trans-acting sequences (e.g., *gag, pol, tat, rev,* and *env* genes) can be deleted and replaced by an expression cassette encoding a transgene.

Efficient integration and replication in non-dividing cells generally requires the presence of two *cis*-acting sequences at the center of the lentiviral genome, the central polypurine tract (cPPT) and the central termination sequence (CTS). These lead to the formation of a triple-stranded DNA structure called the central DNA "flap", which acts as a signal for uncoating of the pre-integration complex at the nuclear pore and efficient importation of the expression cassette into the nucleus of non-dividing cells, such as dendritic cells.

In one embodiment, the invention encompasses a lentiviral vector comprising a central polypurine tract and central termination sequence referred to as cPPT/CTS sequence as described, in particular, in the European patent application EP 2 169 073.

Further sequences are usually present in *cis,* such as the long terminal repeats (LTRs) that are involved in integration of the vector proviral DNA sequence into a host cell genome. Vectors may be obtained by mutating the LTR sequences, for instance, in domain U3 of said LTR (ΔU3) (Miyoshi H et al, 1998, J Virol. 72(10):8150-7; Zufferey et al., 1998, J Virol 72(12):9873-80).

Preferably, the vector does not contain an enhancer. In one embodiment, the invention encompasses a lentiviral vector comprising LTR sequences, preferably with a mutated U3 region (ΔU3) removing promoter and enhancer sequences in the 3' LTR.

The packaging sequence ψ (psi) can also be incorporated to help the encapsidation of the polynucleotide sequence into the vector particles (Kessler et al., 2007, Leukemia, 21(9):1859-74; Paschen et al., 2004, Cancer Immunol Immunother 12(6):196-203).

In one embodiment, the invention encompasses a lentiviral vector comprising a lentiviral packaging sequence ψ (psi).

Further additional functional sequences, such as a transport RNA-binding site or primer binding site (PBS) or a Woodchuck PostTranscriptional Regulatory Element (WPRE), can also be advantageously included in the lentiviral vector polynucleotide sequence of the present invention, to obtain a more stable expression of the transgene in vivo.

In one embodiment, the invention encompasses a lentiviral vector comprising a PBS. In one embodiment, the invention encompasses a lentiviral vector comprising a WPRE and/or an IRES.

Thus, in a preferred embodiment, the lentiviral vector comprises at least one cPPT/CTS sequence, one ψ sequence, one (preferably 2) LTR sequence, and an expression cassette including a transgene under the transcriptional control of a β2m or class I MHC promoter.

### PROMOTER

In various embodiments, the promoter drives high expression in antigen presenting cells, including dendritic cells, to induce maximal immune responses. Preferably, the promoter drives expression in other transduced cell types sufficient for elimination by the induced immune response. Most preferably, the promoter lacks an enhancer element to avoid insertional effects.

Most preferably, the promoter is not a CMV promoter/enhancer. Preferably, the promoter is not a dectin-2 or MHCII promoter.

In various embodiments, the lentiviral vector comprises a β2m or MHC class I promoter. Preferably, the MHC class I promoter is an HLA-A2 promoter, an HLA-B7 promoter, an HLA-Cw5 promoter, an HLA-F, or an HLA-E promoter. In various embodiments, the promoter sequence comprises a polynucleotide sequence that shares more than 90%, preferably more than 95%, more preferably more than 99% identity with the promoter sequence of SEQ ID NO:1, SEQ ID NO:2, SEQ ID NO:3, SEQ ID NO:4, SEQ ID NO:5, or SEQ ID NO:6.

The invention encompasses dendritic cell-specific promoters. A "dendritic cell-specific promoter" is one in which expression of the promoter in BDCA+ dendritic cells is higher than the expression in kidney, smooth muscle, liver, and heart cells. Preferably, expression of the promoter in BDCA+ dendritic cells is at least 2X, 3X, or 4X higher than the expression in kidney, smooth muscle, liver, and/or heart cells. Whether a promoter is "(2X, 3X, or 4X) higher than the expression in kidney, smooth muscle, liver, and/or heart cells" can be determined by reference to the data sets at http://biogps.org (*see, e.g.,* Fig. 24), which are hereby incorporated by reference or by the use of the Affimetrix probes, chips, and methods used to generate these data sets (particularly HG-U133 set). The β2m, HLA-A2, HLA-B7, HLA-Cw5, HLA-F, and HLA-E promoters are "dendritic cell-specific promoters." The EF1 a promoter is not. Thus, preferably the promoter is not an EF1 a promoter.

Preferably, the promoter drives expression in other transduced cell types, such as kidney, smooth muscle, liver, and/or heart cells, and skeletal muscle. Preferably, the expression of the promoter in BDCA+ dendritic cells is 12-100 times the expression of that promoter in skeletal muscle cells. Whether "the expression of the promoter in BDCA+ dendritic cells is 12-100 times the expression of that promoter in skeletal muscle cells" can be determine by reference to the data sets at http://biogps.org (*see, e.g.,* Fig. 24), which are hereby incorporated by reference or by the use of the Affimetrix probes, chips, and methods used to generate these data sets (particularly HG-U133 set). Most preferably, the expression of the promoter in BDCA+ dendritic cells is 12-100 times the expression of that promoter in skeletal muscle cells and expression of the promoter in BDCA+ dendritic cells is higher than the expression in kidney, smooth muscle, liver, and heart cells. The β2m, HLA-A2, HLA-B7, HLA-Cw5, HLA-F, and HLA-E promoters are promoters where expression in BDCA+ dendritic cells is 12-100 times the expression of that promoter in skeletal muscle cells. The HLA-DRα promoter and UBC promoter are not. Thus, preferably the promoter is not an HLA-DRα (MHCII) promoter or UBC (Ubiquitin) promoter.

In some embodiments, the expression of the promoter in BDCA+ dendritic cells is at least 10, 12, 15, 20, 25, 30, 35, 40, 50, or 60 times the expression of that promoter in skeletal muscle cells.

In one embodiment, the invention encompasses lentiviral vector particles comprising a lentiviral vector that comprises a dendritic cell-specific promoter directing expression of a microbial or tumor antigen, wherein the lentiviral vector particles exhibit higher expression of the antigen in BDCM cells than in HEK 293 T cells. Preferably, the lentiviral vector encodes at least one HIV-1 antigen, preferably an HIV-1 Nef, Pol, NC, or CA antigen or immunogenic polypeptide, most preferably comprising the amino acid sequence of SEQ ID NO:30.

In various embodiments, the lentiviral vector comprising the promoter induces a greater CTL response *in vivo* against the encoded immunogenic polypeptide than a vector in which the transgene sequence is under the transcriptional control of a CMV promoter. Within the context of this invention, whether a vector "induces a greater CTL response *in vivo* against the encoded immunogenic polypeptide than a vector in which the transgene sequence is under the transcriptional control of a CMV promoter" can be determined using the assay set forth in the examples. Other assays that provide similar results can also be used.

In various embodiments, the lentiviral vector comprising the promoter induces a greater CTL response *in vivo* against the encoded immunogenic polypeptide than a vector in which the transgene sequence is under the transcriptional control of an EF1 a promoter. Preferably, the CTL response with the promoter is at least 2-fold or 3-fold higher than with the EF1α promoter. Within the context of this invention, whether a vector "induces a greater CTL response *in vivo* against the encoded immunogenic polypeptide than a vector in which the transgene sequence is under the transcriptional control of an EF1 a promoter" can be determined using the assay set forth in the examples. Other assays that provide similar results can also be used.

In various embodiments, the lentiviral vector comprising the promoter induces a greater CTL response *in vivo* against the encoded immunogenic polypeptide than a vector in which the transgene sequence is under the transcriptional control of an Ubiquitin promoter. Within the context of this invention, whether a vector "induces a greater CTL response *in vivo* against the encoded immunogenic polypeptide than a vector in which the transgene sequence is under the transcriptional control of an Ubiquitin promoter" can be determined using the assay set forth in the examples. Other assays that provide similar results can also be used.

The invention encompasses lentiviral vectors containing a promoter that does not contain an enhancer.

The invention encompasses the insertion of an MHC Class I (MHCI) or β2 microglobulin(β2m) promoter into a lentiviral vector. As used herein, an "MHC Class I (MHCI) promoter" or "β2 microglobulin promoter" includes a naturally occurring or synthetic MHC Class I promoter or β2 microglobulin promoter. The term "MHC Class I promoter" does not include a β2m promoter.

In one embodiment, the lentiviral vector particles comprising the promoter exhibit higher expression of the antigen in BDCM cells than in HEK 293 T cells.

The promoter can be a naturally occurring promoter. Examples of naturally occurring promoters are the human β2m, HLA-A2, HLA-B7, HLA-Cw5, HLA-E, HLA-F gene promoters. These naturally occurring MHCI promoters are generally cloned or reproduced from the promoter region of a gene encoding the MHC class I protein, or referred to as putatively encoding such proteins in genome databases (ex: NCBI polynucleotide database http://www.ncbi.nlm.nih.gov/guide/dna-rna). Both β2m and class I MHC proteins enter the Major Histocompatibility Complex (MHC).

The proteins encoded by these genes are found in almost all cell types. MHCI proteins are generally present at the surface of the membrane of leucocytes, where they are associated with the β2-microglobulin (β2m). The role of these associated proteins is to present peptides from endogenous sources to CD8+ T cells. They thus play a central role to the generation of the antigen-specific immune response. Because MHC class I proteins have been widely studied and described for many years, their genes are well characterized and detectable using sequence comparison tools, such as the BLAST method (Altschul, S.F. et a/. (1990). Basic local alignment search tool. J. Mol. Biol. 215(3):403-410).

MHC class I promoters share the ability to be strongly activated in antigen presenting cells, including dendritic cells, as well as, to lower intensity, in the majority of the other human body tissues.

The promoters of the invention can contain further regulatory elements, such as one or more Sp1 and ETs binding sites. In a preferred embodiment, the MHC class I promoter contains 2 Sp1 binding sites and 1 Ets binding site. In other embodiments, Ap1 and/or Ap2 sites are further contained in the promoter.

Preferred promoters are naturally occurring human β2m, HLA-A2, HLA-B7, HLA-Cw5, HLA-E and HLA-F promoters.

Promoters can also be synthetic. Synthetic promoters include promoters that are synthesized using molecular biological techniques to assemble the individual components of a promoter or that are derived from naturally occurring promoters using molecular biological techniques.

In various embodiments, the synthetic promoter comprises a polynucleotide sequence that shares more than 90%, preferably more than 95%, more preferably more than 99% identity, or 100% with the promoter sequence of a β2m or MHC class I gene promoter (e.g., SEQ ID NOs: 1-6 and 19).

The transcription of MHC I class genes are usually mediated by two major regulatory elements: Interferon stimulated response element (ISRE) and the SXY module (encompassing the W/S, X1 X2/Site α and Y/enhancer B regulatory elements) (see figure 1). *See also* Van den Elsen, Immunogenetics (1998) 48:208-211.

These regulatory promoter elements are localized in a region extending approximately from nucleotides -220 to -95 upstream of the transcription initiation site. They mediate tissue-specific and cytokine-induced transcription of MHC class I genes.

The ISRE of MHC class I gene promoters generally contains binding sites for interferon regulatory factor (IRF) family members. It is thus a property of MHC class I promoters to bind to interferon regulatory factor (IRF) family members. This may be verified, for example, by gel shift assays.

Another regulatory element, the enhancer A (containing binding sites for nuclear transcription factor κB (NF-κB)) is present in most cases. It is thus a property of MHC class I promoters to bind to nuclear transcription factor κB (NF-κB). This may be verified, for example, by gel shift assays.

In addition to ISRE, MHC class I promoters generally share another set of conserved upstream sequence motifs, consisting of three regulatory elements: the S or W box, the X1/CREX2 boxes or site α, and the Y box or enhancer B, which together are termed the SXY module. This SXY module is generally cooperatively bound by a multiprotein complex containing regulatory factor X (RFX; consisting of RFX5, RFXB/ANK and RFXAP), cAMP response element binding protein (CREB)/activating transcription factor (ATF), and nuclear factor Y (NFY), which acts as an enhanceosome driving transactivation of these genes. It is thus a property of MHC class I promoters to bind to these factors. This may be verified, for example, by gel shift assays.

In contrast, MHC class II promoters do not display enhancer A nor ISRE elements (Van den Elsen, P.J. et al, 1998, Immunogenetics. 48:208-221). Furthermore, RFX and CIITA in MHC class II gene regulation have been found of crucial importance as illustrated by studies with cell lines established from patients with the bare lymphocyte syndrome (BLS), a severe combined immunodeficiency due to mutations in one of the RFX subunits or CIITA (DeSandro, A. et al., 1999, Am J Hum Genet, 65:279-286). Also, lack of either CIITA or one of the RFX subunits affects the functioning and assembly of the MHC enhanceosome, respectively, leading to a lack of MHC class II and reduced levels of MHC class I transcription (Van den Elsen, P.J. et al. 2004, Current Opinion in Immunology, 16:67-75).

In one embodiment, the invention encompasses a method comprising inserting a promoter of the invention, particularly a β2m or MHC class I promoter, into a lentiviral vector to direct expression of a transgene, which preferably encodes an immunogenic polypeptide, most preferably encoding a microbial or a tumor antigen or therapeutic protein. The method can further comprise inserting any of the other nucleic acid elements mentioned herein, such as a DNA flap sequence.

### TRANSGENE

Within the context of this invention, a "transgene" is a nucleic acid sequence within a lentiviral vector that is not normally present in a cell to be transduced with the lentiviral vector. The lentiviral vector serves to introduce this sequence into the transduced cell. The term "transgene" does not include those sequences of the vector that facilitate transduction of the transgene. The transgene may be a nucleic acid sequence from another organism. Alternatively, the transgene may be a nucleic acid sequence from the same organism, but having different regulatory sequences controlling its expression. The transgene may be a sense or antisense nucleic acid molecule. According to a preferred embodiment of the invention, the transgene sequence encodes an antigen or immunogenic polypeptide.

Preferably, the antigen or immunogenic polypeptide is a microbial or tumor antigen or immunogenic polypeptide. More preferably, the antigen or immunogenic polypeptide is viral, bacterial, or fungal. In one embodiment, the antigen or immunogenic polypeptide is a tumor antigen or immunogenic polypeptide. In one embodiment, the antigen or immunogenic polypeptide is an allergen. In one embodiment, the antigen or immunogenic polypeptide is an HIV-1 antigen or immunogenic polypeptide, preferably an HIV-1 Nef, Pol, NC, or CA antigen or immunogenic polypeptide.

The antigen or immunogenic polypeptide preferably comprises one or several epitope(s) from agents of infectious diseases, for example a human immunodeficiency type 1 virus, such as antigen(s) from Gag, Pol, and/or Nef proteins.

Several epitopes forming a polyepitope may also be encoded by the transgene of the invention.

In a particular embodiment, such epitope is derived from target antigens identified in tumors (i.e., tumor antigen), and can be chosen in such a way that a cell-mediated immune response is obtained against it. Target antigens are well documented in the art, which can be selected with respect to several types of tumors and in particular in melanomas or in carcinomas, including renal carcinomas, bladder carcinomas, colon carcinomas, lung carcinomas, breast cancers, leukemias, myelomas and lymphomas.

Both B and T cell-based immune responses can be induced by the same antigen.

### LENTIVIRAL VECTOR PARTICLES

The present invention provides a method for producing a lentiviral vector particle. A lentiviral vector particle (or lentiviral particle vector) comprises a lentiviral vector in association with viral proteins. The vector is preferably an integrating vector.

In one embodiment, the lentiviral vector particles encode an HIV-1 antigen, preferably an HIV-1 Nef, Pol, NC, or CA antigen or immunogenic polypeptide, most preferably comprising the amino acid sequence of SEQ ID NO:30. Preferably, the lentiviral vector particles comprise the nucleic acid sequence of any of SEQ ID NOs 31-36.

In one embodiment, the lentiviral vector particle comprises HIV-1 Gag and Pol proteins. Preferably, the lentiviral vector particle comprises subtype D, especially HIV-1_{NDK}, Gag and Pol proteins.

According to one embodiment of this method, the lentivector particles are obtained in a host cell transformed with a DNA plasmid.

Such a DNA plasmid can comprise:
- bacterial origin of replication (ex: pUC ori);
- antibiotic resistance gene (ex: KanR) for selection; and more particularly:
- a lentiviral vector comprising at least one transgene transcriptionally linked to a MHC class I promoter.

Such a method allows producing a recombinant vector particle according to the invention, comprising the following steps of:
i) transfecting a suitable host cell with a lentiviral vector;
ii) transfecting said host cell with a packaging plasmid vector, containing viral DNA sequences encoding at least structural and polymerase (+ integrase) activities of a retrovirus (preferably lentivirus); Such packaging plasmids are described in the art (Dull et al., 1998, J Virol, 72(11):8463-71; Zufferey et al., 1998, J Virol 72(12):9873-80).
iii) culturing said transfected host cell in order to obtain expression and packaging of said lentiviral vector into lentiviral vector particles; and
iv) harvesting the lentiviral vector particles resulting from the expression and packaging of step iii) in said cultured host cells.

For different reasons, it may be helpful to pseudotype the obtained retroviral particles, i.e. to add or replace specific particle envelope proteins. For instance, this may be advantageous to have different envelope proteins in order to distinguish the recombinant particle from natural particles or from other recombinant particles. In matter of vaccination strategy, pseudotyped particle vectors are more likely to escape the immune system, when this latter already developed immunity against lentiviruses. This is particularly helpful when successive injections of similar particle vectors are required for immunizing a patient against a disease.

In order to pseudotype the retroviral particles of the invention, the host cell can be further transfected with one or several envelope DNA plasmid(s) encoding viral envelope protein(s), preferably a VSV-G envelope protein, most preferably a VSV Indiana, New Jersey, or Cocal VSV-G envelope protein.

An appropriate host cell is preferably a human cultured cell line as, for example, a HEK cell line.

Alternatively, the method for producing the vector particle is carried out in a host cell, which genome has been stably transformed with one or more of the following components: a lentiviral vector DNA sequence, the packaging genes, and the envelope gene. Such a DNA sequence may be regarded as being similar to a proviral vector according to the invention, comprising an additional promoter to allow the transcription of the vector sequence and improve the particle production rate.

In a preferred embodiment, the host cell is further modified to be able to produce viral particle in a culture medium in a continuous manner, without the entire cells swelling or dying. One may refer to Strang et al., 2005, J Virol 79(3):1165-71; Relander et al., 2005, Mol Ther 11 (3):452-9; Stewart et al., 2009, Gene Ther, 16(6):805-14; and Stuart et al., 2011, Hum gene Ther.*,* with respect to such techniques for producing viral particles.

An object of the present invention consists of a host cell transformed with a lentiviral particle vector.

The lentiviral particle vectors can comprise the following elements, as previously defined:
- cPPT/CTS polynucleotide sequence; and
- a transgene sequence under control of a promoter of the invention, and optionally one of the additional elements described above.

Preferably, the lentiviral vector comprises a β2m or MHC class I promoter.

Preferably, the lentivector particles are in a dose of, at least, 10⁶, 2 x 10⁶, 5x 10⁶, 10⁷, 2 x 10⁷ 5 x 10⁷ 10⁸, 2 x 10⁸, 5 x 10⁸, or 10⁹ TU.

### METHODS FOR EXPRESSING A TRANSGENE IN A CELL

The present invention encompasses methods for expressing a transgene in a cell, preferably a non-dividing cell. The method comprises transducing a cell with a lentiviral vector or lentiviral particle vector of the invention under conditions that allow the expression of the transgene. Preferably, the vector encodes an HIV-1 antigen, preferably an HIV-1 Nef, Pol, NC, or CA antigen or immunogenic polypeptide, most preferably comprising the amino acid sequence of SEQ ID NO:30.

Preferably, the lentiviral vector or lentiviral particle vector comprises or encodes the nucleic acid sequence of any of SEQ ID NOs 31-40.

The cells are preferably mammalian cells, particularly human cells. Particularly preferred are human non-dividing cells.

Preferably, the lentiviral vector comprises a β2m or MHC class I promoter.

The transgene preferably encodes an immunogenic polypeptide, preferably an HIV-1 antigen, more preferably an HIV-1 Nef, Pol, NC, or CA antigen or immunogenic polypeptide, most preferably comprising the amino acid sequence of SEQ ID NO:30. The method can further comprise harvesting or isolating the polypeptide.

The lentiviral vector or lentiviral particle vector preferably comprises a promoter of the invention.

In one embodiment, the invention encompasses a method for expressing a transgene comprising inserting promoter of the invention into a lentiviral vector such that it direct the expression of a transgene and transducing a cell with the vector containing the promoter.

### USE OF LENTIVIRAL VECTORS

The present invention further relates to the use of the lentiviral vectors according to the invention, especially in the form of lentiviral vector particles, for the preparation of immunogenic and/or therapeutic compositions and/or vaccines which are capable of inducing or contributing to the occurrence or improvement of an immunological reaction against a polypeptide comprising an HIV-1 antigen, preferably an HIV-1 Nef, Pol, NC, or CA antigen or immunogenic polypeptide, most preferably comprising the amino acid sequence of SEQ ID NO:30.

The invention encompasses methods for expressing a protein comprising the amino acid sequence SEQ ID NO:30. Preferably, the method comprises administering a nucleic acid encoding SEQ ID NO:30 into a cell under conditions that allow expression of the protein. The nucleic acid can be any of the vectors of the invention, preferably a lentiviral vector. In a preferred embodiment, the method comprises contacting a cell with a lentiviral vector comprising a nucleic acid encoding SEQ ID NO: 30 under conditions that allow entry and reverse transcription of the lentiviral vector and expression of the protein.

### METHODS OF ADMINISTRATION

The invention encompasses methods of administration of a lentiviral vector (or "lentivector") to a human. Preferably, the lentivector contains a promoter that drives high expression of an antigen in antigen presenting cells, including dendritic cells, and drives expression in other transduced cell types sufficient for elimination by the induced immune response. Most preferably, the promoter lacks an enhancer element to avoid insertional effects.

Preferably, the administration is intramuscular. In one embodiment, the lentivector is injected into the muscle using a needle.

Preferably, the antigen is a microbial antigen or tumor antigen. Preferably, the antigen is an HIV-1 antigen, more preferably an HIV-1 Nef, Pol, NC, or CA antigen or immunogenic polypeptide, most preferably comprising the amino acid sequence of SEQ ID NO:30.

Preferably, the lentivector particle is an integrating lentivector particle, comprising a functional integrase protein.

Most preferably, the administration eliminates at least 95%, 99%, 99.9%, or 99.99% of the lentiviral DNA integrated in the muscle cells of an animal model at day 4 after administration by day 21 after administration.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a microbial or tumor antigen, wherein the promoter does not contain an enhancer, and wherein the lentiviral vector particles exhibit higher expression of the antigen in BDCM cells than in HEK 293 T cells; integrating the DNA of the lentiviral vector into cells of the human; expressing the microbial or tumor antigen in the cells of the human; and generating an immune response against the microbial or tumor antigen.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a microbial or tumor antigen, wherein the promoter does not contain an enhancer, wherein expression of the promoter in BDCA+ dendritic cells is higher than the expression in kidney, smooth muscle, liver, and heart cells; integrating the DNA of the lentiviral vector into cells of the human; expressing the microbial or tumor antigen in the cells of the human; and generating an immune response against the microbial or tumor antigen.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a microbial or tumor antigen, wherein the promoter does not contain an enhancer, wherein the expression of the promoter in BDCA+ dendritic cells is 12-100 times the expression of that promoter in skeletal muscle cells; integrating the DNA of the lentiviral vector into cells of the human; expressing the microbial or tumor antigen in the cells of the human; and generating an immune response against the microbial or tumor antigen.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a microbial or tumor antigen, wherein the promoter does not contain an enhancer, wherein expression of the promoter in BDCA+ dendritic cells is higher than the expression in kidney, smooth muscle, liver, and heart cells, and wherein the expression of the promoter in BDCA+ dendritic cells is 12-100 times the expression of that promoter in skeletal muscle cells; integrating the DNA of the lentiviral vector into cells of the human; expressing the microbial or tumor antigen in the cells of the human; and generating an immune response against the microbial or tumor antigen.

In some embodiments, the method comprises eliminating at least 95%, 99%, 99.9% or 99.99% of the lentiviral DNA integrated in the muscle cells of an animal model at day 4 after administration by day 21, day 25, day 33, or day 66 after administration. Elimination of integrated lentiviral vector particles in muscle cells can be measured as described in the examples and by other similar techniques. For example, a biopsy can be taken at the site of the injection of a mouse or rat at 4 days and at 21 days after injection and the amount of integrated DNA in the cells determined at these timepoints by qPCR.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a microbial or tumor antigen, wherein the promoter does not contain an enhancer; integrating the DNA of the lentiviral vector into cells of the human; expressing the microbial or tumor antigen in the cells of the human; and generating an immune response.

In various embodiments, the administration induces T-specific responses against the antigen at 100, 200, 300, 400, 420, 500, or 600 days after administration that are more than 10%, 20%, 25%, 30%, 40%, 50%, 60%, 70%, 75%, 80%, 90% or 100% of the responses at 11, 28, 42, or 105 days after administration. T-specific responses can be measured as set forth in the examples.

Preferably, the lentivector particles are in a dose of, at least, 10⁶, 2 x 10⁶, 5x 10⁶, 10⁷ , 2 x10⁷, 5 x10⁷, 10⁸, 2 x 10⁸, 5 x 10⁸, or 10⁹ TU.

Most preferably, the lentiviral vector particles are in a dose of 5x 10⁶ to 5 x 10⁷, 5 x 10⁷ to 5 x 10⁸, or 5 x 10⁸ to 10⁹ TU.

The immune response induced by the lentiviral vector can be a B cell response, an NK response, a dendritic cell (e.g., pDC) response, a CD4+ T cell response, and/or a CD8+ T cell response.

The lentivector particles can be administered to the subject in a single dosage, or in multiple (i. e., 2, 3, 4, etc.) dosages. The lentivector particles can be administered in a first (priming) and second (boosting) administration. In one embodiment, the first dosage comprises 10⁷ to 10⁸ TU of lentivector particles and the second dosage comprises 10⁷ to 10⁸ TU of lentivector particles.

Preferably, the first dosage comprises a dose of 5x 10⁶ to 5 x 10⁷, 5 x 10⁷ to 5 x 10⁸, or 5 x 10⁸ to 10⁹ TU of lentivector particles and the second dosage comprises a dose of 5x 10⁶ to 5 x 10⁷, 5 x 10⁷ to 5 x 10⁸, or 5 x 10⁸ to 10⁹ TU of lentivector particles.

In one embodiment, the first dosage comprises 10⁸ to 10⁹ TU of lentivector particles and the second dosage comprises 10⁸ to 10⁹ TU of lentivector particles.

In one embodiment, the lentiviral vector particles are in a dose of at least 5x 10⁶, 10⁷, 5 x10⁷, 10⁸, 5 x 10⁸, or 10⁹ TU.

The time between the first and second administrations and between an administration and a subsequent administration can vary. In one embodiment, the time between administrations is two to six weeks. In various embodiments, the time between administrations is at least 2, 4, 6, 8, 10, 12, 15, 30, or 52 weeks. In various embodiments, the time between administrations is at least 1, 3, 6, 9, 12, 24, 36, or 48 months. In various embodiments, the time between administrations is at least 1, 2, 3, 4, 5, 6, 7, 8, 9, or 10 years.

In one embodiment, the invention comprises method for inducing a long term immune response in a human comprising intramuscularly administering in a priming dose and a boosting dose lentiviral vector particles comprising a functional integrase protein and a lentiviral vector to a human; wherein the DNA of the lentiviral vector comprises a promoter directing expression of a microbial or tumor antigen, wherein the promoter does not contain an enhancer, wherein expression of the promoter in BDCA+ dendritic cells is higher than the expression in kidney, smooth muscle, liver, and heart cells, and wherein the expression of the promoter in BDCA+ dendritic cells is 12-100 times the expression of that promoter in skeletal muscle cells; integrating the DNA of the lentiviral vector into cells of the human; expressing the microbial or tumor antigen in the cells of the human; and generating an immune response against the microbial or tumor antigen. Preferably, the antigen is an HIV-1 antigen, more preferably an HIV-1 Nef, Pol, NC, or CA antigen or immunogenic polypeptide, most preferably comprising the amino acid sequence of SEQ ID NO:30

Preferably, the lentiviral vector comprises a β2m or MHC class I promoter.

### THERAPEUTIC USE OF LENTIVIRAL VECTORS

The present invention further relates to the use of the lentiviral vectors according to the invention, especially in the form of lentiviral vector particles, for the preparation of therapeutic compositions or vaccines which are capable of inducing or contributing to the occurrence or improvement of an immunogical reaction against epitopes, more particularly those encoded by the transgene present in the vectors under the transcriptional control of any of the promoters of the invention.

The present invention thus provides vectors that are useful as a medicament or vaccine, particularly for intramuscular administration.

Preferably, the lentiviral vector comprises a β2m or MHC class I promoter.

These vectors are preferentially used for the treatment or prophylaxis of infectious diseases, especially diseases associated with virus infection and more particularly, with retrovirus infection, such as AIDS and other immunodeficiencies.

The invention can also be used in treatment protocols against tumors and cancers and especially could be used in protocols for immunotherapy or vaccination therapy against tumors.

As the vectors of the invention more specifically target dendritic cells to obtain a cell-mediated immune response and especially the CTL response associated with the antigen expressed by the transgene in these cells, they are particularly useful as vaccines targeting slow or endogenous pathogenic microorganisms such as Mycobacteria or HIV virus.

Accordingly, the invention relates to an immunogenic composition comprising a lentiviral vector as previously defined.

The immunogenic compositions of the invention preferably contain cPPT and CTS sequences in the vector and vector particles to induce or to stimulate the nuclear import of the vector genome in the target cells.

During reverse transcription, cPPT and CTS sequences induce the formation of a three stranded DNA structure referred as DNA triplex, which stimulates the nuclear import of DNA vector sequence. Preferably, the vector comprises a transgene and regulatory signals of retrotranscription, expression and encapsidation of retroviral or retroviral-like origin, wherein the composition is capable of inducing or of stimulating a CTL and/or a CD4 response against one or several epitopes encoded by the transgene sequence present in the vector.

The expression of the transgene is greatly improved by inclusion of a promoter of the invention in the vector.

Thus, the lentiviral vectors according to the invention have the ability to induce, improve, or in general be associated with the occurrence of a memory CTL response. In other words, they can be used for the preparation of therapeutic composition for the treatment of allergies, autoimmune diseases, tumor diseases, or infectious diseases, by induction of, stimulation of, or participation in the occurrence of a cell-mediated immune response, especially a CTL response or a memory response.

The lentiviral vectors of the invention can be used in methods of treatment and methods of inducing an immune response comprising administering the lentiviral vector to a host and generating a specific immune response against the transgene in the host. The cells and antibodies generated in these hosts can be used as diagnostic reagents.

The lentiviral vectors according to the invention are preferably for intramuscular administration, most preferably by injection with a needle.

In a particular embodiment, the immunogenic composition according to the invention can be directly administered to the patient, in such a way that it will induce, improve, or participate in vivo in the occurrence of a cell-mediated immune response, especially a CTL-mediated immune response.

In another embodiment, the immunogenic compositions are used once or upon repeated administration so that they can enable the occurrence of a long-term memory cell mediated response.

A particular advantage of the immunogenic compositions of the invention is that they can be used to elicit or stimulate a cell-mediated immune response against multiple epitopes encoded by the nucleotide sequence of interest or transgene present in the vector or vector particles, and they can also be used to elicit or stimulate a cell-mediated immune response against the product of the entire sequence of a gene, for instance a gene of a pathogenic agent or fragments of said gene capable to encode at least 8 to 15 amino acids preferably 9 to 12 amino acids.

The invention also encompasses a lentiviral vector comprising a nucleotide sequence encoding a multiple repeat (at least 2 identical sequences) of said amino acid sequence inducing a cellular response and/or an amino acid sequence containing at least 2 different sequences corresponding to 2 epitopes of different pathogens or tumoral antigens.

As a result, the invention encompasses a composition that could be used in prophylactic and/or therapeutic vaccination protocols, for the treatment of tumors and especially as anti-cancer or anti-infectious diseases treatment.

In particular, it can be used in combination with adjuvants, other immunogenic compositions, chemotherapy, or any other therapeutic treatment.

The invention encompasses a composition for intramuscular administration to a human comprising lentiviral vector particles comprising a functional integrase protein and a lentiviral vector; wherein the DNA of the lentiviral vector comprises a promoter of the invention directing expression of a microbial or tumor antigen.

In one embodiment, the invention encompasses a composition for intramuscular administration to a human comprising lentiviral vector particles comprising a functional integrase protein and a lentiviral vector; wherein the DNA of the lentiviral vector comprises a promoter directing expression of a microbial or tumor antigen, wherein the promoter does not contain an enhancer, and wherein the expression of the promoter in BDCA+ dendritic cells is 12-100 times the expression of that promoter in skeletal muscle cells and expression of the promoter in BDCA+ dendritic cells is higher than the expression in kidney, smooth muscle, liver, and heart cells.

The invention encompasses use of a composition comprising lentiviral vector particles for intramuscular administration to a human, wherein the lentiviral vector particles comprise a functional integrase protein and a lentiviral vector; wherein the DNA of the lentiviral vector comprises a promoter of the invention directing expression of a microbial or tumor antigen.

In one embodiment, the invention encompasses use of a composition comprising lentiviral vector particles for intramuscular administration to a human, wherein the lentiviral vector particles comprise a functional integrase protein and a lentiviral vector; wherein the DNA of the lentiviral vector comprises a promoter directing expression of a microbial or tumor antigen, wherein the promoter does not contain an enhancer, and wherein the expression of the promoter in BDCA+ dendritic cells is 12-100 times the expression of that promoter in skeletal muscle cells and expression of the promoter in BDCA+ dendritic cells is higher than the expression in kidney, smooth muscle, liver, and heart cells.

Preferably, the composition induces T-specific responses against the antigen at 100, 200, 300, 400, 420, 500, or 600 days after administration that are more than 10%, 20%, 25%, 30%, 40%, 50%, 60%, 70%, 75%, 80%, 90% or 100% of the responses at 11, 28, 42, or 105 days after administration. T-specific responses can be measured as set forth in the examples.

The invention encompasses compositions comprising a dose of 5 x 10⁶ to 5 x 10⁸ TU of lentiviral vector particles for use in the treatment of a microbial infection or a cancer by induction of a CD4+ and/or CD8+ T cell response against an antigen encoded by the lentiviral vector. Preferably, the treatment is by repetitive intramuscular administration to a human,

The lentiviral vector particles can comprise a functional integrase protein and a lentiviral vector. The DNA of the lentiviral vector can comprise a β2m or MHC class I promoter directing expression of a microbial or tumor antigen, wherein the promoter does not contain an enhancer. Preferably, the repetitive intramuscular administration induces CD4+ and/or CD8+ T-specific responses against the antigen.

The invention encompasses uses of a composition comprising a dose of 5 x 10⁶ to 5 x 10⁸ TU of lentiviral vector particles to induce CD4+ and/or CD8+ T cell responses against an antigen encoded by the lentiviral vector by repetitive intramuscular administration to a human. Preferably, the lentiviral vector particles comprise a functional integrase protein and a lentiviral vector; wherein the DNA of the lentiviral vector comprises a β2m or MHC class I promoter directing expression of a microbial or tumor antigen, wherein the promoter does not contain an enhancer, and wherein the repetitive intramuscular administration induces CD4+ and/or CD8+ T-specific responses against the antigen.

The invention encompasses methods for inducing a CD4+ and/or CD8+T cell response in a human comprising intramuscularly administering in a priming dose5 x 10⁶ to 5 x 10⁸ TU of lentiviral vector particles and a boosting dose of 5 x 10⁶ to 5 x 10⁸ TU of lentiviral vector particles. Preferably, the lentiviral vector particles comprise a functional integrase protein and a lentiviral vector to a human. Preferably, the DNA of the lentiviral vector comprises a β2m or MHC class I promoter directing expression of a microbial or tumor antigen, wherein the promoter does not contain an enhancer. The method can comprise integrating the DNA of the lentiviral vector into cells of the human, expressing the microbial or tumor antigen in the cells of the human; and generating a CD4+ and/or CD8+ T cell response against the microbial or tumor antigen.

Preferably, the lentivector contains a promoter that drives high expression of a polypeptide comprising an HIV-1 Nef, Pol, NC, or CA antigen or immunogenic polypeptide, most preferably comprising the amino acid sequence of SEQ ID NO:30, in antigen presenting cells, including dendritic cells, and drives expression in other transduced cell types sufficient for elimination by the induced immune response. Most preferably, the promoter lacks an enhancer element to avoid insertional effects.

The lentiviral vectors according to the invention can be directly administered to a patient through known routes of administration, including systemic, local, or cutaneous, intradermal, for instance intramuscular, subcutaneous, intranasal, intravaginal and intrarectal administration routes. *Ex vivo* administration, for instance ex *vivo* transduction of target cells followed by administration of the treated cells to the patient to be treated, is also encompassed by the invention

Preferably, the administration is intramuscular. In one embodiment, the lentivector is injected into the muscle using a needle.

Preferably, the lentivector particle is an integrating lentivector particle, comprising a functional integrase protein.

Preferably, the lentiviral vector or lentiviral vector particle comprises or encodes the nucleic acid sequence of any of SEQ ID NOs 31-40.

Most preferably, the administration eliminates at least 95%, 99%, 99.9%, or 99.99% of the lentiviral DNA integrated in the muscle cells of an animal model at day 4 after administration by day 21 after administration.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of an HIV-1 (e.g., Gag, Pol, or Nef) polypeptide, most preferably comprising the amino acid sequence of SEQ ID NO:30, wherein the promoter does not contain an enhancer; integrating the DNA of the lentiviral vector into cells of the human; expressing the polypeptide in the cells of the human; and generating an immune response against the HIV-1 polypeptide.

Preferably, the HIV-1 Gag, Pol, or Nef polypeptide comprises the Gag sequence AA 132-496, the Pol sequence AA 258-355 and/or AA 463-473, and/or the Nef sequence AA 106-148 and/or AA 175-203.

Preferably, the lentiviral vector comprises a β2m or MHC class I promoter.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a polypeptide comprising a microbial or tumor antigen, preferably a polypeptide comprising an HIV-1 Nef, Pol, NC, or CA antigen or immunogenic polypeptide, most preferably comprising the amino acid sequence of SEQ ID NO:30, wherein the promoter does not contain an enhancer, and wherein the lentiviral vector particles exhibit higher expression of the antigen in BDCM cells than in HEK 293 T cells; integrating the DNA of the lentiviral vector into cells of the human; expressing the polypeptide in the cells of the human; and generating an immune response against the polypeptide.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a polypeptide comprising a microbial or tumor antigen, preferably a polypeptide comprising an HIV-1 Nef, Pol, NC, or CA antigen or immunogenic polypeptide, most preferably comprising the amino acid sequence of SEQ ID NO:30, wherein the promoter does not contain an enhancer, wherein expression of the promoter in BDCA+ dendritic cells is higher than the expression in kidney, smooth muscle, liver, and heart cells; integrating the DNA of the lentiviral vector into cells of the human; expressing the polypeptide in the cells of the human; and generating an immune response against the polypeptide.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a polypeptide comprising a microbial or tumor antigen, preferably a polypeptide comprising an HIV-1 Nef, Pol, NC, or CA antigen or immunogenic polypeptide, most preferably comprising the amino acid sequence of SEQ ID NO:30, wherein the promoter does not contain an enhancer, wherein the expression of the promoter in BDCA+ dendritic cells is 12-100 times the expression of that promoter in skeletal muscle cells; integrating the DNA of the lentiviral vector into cells of the human; expressing the polypeptide in the cells of the human; and generating an immune response against the polypeptide.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression of a polypeptide comprising a microbial or tumor antigen, preferably a polypeptide comprising an HIV-1 Nef, Pol, NC, or CA antigen or immunogenic polypeptide, most preferably comprising the amino acid sequence of SEQ ID NO:30, wherein the promoter does not contain an enhancer, wherein expression of the promoter in BDCA+ dendritic cells is higher than the expression in kidney, smooth muscle, liver, and heart cells, and wherein the expression of the promoter in BDCA+ dendritic cells is 12-100 times the expression of that promoter in skeletal muscle cells; integrating the DNA of the lentiviral vector into cells of the human; expressing the polypeptide in the cells of the human; and generating an immune response against the polypeptide.

In some embodiments, the method comprises eliminating at least 95%, 99%, 99.9% or 99.99% of the lentiviral DNA integrated in the muscle cells of an animal model at day 4 after administration by day 21, day 25, day 33, or day 66 after administration. Elimination of integrated lentiviral in muscle cells can be measured as described in the examples and by other similar techniques. For example, a biopsy can be taken at the site of the injection of a mouse or rat at 4 days and at 21 days after administration and the amount of integrated DNA in the cells determined at these timepoints by PCR.

In one embodiment, the invention comprises a method for inducing an immune response in a human comprising intramuscularly administering lentiviral vector particles comprising a functional integrase protein and a lentiviral vector to a human; wherein the integrating lentiviral vector comprises a promoter directing expression a polypeptide comprising a microbial or tumor antigen, preferably a polypeptide comprising an HIV-1 Nef, Pol, NC, or CA antigen or immunogenic polypeptide, most preferably comprising the amino acid sequence of SEQ ID NO:30, the promoter does not contain an enhancer; integrating the DNA of the lentiviral vector into cells of the human; expressing the polypeptide in the cells of the human; and generating an immune response.

Preferably, the lentivector particles are in a dose of, at least, 10⁶, 2 x 10⁶, 5x 10⁶, 10⁷, 2 x 10⁷, 5 x 10⁷, 10⁸, 2 x 10⁸, 5 x 10⁸, or 10⁹ TU.

The immune response induced by the lentiviral vector can be a B cell response, a CD4+ T cell response, and/or a CD8+ T cell response.

The present invention thus provides vectors that are useful as a medicament or vaccine, particularly for intramuscular administration.

These vectors are preferentially used for the treatment or prophylaxis of infectious diseases, especially diseases associated with virus infection and more particularly, with retrovirus infection, such as AIDS and other immunodeficiencies.

As the vectors of the invention more specifically target dendritic cells to obtain a cell-mediated immune response and especially the CTL response associated with the antigen expressed by the transgene in these cells, they are particularly useful as vaccines targeting slow or endogenous pathogenic microorganisms such as HIV virus.

Accordingly, the invention relates to an immunogenic composition comprising a lentiviral vector as previously defined.

The immunogenic compositions of the invention preferably contain cPPT and CTS sequences in the vector and vector particles to induce or to stimulate the nuclear import of the vector genome in the target cells.

During reverse transcription, cPPT and CTS sequences induce the formation of a three stranded DNA structure referred as DNA triplex, which stimulates the nuclear import of DNA vector sequence. Preferably, the vector comprises a transgene and regulatory signals of retrotranscription, expression and encapsidation of retroviral or retroviral-like origin, wherein the composition is capable of inducing or of stimulating a CTL and/or a CD4 response against one or several epitopes encoded by the transgene sequence present in the vector.

The expression of the transgene is greatly improved by inclusion of a promoter of the invention in the vector.

Thus, the lentiviral vectors according to the invention have the ability to induce, improve, or in general be associated with the occurrence of a memory CTL response. In other words, they can be used for the preparation of therapeutic composition for induction of, stimulation of, or participation in the occurrence of a cell-mediated immune response, especially a CTL response or a memory response.

The lentiviral vectors of the invention can be used in methods of treatment and methods of inducing an immune response comprising administering the lentiviral vector to a host and generating a specific immune response against the transgene in the host. The cells and antibodies generated in these hosts can be used as diagnostic reagents.

The lentiviral vectors according to the invention are preferably for intramuscular administration, most preferably by injection with a needle.

In a particular embodiment, the immunogenic composition according to the invention can be directly administered to the patient, in such a way that it will induce, improve, or participate in vivo in the occurrence of a cell-mediated immune response, especially a CTL-mediated immune response.

In another embodiment, the immunogenic compositions are used once or upon repeated administration so that they can enable the occurrence of a long-term memory cell mediated response.

A particular advantage of the immunogenic compositions of the invention is that they can be used to elicit or stimulate a cell-mediated immune response against multiple epitopes encoded by the nucleotides sequence of interest or transgene present in the vector or vector particles, and they can also be used to elicit or stimulate a cell-mediated immune response against the product of the entire sequence of a gene.

The invention also encompasses a lentiviral vector comprising a nucleotide sequence encoding a multiple repeat (at least 2 identical sequences) of said amino acid sequence inducing a cellular response and/or an amino acid sequence containing at least 2 different sequences.

As a result, the invention encompasses a composition that could be used in prophylactic and/or therapeutic vaccination protocols.

In particular, it can be used in combination with adjuvants, other immunogenic compositions, chemotherapy, or any other therapeutic treatment.

The invention encompasses a composition for intramuscular administration to a human comprising lentiviral vector particles comprising a functional integrase protein and a lentiviral vector; wherein the DNA of the lentiviral vector comprises a promoter directing expression of an amino acid comprising or consisting of SEQ ID NO:30.

In one embodiment, the invention encompasses a composition for intramuscular administration to a human comprising lentiviral vector particles comprising a functional integrase protein and a lentiviral vector; wherein the DNA of the lentiviral vector comprises a promoter directing expression of an amino acid comprising or consisting of SEQ ID NO:30, wherein the promoter does not contain an enhancer, and wherein the expression of the promoter in BDCA+ dendritic cells is 12-100 times the expression of that promoter in skeletal muscle cells and expression of the promoter in BDCA+ dendritic cells is higher than the expression in kidney, smooth muscle, liver, and heart cells.

The invention encompasses use of a composition comprising lentiviral vector particles for intramuscular administration to a human, wherein the lentiviral vector particles comprise a functional integrase protein and a lentiviral vector; wherein the DNA of the lentiviral vector comprises a promoter directing expression of a microbial or tumor antigen, preferably a polypeptide comprising an HIV-1 Nef, Pol, NC, or CA antigen or immunogenic polypeptide, most preferably an amino acid comprising or consisting of SEQ ID NO:30.

In one embodiment, the invention encompasses use of a composition comprising lentiviral vector particles for intramuscular administration to a human, wherein the lentiviral vector particles comprise a functional integrase protein and a lentiviral vector; wherein the DNA of the lentiviral vector comprises a promoter directing expression of a microbial or tumor antigen, preferably polypeptide comprising an HIV-1 Nef, Pol, NC, or CA antigen or immunogenic polypeptide, most preferably comprising the amino acid sequence of SEQ ID NO:30, wherein the promoter does not contain an enhancer, and wherein the expression of the promoter in BDCA+ dendritic cells is 12-100 times the expression of that promoter in skeletal muscle cells and expression of the promoter in BDCA+ dendritic cells is higher than the expression in kidney, smooth muscle, liver, and heart cells.

In some embodiments, the administration to a human comprises administering the composition comprising lentiviral vector particles to a patient infected with HIV-1.

Having thus described different embodiments of the present invention, it should be noted by those skilled in the art that the disclosures herein are exemplary only and that various other alternatives, adaptations, and modifications may be made within the scope of the present invention. Accordingly, the present invention is not limited to the specific embodiments as illustrated herein.

### EXAMPLES

### Example 1. Molecular constructions

PCR amplification of the proviral region of the pTRIPΔU3-CMV-GFP(15) was performed using direct (5'-CTTACTAGTTGGAAGGGCTAATTCACTCCCAAC-3'; SEQ ID NO:7) and reverse (5'-CATTCTAGAACTGCTAGAGATTTTCCACACTG-3'; SEQ ID NO:8) oligonucleotides encompassing respectively the Spel and Xbal restriction sites. The resulting fragment was digested and cloned between the Spel and Xbal sites of the pVAX-1 plasmid (Invitrogen, Lifetech) from which the Mlul site have been deleted. The resulting plasmid was named pFLAP-CMV-GFP. The SV40 sequence was amplified by PCR from the pTRIPΔU3-CMV-GFP plasmid (using the 5'-TACCCCGGGCCATGGCCTCCAAAAAAGCCTCCTCACTACTTC-3' (SEQ ID NO:9) and 5'-ACTCCCGGGTAATTTTTTTTATTTATGCAGAGGCCGAGGCCGCC-3' (SEQ ID NO:10) oligonucleotides), and cloned into the Pml1 site of the pFLAP-CMV-GFP, the resulting plasmid being then named pFLAP-CMV-GFP-SV. The CMV promoter was amplified with direct (5'-TACACGCGTGGAGTTCCGCGTTACATAACTTACGG-3'; SEQ ID NO:11) and reverse (5'-CGTGGATCCGATCGCGGTGTCTTCTATGGAGGTCAAAAC-3'; SEQ ID NO:12) oligonucleotides encompassing the Mlul and BamHI sites, respectively. The resulting fragment was cloned back between the Mlul and BamHI sites of the pFlap-CMV-GFP-SV allowing the easy replacement of the promoters inside the lentiviral vectors. The various promoters were then amplified by PCR from HEK 293T cells DNA (with 5'-TACACGCGTGGCCTCCGCGCCGGGTTTTGGCGCCTCC-3' (SEQ ID NO:13) and 5'-CGTGGATCCGATCGGTCTAACAAAAAAGCCAAAAACGGCC-3' (SEQ ID NO:14) for the UBC promoter; 5'-TACACGCGTTGCGTGAGGCTCCGGTGCCCGTCAGTGG-3' (SEQ ID NO:15) and 5'-CGTGGATCCGATCGTCACGACACCTGAAATGGAAG-3' (SEQ ID NO:16) for the EF1α1 promoter and 5'-GCCGGCGCGCCGAGAAACCCTGCAGGGAATTCCC-3' (SEQ ID NO:17) and 5'-CGTGGATCCGATCGCTCGGCCCGAATGCTGTCAGCTTCAGG-3' (SEQ ID NO:18) for the β2m promoter and cloned between the Mlul and BamH1 sites of the pFLAP-CMV-GFP. The amplified β2m promoter sequence is the following : GAGAAACCCTGCAGGGAATTCCCCAGCTGTAGTTATAAACAGAAGTTCTCCTTCTG CTAGGTAGCATTCAAAGATCTTAATCTTCTGGGTTTCCGTTTTCTCGAATGAAAAAT GCAGGTCCGAGCAGTTAACTGGCGGGGGCACCATTAGCAAGTCACTTAGCATCTC TGGGGCCAGTCTGCAAAGCGAGGGGGCAGCCTTAATGTGCCTCCAGCCTGAAGT CCTAGAATGAGCGCCCGGTGTCCCAAGCTGGGGCGCGCACCCCAGATCGGAGGG CGCCGATGTACAGACAGCAAACTCACCCAGTCTAGTGCATGCCTTCTTAAACATCA CGAGACTCTAAGAAAAGGAAACTGAAAACGGGAAAGTCCCTCTCTCTAACCTGGCA CTGCGTCGCTGGCTTGGAGACAGGTGACGGTCCCTGCGGGCCTTGTCCTGATTG GCTGGGCACGCGTTTAATATAAGTGGAGGCGTCGCGCTGGCGGGCATTCCTGAA GCTGACAGCATTCGGGCCGAG (SEQ ID NO:19). The HIV antigen (Gag sequence AA 132-496, Pol sequence AA 258-355 and AA 463-473 and Nef sequence AA 106-148 and AA 175-203) was synthetized by GeneArt (Lifetech) and cloned between the BamHI and Xhol sites of the pFlap-β2m-SV, in place of the GFP gene, giving the pFLAP-DeltaU3-pβm-HIV(P24-NC-Frag) SV. This vector is referred to as "β2m-HIV." .

The β2m-HIV vector expresses the following amino acid sequence: 
MPIVQNLQGQMVHQAISPRTLNAWVKVVEEKAFSPEVIPMFSALSEGATPQDLNTMLNTVGGHQ
AAMQMLKETINEEAAEWDRLHPVHAGPIAPGQMREPRGSDIAGTTSTLQEQIGWMTNNPPIPVG
EIYKRWIILGLNKIVRMYSPTSILDIRQGPKEPFRDYVDRFYKTLRAEQASQEVKNWMTETLLV
QNANPDCKTILKALGPAATLEEMMTACQGVGGPGHKARVLAEAMSQVTNSATIMMQRGNFRNQR
KTVKCFNCGKEGHIAKNCRAPRKKGCWKCGKEGHQMKDCTERQANFLGKIWPSHKGRPGNFLQS
RPEPTAPPEESFRFGEETTTPSQKQEPIDKELYPLASLRSLFGNDKKSVTVLDVGDAYFSVPLD
KDFRKYTAFTIPSINNETPGIRYQYNVLPQGWKGSPAIFQSSMTKILEPFRKQNPDIVIYQYMD
DLYVGSDLEIGQHRTEILKEPVHGVYVGFPVRPQVPLRPMTYKAAVDLSHFLKEKGGLEGLIYS
QKRQDILDLWVYHTQGYFPDWQNYTPGPGIRYPLTFGWCFKLVPVDPEKEVLVWKFDSRLAFHH
MARELHPEYY (SEQ ID NO:30).
 This sequence comprises Gag, Pol, and Nef sequences, in that order.

Mock vector: pFlap-β2m-GFP-SVwas digested by BamHI and Xhol, and a DNA linker containing a Mutiple Cloning Site (MCS, carrying SalI, SacII, Ndel, AscI and Nhel restriction sites) was cloned between those sites, in place of the GFP gene.

The packaging plasmid pTHV-GP-N was constructed by amplifying the HIV-1 NDK genome by PCR (using the following oligonucleotides with 5'-atgcatgcgtcgacctcgagttaatcctcatcctgtctacttgccac-3' (SEQ ID NO:20) and 5'-This sequence comprises Gag, Pol, and Nef gcatgcatcggccggggcggcgactgGTgagagGCCACCatgggtgcgagagcgtcagtattaag-3' (SEQ ID NO:21)). The resulting fragment has been digested by Eagl and SalI restriction enzymes and inserted in the p8.74 packaging plasmid(15) from which the Eag1-SalI fragment had been previously removed.

Pseudotyping plasmids were generated by synthesizing the codon optimized genes corresponding to the vesiculo stomatitis virus Indiana (GenBank #CAX62728.1), New Jersey GenBank #CAX62729.1) and Cocal (GenBank # CAX62731.1) strains. Those genes were then digested with EcoR1 and BamH1 and cloned between the corresponding restriction sites of the pVAX1 plasmid (Invitrogen, Lifetech).

pFlap-β2m-HIV-IRES-Luc, an IRES sequence (pQCXIX, Clontech) has been cloned between Ndel and AscI sites in the mock vector. Then, the HIV antigen is cloned between BamHI and SalI sites and the luciferase gene (pGL3-promoter vector, Promega) was amplified by PCR (with 5'-GCCGGCGCGCCGAGAAACCCTGCAGGGAATTCCC-3' (SEQ ID NO:22) and 5'-TTCTCGAGCATTACACGGCGATCTTTCCGC-3' (SEQ ID NO:23)) and cloned between Nhel and Xhol sites.

For R&D and pilot vector productions, the plasmids were produced using Nucleobond Xtra Maxi EF column according to manufacturer's instructions (Macherey Nagel). For GMP productions, high-quality grade plasmids manufactured by Plasmid Factory (Germany) were used.

### Example 2. Lentiviral production

R&D productions: Vectors were produced by transient calcium-phosphate transfection of HEK 293T as previously describe (25).

Preclinical and GMP productions: The day before transfection, HEK 293T cells were seeded in culture medium on 24 units of Cell Factory 10 (CF-10, Nunc). Cells were transfected by a calcium-phosphate method as reported previously (25). 18 to 24 hours post-transfection, culture medium was changed with production medium corresponding to Dulbecco's modified Eagle's medium (DMEM/ High modified, Hyclone) supplemented with 2% heat-inactivated fetal calf serum (FCS, PAA), 1% L-Glutamine (Gibco by Life technologies), 1% Penicillin-Streptomycin (Gibco by Life technologies), 1% Sodium Pyruvate (Gibco by Life technologies), BENZONASE® (pharma grade I, 100 000U, Merck Millipore) and MgCL2 1 M. Minimum 24 hours after medium renewal, supernatant of the 24 CF-10 is harvested and pooled. After a second BENZONASE® treatment, supernatant is clarified by filtration on Kleenpak Nova Profile II cartridge (Pall). After clarification, a third BENZONASE® treatment was applied overnight at +2/+8°C. Viral vector were purified using Anion exchange chromatography on Mustang-Q XT cassette (Pall). Lentiviral particles were eluted in two steps with 0,5M and 1,2M NaCl. Both fractions were diluted to decrease NaCl concentration up to ± 150mM before pooling. IEX eluate was further concentrated approximately 40 fold by ultrafiltration using a 100KDa Omega T series filter, 0,1m² (Pall) and diafiltrated with PBS-Lactose 40 mg.L-1. Purified bulk (Drug substance) was finally filtered through a 0.2 µM Sartobran H5 filter, 300cm² (Sartorius Stedim) and aseptically distributed on 2R 3mL-glass vials with a target filling volume of 650µL (1200µL for pilot batches). After visual inspection of all the vials (about 350 vials by clinical batch), drug product was stored at -70°C±10°C.

For product characterization and pharmaceutical release, quality tests were performed according to regulatory texts on vaccines: the quality control required for vaccines as per the European Pharmacopeia (section 6.16), the "guideline on quality, non-clinical and clinical aspects of live recombinant viral vectored vaccines" (EMA/CHMP/141697/2009), the "guideline on development and manufacture of lentiviral vectors" (CHMP/BWP/2458/03); regulatory text on gene therapy medicinal products: the quality controls required for gene transfer medicinal products for human use as per the European Pharmacopeia (section 5.14), the quality controls specific to gene therapy products as defined in the "note for guidance on the quality, preclinical and clinical aspects of gene transfer medicinal products" (CHMP/BWP/3088/99); regulatory texts on biotechnological products (ICH Q5A to ICH Q5E); regulatory texts on specifications (ICH Q6A and ICH Q6B) and the quality controls required for parenteral preparations as per the European Pharmacopeia (section 7.0).

### Example 3. Lentiviral vector titration

qPCR reactions: HEK 293T cells were seeded in 6-well plates (BD Falcon) in culture medium and incubated for 4 h at 37°C, 5% CO2 in moist atmosphere. Cells were transduced with 3 successive dilutions of lentiviral vector. 72h post-incubation, cells are harvested and transduced HEK 293T cell pellets are produced. Total genomic DNA from transduced cell-pellets is extracted using a method based on QIAGEN QIAamp DNA mini kit handbook. Proviral quantification is performed using Taqman qPCR. The amplification is performed with the Master Mix (Fermentas Thermo Scientific), the Flap A (CCCAAGAACCCAAGGAACA; SEQ ID NO:24) and Flap S (AGACAA GATAGAGGAAGAGCAAAAC; SEQ ID NO:25) primers and LENTI TM probe (6FAM-AACCATTAGGAGTAGCACCCACCAAGG-BBQ; SEQ ID NO:26). Normalization is performed with the quantification of the actin gene (same Mix, Actine A - CGGTGAGGATCTTCATGAGGTAGT- (SEQ ID NO:27), Actine S - AACACCCCAGCCATGTACGT-(SEQ ID NO:28) primers and HUMURA ACT TM probe -6FAM-CCAGCCAGGTCCAGACGCAGGA-BBQ-(SEQ ID NO:29). Both reactions are achieved on MasterCycler Ep Realplex S (Eppendorf, 2 min at 50°C, 10 min at 95°C and 40 cycles of 15 seconds at 95°C and 1 min at 63°C). The analysis is performed on MasterCycler Ep Realplex Software.

FACS analyses were performed as described elsewhere (20).

Animals: For non-GLP studies, C57BL/6J Rj (C57BI/6J) female mice of four weeks or Sprague Dawley RjHan:SD (Sprague Dawley) female mice of eight weeks were purchased from Janvier Laboratories (France). The animals were housed in Institute Pasteur animal facility in accordance with Institute regulations on the respect of animal experimentation ethical procedures. For GLP studies males and females of CRL: CD strain of rats were purchased from Charles River Italia, Calco (LC), Italy (aged of at least 10 weeks). Rats were housed in our CRO (APTUIT) facilities.

Sample collection and cell isolation: after CO2 euthanasia, rats' spleens were perfused and chopped into small pieces before being gently squished on a 100 µm cell stainer whilst mice spleens were directly squished on the cell stainer. Red Blood Cell Lysing Buffer Hybri-Max (Sigma Aldrich) was used to lyse red blood cells. Following three further washes splenocytes were resuspended into RPMI1640 medium containing 10% FBS and automatically counted using Nucleocounter Chemometec cells counter.

### Example 4. GFP expression driven by various promoters in two different cell types.

Lentivector constructions encompassing various promoters were used to transduce HEK293T (a fibroblast cell line) and BDCM (a dendritic-like cell line) cells, and the promoters' ability to drive the GFP expression was evaluated by flow cytometry. MFI (Mean Fluorescence intensity) in BDCM cells are represented in % of the MFI obtained in 293T cells.

Viral (CMV) and ubiquitous (UBC and EF1α) promoters appear to be repressed in BDCM, while MHC I (HLA-A2, HLA-B7 and HLA-E) and MHC II (HLA-DRα) promoters are overexpressed in this cell type. The β2m promoter was also overexpressed in this cell type.

Cells were seeded in 24-well plates at a density of 1×10⁵ cells per well in complete medium containing 10% FBS. For each cell type, 3 wells of 1×10⁵ cells were transduced by replacing the culture medium with 300 µl of the dilution of viral samples which allowed a percentage of transduced cells included between 5 and 30%. The cells were then incubated 2h at 37°C, 5% CO₂ and 1 ml of complete medium was added per well. 72h post transduction, the cells were trypsinized and resuspended, and the GFP MFI was measured with a FACScalibur flow cytometer, using the FL1 channel.

### Example 5. T-specific response (median) in C57BI/6j mice

C57BI/6j mice were immunized with 1 x 10⁶TU of lentivectors in which a HIV antigen expression is driven by various promoters (viral, ubiquitous, MHCl and MHCII). 12 days after immunization, the specific T-cell responses were monitored in mice splenocytes by IFN-γ ELISPOT. As shown in figure 2, immunization with lentivectors encompassing a MHCI promoter give higher specific T-cell responses than any other lentivectors used, even when a viral promoter is driving the antigen expression.

Splenocytes were isolated from the mice spleens immunized with lentivectors. 12 days after immunization and were used for the ELISPOT assays. Ninety-six-well tissue culture plates (Millipore) were coated overnight at 4 °C with 50 µl/well of 5 µg/ml anti-mouse IFNγ mAb (Mouse IFNγ Elispot pair; BD Biosciences Pharmingen). The plates were washed three times with 200 µl DPBS/well and blocked with 200 µl/well of DPBS/10% fetal bovine serum for 2 h at 37°C. The plates were washed three times with 200 µl DPBS/well. Splenocytes were added to the plates in triplicate at 2.5, 4.1, or 5.1 ×105 cells/well and stimulated with 2 µg/ml of stimulatory peptides (specific to the antigen), concanavalin A (5 µg/ml; source), or culture medium alone. The plates were incubated for 18 h at 37°C and then rinsed three times with 200 µl/well of DPBS/0.05 % Tween 20 and three times with 200 µl/well of DPBS. For detection, 50 µl/well of 2 µg/ml anti-mouse IFNγ-biotinylated monoclonal antibody (BD Pharmingen) were added for 2 h at room temperature. Plates were washed and 100 µl/well of streptavidin-alkaline phosphatase (Roche) diluted 1:2000 in Dulbecco's PBS for 90 min at room temperature. After washing the plates, spots (IFNγ -secreting cells) were visualized by adding 60 µl/well of BCIP/NBT solution (Sigma). Plates were incubated for 15-30 min at room temperature until blue spots developed and then thoroughly washed with running tap water and air-dried for 24 h. Finally, the spots were counted using a Bioreader 2000 (Biosys).

Depletion of CD4+ and CD8+ T cells: Rats spleen cells were mixed with washed magnetic beads coated with either anti-rat CD4 or anti-CD8 using the Deplete S program (MACS, Miltenyi Biotec). Negative and positive CD4 and CD8 cellular fractions were obtained in separated tubes. Untreated and CD4 and CD8 depleted cells were stained with anti-rat CD3-FITC, CD4-APC and CD8-PerCP BD Biosciences antibodies to confirm the complete depletion. Cells were analyzed by FACS using a FACSCalibur with FlowJO package (BD Biosciences).

In vivo assay of targeting cells by lentiviral vectors: The concentrated lentiviral vectors: CMV.GFPbis and β2m.GFP.WThV (1.10e8 TU/mouse resuspended in 50µL PBS-lactose) were injected intramuscularly into the left caudal thigh of the C57BI/6j mice. The inguinal lymph nodes and the muscles corresponding to the injection sites as well as the ones from a naïve mouse were isolated for cells harvesting. Lymph nodes and muscles were digested into RPMI1640 media containing 10% collagenase and 0,5% DNA-se, chopped into small pieces before being gently squished on a 100 µm cell stainer. Collagenase action was reduced adding RPMI1640, 10% FBS. Following two further washes cells were resuspended into RPMI1640 medium containing 10% FBS. Viable mononuclear cells were isolated from muscle suspensions underlying them on Histopaque 1077 (Sigma, United Kingdom) before centrifugation at 1,000 x g for 10 min at RT. Cells at the interface were collected, washed two times with PBS and resuspended into RPMI1640 medium containing 10% FBS. Finally lymph node and muscle cells were automatically counted using Nucleocounter Chemometec cells.

ELISPOT Assay. MultiScreen HTS 96 microplates (MSIPS4W10, Millipore) were coated with capture antibody (Mouse and Rat IFNg Elispot Pair, BD Biosciences) and blocked with complete medium. Cells were cultured at the concentration of 0,2 million/well for rat studies and 0,1 million/well for mice studies. Subsequently they were incubated with 2µg/mL of peptides (pool stimulation) and with 2.5 µg/mL of concanavaline A used as positive control. Eighteen hours later, spots were revealed with the biotine-conjugated antibody (Mouse and Rat IFNg Elispot pair, BD Biosciences) followed by streptavidin-AP (Roche) and BCIP/NBT substrate solution (Sigma Aldrich). Spots were counted using an AID reader (Autoimmun Diagnostika GmbH, Germany). Mean number of IFNg spots-forming-cells (SFC) per million was calculated from triplicate wells after subtracting the one from control wells (cultured in medium without peptides).

HIV-1 consensus B subtype Gag, Pol and Nef complete sets of overlapping synthetic peptides were kindly provided by NIH AIDS Reagent Program.

Imaging and quantification of bioluminescence in vivo: Measurements were performed with an ultrasensitive cooled CDD camera mounted with in a light-tight camera box (IVIS 200, Xenogen). Images and measurements of bioluminescent signals were acquired and analyzed using Living image software 2.5 (Xenogen). To image, mice were anesthetized by isoflurane inhalation and given 150µL of 15mg/mL D-luciferin (Caliper Lifesciences) via intraperitoneal injection. Images were taken between 2 to 5 minutes after D-luciferin injection.

### Example 6. T cell responses of integrating and non-integrating vectors

Lentiviral vectors are produced as previously described. Zennou, V. et al. HIV-1 genome nuclear import is mediated by a central DNA flap. Cell. 101, 173-185 (2000). For non-integrating lentiviral vector, the packaging plasmid used is an integrase defective pNDKD64-V.

Animals: C57BL/6J Rj (C57BI/6J) female mice of four weeks were purchased from Janvier Laboratories (France). The animals were housed in Institute Pasteur animal facility in accordance with Institute regulations on the respect of animal experimentation ethical procedures.

Sample collection and cell isolation: after CO2 euthanasia, mice spleens were directly squished on the cell stainer. Red Blood Cell Lysing Buffer Hybri-Max (Sigma Aldrich) was used to lyse red blood cells. Following three further washes splenocytes were resuspended into RPMI1640 medium containing 10% FBS and automatically counted using Nucleocounter Chemometec cells counter.

ELISPOT Assay. MultiScreen HTS 96 microplates (MSIPS4W10, Millipore) were coated with capture antibody (Mouse IFNg Elispot Pair, BD Biosciences) and blocked with complete medium. Cells were cultured at the concentration of 0,2 million/well. Subsequently they were incubated with 2µg/mL of peptides (pool stimulation) and with 2.5 µg/mL of concanavaline A used as positive control. Eighteen hours later, spots were revealed with the biotine-conjugated antibody (Mouse IFNg Elispot pair, BD Biosciences) followed by streptavidin-AP (Roche) and BCIP/NBT substrate solution (Sigma Aldrich). Spots were counted using an AID reader (Autoimmun Diagnostika GmbH, Germany). Mean number of IFNg spots-forming-cells (SFC) per million was calculated from triplicate wells after subtracting the one from control wells (cultured in medium without peptides).

Four individual peptides synthesized by Genscript and identified as immunogenic in C57BL/6J mice were used in this test.

Statistical analysis: Data are expressed as median ± confidence interval.

### Example 7. GLP biodistribution

The titration qPCR method was used for GLP biodistribution (without the actin normalization), the method being analytically validated by our CRO APTUIT.

Tissue samples were collected in Qiagen collection plates in dry ice and stored at approximately -80°C until analyzed. Genomic DNA was isolated and extracted from tissues using Qiagen DNeasy 96 blood and Tissues kit handbook on a robotic station (Biorobot 8000 Universal, Qiagen) using reagents and protocols recommended by the manufacturer. Multifuge 3SR, Heraeus was used in the centrifugation steps. DNA quantification was based on Quant-iT™ PICOGREEN® dsDNA kit (Molecular Probes) user manual. The concentration of the eluted DNA was determined using the Fluostar OPTIMA (BMG) fluorescence reader. Concentrations of the total DNA isolates (µg/mL) are interpolated from the standard curve and multiplied by the sample dilution factor. A real-time quantitative modification of the TaqMan PCR technique in which the amplicon is detected by a sequence-specific fluorogenic probe was used. Amplification of the target sequence was performed in the ABI PRISM 7900HT sequence detection system and qPCR was realized as following: adjustment of primers at a final concentration of 900nM and the Taqman probe at: 200nM. Cycling conditions were 50°C for 2 min, 95°C for 10 minutes for the Taq polymerase activating step, a denaturation step of 0.15 minutes at 95°C and 45 cycles of combined annealing/extension step of 1 minute at 63°C. The qPCR TaqMan assay is the same than the one used for vector titration, without actin normalization.

### Example 8. Persistence of antigen expression

Measurements were performed with an ultrasensitive cooled CDD camera mounted with in a light-tight camera box (IVIS 200, Xenogen). Images and measurements of bioluminescent signals were acquired and analyzed using Living image software 2.5 (Xenogen). To image, mice were anesthetized by isoflurane inhalation and given 150µL of 15mg/mL D-luciferin (Caliper Lifesciences) via intraperitoneal injection. Images were taken between 2 to 5 minutes after D-luciferin injection.

### Example 9. Persistence of immune response

The persistence of the immune response was measured by ELISPOT INF-gamma as previously described (Example 5).

### Example 10. Transduction of DCs

The concentrated lentiviral vectors: CMV.GFPbis and β2m.GFP.WThV (1.10e8 TU/mouse resuspended in 50µL PBS-lactose) were injected intramuscularly into the left caudal thigh of the C57BI/6j mice. The inguinal lymph nodes and the muscles corresponding to the injection sites as well as the ones from a naïve mouse were isolated for cells harvesting. Lymph nodes and muscles were digested into RPMI1640 media containing 10% collagenase and 0,5% DNAse, chopped into small pieces before being gently squished on a 100 µm cell stainer. Collagenase action was reduced adding RPMI1640, 10% FBS. Following two further washes cells were resuspended into RPMI1640 medium containing 10% FBS. Viable mononuclear cells were isolated from muscle suspensions underlying them on Histopaque 1077 (Sigma, United Kingdom) before centrifugation at 1,000 × g for 10 min at RT. Cells at the interface were collected, washed two times with PBS and resuspended into RPMI1640 medium containing 10% FBS. Finally lymph node and muscle cells were automatically counted using Nucleocounter Chemometec cells.

### Example 11. Shedding Studies

Urine samples were collected using metabolic cages. RNA extraction was achieved according to the Qiagen kit "QiaAmp Viral RNA Mini". The Reverse transcription reactions have been realized in 96-Well plates placed on a refrigerated rack and all incubation steps have been performed using a THERMOCYCLER MASTERCYCLER™ (Eppendorf) and RT-qPCR was realized as following: First a DNase treatment has been performed, to remove eventual DNA contamination, using DNase I Recombinant (Roche) during 30min at 37°C followed by an inactivation step at 75°C during 5min in presence of EDTA (30mM). Then, a first incubation step of the RNA sample with DNTP mix (10mM each) and Oligo(dT)18mers (100µM) at 65°C during 5min allows the disruption of secondary structures. The reverse transcription was achieved using the reverse transcriptase kit "REVERTAID™ Premium" (Fermentas, Thermo Scientific) and an RNase inhibitor RIBOLOCK™ (Fermentas, Thermo Scientific) by an incubation of 30min at 50°C. A last incubation step at 85°C during 5min allows the reverse transcriptase inactivation. The qPCR TaqMan assay is the same than the one used for vector titration, without actin normalization.

Statistical analysis: Data are expressed as means ± standard deviation or as median ± confidence interval.

### Example 12. Kinetics of prime-boost immunization (influenza vaccine)

Animals: C57BI/6j female mice of four weeks were purchased from Janvier Laboratories (France). The animals were housed in Institute Pasteur animal facility in accordance with Institute regulations on the respect of animal experimentation ethical procedures. (5 mice/group of vaccinated animals and 8 mice/group of PBS-lactose).Vectors and administration. Animals: C57BI/6j female mice of four weeks were purchased from Janvier Laboratories (France). The animals were housed in Institute Pasteur animal facility in accordance with Institute regulations on the respect of animal experimentation ethical procedures. (5 mice/group of vaccinated animals and 8 mice/group of PBS-lactose). Mice were intramuscularly immunized with 5 x 10⁶TU in prime and 3 x 10⁶TU in boost of lentivectors in which a M1 NP antigen expression is driven by a B2m promoter. 12 days after prime immunization, 54 days after prime and 8 days before boost immunization, 69 days after prime and 7 days after boost immunization and 88 days after prime and 26 days after boost immunization the specific T-cell responses were monitored in mice splenocytes by IFN-γ ELISPOT.

Sample collection and cell isolation: Splenocytes were isolated from the mice spleens on days of analyses. Sample collection and cell isolation: after CO2 euthanasia, mice' spleens were gently squished on a 100 µm cell stainer. Lysis Buffer was used to lyse red blood cells. Following three further washes splenocytes were resuspended into FBS 10% media and were used for the ELISPOT assays.

ELISPOT assay: Ninety-six-well tissue culture plates (Millipore) were coated overnight at 4 °C with 50 µl/well of 5 µg/ml anti-mouse IFNγ mAb (Mouse IFNγ Elispot pair; BD Biosciences Pharmingen). The plates were washed three times with 200 µl DPBS/well and blocked with 200 µl/well of DPBS/10% fetal bovine serum for 2 h at 37°C. The plates were washed three times with 200 µl DPBS/well. Splenocytes were added to the plates in duplicate at 2×10⁵ cells/well and stimulated with 2 µg/ml of stimulatory peptides (specific to the antigen), concanavalin A (5 µg/ml; source), or culture medium alone. The plates were incubated for 18 h at 37°C and then rinsed three times with 200 µl/well of DPBS/0.05 % Tween 20 and three times with 200 µl/well of DPBS. For detection, 50 µl/well of 2 µg/ml anti-mouse IFNγ-biotinylated monoclonal antibody (BD Pharmingen) were added for 2 h at room temperature. Plates were washed and 100 µl/well of streptavidin-alkaline phosphatase (Roche) diluted 1:2000 in Dulbecco's PBS for 90 min at room temperature. After washing the plates, spots (IFNγ - secreting cells) were visualized by adding 60 µl/well of BCIP/NBT solution (Sigma). Plates were incubated for 15-30 min at room temperature until blue spots developed and then thoroughly washed with running tap water and air-dried for 24 h. Spots were counted using an AID reader (Autoimmun Diagnostika GmbH, Germany). Mean number of IFNg spots-forming-cells (SFC) per million was calculated from duplicate wells after subtracting the one from control wells (cultured in medium without peptides). Overlapping synthetic peptides covering influenza complete sequence used for vaccination were used (Eurogentec). Statistical analysis: Data are expressed as median ± confidence interval.

### Example 13. Human Administration

THV01-1: β2m-HIV pseudotyped with Indiana VSV-G and THV01-2: β2m-HIV pseudotyped with New Jersey VSV-G were injected into humans as depicted in Figure 21.

The THV01 treatment administrated to patients enrolled in this Phase I/II study was composed of two vaccines: THV01-1 and THV01-2. They have been injected 8 weeks apart, intramuscularly.

THV01-1 and THV01-2 are lentiviral vectored vaccines derived from the NL4-3 strain of the HIV-1. They are non-replicative and self-inactivating vectors. They both encode the same HIV antigen (SEQ ID NO:30), composed of clustered epitopes of the HIV-1 clade B, Gag, Pol and Nef proteins under the regulation of a human β2m promoter. THV01-1 (β2m-HIV pseudotyped with Indiana VSV-G ) and THV01-2 (β2m-HIV pseudotyped with New Jersey VSV-G) are pseudotyped by the G protein of the vesicular stomatitis virus (VSV.G). This allows the production of more robust lentiviral particles and induces a broader tropism of the vectors. Since THV01-1 and THV01-2 are however pseudotyped by two different serotypes of the VSV.G (Indiana & New Jersey), successive injections of them occurs without a reduction in the induced immunogenicity due to antibodies generated against the envelope of the previously injected vectored vaccine.

Enrolled patients were randomized in a 3:1 ratio (vaccine:placebo) for each dose group. Eligible patients for this first-in human were HIV-1 infected patients, treated with HAART (2 nucleosidic reverse transcriptase inhibitors plus 1 boosted protease inhibitor or 2 nucleosidic reverse transcriptase inhibitors plus 1 non nucleosidic inhibitor) for more than 12 months at baseline and treated with 2 nucleosidic reverse transcriptase inhibitors plus a ritonavir boosted protease inhibitor among darunavir+ritonavir or lopinavir+ritonavir, who have CD4+ T cells count ≥600 cells mm-3 at baseline with a nadir ≥300 cells mm-3 and who had CD4+ T cells count < 500 cells mm-3 at least once from diagnosis to initiation of treatment.

This trial is a randomized, placebo-controlled, 4-parallel groups trial to compare the safety, tolerability and immunogenicity of the THV01 vaccination versus placebo.

Study enrollment design is detailed in Figure 28.

Twelve patients were randomized in a 3:1 ratio (vaccine:placebo) for each dose group, with four groups (three doses to be assessed and a placebo group):
- Patients randomized in group 1 have received intramuscular injections of THV01-1 and THV01-2, 8 weeks apart, at 5.10⁶ TU, or matching placebo;
- Patients randomized in group 2 have received intramuscular injections of THV01-1 and THV01-2, 8 weeks apart, at 5.10⁷ TU or matching placebo;
- Patients randomized in group 3 have received intramuscular injections THV01-1 and THV01-2, 8 weeks apart, at 5.10⁸ TU or matching placebo.

2 or 7 weeks before the first vaccine injection, patients had to stop reverse transcriptase inhibitors:
- Patients taking Tenofovir had to stop it 7 weeks before the first vaccination (W-7);
- Patients not taking Tenofovir will had to stop taking reverse transcriptase inhibitors 2 weeks before the first vaccination (W-2).

During this treatment alleviation, patients had to continue being treated by their ritonavir boosted protease inhibitor. Patients had to resume their initial HAART 1 week after the second injection.

HAART have been reintroduced in case of:
▪ CD4+ T cell count ≤ 500 cells mm-3 confirmed by a second measurement 14 days later or,
▪ HIV viral load > 15,000 copies mL-1 from confirmed twice respectively 14 and 28 days later.

As per study protocol, an interim analysis has been performed upon week 36 of the last patient of second treatment group.

### Example 14. Safety of Lentiviral Administration in Humans

### EXTENT OF EXPOSURE

All patients (n=24) from Group 1&2 have adequately received their prime (W0) and boost (W8) injections except one patient - #102-001 (THV01/Group 1) - that has been treated at Week 8 with a vaccine dose of 600µL instead of 1000µL.

### DISPLAY OF ADVERSE EVENTS

Adverse events are described by SOC and preferred term according to the MedDRA classification and by treatment/dose group.

All AE are presented hereafter regardless of the causal relationship rated by the Principal Investigator.

### Serious Adverse Events / Deaths

To date (July 22nd 2014), neither Serious Adverse Event nor death has been reported to THERAVECTYS regarding patients treated with THV01 regardless of the dose. Of note, the last patient of the third dose group has been vaccinated (i.e. boost injection) on March 23rd 2014.

### Adverse Events of Mild Intensity

Table 3 displays the percentage of patients experiencing at least one Adverse Event of mild intensity by SOC and preferred term and treatment groups.

**Table 3 : Adverse Events of Mild Intensity**

| **System Organ Class/Preferred Term** | **Placebo (N=6)** | **THV01- Dose 1 (N=9)** | **THV01-Dose 2 (N=9)** |
|---|---|---|---|
| At least one AE | 6 (100.0%) | 9 (100.0%) | 8 (88.9%) |
| **Blood and lymphatic system disorders** | **1 (16.7%)** | 1 **(11.1%)** | **1 (11.1%)** |
| Lymphadenopathy | 1 (16.7%) | 1 (11.1%) | 1 (11.1%) |
| **Gastrointestinal disorders** | **1 (16.7%)** | **4 (44.4%)** | **3 (33.3%)** |
| Abdominal distension | 0 (0.0%) | 1 (11.1%) | 0 (0.0%) |
| Diarrhoea | 0 (0.0%) | 3 (33.3%) | 2 (22.2%) |
| Haemorrhoids | 0 (0.0%) | 0 (0.0%) | 1 (11.1%) |
| Nausea | 1 (16.7%) | 0 (0.0%) | 0 (0.0%) |
| Toothache | 0 (0.0%) | 1 (11.1%) | 0 (0.0%) |
| **General disorders and administration site conditions** | **3 (50.0%)** | **0 (0.0%)** | **4 (44.4%)** |
| **Asthenia** | 1 (16.7%) | 0 (0.0%) | 1 (11.1%) |
| Chest pain | 2 (33.3%) | 0 (0.0%) | 0 (0.0%) |
| Fatigue | 2 (33.3%) | 0 (0.0%) | 1 (11.1%) |
| Injection site pain | 0 (0.0%) | 0 (0.0%) | 1 (11.1%) |
| Pain | 0 (0.0%) | 0 (0.0%) | 2 (22.2%) |
| **Infections and infestations** | **3 (50.0%)** | **1 (11.1%)** | **5 (55.6%)** |
| Bronchitis | 1 (16.7%) | 1 (11.1%) | 0 (0.0%) |
| Gastroenteritis | 0 (0.0%) | 0 (0.0%) | 1 (11.1%) |
| Herpes pharyngitis | 0 (0.0%) | 0 (0.0%) | 1 (11.1%) |
| Herpes simplex | 0 (0.0%) | 0 (0.0%) | 1 (11.1%) |
| Herpes virus infection | 0 (0.0%) | 0 (0.0%) | 1 (11.1%) |
| Nasopharyngitis | 1 (16.7%) | 0 (0.0%) | 0 (0.0%) |
| Oral herpes | 0 (0.0%) | 0 (0.0%) | 1 (11.1%) |
| Pharyngitis | 1 (16.7%) | 0 (0.0%) | 0 (0.0%) |
| Rhinitis | 1 (16.7%) | 0 (0.0%) | 0 (0.0%) |
| Tooth infection | 0 (0.0%) | 0 (0.0%) | 1 (11.1%) |
| Tracheitis | 1 (16.7%) | 0 (0.0%) | 0 (0.0%) |
| **Investigations** | **6 (100.0%)** | **8 (88.9%)** | **8 (88.9%)** |
| Blood HIV RNA increased | 6 (100.0%) | 8 (88.9%) | 8 (88.9%) |
| CD4 lymphocytes decreased | 1 (16.7%) | 2 (22.2%) | 2 (22.2%) |
| Platelet count decreased | 1 (16.7%) | 0 (0.0%) | 0 (0.0%) |
| Weight increased | 0 (0.0%) | 3 (33.3%) | 1 (11.1%) |
| **Musculoskeletal and connective tissue disorders** | **2 (33.3%)** | **3 (33.3%)** | **0 (0.0%)** |
| Arthralgia | 0 (0.0%) | 2 (22.2%) | 0 (0.0%) |
| Back pain | 0 (0.0%) | 1 (11.1%) | 0 (0.0%) |
| Myalgia | 0 (0.0%) | 1 (11.1%) | 0 (0.0%) |
| Neck pain | 1 (16.7%) | 0 (0.0%) | 0 (0.0%) |
| Sacroiliitis | 1 (16.7%) | 0 (0.0%) | 0 (0.0%) |
| Spinal osteoarthritis | 1 (16.7%) | 0 (0.0%) | 0 (0.0%) |
| **Neoplasms benign, malignant and unspecified (incl cysts and polyps)** | **0 (0.0%)** | **1 (11.1%)** | **0 (0.0%)** |
| Anogenital warts | 0 (0.0%) | 1 (11.1%) | 0 (0.0%) |
| **Nervous system disorders** | **2 (33.3%)** | **2 (22.2%)** | **1 (11.1%)** |
| Headache | 1 (16.7%) | 2 (22.2%) | 1 (11.1%) |
| Presyncope | 1 (16.7%) | 0 (0.0%) | 0 (0.0%) |
| **Psychiatric disorders** | **0 (0.0%)** | **0 (0.0%)** | **2 (22.2%)** |
| Anxiety | 0 (0.0%) | 0 (0.0%) | 1 (11.1%) |
| Insomnia | 0 (0.0%) | 0 (0.0%) | 1 (11.1%) |
| **Respiratory, thoracic and mediastinal disorders** | **1 (16.7%)** | **3 (33.3%)** | **0 (0.0%)** |
| Asthma exercise induced | 0 (0.0%) | 1 (11.1%) | 0 (0.0%) |
| Cough | 1 (16.7%) | 0 (0.0%) | 0 (0.0%) |
| Nasal obstruction | 1 (16.7%) | 0 (0.0%) | 0 (0.0%) |
| Oropharyngeal pain | 0 (0.0%) | 2 (22.2%) | 0 (0.0%) |
| **Skin and subcutaneous tissue disorders** | **0 (0.0%)** | **1 (11.1%)** | **0 (0.0%)** |
| Eczema | 0 (0.0%) | 1 (11.1%) | 0 (0.0%) |
| **Surgical and medical procedures** | **0 (0.0%)** | **0 (0.0%)** | **1 (11.1%)** |
| Lesion excision | 0 (0.0%) | 0 (0.0%) | 1 (11.1%) |
| **Vascular disorders** | **1 (16.7%)** | **0 (0.0%)** | **0 (0.0%)** |
| Hot flush | 1 (16.7%) | 0 (0.0%) | 0 (0.0%) |
| Hypotension | 1 (16.7%) | 0 (0.0%) | 0 (0.0%) |

Nearly all patients (95,9%) have experienced at least one Adverse Event of Mild intensity during the course of the study. Neither a clinically meaningful Adverse Event nor a significant imbalance over the treatment groups can be observed.

### Adverse Events of Moderate Intensity

Table 4 displays the percentage of patients experiencing at least one Adverse Event of moderate intensity by SOC and preferred term and treatment groups.

**Table 4: Adverse Events of Moderate Intensity**

| **System Organ Class/Preferred Term** | **Placebo (N=6)** | **THV01- Dose 1 (N=9)** | **THV01 Dose 2 (N=9)** |
|---|---|---|---|
| At least one AE | 4 (66.7%) | 3 (33.3%) | 6 (66.7%) |
| **General disorders and administration site conditions** | **1 (16.7%)** | **2 (22.2%)** | **3 (33.3%)** |
| Asthenia | 0 (0.0%) | 0 (0.0%) | 3 (33.3%) |
| Fatigue | 1 (16.7%) | 2 (22.2%) | 0 (0.0%) |
| **Infections and infestations** | **2 (33.3%)** | **2 (22.2%)** | **2 (22.2%)** |
| Acute HIV infection | 0 (0.0%) | 0 (0.0%) | 1 (11.1%) |
| Bronchitis | 1 (16.7%) | 0 (0.0%) | 0 (0.0%) |
| Gingivitis | 1 (16.7%) | 0 (0.0%) | 0 (0.0%) |
| Influenza | 0 (0.0%) | 1 (11.1%) | 0 (0.0%) |
| Laryngitis | 0 (0.0%) | 1 (11.1%) | 0 (0.0%) |
| Nasopharyngitis | 0 (0.0%) | 0 (0.0%) | 1 (11.1%) |
| Upper respiratory tract infection | 0 (0.0%) | 1 (11.1%) | 0 (0.0%) |
| Urinary tract infection | 1 (16.7%) | 0 (0.0%) | 0 (0.0%) |
| **Injury, poisoning and procedural complications** | **1 (16.7%)** | **0 (0.0%)** | **1 (11.1%)** |
| Hand fracture | 0 (0.0%) | 0 (0.0%) | 1 (11.1%) |
| Ligament sprain | 1 (16.7%) | 0 (0.0%) | 0 (0.0%) |
| **Investigations** | **2 (33.3%)** | **3 (33.3%)** | **4 (44.4%)** |
| Blood HIV RNA increased | 2 (33.3%) | 3 (33.3%) | 4 (44.4%) |
| **Musculoskeletal and connective tissue disorders** | **1 (16.7%)** | **0 (0.0%)** | **0 (0.0%)** |
| Tendonitis | 1 (16.7%) | 0 (0.0%) | 0 (0.0%) |
| **Nervous system disorders** | **1 (16.7%)** | **0 (0.0%)** | **0 (0.0%)** |
| Paraesthesia | 1 (16.7%) | 0 (0.0%) | 0 (0.0%) |
| **Reproductive system and breast disorders** | **0 (0.0%)** | **1 (11.1%)** | **0 (0.0%)** |
| Prostatitis | 0 (0.0%) | 1 (11.1%) | 0 (0.0%) |

54,2 % of the patients have experienced at least one Adverse Event of Moderate intensity during the course of the study. No significant imbalance in Adverse Event incidence over the treatment groups can be observed. Of note, fatigue/asthenia, which is often reported as an Adverse Event in vaccination trials, tends to increase according to the treatment and the dose group.

### Adverse Events of Severe Intensity

Table 5 displays the percentage of patients experiencing at least one Adverse Event of Severe intensity by SOC and preferred term and treatment groups.

**Table 5: Adverse Events of Severe Intensity**

| **System Organ Class/Preferred Term** | **Placebo (N=6)** | **THV01- Dose 1 (N=9)** | **THV01**- **Dose 2 (N=9)** |
|---|---|---|---|
| At least one AE | 0 (0.0%) | 1 (11.1%) | 1 (11.1%) |
| **General disorders and administration site conditions** | **0 (0.0%)** | **1 (11.1%)** | **1 (11.1%)** |
| Asthenia | 0 (0.0%) | 0 (0.0%) | 1 (11.1%) |
| Pyrexia | 0 (0.0%) | 1 (11.1%) | 0 (0.0%) |

Two Adverse Events of severe intensity have been reported among patients treated with dose 1&2. The two cases are detailed hereafter:
- Patient #0202-001 (THV01 Dose 1): Pyrexia episode occurred from December 3rd 2013 to December 4th 2013. The Adverse Event has been assessed as not related to the study vaccine by the investigator. The patient has been treated by THV01 respectively on May 21^{st} 2013 for the prime injection and on July 19^{th} for the boost injection. The pyrexia has been treated with Aspirin and resolved on December 4^{th} without sequelae.
- Patient #105-001(THV01 Dose 2): Asthenia episode occurred from June 10^{th} 2014 and was still on-going upon the database lock. This episode occurred far after the boost injection (October 28^{th} 2013). It has been assessed as definitively related to the study treatment by the investigator. According to the delay between the last vaccine injection and the date of the event, the sponsor does not share this opinion.

### Laboratory Findings

Based on laboratory workups results (Detailed in Table 6), no significant change in lab parameters has been observed in both treatment groups.

**Table 6: List of Lab parameters studied**

| **HEMATOLOGY** | **BIOCHEMISTRY** |
|---|---|
| Hemoglobin | Sodium |
| Hematocrit | Potassium |
| WBC | Calcium |
| Neutrophils | Glucose |
| Lymphocytes | BUN or urea |
| Monocytes | Creatinine |
| Eosinophils | AST |
| Basophils | ALT |
| Platelet count | Alkaline phosphatase |
| | Total bilirubin |
| | Total protein |
| | Albumin |
| | LDH |
| | gGT |
| | CRP |
| | Prothrombin time (PT) |

### Conclusions

According to these results from dose 1 and 2 patients, the safety profile of THV01 appears comparable to patients that have received the Placebo.

Moreover, neither Serious Adverse Event nor additional severe Adverse Events have been reported to date among the patients treated with the third dose. We therefore anticipate that the Primary endpoints of this study will be met.

### Example 15. Effects on Viral Replication of Lentiviral Administration in Humans

Samples were collected from patients at various timepoints and subjected to quantitation of viral load (PCR) and CD4+ T cell counts. The results are depicted in Figures 29-38.

### Example 16. Effects on Human Immune Response of Lentiviral Administration in Humans

For each patient, the immunogenicity from baseline(s) to week 24 was evaluated and the % of cells was calculated from the global cell population.

The following graphs were generated for each patients, and for the CD4+ and CD8+:
CD4+/CD69+; CD8+/CD69+; CD4+/CD69+/IFN+; CD8+/CD69+/IFN+;
CD4+/CD69+/IL2+ ; CD8+/CD69+/IL2+ ; CD4+/CD69+/TNF+; CD8+/CD69+/TNF+;
CD4+/CD69+/IFN+/IL2+; CD8+/CD69+/IFN+/IL2+; CD4+/CD69+/TNF+/IL2+;
CD8+/CD69+/TNF+/IL2+ ; CD4+/CD69+/IFN+/TNF+; CD8+/CD69+/IFN+/TNF+;
CD4+/CD69+/IFN+/TNF+/IL2+; and CD8+/CD69+/IFN+/TNF+/IL2+ .

The stimulations with peptides from the antigens included in the vaccine (GAG, POL and Nef pools) were measured, and the analysis also included ENV pools from W0 to W24.

The following evaluation criteria were used:
- For each timepoint, all the peptide pools were analyzed independently
- Absolute % values of positive cells were considered
- Positivity: ratio between the % of positive cells detected after peptide stimulation and the % of cells non stimulated (negative). If >2, it is considered as « positive » (it allows the normalization as variations between the neg values can be observed)
- General immunogenicity profile:
   If only one timepoint is positive, immunogenicity is « punctual »
   If it is detectable up to 24 weeks and for more than one timepoint, immunogenicity is « long-lasting »
   If it appears after the boost, immunogenicity is « late» and « early » if appearing from the prime
   Immunogenicity is « intense » or « weak » depending on the absolute percentage of positive cells
   « Multispecific » means immunogenicity against more than one peptide pool at one timepoint
   « Polyfunctional » means immunogenicity with cells secreting two or three cytokines

The results are depicted in Fig. 27A-U and summarized in Fig. 39 and 40..

## Claims

1. A composition comprising a dose of 5 x 10⁶ to 5 x 10⁸ TU of lentiviral vector particles for use in the treatment of a microbial infection or a cancer by induction of a CD4+ and/or CD8+ T cell response against an antigen encoded by the lentiviral vector by repetitive intramuscular administration to a human,
wherein the lentiviral vector particles comprise a functional integrase protein and a lentiviral vector;
wherein the DNA of the lentiviral vector comprises a β2m or MHC class I promoter directing expression of a microbial or tumor antigen,
wherein the promoter does not contain an enhancer, and
wherein the repetitive intramuscular administration induces CD4+ and/or CD8+ T-specific responses against the antigen.

2. The composition of claim 1, wherein the lentiviral vector comprises a β2m promoter.

3. The composition of claim 1, wherein the lentiviral vector comprises an MHC class I promoter.

4. The composition of any of claims 1-3, wherein the lentiviral vector comprises a viral antigen.

5. The composition of any of claims 1-3, wherein the lentiviral vector encodes a bacterial antigen.

6. The composition of any of claims 1-3, wherein the lentiviral vector encodes a tumor antigen.

7. The composition of any of claims 1-3, wherein the lentiviral vector encodes a human telomerase antigen.

8. The composition of any of claims 1-8, comprising at least 10⁷ TU of lentiviral vector particles.

9. The composition of any of claims 1-8, comprising at least 5 x 10⁷ TU of lentiviral vector particles.

10. Use of a composition comprising a dose of 5 x 10⁶ to 5 x 10⁸ TU of lentiviral vector particles to induce CD4+ and/or CD8+ T cell responses against an antigen encoded by the lentiviral vector by repetitive intramuscular administration to a human,
wherein the lentiviral vector particles comprise a functional integrase protein and a lentiviral vector;
wherein the DNA of the lentiviral vector comprises a β2m or MHC class I promoter directing expression of a microbial or tumor antigen,
wherein the promoter does not contain an enhancer, and
wherein the repetitive intramuscular administration induces CD4+ and/or CD8+ T-specific responses against the antigen.

11. The use of claim 10, wherein the lentiviral vector comprises a β2m promoter.

12. The use of claim 10, wherein the lentiviral vector comprises an MHC class I promoter.

13. The use of any of claims 10-12, wherein the lentiviral vector comprises a viral antigen.

14. The use of any of claims 10-12, wherein the lentiviral vector encodes a bacterial antigen.

15. The use of any of claims 10-12, wherein the lentiviral vector encodes a tumor antigen.

16. The use of any of claims 10-12, wherein the lentiviral vector encodes a human telomerase antigen.

17. The use of any of claims 10-16, comprising at least 10⁷ TU of lentiviral vector particles.

18. The use of any of claims 10-16, comprising at least 5 x 10⁷ TU of lentiviral vector particles.

19. A method for inducing a CD4+ and/or CD8+T cell response in a human comprising:
intramuscularly administering in a priming dose and a boosting dose a dose of 5 x 10⁶ to 5 x 10⁸ TU of lentiviral vector particles comprising a functional integrase protein and a lentiviral vector to a human;
wherein the DNA of the lentiviral vector comprises a β2m or MHC class I promoter directing expression of a microbial or tumor antigen,
wherein the promoter does not contain an enhancer,
integrating the DNA of the lentiviral vector into cells of the human;
expressing the microbial or tumor antigen in the cells of the human; and
generating a CD4+ and/or CD8+ T cell response against the microbial or tumor antigen.

20. The method of claim 19, comprising administering a priming dose comprising 10⁷ to 5 x 10⁸ TU of lentivector particles and a boosting dose comprising 10⁷ to 5 x 10⁸ TU of lentivector particles.

21. The method of claim 18 or 19, comprising administering the boosting dose at least 1 year after the priming dose.

22. The composition of claim 1, wherein the lentiviral vector encodes an HIV-1 protein.

23. The composition of claim 1, wherein the lentiviral vector encodes a protein comprising the amino acid sequence of SEQ ID NO:30.

24. The use of claim 10, wherein the lentiviral vector encodes an HIV-1 polypeptide.

25. The use of claim 10, wherein the lentiviral vector encodes a protein comprising the amino acid sequence of SEQ ID NO:30.

26. The method of claim 19, wherein the lentiviral vector encodes an HIV-1 polypeptide.

27. The method of claim 19, wherein the lentiviral vector encodes a protein comprising the amino acid sequence of SEQ ID NO:30
